# EUROPEAN PATENT SPECIFICATION

(11) **EP 1 257 553 B1**
(45) Date of publication and mention of the grant of the patent: **26.05.2004**
(21) Application number: 01908397.1
(22) Date of filing: 15.02.2001
(51) Int. Cl.: C07D 487/04, A61K 31/519, C07D 207/34

(54) **PYRROLOPYRIMIDINONE DERIVATIVES, PROCESS OF PREPARATION AND USE**
PYRROLOPYRIMIDINON-DERIVATE, VERFAHREN ZU IHRER HERSTELLUNG UND VERWENDUNG
DERIVES DE PYRROLOPYRIMIDINONE, PROCEDES D'ELABORATION ET D'UTILISATION

(30) Priority: 17.02.2000 KR 2000007625
(43) Date of publication of application: 20.11.2002
(73) Proprietor: SK Chemicals Co., Ltd., Suwon-si, Kyungki-do 440-301 (KR); In2gen Co., Ltd., Chongno-gu, Seoul 110-799 (KR)
(72) Inventor: KIM, Dae-Kee, Seoul 156-060 (KR); LEE, Ju Young, Suwon-shi Kyungki-do 440-300 (KR); RYU, Do Hyun, Suwon-shi, Kyungki-do 441-390 (KR); LEE, Nam Kyu, Suwon-shi, Kyungki-do 440-330 (KR); LEE, Suk Ho, Kwangmyung-shi, Kyungki-do 423-033 (KR); KIM, Nam-Ho, Sungnam-shi, Kyungki-do 462-150 (KR); KIM, Jae-Sun, Suwon-shi, Kyungki-do 441-112 (KR); RYU, Je Ho, Youngdeungpo-ku, Seoul 150-073 (KR); CHOI, Jin-Young, Jangan-ku, Suwon-shi, Kyungki-do 440-330 (KR); IM, Guang-Jin, Ansan-shi, Kyungki-do 425-170 (KR); CHOI, Won-Son, Dongan-ku Anyang-shi, Kyungki-do 431-070 (KR); KIM, Tae, Kon, Suwon-shi, Kyungki-do 440-300 (KR); CHA, Hoon, Sungdong-ku, Seoul 133-767 (KR)
(74) Representative: Weber, Dieter, Dr.
(86) International application number: PCT/KR2001/000227
(87) International publication number: WO 2001/060825

(56) References cited:
- T. MURATA ET AL.: 'Intramolecular reactions of enaminonitriles. A new synthesis of beta-aminopyrroles and related heterocycles' CHEM. PHARM. BULL. vol. 26, no. 10, 1978, pages 3080 - 3100, XP002955436
- T. MURATA ET AL.: 'Intramolecular reactions of enaminonitriles. II. Synthesis of new 3-aminopyrrole-2-carboxamides - A new route to pyrrolo(3,2-d)pyrimidines' CHEM. PHARM. BULL. vol. 22, no. 1, pages 240 - 241, XP002955444
- J. HAUSLER: 'Darstellung von cis- und trans-C-3-substituierten prolinverbundungen' LIEBIGS ANN. CHEM. 1981, pages 1073 - 1088, XP002955437
- DATABASE WPI Week 198304, Derwent Publications Ltd., London, GB; AN 1975-83762W, XP002955424 & JP 57 061 734 B (TAKEDA CHEM IND LTD) 25 December 1982

## Description

### BACKGROUND OF THE INVENTION

### Field of the Invention

The invention relates to a series of pyrrolopyrimidinone derivatives of the formula (1), processes for their preparation, intermediates in their preparation, their use as therapeutic agents, and pharmaceutical compositions containing them, 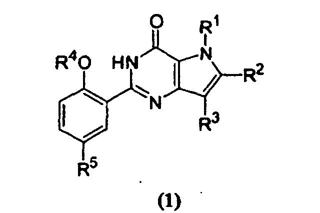 wherein R¹ is H; C₁-C₃ alkyl optionally substituted with one or more fluoro atoms; or C₃-C₆ cycloalkyl;
R² is H; a halogen atom; C₁-C₆ alkyl optionally substituted with OH, C₁-C₃ alkoxy, C₃-C₆ cycloalkyl, or with one or more fluoro atoms; C₃-C₆ cycloalkyl; C₂-C₆ alkenyl; or C₂-C₆ alkynyl;
R³ is H; C₁-C₆ alkyl optionally substituted with OH, C₁-C₃ alkoxy, C₃-C₆ cycloalkyl, or with one or more fluoro atoms; C₃-C₆ cycloalkyl; C₂-C₆ alkenyl; or C₂-C₆ alkynyl;
R⁴ is C₁-C₆ alkyl optionally substituted with C₃-C₆ cycloalkyl or with one or more fluoro atoms; C₂-C₆ alkenyl; C₂-C₆ alkynyl; or C₃-C₆ cycloalkyl;
R⁵ is SO₂NR⁶R⁷; NHSO₂NR⁶R⁷; NHCOCONR⁶R⁷; NHSO₂R⁸; NHCOR⁸; or phenyl or heterocyclyl either of which is optionally substituted with one or more fluoro atoms or C₁-C₃ alkyl;
R⁶ and R⁷ are each independently H or C₁-C₆ alkyl optionally substituted with OH, CO₂H, C₁-C₃ alkoxy, C₃-C₆ cycloalkyl, or with one or more fluoro atoms; or together with the nitrogen atom to which they are attached form either a mono-cyclic ring such as imidazole, aziridene (aziridine), azeridine (azetidine), pyrrolidine, piperidine, morpholine, piperazine and homopiperazine, or a bicyclic ring such as 2,5-diazabicyclo[2. 2. 1]heptane and 3,7-diazabicyclo[3. 3. 0]octane, wherein said group is optionally substituted with R⁹;
R⁸ is C₁-C₆ alkyl optionally substituted with one or more fluoro atoms; or C₃-C₇ cycloalkyl;
R⁹ is C₁-C₆ alkyl optionally substituted with one or more halide atoms, OH, C₁-C₃ alkoxy which is optionally substituted with one or more fluoro atoms, CO₂R¹⁰, NR¹¹R¹², C=NR¹³(NR¹⁴R¹⁵), or with a tetrazole group which is optionally substituted with C₁-C₃ alkyl; or one or more nitrogen containing heteroaryl group which is optionally substituted with one or more fluoro atoms;
R¹⁰ is H; or C₁-C₄ alkyl optionally substituted with OH, NR¹¹R¹², one or more fluoro atoms, or with a nitrogen containing heterocyclic ring such as pyrrolidine, piperidine, piperazine, morpholine, pyrrole, and imidazole wherein nitrogen atom is directly bound to C₁-C₄ alkyl;
R¹¹ and R¹² are each independently H or C₁-C₄ alkyl;
R¹³ is H; C₁-C₄ alkyl optionally substituted with one or more fluoro atoms; or C₃-C₆ cycloalkyl;
R¹⁴ and R¹⁵ are each independently H or C₁-C₄ alkyl optionally substituted with one or more fluoro atoms; C₃-C₆ cycloalkyl; or together with the nitrogen atom to which they are attached form a pyrrolidinyl, piperidino, morpholino, piperazinyl, or homopiperazinyl group wherein said group is optionally substituted with C₁-C₃ alkyl.

### Description of the Prior Art

European patent applications EP-A-0463756 and EP-A-0526004 disclose certain pyrazolo[4,3-*d*]pyrimidin-7-ones as cGMP PDE inhibitors, useful in the treatment of cardiovascular disorders such as angina, hypertension and heart failure. International application WO 94/28902 discloses their use for the treatment of impotence.

The present inventors have recently disclosed a series of pyrazolo[4,3-*d*]pyrimidin-7-one derivatives as PDE V inhibitors (Appln. No. KR 98-60436 and KR 99-7580). Herein a new series of pyrrolo[3,2-*d*]pyrimidin-4-one derivatives are prepared as PDE V inhibitors. However, none of the compounds of this invention are specifically disclosed.

### SUMMARY OF THE INVENTION

The compounds (1) of this invention are potent and selective inhibitors of cyclic guanosine 3',5'-monophosphate specific phosphodiesterase (cGMP specific PDE; PDE V) having utility in a variety of therapeutic areas where such inhibition is thought to be beneficial, including the treatment of impotence (male erectile dysfunction), sexual dysfunction in female, and various cardiovascular disorders such as angina, hypertension, heart failure and atherosclerosis.

As a consequence of the selective PDE V inhibition exhibited by compounds of this invention, cGMP levels are elevated, which in turn can give rise to beneficial vasodilatory, anti-vasospastic, anti-platelet, anti-neutrophil, natriuretic and diuretic activities as well as potentiation of the effects of endothelium-derived relaxing factor (EDRF), nitrovasodilators, atrial natriuretic factor (ANF), brain natriuretic peptide (BNP), C-type natriuretic peptide (CNP) and endothelium-dependent relaxing agents such as bradykinin, acetylcholine and 5-HT₁.

The compounds of this invention therefore have utility in the treatment of a number of disorders, including impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases characterized by disorders of gut motility (e. g. irritable bowel syndrome).

### Detailed Description of the Invention

Thus, according to a first aspect, this invention provides compounds of the formula (**1**) and pharmaceutically acceptable salts and solvates (e.g. hydrates) thereof, 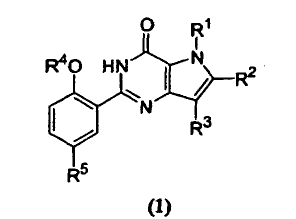 wherein R¹, R², R³, R⁴ and R⁵ are as previously defined.

In the above definition, unless otherwise indicated, alkyl groups having three or more carbon atoms may be straight or branched chain. In addition, alkenyl or alkynyl groups having four or more carbon atoms, or alkoxy groups having three carbon atoms, may be straight or branched chain.

Compounds of the formula **(1)** may contain one or more asymmetric centers and thus can exist as enantiomers or diastereomers. It is to be understood that the invention includes both mixtures and separate individual isomers of compounds of the formula **(1)**. Furthermore certain compounds of the formula **(1)** which contain alkenyl groups may exist as cis- or trans-isomers. In each instance, the invention includes both mixtures and separate individual isomers.

Compounds of the formula (**1**) may also exist in tautomeric forms and the invention includes both mixtures and separate individual tautomers thereof.

Also included in the invention are radiolabelled derivatives of compounds of formula **(1)** which are suitable for biological studies.

Compounds of the formula **(1)** wherein one or more basic nitrogen atoms are present may form pharmaceutically acceptable salts with acids such as hydrochloric, hydrobromic, sulfuric, phosphoric, methanesulfonic, acetic, citric, fumaric, lactic, maleic, succinic and tartaric acids.

Compounds of the formula **(1)** may form pharmaceutically acceptable salts with metal ions, such as alkali metals for example sodium and potassium, or with an ammonium ion.

A preferred group of compounds of the formula **(1)** is that
wherein
R¹ is H; methyl; or ethyl;
R² is H; methyl; or a halogen atom;
R³ is C₁-C₄ alkyl optionally substituted with one or more fluoro atoms;
R⁴ is ethyl; *n*-propyl; or allyl;
R⁵ is SO₂NR⁶R⁷; or NHSO₂R⁸;
R⁶ and R⁷ taken together with the nitrogen atom to which they are attached form a piperidino, piperazinyl or homopiperazinyl group wherein said group is substituted with R⁹;
R⁸ is methyl;
R⁹ is C₁-C₄ alkyl optionally substituted with one or more halide atoms, OH, CO₂R¹⁰, or with a tetrazole group which is optionally substituted with C₁-C₃ alkyl; R¹⁰ is H.

A particularly preferred group of compounds of the formula (1) is that
wherein
R¹ is methyl; or ethyl;
R² is H;
R³ is ethyl; *n*-propyl; 3-fluoropropyl; or cyclopropylmethyl;
R⁴ is ethyl; or *n*-propyl;
R⁵ is SO₂NR⁶R⁷; or NHSO₂R⁸;
R⁶ and R⁷ taken together with the nitrogen atom to which they are attached form a piperidino or piperazinyl group wherein said group is substituted with R⁹;
R⁸ is methyl;
R⁹ is C₁-C₄ alkyl optionally substituted with one or more fluoro or chloro atoms, OH, CO₂R¹⁰, or with a tetrazole group;
R¹⁰ is H.

Especially preferred individual compounds of the invention include:
2-(2-ethoxy-5-(4-methy)piperazin-1-ylsulfonyl)phenyl)-5-methyl-7-*n*-propyl-3,5-dihydro-4*H*-pyrrolo[3,2-*d*]pyrimidin-4-one;
2-(5-(4-methylpiperazin-1-ylsulfonyl)-2-*n*-propoxyphenyl)-5-methyl-7-*n*-propyl-3, 5-dihydro-4*H*-pyrrolo[3,2-*d*]pyrimidin-4-one;
2-(2-ethoxy-5-(4-*n*-propylpiperazin-1-ylsulfonyl)phenyl)-5-methyl-7-*n*-propyl-3,5-dihydro-4*H*-pyrrolo[3,2-*d*]pyrimidin-4-one;
2-(2-ethoxy-5-(4-isopropylpiperazin-1-ylsulfonyl)phenyl)-5-methyl-7-*n*-propyl-3,5 - dihydro-4*H*-pyrrolo[3,2-*d*]pyrimidin-4-one;
2-(2-ethoxy-5-(4-(2-fluoroethyl)piperazin-1-ylsulfonyl)phenyl)-5-methyl-7-*n*-prop yl-3,5-dihydro-4*H*-pyrrolo[3,2-*d*]pyrimidin-4-one;
2-(5-(4-(2-fluoroethyl)piperazin-1-ylsulfonyl)-2-*n*-propoxyphenyl)-5-methyl-7-*n*-p ropyl-3,5-dihydro-4*H*-pyrrolo[3,2-*d*]pyrimidin-4-one;
2-(2-ethoxy-5-(4-(3-fluoropropyl)piperazin-1-ylsulfonyl)phenyl)-5-methyl-7-*n*-pro pyl-3,5-dihydro-4*H*-pyrrolo[3,2-*d*]pyrimidin-4-one;
2-(2-ethoxy-5-(4-(3-fluoropropyl)piperazin-1-ylsulfonyl)phenyl)-7-ethyl-5-methyl -3,5-dihydro-4*H*-pyrrolo[3,2-*d*]pyrimidin-4-one;
2-(2-ethoxy-5-(4-(3-fluoropropyl)piperazin-1-ylsulfonyl)phenyl)-7-(3-fluoropropyl)-5-methyl-3,5-dihydro-4*H*-pyrrolo[3,2-*d*]pyrimidin-4-one;
7-cyclopropylmethyl-2-(2-ethoxy-5-(4-(3-fluoropropyl)piperazin-1-ylsulfonyl)phenyl) -5-methyl-3,5-dihydro-4*H*-pyrrolo[3,2-*d*]pyrimidin-4-one;
2-(2-ethoxy-5-(4-(3-fluoropropyl)piperazin-1-ylsulfonyl)phenyl)-5-ethyl-7-ethyl-3,5-d ihydro-4*H*-pyrrolo[3,2-*d*]pyrimidin-4-one;
2-(2-ethoxy-5-(4-(3-fluoropropyl)piperazin-1-ylsulfonyl)phenyl)-5-ethyl-7-*n*-propyl-3 5-dihydro-4*H*-pyrrolo[3,2-*d*]pyrimidin-4-one;
2-(2-ethoxy-5-(4-isopropylpiperazin-1-ylsulfonyl)phenyl)-5-ethyl-7-*n*-propyl-3,5-dihydro-4*H*-pyrrolo[3,2-*d*]pyrimidin-4-one;
2-(2-ethoxy-5-(4-(3-fluoropropyl)piperazin-1-ylsulfonyl)phenyl)-5-ethyl-7-*n*-propyl-3, 5-dihydro-4*H*-pyrrolo[3,2-*d*]pyrimidin-4-one;
2-(2-ethoxy-5-(4-(2-hydroxyethyl)piperazin-1-ylsulfonyl)phenyl)-5-ethyl-7-*n*-propyl-3 ,5-dihydro-4*H*-pyrrolo[3,2-*d*]pyrimidin-4-one;
2-(2-ethoxy-5-(4-ethylpiperazin-1-ylsulfonyl)phenyl)-5-ethyl-7-(3-fluoropropyl)-3,5-d-ihydro-4*H*-pyrrolo[3,2-*d*]pyrimidin-4-one;
2-(5-(4-ethylpiperazin-1-ylsulfonyl)-2-*n*-propoxyphenyl)-5-ethyl-7-(3-fluoropropyl)-3-5-dihydro-4*H*-pyrrolo[3,2-*d*]pyrimidin-4-one;
2-(2-ethoxy-5-(4-isopropylpiperazin-1-ylsulfonyl)phenyl)-5-ethyl-7-(3-fluoropropyl)-3,5-dihydro-4*H*-pyrrolo[3,2-*d*]pyrimidin-4-one;
2-(2-ethoxy-5-(4-(3-fluoropropyl)piperazin-1-ylsulfonyl)phenyl)-5-ethyl-7-(3-fluoropr opyl)-3,5-dihydro-4*H*-pyrrolo[3,2-*d*]pyrimidin-4-one;
2-(5-(4-(3-fluoropropyl)piperazin-1-ylsulfonyl)-2-*n*-propoxyphenyl)-5-ethyl-7-(3-fluoropropyl)-3,5-dihydro-4*H*-pyrrolo[3,2-*d*]pyrimidin-4-one;
2-(2-ethoxy-5-(4-(2-hydroxyethyl)piperazin-1-ylsulfonyl)phenyl)-5-ethyl-7-(3-fluorop ropyl)-3,5-dihydro-4*H*-pyrrolo[3,2-*d*]pyrimidin-4-one;
2-(5-(4-(2-hydroxyethyl)piperazin-1-ylsulfonyl)-2-*n*-propoxyphenyl)-5-ethyl-7-(3-fluoropropyl)-3,5-dihydro-4*H*-pyrrolo[3,2-*d*]pyrimidin-4-one;
2-(5-(4-(3-hydroxypropyl)piperazin-1-ylsulfonyl)-2-*n*-propoxyphenyl)-5-ethyl-7-(3-fluoropropyl)-3,5-dihydro-4*H*-pyrrolo[3,2-*d*]pyrimidin-4-one;
2-(5-(4-(2-hydroxyethyl)piperazin-1-ylsulfonyl)-2-*n*-propoxyphenyl)-5-ethyl-7-*n*-propyl-3,5-dihydro-4*H*-pyrrolo[3,2-*d*]pyrimidin-4-one;
2-(5-(4-(3-fluoropropyl)piperazin-1-ylsulfonyl)-2-*n*-propoxyphenyl)-5-methyl-7-*n* -propyl-3,5-dihydro-4*H*-pyrrolo[3,2-*d*]pyrimidin-4-one;
2-(2-ethoxy-5-(4-(2-hydroxyethyl)piperazin-1-ylsulfonyl)phenyl)-5-methyl-7-*n*-propyl-3,5-dihydro-4*H*-pyrrolo[3,2-*d*]pyrimidin-4-one;
2-(5-(4-(2-hydroxyethyl)piperazin-1-ylsulfonyl)-2-*n*-propoxyphenyl)-5-methyl-7-*n* -propyl-3,5-dihydro-4*H*-pyrrolo[3,2-*d*]pyrimidin-4-one;
2-(5-(2-ethoxy-4-(3-hydroxypropyl)piperazin-1-ylsulfonyl)phenyl)-5-methyl-7-*n*-p ropyl-3,5-dihydro-4*H*-pyrrolo[3,2-*d*]pyrimidin-4-one;
2-(5-(4-(3-hydroxypropyl)piperazin-1-ylsulfonyl)-2-*n*-propoxyphenyl)-5-methyl-7 -*n*-propyl-3,5-dihydro-4*H*-pyrrolo[3,2-*d*]pyrimidin-4-one;
2-(2-ethoxy-5-(4-(hydroxycarbonylmethyl)piperidin-1-ylsulfonyl)phenyl)-5-methyl-7-*n*-propyl-3,5-dihydro-4*H*-pyrrolo[3,2-*d*]pyrimidin-4-one;
2-(5-(4-(hydroxycarbonylmethyl)piperidin-1-ylsulfonyl)-2-*n*-propoxyphenyl)-5-methyl-7-*n*-propyl-3,5-dihydro-4*H*-pyrrolo[3,2-*d*]pyrimidin-4-one;
2-(2-ethoxy-5-(4-(2-hydroxycarbonylethyl)piperidin-1-ylsulfonyl)phenyl)-5-methyl-7-*n*-propyl-3,5-dihydro-4*H*-pyrrolo[3,2-*d*]pyrimidin-4-one;
2-(5-(4-(2-hydroxycarbonylethyl)piperidin-1-ylsulfonyl)-2-*n*-propoxyphenyl)-5-methyl-7-*n*-propyl-3,5-dihydro-4*H*-pyrrolo[3,2-*d*]pyrimidin-4-one;
2-(2-ethoxy-5-(4-(1*H*-tetrazol-5-ylmethyl)piperazin-1-ylsulfonyl)phenyl)-5-methyl -7-*n*-propyl-3,5-dihydro-4*H*-pyrrolo[3,2-*d*]pyrimidin-4-one;
5-methyl-2-(2-*n*-propoxy-5-(4-(2-(1*H*-tetrazol-5-yl)ethyl)piperazin-1-ylsulfonyl)phenyl)-7-*n*-propyl-3,5-dihydro-4*H*-pyrrolo[3,2-*d*]pyrimidin-4-one;
2-(2-ethoxy-5-(4-(1*H*-tetrazol-5-ylmethyl)piperidin-1-ylsulfonyl)phenyl)-5-methyl -7-*n*-propyl-3,5-dihydro-4*H*-pyrrolo[3,2-*d*]pyrimidin-4-one;
5-methyl-2-(2-*n*-propoxy-5-(4-(1*H*-tetrazol-5-ylmethyl)piperidin-1-ylsulfonyl)phenyl)-7-*n*-propyl-3,5-dihydro-4*H*-pyrrolo[3,2-*d*]pyrimidin-4-one;
and physiologically acceptable salts and solvates (e. g. hydrates) thereof.

In another aspect, this invention provides processes for the preparation of compounds of the formula **(1)** or pharmaceutically acceptable salts thereof. Compounds of the formula **(1)** may be prepared from compounds of the formula **(2), (3)** or **(4):** 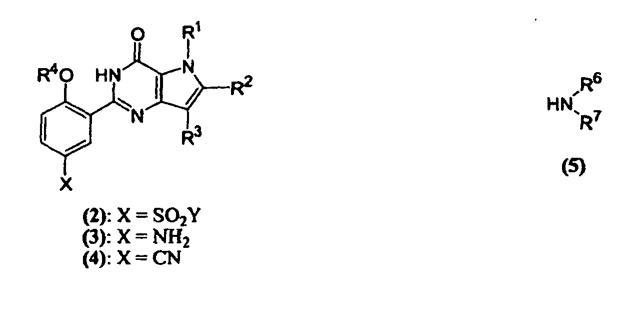 wherein R¹, R², R³ and R⁴ are as previously defined, and X represents sulfonyl halide, cyano or amino group, and Y represents a halogen atom, preferably a chloro atom. The coupling reaction of compounds of the formula (**2**) with a compound of the formula (**5**) (wherein R⁶ and R⁷ are as previously defined) is generally carried out at 0 °C to room temperature for 1-24 hours in a suitable solvent such as a C₁-C₃ alkanol, dichloromethane, DMF, or water using an excess amount of (**5**) or in the presence of an organic tertiary amine, preferably triethylamine to scavenge the acid by-product. For compounds of the formula (1) wherein R⁹ is C₁-C₆ alkyl substituted with C=NR¹³(NR¹⁴R¹⁵), a cyano group in the precursor compound may be transformed into the amidine functionality. The reaction can be affected by treating a cyano compound with saturated HCl gas in an anhydrous alcohol such as methanol and ethanol, at -20 ° to 0 °C, and the subsequent reaction of the resulting alkyl imidate intermediate with an appropriate amine at 0 °C to room temperature.

The reaction of amine compounds of the formula (**3**) with a compound of the formula (**6**), (**7**) or (**8**): 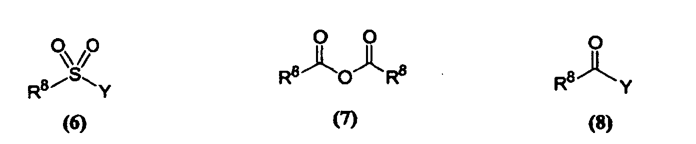 (wherein R⁸ is as previously defined, and Y represents a halogen atom, preferably a chloro atom) is conveniently carried out at 0 °C to room temperature for 1-24 hours in an inert anhydrous solvent such as dichloromethane or THF using an excess amount of (**6**), (**7**) or (**8**), in the presence of an organic tertiary amine, preferably triethylamine to scavenge the acid by-product. The sulfonyl halide of the formula (**6**), the carboxylic acid anhydride of the formula (**7**) and the acyl halide of the formula (**8**) are either commercially available or readily obtainable by conventional synthetic procedures.

The cyano compounds of the formula **(4)** can be effectively converted to the corresponding tetrazole derivatives by reacting with NaN₃ in the presence of *n*-Bu₃SnCl as a Lewis acid at refluxing temperature in an anhydrous hydrocarbon solvent such as toluene.

Compounds of the formula **(2), (3)** and **(4)** may be prepared from compounds of the formula **(9), (10)** and **(11),** respectively: 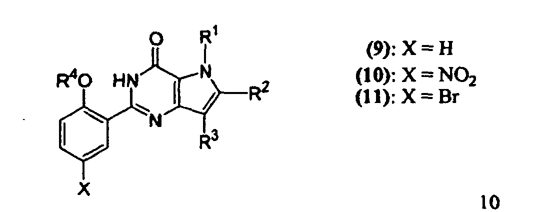 (wherein R¹, R², R³ and R⁴ are as previously defined, and X represents hydrogen, nitro or bromide group) by adopting precedent procedures.

Compounds of the formula **(2)** may be prepared from compounds of the formula **(9)** by using known methods for the introduction of a sulfonyl halide group into an aromatic ring, for example, when halide represents a chloro atom, by the action of chlorosulfonic acid at 0 °C to room temperature for 3-24 hours without any solvent.

The amines of the formula (**3**) can be readily obtained by reduction of the corresponding nitro compounds of the formula (**10**) using well-known methods such as catalytic hydrogenation in an alcoholic solvent, or tin(II) chloride reduction, and so on.

The cyano compounds of the formula (**4**) may be readily prepared from the bromide compounds of the formula (**11**) by displacement of the bromide with CuCN at 150-200 °C in a high boiling solvent such as 1-methyl-2-pyrrolidinone.

Compounds of the formula (**9**), (**10**) and (**11**) may be prepared from compounds of the formula (**12**), (**13**) and (**14**), respectively: 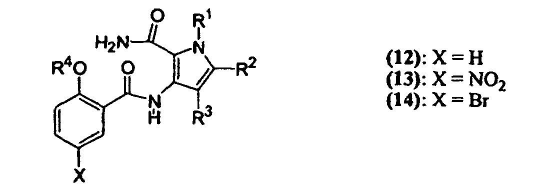 (wherein R¹, R², R³ and R⁴ are as previously defined, and X represents hydrogen, nitro or bromide group) by the application of known cyclization methods for pyrimidinone ring formation.

A cyclization reaction is generally carried out by heating at an elevated temperature, for example 50-150 °C, in the presence of an acid or a base in a suitable solvent such as an aqueous C₁-C₄ alkanol, water, a halogenated hydrocarbon, or acetonitrile. Thus, for example, the cyclization may be affected by treatment of a compound of the formulae (**12**)-(**14**) with an inorganic or organic base such as sodium hydroxide, potassium carbonate or potassium tert-butoxide, in an alcoholic aqueous medium, preferably potassium *tert*-butoxide in *tert*-butanol at 60 °C to reflux temperature.

For compounds of the formula (**9**) (wherein R¹, R³ and R⁴ are as previously defined, and R² is halide), the introduction of a halide atom to compounds of the formula (**12**) is carried out prior to subsequent cyclization. Halogenations may be affected by applying appropriate conditions for each halide, for example, N-chlorosuccinimide (NCS) in a halogenated solvent such as CH₂Cl₂ at -10 °C to room temperature for chlorination, bromine in acetic acid in the presence of sodium acetate at room temperature for bromination, and iodine along with mercuric oxide (HgO) in a hydrocarbon solvent such as benzene at 0 °C to room temperature for iodination.

Compounds of the formulae (**12**)-(**14**) may be prepared from compounds of the formula (**15**) and (**16**), (**17**) or (**18**), respectively: 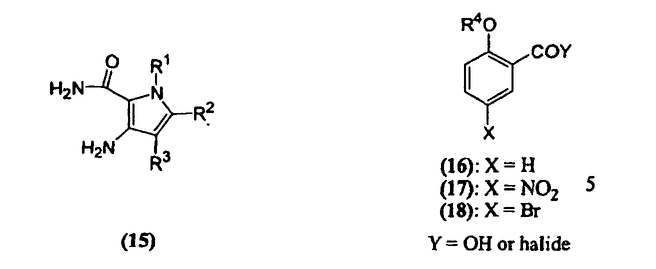 wherein R¹, R², R³ and R⁴ are as previously defined, X represents hydrogen, nitro or bromide group and Y represents a hydroxyl group or a halogen atom, preferably a chloro atom.

The reaction is generally carried out by first converting a carboxylic acid of the formulae **(16)-(18)** (Y = OH) to the corresponding acyl chloride using excess amounts of well-known reagents in the literature, preferably thionyl chloride or oxalyl chloride, in the presence of an inert solvent such as dichloromethane or benzene, at room temperature to reflux temperature. The coupling reaction with a compound of the formula (**15**) is generally affected by using an excess of the resulting acyl chloride (**16**)-(**18**) (Y = Cl) in the presence of an excess of an organic tertiary amine such as triethylamine to act as scavenger for the acid by-product (HY), optionally in the presence of a catalyst such as 4-dimethylaminopyridine (DMAP), in an inert anhydrous solvent such as dichloromethane at 0 °C to room temperature for 2-6 hours. The starting materials of the formulae (**16**)-(**18**) (Y = OH) are either commercially available or readily obtainable from the compound of the formula (**16**) by the methods known in the literature. For example, the nitro compounds of the formula (**17**) can be efficiently prepared from compounds of the formula (**16**) by using known methods for the nitration of an aromatic ring, and the reaction is generally carried out using sodium nitrite or fuming nitric acid under a strongly acidic medium such as concentrated sulfuric acid or trifluoroacetic acid, preferably trifluoroacetic acid, at -10 °C to room temperature for 1-24 hours.

Compounds of the formula **(15)** may be prepared from compounds of the formula **(20)** in two steps: 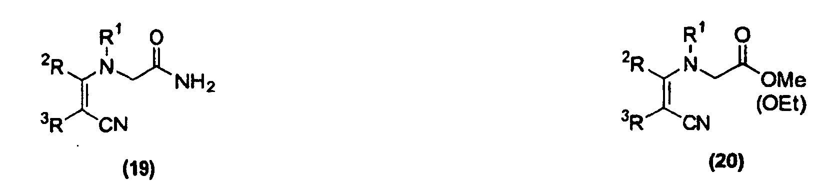 (wherein R¹, R² and R³ are as previously defined) by converting compounds of the formula **(20)** into the corresponding amide compounds of the formula **(19)** and the subsequent cyclization of compounds of the formula **(19)** for pyrrole ring formation. Amide formation may be affected by using ammonia either in an alcoholic solvent or water, preferably water, at room temperature to 100 °C, in the presence or absence of sodium cyanide as a catalyst.

A cyclization reaction is effectively carried out by heating at an elevated temperature, for example 50-150 °C, in the presence of a base in a suitable solvent such as aqueous C₁-C₄ alkanol or acetonitrile. Thus, for example, the cyclization may be affected by treatment of a compound of the formula **(20)** with an alkoxide base such as sodium ethoxide or potassium *tert*-butoxide, in an alcoholic medium, preferably sodium ethoxide in ethanol at 60 °C.

Compounds of the formula **(20)** may be prepared from compounds of the formula **(21)** and **(22):** 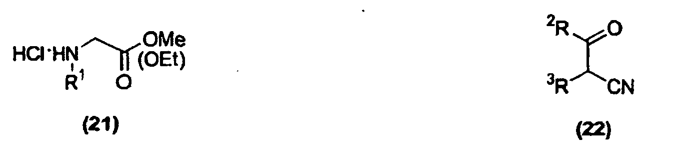 wherein R¹, R² and R³ are as previously defined.

A condensation reaction between compounds of the formula **(21)** and **(22)** is generally performed in a mixture of an alcohol and water, preferably methanol alone, in the presence of a weak base such as sodium acetate, at room temperature for 1-3 days. Compounds of the formula **(21)** are either commercially available or readily obtainable from glycine using well-documented synthetic methods.

Compounds of the formula **(22)** may be prepared from compounds of the formula **(23)** and **(24):** 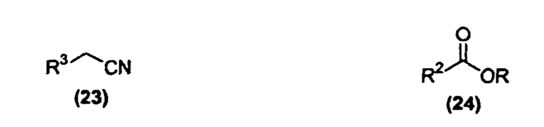 wherein R² and R³ are as previously defined, and R is C₁-C₃ alkyl.

Acylation reaction of compounds of the formula (**23**) is efficiently carried out by trapping the anionic species of compounds of the formula (**23**) with compounds of the formula (**24**) at -78 °C to room temperature. Generation of the anionic intermediate from compounds of the formula (**23**) may be affected by the action of a strong amide base such as sodium amide, alkali metal hexamethyldisilazide (Li, Na, or KHMDS) or lithium diisopropylamide (LDA), preferably LDA, in an anhydrous ethereal solvent such as tetrahydrofuran, at low temperature, ranging from -78 ° to 0 °C.

Compounds of the formula **(1)** may be also prepared by cyclizing compounds of the formula (**25**): 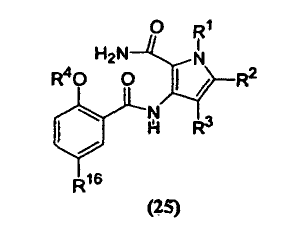 wherein R¹, R², R³ and R⁴ are as previously defined, and R¹⁶ is a group R⁵ as hereinbefore defined or a precursor to a group R⁵.

A cyclization reaction is generally carried out by heating at an elevated temperature, for example 50-150 °C, in the presence of an acid or a base in a suitable solvent such as an aqueous C₁-C₄ alkanol, water, a halogenated hydrocarbon, or acetonitrile. Thus, for example, the cyclization may be affected by treatment of a compound of the formula **(25)** with an inorganic or organic base such as sodium hydroxide, potassium carbonate or potassium *tert*-butoxide, in an aqueous alcoholic medium. Examples of R¹⁶ being a precursor to a group R⁵ are when R⁵ contains a carboxylic acid since an ester group of the formula (**25**) can be converted to the corresponding carboxylic acid under the basic cyclization condition.

Compounds of the formula (**25**) may be prepared from compounds of the formula (**15**) and (**26**): 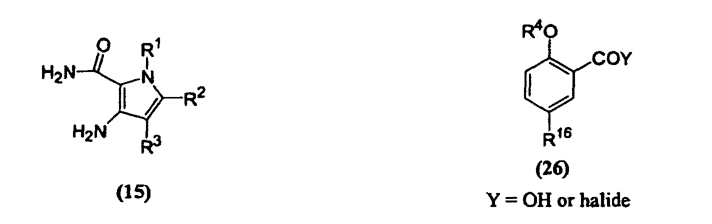 wherein R¹, R², R³, R⁴ and R¹⁶ are as previously defined, and Y represents a hydroxyl group or a halogen atom, preferably a chloro atom.

The reaction is generally carried out by first converting a carboxylic acid of the formula (**26**) (Y = OH) to the corresponding acyl chloride using excess amounts of well-known reagents in the literature, preferably thionyl chloride or oxalyl chloride, in the presence or absence of an inert solvent such as dichloromethane or benzene, at room temperature to reflux temperature. The coupling reaction with a compound of the formula (**15**) is generally affected by using an excess of the resulting acyl chloride (**26**) (Y = Cl) in the presence of an excess of an organic tertiary amine such as triethylamine to act as scavenger for the acid by-product (HY), optionally in the presence of a catalyst such as 4-dimethylaminopyridine (DMAP), in an inert anhydrous solvent such as dichloromethane at 0 °C to room temperature for 2-6 hours. The starting materials of the formula (**26**) (Y = OH) are readily obtainable from compound of the formula (**16**) (X = H, Y = OH) by the methods known in the literature.

Amines of the formula (**5**), when not commercially available, can be prepared by conventional synthetic procedures, in accordance with literature precedents, from readily accessible starting materials using standard reagents and reaction conditions.

Certain compounds of the formula (**5**), (**5a**), wherein R⁶ and R⁷ taken together with the nitrogen atom to which they are attached form a piperazinyl or homopiperazinyl group substituted with R⁹ (R⁹ is as previously defined), can be synthesized readily from the compounds of the formula (**27**): 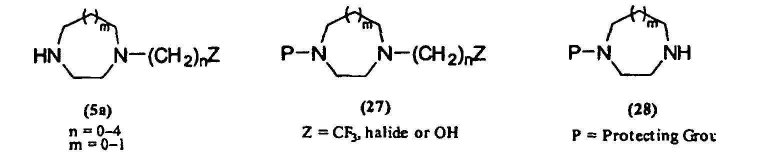 wherein Z is a group CF₃, hydroxyl, or a halogen, preferably fluoro atom and P represents an appropriate protecting group, for example, benzyl, benzyloxycarbonyl (Cbz) or *tert*-butoxycarbonyl (Boc).

Removal of benzyl or benzyloxycarbonyl (Cbz) group in the compounds of the formula (**27**) can be performed under a hydrogenation condition using a catalytic amount of palladium on carbon in an alcoholic solvent such as methanol or ethanol, at room temperature to afford the corresponding compound of the formula (**5a**). Cleavage of *tert*-butoxycarbonyl (Boc) group in the compounds of the formula (**27**) can be affected under the acidic conditions using aqueous HCl or trifluoroacetic acid in an aprotic solvent such as tetrahydrofuran or dichloromethane at room temperature to afford the corresponding salt of the formula (**5a**). Starting materials of the formula (**27**) can be prepared from 1-benzylpiperazine or 1-*tert*-butoxycarbonylhomepiperazine of the formula (**28**) by direct N-alkylation with an appropriate alkyl halide containing a CF₃, hydroxyl or halogen group.

Another compounds of the formula (**5**), (**5b**), wherein R⁶ and R⁷ taken together with the nitrogen atom to which they are attached form a piperazinyl or piperidino group substituted with R⁹ (R⁹ is as previously defined and is substituted with a 1*H*-(tetrazol-5-yl) group), can be synthesized readily from the compounds of the formula (29): 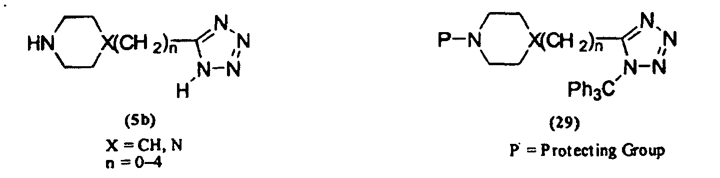 wherein X is a group methine or nitrogen atom and P represents an appropriate protecting group, for example, preferably *tert*-butoxycarbonyl (Boc).

Removal of *tert*-butoxycarbonyl (Boc) and triphenylmethyl (Trityl) groups in the compounds of the formula (**29**) can be affected simultaneously under the acidic conditions using aqueous HCl or trifluoroacetic acid in an aprotic solvent such as tetrahydrofuran at room temperature, in the presence of excess 1*H*-tetrazole as a carbocation scavenger, to afford the corresponding salt of the formula (**5b**).

Compounds of the formula (**29**) can be prepared from the compounds of the formula (**30**): 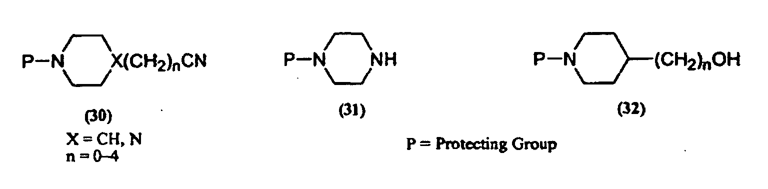 wherein X and P are as previously defined.

Conversion of the cyano group of the formula (**30**) is generally affected by using tributyltin chloride and sodium azide in a hydrocarbon solvent, preferably toluene, at refluxing temperature to afford corresponding tetrazole compounds of the formula (**29**). Compounds of the formula (**30**) are readily prepared either from 1-*tert*-butoxycarbonylpiperazine of the formula (**31**) by direct N-alkylation with an appropriate alkyl halide containing a cyano group, or by conversion of the hydroxyl functionality of the formula **(32)** to a cyano group, using well-documented procedures. Starting materials of the formula **(31)** and **(32)** are either commercially available or readily accessible by conventional synthetic procedures in accordance with literature precedents.

The resulting compounds of this invention represented by the formula (**1**)-(**5**), (**9**)-(**15**) and (**19**)-(**22**), can be separated and purified by appropriate conventional methods such as column chromatography and recrystallization.

Compounds of the invention may be administered by any suitable route, for example by oral, buccal, sub-lingual, rectal, vaginal, nasal, topical or parenteral (including intravenous, intramuscular, subcutaneous and intracoronary) administration.

For administration to man in the curative or prophylactic treatment of the disorders identified above, oral, buccal or sub-lingual dosages of a compound of the formula (**1**) will generally be in the range of from 0. 1-400 mg daily for an average adult patient (70 Kg). Thus for a typical adult patient, individual tablets or capsules contain from 0. 05-200 mg of active compound, in a suitable pharmaceutically acceptable vehicle or carrier, for administration in single or multiple doses, once or several times per day. Dosages for parenteral administration will typically be within the range of from 0. 01-100 mg per single dose as required. In practice the physician will determine the actual dosing regimen which will be most suitable for an individual patient and it will vary with the age, weight and response of the particular patient. The above dosages are exemplary of the average case but there can be individual instances in which higher or lower dosage ranges may be merited, and such are within the scope of this invention.

For human use, a compound of the formula **(1)** can be administered alone, but will generally be administered in admixture with a pharmaceutical carrier selected with regard to the intended route of administration and standard pharmaceutical practice. For example, the compound may be administered orally, buccally or sublingually, in the form of tablets containing excipients such as starch or lactose, or in capsules or ovules either alone or in admixture with excipients, or in the form of elixirs or suspensions containing flavouring or colouring agents. Such liquid preparations may be prepared with pharmaceutically acceptable additives such as suspending agent (e. g. methylcellulose, a semi-synthetic glyceride such as witepsol or mixtures of glycerides such as a mixture of apricot kernel oil and PEG-6 esters or mixtures of PEG-8 and caprylic/capric glycerides). A compound may also be injected parenterally, for example intravenously, intramuscularly, subcutaneously or intracoronarily. For parenteral administration, the compound is best used in the form of a sterile aqueous solution which may contain other substances, for example salts, or monosaccharides such as mannitol or glucose, to make the solution isotonic with blood.

Thus, the invention provides in a further aspect a pharmaceutical composition comprising a compound of the formula **(1),** or a pharmaceutically acceptable salt thereof, together with a pharmaceutically acceptable diluent or carrier therefor.

The invention also provides a compound of the formula **(1),** or a pharmaceutically acceptable salt thereof, or a pharmaceutical composition containing either entity, for use in the treatment of impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases characterized by disorders of gut motility (e. g. irritable bowel syndrome).

The invention further provides the use of a compound of the formula **(1),** or a pharmaceutically acceptable salt thereof, or a pharmaceutical composition containing either entity, for the manufacture of a medicament for the treatment of impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases characterized by disorders of gut motility (e. g. irritable bowel syndrome).

In a further aspect, the invention provides a method of treating or preventing impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases characterized by disorders of gut motility (e. g. irritable bowel syndrome), in a mammal (including a human being), which comprises administering to said mammal a therapeutically effective amount of a compound of formula **(1),** or a pharmaceutically acceptable salt thereof, or a pharmaceutical composition containing either entity.

The invention also includes any novel intermediates of the formulae (**2**)-(**4**), (**9**)-(**15**) and (**25**) disclosed herein.

### EXAMPLES

The present invention is further illustrated in the following Preparative Examples and Examples, which should not be taken to limit the scope of the invention.

### Preparative Example 1

### Preparation of 1-tert-butoxycarbonyl-4-(4-fluoro-n-butyl)piperazine (a compound of the formula (27) wherein n = 4, m = 0, Z = F)

A mixture of 1-*tert*-butoxycarbonylpipetazine (200 mg, 1. 07 mmol), 1-bromo-4-fluoropropane (170 mg, 1. 13 mmol) and sodium bicarbonate (680 mg, 8. 06 mmol) in anhydrous *N*,*N-*dimethylforamide (DMF) (6 mL) was stirred overnight at 40-50 °C and was cooled to room temperature. The reaction mixture was filtered through a Celite pad, and the filtrate was evaporated to dryness under vacuum to afford yellowish oil. The crude product was purified by MPLC on silica gel (1% MeOH in CHCl₃) to afford the titled compound (182 mg, 66%) as a pale yellow oil.
IR (neat) 1700 (C=O), 1176 (C-F) cm⁻¹;
¹H NMR (DMSO-*d*₆) δ 1. 46 (s, 9 H, C(CH₃)₃), 1. 58-1. 78 (m, 4 H, NCH₂C*H*₂C*H*₂CH₂F), 2. 35-2. 42 (m, 6 H, 3 NCH₂), 3. 43 (dd, *J =* 5. 4 Hz, 4. 8 Hz, 4 H, 2 BocNCH₂), 4. 46 (dt, *J =* 47. 4 Hz, 6. 0 Hz, 2 H, CH₂C*H*₂F); MS (FAB) *m*/*z* 261 (MH⁺).

### Preparative Example 2

### Preparation of 1-(4-fluoro-n-butyl)piperazine trifluoroacetic acid (a compound of the formula (5a) wherein n = 4, m = 0, Z = F)

A mixture of 1-*tert*-butoxycarbonyl-4-(4-fluoro-*n*-butyl)piperazine (3. 50 g, 10. 74 mmol) in trifluoroacetic acid (20 mL) was stirred at room temperature for 1 h. The reaction mixture was evaporated to dryness under vacuum and the resulting residue was triturated from Et₂O to afford the titled compound (2. 61 g, 87%) as a white solid.
mp 108. 5-109. 5 °C;
IR (neat) 1665 (C=O), 1118 (C-F) cm⁻¹;
¹H NMR (CDCl₃/TMS) δ 1. 58-1. 78 (m, 4 H, NCH₂C*H*₂C*H*₂CH₂F), 3. 11 (t, *J* = 6. 9 Hz, NC*H*₂CH₂), 3. 30-3. 50 (m, 8 H, 2 NCH₂ and 2 BocNCH₂), 4. 46 (dt, *J* = 47. 7 Hz, 5.4 Hz, 2 H, CH₂C*H*₂F); MS (FAB) *m*/*z* 161 (MH⁺).

### Preparative Example 3

### Preparation of 1-tert-butoxycarbonyl-4-(2-fluoroethyl)homopiperazine (a compound of the formula (27) wherein n = 2, m = 1, Z = F)

A mixture of 1-*tert*-butoxycarbonylhomopiperazine (800 mg, 3. 99 mmol), 1-bromo-2-fluoroethane (0. 46 mL, 11. 98 mmol) and potassium carbonate (1. 10 g, 7. 98 mmol) in anhydrous tetrahydrofuran (20 mL) was stirred overnight at 70 °C and was cooled to room temperature. The reaction mixture was filtered through a Celite pad, and the filtrate was evaporated to dryness under vacuum to afford yellowish oil. The crude product was purified by MPLC on silica gel (3% MeOH in CHCl₃) to afford the titled compound (653 mg, 67%) as a pale yellow oil.
IR (neat) 1686 (C=O), 1163 (C-F) cm⁻¹;
¹H NMR (CDCl₃/TMS) δ 1. 46 (s, 9 H, C(CH₃)₃), 1. 78-1. 90 (m, 2 H, NCH₂C*H*₂CH₂N), 2. 68-2. 78 (m, 4 H, 2 NCH₂), 2. 84 (dt, *J =* 26. 7 Hz, 5. 1 Hz, 2 H, NC*H*₂CH₂F), 3. 40-3. 54 (m, 4 H, 2 BocNCH₂), 4. 53 (dt, *J =* 47. 7 Hz, 5. 1 Hz, 2 H, NCH₂C*H*₂F); MS (FAB) *m*/*z* 247 (MH⁺).

### Preparative Example 4

### Preparation of 1-(2-fluoroethyl)homopiperazine dihydrochloride (a compound of the formula (5a) wherein n = 2, m = 1, Z = F)

A mixture of 1-*tert*-butoxycarbonyl-4-(2-fluoroethyl)homopiperazine (550 mg, 2. 23 mmol) in 10% aqueous hydrochloric acid (2 mL) and tetrahydrofuran (4 mL) was stirred at room temperature for 2 h, and the reaction mixture was evaporated to dryness under vacuum. Resulting residue was triturated from Et₂O/MeOH to afford the titled compound (475 mg, 97%) as a white solid.
mp 185-186 °C;
IR (neat) 1069 (C-F) cm⁻¹;
¹H NMR (DMSO-*d*₆) δ 2. 06-2. 14 (m, 2 H, NCH₂C*H*₂CH₂N), 3. 16-3. 76(m, 10 H, 5 NCH₂), 4. 54(dt, *J=* 47. 1 Hz, 5. 7 Hz, 2 H, NCH₂C*H*₂F), 9. 57(br s, 1 H, NH⁺), 9. 91 (br s, 1 H, NH⁺), 11. 70 (br s, 1 H, NH⁺); MS (FAB) *m*/*z* 147 (MH⁺).

### Preparative Example 5

### Preparation of 1-tert-butoxycarbonyl-4-(3-fluoro-n-propyl)homopiperazine (a compound of the formula (27) wherein n = 3, m = 1, Z = F)

The titled compound was prepared as described in Preparative Example 3 by using 1-bromo-3-fluoropropane in place of 1-bromo-2-fluoroethane.
yield: 76%
IR (neat) 1700 (C=O), 1174 (C-F) cm⁻¹;
¹H NMR (CDCl₃/TMS) δ 1. 46 (s, 9 H, C(CH₃)₃), 1. 78-1. 91 (m, 4 H, NCH₂C*H*₂CH₂N and NCH₂C*H*₂CH₂F), 2. 58-2. 68 (m, 4 H, 2 NCH₂), 2. 61 (t, *J=* 7. 2 Hz, 2 H, NC*H*₂CH₂), 3. 40-3. 52 (m, 4 H, 2 BocNCH₂), 4. 50 (dt, *J=* 47. 1 Hz, 6. 0 Hz, 2 H, CH₂C*H*₂F); MS (FAB) *m*/*z* 261 (MH⁺).

### Preparative Example 6

### Preparation of 1-(3-fluoro-n-propyl)homopiperazine dihydrochloride (a compound of the formula (5a) wherein n = 3, m = 1, Z = F)

The titled compound was prepared as described in Preparative Example 4 by using 1-*tert*-butoxycarbonyl-4-(3-fluoro-*n*-propyl)homopiperazine in place of 1-*tert*-butoxycarbonyl-4-(2-fluoroethyl)homopiperazine.
yield: 88%
mp 194-195 °C (Et₂O/MeOH);
IR (neat) 1053 (C-F) cm⁻¹;
¹H NMR (DMSO-*d*₆) δ 2. 06-2. 14 (m, 4 H, NCH₂C*H*₂CH₂N and NCH₂C*H*₂CH₂F), 3. 16-3. 76 (m, 10 H, 5 NCH₂), 4. 54 (dt, *J =* 47. 1 Hz, 5. 7 Hz, 2 H, NCH₂C*H*₂F), 9. 58 (br s, 1 H, NH⁺), 9. 91 (br s, 1 H, NH⁺), 11. 70 (br s, 1 H, NH⁺); MS (FAB) *m*/*z* 161 (MH⁺).

### Prenarative Example 7

### Preparation of N-(2-cyano-2-n-propylvinyl)-N-methylglycine ethyl ester (a compound of the formula (20) wherein R¹ = CH₃, R² = H, R³ = CH₂CH₂CH₃)

A suspension of 2-cyanopentanal (33. 12 g, 0. 30 mol), *N*-methylglycine ethyl ester hydrochloride (62. 39 g, 0. 45 mol) and sodium acetate (36. 67 g, 0. 45 mol) in MeOH (600 mL) was stirred at room temperature for 21 h, and the mixture was evaporated to dryness under reduced pressure. Resulting residue was diluted with water (200 mL) and was extracted with ethyl acetate (200 mL × 3). Combined organic layer was dried (Na₂SO₄), filtered and the filtrate was evaporated to dryness under reduced pressure to give yellow oil. The crude product was purified by MPLC on silica gel (1% MeOH in CHCl₃) to afford the titled compound (47. 02 g, 80%) as a pale yellow oil.
IR (neat) 2180 (CN), 1745 (C=O) cm⁻¹;
¹H NMR (CDCl₃/TMS) δ 0. 90 (t, *J=* 7.5 Hz, 3 H, CH₂CH₂C*H*₃), 1. 30 (t, *J=* 6. 9 Hz, 3 H, OCH₂C*H*₃), 1. 45-1. 57 (m, 2 H, CH₂C*H*₂CH₃), 2. 03 (t, *J* = 7. 5 Hz, 2 H, C*H*₂CH₂CH₃), 3. 12 (s, 3 H, NCH₃), 3. 97 (s, 2 H, NCH₂CO), 4. 24 (q, *J =* 6. 9 Hz, 2 H, OC*H*₂CH₃), 6: 12 (s, 1 H, CH); MS (FAB) *m*/*z* 211 (MH⁺).

### Preparative Example 8

### Preparation of N-(2-cyano-2-ethylvinyl)-N-methylglycine methyl ester (a compound of the formula (20) wherein R¹ = CH₃, R² = H, R³ = CH₂CH₃)

The titled compound was prepared as described in Preparative Example 7 by using 2-cyanobutyraldehyde and *N*-methylglycine methyl ester hydrochloride in place of 2-cyanopentanal and *N*-methylglycine ethyl ester hydrochloride. yield: 62%
IR (neat) 2184 (CN), 1751 (C=O) cm⁻¹;
¹H NMR (CDCl₃/TMS) δ 1. 10 (t, *J* = 7. 5 Hz, 3 H, CH₂C*H*₃), 2. 10 (q, *J=* 7. 5 Hz, 2 H, C*H*₂CH₃), 3. 12 (s, 3 H, NCH₃), 3. 78 (s, 3 H, OCH₃), 3. 99 (s, 2 H, NCH₂CO), 6. 12 (s, 1 H, CH); MS (FAB) *m*/*z* 183 (MH⁺).

### Preparative Example 9

### Preparation of N-(2-cyano-2-(3-fluoropropyl)vinyl)-N-methylglycine methyl ester (a compound of the formula (20) wherein R¹ = CH₃, R² = H, R³ = CH₂CH₂CH₂F)

The titled compound was prepared as described in Preparative Example 7 by using 2-cyano-5-fluoropentanal and *N*-methylglycine methyl ester hydrochloride in place of 2-cyanopentanal and *N*-methylglycine ethyl ester hydrochloride.
yield: 73%
IR (neat) 2180 (CN), 1741 (C=O) cm⁻¹;
¹H NMR (CDCl₃/TMS) δ 1. 78-1. 97 (m, 2 H, CH₂C*H*₂CH₂F), 2. 22 (t, *J* = 7. 2 Hz, 2 H, C*H*₂CH₂CH₂F), 3. 13 (s, 3 H, NCH₃), 3. 79 (s, 3 H, OCH₃), 4. 01 (s, 2 H, NCH₂CO), 4. 48 (dt, J= 47. 1 Hz, 5. 7 Hz, 2 H, CH₂CH₂C*H*₂F), 6. 28 (s, 1 H, CH); MS (FAB) *m*/*z* 215 (MH⁺).

### Preparative Example 10

### Preparation of N-(2-cyano-2-(cyclopropylmethyl)vinyl)-N-methylglycine methyl ester (a compound of the formula (20) wherein R¹ = CH₃, R² = H, R³ = cyclopropylmethyl)

The titled compound was prepared as described in Preparative Example 7 by using 3-cyclopropyl-2-cyanopropionaldehyde and *N*-methylglycine methyl ester hydrochloride in place of 2-cyanopentanal and *N*-methylglycine ethyl ester hydrochloride.
yield: 70%
IR (neat) 2185 (CN), 1759 (C=O) cm⁻¹;
¹H NMR (CDCl₃/TMS) δ 0. 13-0. 18 (m, 2 H, c-C₃H₅), 0. 46-0. 53 (m, 2 H, c-C₃H₅), 0. 79-0. 93 (m, 1 H, c-C₃H₅), 1. 99 (d, *J* = 6. 9 Hz, 2 H, CHC*H*₂), 3. 13 (s, 3 H, NCH₃), 3. 78 (s, 3 H, OCH₃), 4. 01 (s, 2 H, NCH₂CO), 6. 27 (s, 1 H, CH); MS (FAB) *m*/*z* 209 (MH⁺).

### Preparative Example 11

### Preparation of N-(2-cyano-2-ethylvinyl)-N-ethylglycine methyl ester (a compound of the formula (20) wherein R¹ = CH₂CH₃, R² = H, R³ = CH₂CH₃)

The titled compound was prepared as described in Preparative Example 7 by using 2-cyanobutyraldehyde and *N*-ethylglycine methyl ester hydrochloride in place of 2-cyanopentanal and *N*-methylglycine ethyl ester hydrochloride.
yield: 51%
IR (neat) 2182 (CN), 1752 (C=O) cm⁻¹;
¹H NMR (CDCl₃/TMS) δ 1. 10 (t, *J*= 7. 5 Hz, 3 H, CH₂C*H*₃), 1. 19 (t, *J*= 7. 2 Hz, 3 H, NCH₂C*H*₃), 2. 10 (q, *J*=7. 5 Hz, 2 H, C*H*₂CH₃), 3. 34 (q, *J*=7. 2 Hz, 2 H, NC*H*₂CH₃), 3. 78 (s, 3 H, OCH₃), 4. 09(s, 2 H, NCH₂CO), 6. 28 (s, 1 H, CH); MS (FAB) *m*/*z* 197 (MH⁺).

### Preparative Example 12

### Preparation of N-(2-cyano-2-n-propylvinyl)-N-ethylglycine methyl ester (a compound of the formula (20) wherein R¹ = CH₂CH₃, R² = H, R³ = CH₂CH₂CH₃)

The titled compound was prepared as described in Preparative Example 7 by using 2-cyanopentanal and *N*-ethylglycine methyl ester hydrochloride in place of 2-cyanopentanal and *N*-methylglycine ethyl ester hydrochloride.
yield: 61 %
IR (neat) 2179 (CN), 1750 (C=O) cm⁻¹;
¹H NMR (CDCl₃/TMS) δ 0. 90 (t, *J =* 7. 5 Hz, 3 H, CH₂CH₂C*H*₃), 1. 19 (t, *J* = 7. 2 Hz, 3 H, NCH₂C*H*₃), 1. 44-1. 57 (m, 2 H, CH₂C*H*₂CH₃), 2. 03 (t, J = 7. 5 Hz, 2 H, C*H*₂CH₂CH₃), 3. 34 (q, *J* = 7. 2 Hz, 2 H, NC*H*₂CH₃), 3. 79 (s, 3 H, OCH₃), 4. 10 (s, 2 H, NCH₂CO), 6. 25 (s, 1 H, CH); MS (FAB) *m*/*z* 211 (MH⁺).

### Preparative Example 13

### Preparation of N-(2-cyano-2-(3-fluoropropyl)vinyl)-N-ethylglycine methyl ester (a compound of the formula (20) wherein R¹ = CH₂CH₃, R² = H, R³ = CH₂CH₂CH₂F)

The titled compound was prepared as described in Preparative Example 7 by using 2-cyano-5-fluoropentanal and *N*-ethylglycine methyl ester hydrochloride in place of 2-cyanopentanal and *N*-methylglycine ethyl ester hydrochloride.
yield: 50%
IR (neat) 2177 (CN), 1743 (C=O) cm⁻¹;
¹H NMR (CDCl₃/TMS) δ 1. 20 (t, *J*=7. 2 Hz, 3 H, NCH₂C*H*₃), 1. 78-1. 96 (m, 2 H, CH₂C*H*₂CH₂F), 2. 22 (t, *J=* 7. 2 Hz, 2 H, C*H*₂CH₂CH₂F), 3. 35 (q, *J*=7. 2 Hz, 2 H, N C*H*₂CH₃), 3. 79 (s, 3 H, OCH₃), 4. 10 (s, 2 H, NCH₂CO), 4. 48 (dt, *J*= 47. 4 Hz, 5. 7 Hz, 2 H, CH₂CH₂C*H*₂F), 6. 32 (s, 1 H, CH); MS (FAB) *m*/*z* 229 (MH⁺).

### Preparative Example 14

### Preparation of N-(2-cyano-2-n-propylvinyl)-N-methylglycine amide (a compound of the formula (19) wherein R¹ = CH₃, R² = H, R³ = CH₂CH₂CH₃)

A suspension of *N*-(2-cyano-2-*n*-propylvinyl)-*N*-methylglycine ethyl ester (6.00 g, 28. 53 mmol) in 29% aqueous ammonia solution (50 mL) was stirred overnight at room temperature. Resulting precipitates were collected by filtration, which were washed with cold water and diethyl ether to afford the titled compound (3. 20 g, 62%) as a white solid. Ether layer was separated from the filtrate, and the aqueous layer was further extracted with 3% MeOH in CHCl₃ (50 mL). Combined organic layer was dried (Na₂SO₄), filtered and the filtrate was evaporated to dryness under reduced pressure to give a pale yellow solid (1. 31 g, 25%).
mp 106-106. 5 °C;
IR (neat) 3375, 3188 (NH₂), 2179 (CN), 1660, 1636 (C=O) cm⁻¹;
¹H NMR (CDCl₃/TMS) δ 0. 91 (t, *J=* 7. 5 Hz, 3 H, CH₂CH₂CH₃), 1. 47-1. 55 (m, 2 H, CH₂C*H*₂CH₃), 2. 05 (t, *J =* 7. 5 Hz, 2 H, C*H*₂CH₂CH₃), 3. 17 (s, 3 H, NCH₃), 3. 92 (s, 2 H, NCH₂CO), 5. 67 (br s, 1 H, CONH), 5. 91 (br s, 1 H, CONH), 6. 27 (s, 1 H, CH); MS (FAB) *m*/*z* 182 (MH⁺).

### Prenarative Example 15

### Preparation of N-(2-cyano-2-ethylvinyl)-N-methylglycine amide (a compound of the formula (19) wherein R¹ = CH₃, R² = H, R³ = CH₂CH₃)

The titled compound was prepared as described in Preparative Example 14 by using *N*-(2-cyano-2-ethylvinyl)-*N*-methylglycine methyl ester in place of *N*-(2-cyano-2-*n*-propylvinyl)-*N*-methylglycine ethyl ester.
yield: 81%
mp 101-102°C (MeOH/CH₂Cl₂/ether);
IR (neat) 3429, 3300 (NH), 2173 (CN), 1694, 1669 (C=O) cm⁻¹;
¹H NMR (CDCl₃/TMS) δ 1. 11 (t, *J =* 7. 5 Hz, 3 H, CH₂C*H*₃), 2. 11 (q, *J =* 7. 5 Hz, 2 H, C*H*₂CH₃), 3. 16 (s, 3 H, NCH₃), 3. 92 (s, 2 H, NCH₂CO), 5. 90 (br s, 1 H, CONH), 5. 99 (br s, 1 H, CONH), 6. 29 (s, I H, CH); MS (FAB) *m*/*z* 168 (MH⁺).

### Preparative Example 16

### Preparation of N-(2-cyano-2-(3-fluoropropyl)vinyl)-N-methylglycine amide (a compound of the formula (19) wherein R¹ = CH₃, R² = H, R³ = CH₂CH₂CH₂F)

The titled compound was prepared as described in Preparative Example 14 by using *N*-(2-cyano-2-(3-fluoropropyl)vinyl)-*N*-methylglycine methyl ester in place of *N*-(2-cyano-2-*n*-propylvinyl)-*N*-methylglycine ethyl ester.
yield: 70%
mp 83. 5-85°C (CH₂Cl₂/EtOAc/hexanes);
IR (neat) 3346, 3173 (NH), 2183 (CN), 1653, 1633 (C=O) cm⁻¹;
¹H NMR (CDCl₃/TMS) δ 1. 89-1. 99 (m, 2 H, CH₂C*H*₂CH₂F), 2. 23 (t, *J* = 7. 8 Hz, 2 H, C*H*₂CH₂CH₂F), 3. 17 (s, 3 H, NCH₃), 3. 94 (s, 2 H, NCH₂CO), 4. 49 (dt, *J* = 47. 4 Hz, 5. 7 Hz, 2 H, CH₂CH₂C*H*₂F), 5. 94 (br s, 2 H, CONH₂), 6. 35 (s, 1 H, CH); MS (FAB) *m*/*z* 180 (MH⁺ - H₂O).

### Preparative Example 17

### Preparation of N-(2-cyano-2-(cyclopropylmethyl)vinyl)-N-methylglycine amide (a compound of the formula (19) wherein R¹ = CH₃, R² = H, R³ = cyclopropylmethyl)

The titled compound was prepared as described in Preparative Example 14 by using *N*-(2-cyano-2-(cyclopropylmethyl)vinyl)-*N*-methylglycine methyl ester in place of *N*-(2-cyano-2-*n*-propylvinyl)-*N*-methylglycine ethyl ester.
yield: 85%
mp 100. 5-102°C (CH₂Cl₂/ether);
IR (neat) 3352, 3173 (NH), 2179 (CN), 1656, 1639 (C=O) cm⁻¹;
¹H NMR (CDCl₃/TMS) δ 0. 13-0. 19 (m, 2 H, c-C₃H₅), 0. 48-0. 56 (m, 2 H, c-C₃H₅), 0. 79-0. 93 (m, 1 H, c-C₃H₅), 2. 00 (d, *J =* 6. 9 Hz, 2 H, CHC*H*₂), 3. 18 (s, 3 H, NCH₃), 3. 93 (s, 2 H, NCH₂CO), 5. 81 (br s, 1 H, CONH), 5. 98 (br s, 1 H, CONH), 6. 32 (s, 1 H, CH); MS (FAB) m/z 194 (MH⁺).

### Preparative Example 18

### Preparation of N-(2-cyano-2-ethylvinyl)-N-ethylglycine amide (a compound of the formula (19) wherein R¹ = CH₂CH₃, R² = H, R³ = CH₂CH₃)

The titled compound was prepared as described in Preparative Example 14 by using *N*-(2-cyano-2-ethylvinyl)-*N*-ethylglycine methyl ester in place of *N*-(2-cyano-2-*n*-propylvinyl)-*N*-methylglycine ethyl ester.
yield: 77%
mp 89-90°C (ether/hexanes);
IR (neat) 3386, 3186 (NH), 2178 (CN), 1664, 1634 (C=O) cm⁻¹;
¹H NMR (CDCl₃/TMS) δ 1. 11 (t, *J =* 7. 5 Hz, 3 H, CH₂CH₃), 1. 22 (t, *J =* 7. 2 Hz, 3 H, NCH₂C*H*₃), 2. 11 (q, *J* = 7. 5 Hz, 2 H, C*H*₂CH₃), 3. 41 (q, *J*=7. 2 Hz, 2 H, N C*H*₂CH₃), 3. 98 (s, 2 H, NCH₂CO), 5. 77 (br s, 1 H, CONH), 6. 01 (br s, 1 H, CONH), 6. 30 (s, 1 H, CH); MS (FAB) *m*/*z* 182 (MH⁺).

### Preparative Example 19

### Preparation of N-(2-cyano-2-n-propylvinyl)-N-ethylglycine amide (a compound of the formula (19) wherein R¹ = CH₂CH₃, R² = H, R³ = CH₂CH₂CH₃)

The titled compound was prepared as described in Preparative Example 14 by using *N*-(2-cyano-2-*n*-propylvinyl)-*N*-ethylglycine methyl ester in place of *N*-(2-cyano-2-*n*-propylvinyl)*-N*-methylglycine ethyl ester.
yield: 90%
mp 71. 5-73 °C(ether/hexanes);
IR (neat) 3392, 3188 (NH₂), 2174 (CN), 1675, 1633 (C=O) cm⁻¹;
¹H NMR (CDCl₃/TMS) δ 0. 91 (t, *J=* 7. 5 Hz, 3 H, CH₂CH₂C*H*₃), 1. 22 (t, *J* = 7. 2 Hz, 3 H, NCH₂C*H*₃), 1. 46-1. 58 (m, 2 H, CH₂C*H*₂CH₃), 2. 05 (t, *J* = 7. 5 Hz, 2 H, C*H*₂CH₂CH₃), 3. 42 (q, *J =* 7. 2 Hz, 2 H, NC*H*₂CH₃), 3. 99 (s, 2 H, NCH₂CO), 5. 64 (br s, 1 H, CONH), 5. 97 (br s, 1 H, CONH), 6. 28 (s, I H, CH); MS (FAB) *m*/*z* 196 (MH⁺).

### Preparative Example 20

### Preparation of N-(2-cyano-2-(3-fluoropropyl)vinyl)-N-ethylglycine amide (a compound of the formula (19) wherein R¹ = CH₂CH₃, R² = H, R³ = CH₂CH₂CH₂F)

The titled compound was prepared as described in Preparative Example 14 by using *N*-(2-cyano-2-(3-fluoropropyl)vinyl)-*N*-ethylglycine methyl ester in place of *N*-(2-cyano-2-*n*-propylvinyl)-*N*-methylglycine ethyl ester.
yield: 86%
mp 70-71°C (ether/hexanes);
IR (neat) 3324, 3198 (NH), 2177 (CN), 1684, 1617 (C=O) cm⁻¹;
¹H NMR (CDCl₃/TMS) δ 1. 23 (t, *J=* 7. 2 Hz, 3 H, NCH₂C*H*₃), 1. 79-1. 97 (m, 2 H, CH₂C*H*₂CH₂F), 2. 24 *(t, J =* 7. 2 Hz, 2 H, C*H*₂CH₂CH₂F), 3. 41 (q, *J =* 7. 2 Hz, 2 H, NC*H*₂CH₃), 4. 01 (s, 2 H, NCH₂CO), 4. 48 (dt, *J* = 47. 1 Hz, 5. 7 Hz, 2 H, CH₂CH₂C*H*₂F), 5. 93 (br s, 2 H, CONH₂), 6. 35 (s, 1 H, CH); MS (FAB) *m*/*z* 214 (MH⁺).

### Prenarative Example 21

### Preparation of 4-amino-1-methyl-3-n-propylpyrrole-5-carboxamide (a compound of the formula (15) wherein R' = CH₃, R² = H, R³ = CH₂CH₂CH₃)

A solution of *N*-(2-cyano-2-*n*-propylvinyl)-*N*-methylglycine amide (8. 26 g, 45. 57 mmol) in freshly prepared NaOEt in EtOH (0. 5 M, 190 mL, 95. 71 mmol) was heated at 60 °C for 1. 5 h, cooled to room temperature and then the mixture was quenched with acetic acid (5. 4 mL). Resulting mixture was diluted with water (50 mL) and was extracted with CH₂Cl₂ (100 mL × 3). Combined organic layer was dried (Na₂SO₄), filtered and the filtrate was evaporated to dryness under reduced pressure. The crude product was dissolved in a minimum amount of CH₂Cl₂, and then solidified by adding diethyl ether and hexanes to afford the titled compound (7. 64 g, 92%) as a pale violet solid.
mp 123-124°C;
IR (neat) 3353, 3181 (NH₂), 1647 (C=O) cm⁻¹;
¹H NMR (CDCl₃/TMS) δ 0. 96 (t, *J* = 7.5 Hz, 3 H, CH₂CH₂C*H*₃), 1. 49-1. 61(m, 2 H, CH₂C*H*₂CH₃), 2. 31 (t, *J* = 7. 5 Hz, 2 H, C*H*₂CH₂CH₃), 3. 17 (br s, 2 H, NH₂), 3. 83 (s, 3 H, NCH₃), 6. 34 (br s, 2 H, CONH₂), 6. 37(s, 1 H, H-2); MS (FAB) *m*/*z* 182 (MH⁺).

### Preparative Example 22

### Preparation of 4-amino-3-ethyl-1-methylpyrrole-5-carboxamide (a compound of the formula (15) wherein R¹ = CH₃, R² = H, R³ = CH₂CH₃)

The titled compound was prepared as described in Preparative Example 21 by using *N*-(2-cyano-2-ethylvinyl)-*N*-methylglycine amide in place of *N*-(2-cyano-2-*n*-propylvinyl)-*N*-methylglycine amide.
yield: 79%
mp 113. 5-115°C (MeOH/ EtOAc/hexanes);
IR (neat) 3373, 3180 (NH), 1653, 1611 (C=O) cm⁻¹;
¹H NMR (CDCl₃/TMS) δ 1. 18 (t, *J* = 7. 5 Hz, 3 H, CH₂C*H*₃), 2. 36 (q, *J* = 7. 5 Hz, 2 H, C*H*₂CH₃), 3. 18 (br s, 2 H, NH₂), 3. 83 (s, 3 H, NCH₃), 6. 35 (br s, 2 H, CONH₂), 6. 38 (s, 1 H, H-2); MS (FAB) *m*/*z* 168 (MH⁺).

### Prenarative Example 23

### Preparation of 4-amino-3-(3-fluoropropyl)-1-methylpyrrole-5-carboxamide (a compound of the formula (15) wherein R' = CH₃, R² = H, R³ = CH₂CH₂CH₂F)

The titled compound was prepared as described in Preparative Example 21 by using *N*-(2-cyano-2-(3-fluoropropyl)vinyl)-*N*-methylglycine amide in place of *N*-(2-cyano-2-*n*-propylvinyl)-*N*-methylglycine amide.
yield: 66%
mp 124-125. 5°C (CH₂Cl₂/EtOAc/ether);
IR (neat) 3347, 3175 (NH), 1642, 1601 (C=O) cm⁻¹;
¹H NMR (CDCl₃/TMS) δ 1. 91-2. 00 (m, 2 H, CH₂C*H*₂CH₂F), 2. 50 (t, *J* = 7. 5 Hz, 2 H, C*H*₂CH₂CH₂F), 3. 16 (br s, 2 H, NH₂), 3. 84 (s, 3 H, NCH₃), 4. 47 (dt, *J* = 47. 1 Hz, 5. 7 Hz, 2 H, CH₂CH₂C*H*₂F), 6. 34 (br s, 2 H, CONH₂), 6. 40 (s, 1 H, H-2); MS (FAB) *m*/*z* 200 (MH⁺).

### Preparative Example 24

### Preparation of 4-amino-3-cyclopropylmethyl-1-methylpyrrole-5-carboxamide (a compound of the formula (15) wherein R' = CH₃, R² = H, R³ = cyclopropylmethyl)

The titled compound was prepared as described in Preparative Example 21 by using *N*-(2-cyano-2-(cyclopropylmethyl)vinyl)*-N*-methylglycine amide in place of *N*-(2-cyano-2-*n*-propylvinyl)-*N*-methylglycine amide.
yield: 73%
mp 135-137°C (CH₂Cl₂/ether/hexanes);
IR (neat) 3348, 3150 (NH), 1655 (C=O) cm⁻¹;
¹H NMR (CDCl₃/TMS) δ 0. 11-0. 16 (m, 2 H, c-C₃H₅), 0. 48-0. 54 (m, 2 H, c-C₃H₅), 0. 83-0. 97 (m, 1 H, c-C₃H₅), 2. 30 (d, *J* = 6. 3 Hz, 2 H, CHC*H*₂), 3. 20 (br s, 2 H, NH₂), 3. 84 (s, 3 H, NCH₃), 6. 34 (br s, 2 H, CONH₂), 6. 47 (s, 1 H, H-2); MS (FAB) *m*/*z* 193 (M⁺).

### Preparative Example 25

### Preparation of 4-amino-1-ethyl-3-ethylpyrrole-5-carboxamide (a compound of the formula (15) wherein R¹ = CH₂CH₃, R² = H, R³ = CH₂CH₃)

The titled compound was prepared as described in Preparative Example 21 by using *N*-(2-cyano-2-ethylvinyl)-*N*-ethylglycine amide in place of *N*-(2-cyano-2-*n*-propylvinyl)-*N*-methylglycine amide.
yield: 89%
mp 94°C dec (CH₂Cl₂/ether/hexanes);
IR (neat) 3367, 3178 (NH), 1608 (C=O) cm⁻¹;
¹H NMR (CDCl₃/TMS) δ 1. 19 (t, *J =* 7. 5 Hz, 3 H, CH₂C*H*₃), 1. 34 (t, *J* = 7. 2 Hz, 3 H, NCH₂C*H*₃), 2. 37 (q, *J=* 7. 5 Hz, 2 H, C*H*₂CH₃), 3. 11 (br s, 2 H, NH₂), 4. 28 (q, *J* = 7. 2 Hz, 2 H, NC*H*₂CH₃), 6. 38 (br s, 2 H, CONH₂), 6. 47 (s, 1 H, H-2); MS (FAB) *m*/*z* 182 (MH⁺).

### Preparative Example 26

### Preparation of 4-amino-1-ethyl-3-n-propylpyrrole-5-carboxamide (a compound of the formula (15) wherein R¹ = CH₂CH₃, R² = H, R³ = CH₂CH₂CH₃)

The titled compound was prepared as described in Preparative Example 21 by using *N*-(2-cyano-2-n-propylvinyl)-*N*-ethylglycine amide in place of *N*-(2-cyano-2-*n*-propylvinyl)-*N*-methylglycine amide.
yield: 89%
mp 108.5-110°C (ether/hexanes);
IR (neat) 3366, 3183 (NH), 1628 (C=O) cm⁻¹;
¹H NMR (CDCl₃/TMS) δ 0. 96 (t, *J* = 7. 5 Hz, 3 H, CH₂CH₂C*H*₃), 1. 33 (t, *J =* 7. 2 Hz, 3 H, NCH₂C*H*₃), 1. 49-1. 62 (m, 2H, CH₂C*H*₂CH₃), 2. 32 (t, *J* = 7. 5 Hz, 2 H, C*H*₂CH₂CH₃), 3. 10 (br s, 2 H, NH₂), 4.28 (q, *J* = 7. 2 Hz, 2 H, NC*H*₂CH₃), 6. 38 (br s, 2 H, CONH₂), 6. 46 (s, 1 H, H-2); MS (FAB) *m*/*z* 196 (MH⁺).

### Preparative Example 27

### Preparation of 4-amino-1-ethyl-3-(3-fluoropropyl)pyrrole-5-carboxamide (a compound of the formula (15) wherein R' = CH₂CH₃, R² = H, R³ = CH₂CH₂CH₂F)

The titled compound was prepared as described in Preparative Example 21 by using *N*-(2-cyano-2-(3-fluoropropyl)vinyl)-*N*-ethylglycine amide in place of *N*-(2-cyano-2-*n*-propylvinyl)-*N*-methylglycine amide.
yield: 90%
mp 94. 5-96°C (CH₂Cl₂/ether);
IR (neat) 3346, 3179 (NH), 1629 (C=O) cm⁻¹;
¹H NMR (CDCl₃/TMS) δ 1. 34 (t, *J* = 7. 2 Hz, 3 H, NCH₂C*H*₃), 1. 82-2. 00 (m, 2 H, CH₂C*H*₂CH₂F), 2. 51 (t, *J* = 7. 5 Hz, 2 H, C*H*₂CH₂CH₂F), 3. 14 (br s, 2 H, NH₂), 4. 28 (q, *J=* 7. 2 Hz, 2 H, NC*H*₂CH₃), 4. 47 (dt, *J* = 47. 4 Hz, 5. 7 Hz, 2 H, CH₂CH₂C*H*₂F), 6. 38 (br s, 2 H, CONH₂), 6. 48 (s, 1 H, H-2); MS (FAB) *m*/*z* 214 (MH⁺).

### Preparative Example 28

### Preparation of 4-(2-ethoxybenzamido)-1-methyl-3-n-propylpyrrole-5-carboxamide (a compound of the formula (12) wherein R' = CH₃, R² = H, R³ = CH₂CH₂CH₃, R⁴ = CH₂CH₃)

To a cooled mixture of 4-amino-1-methyl-3-*n*-propylpyrrole-5-carboxamide (3. 00 g, 16. 55 mmol), DMAP (101 mg, 0. 83 mmol) and triethylamine (4. 6 mL, 33. 00 mmol) in CH₂Cl₂ (50 mL) in an ice bath was added dropwise 2-ethoxybenzoyl chloride in CH₂Cl₂ (30 mL) over a period of 10 minutes, and the reaction mixture was stirred in an ice bath for 1 h. The reaction was quenched with 1 N HCl solution (100 mL), and was extracted with 3% MeOH in CHCl₃ (3 × 100 mL). The combined organic layer was washed with saturated aqueous NaHCO₃ (50 mL), dried (MgSO₄), and filtered. The filtrate was evaporated to dryness in vacuo to afford an off-white solid, and the crude product was purified by MPLC on silica gel (2% MeOH in CHCl₃) to afford the titled compound (5. 01 g, 92%) as a white solid. Analytically pure compound was obtained by crystallization from ethyl acetate/hexanes.
mp 166-167 °C;
IR (neat) 3334, 3149 (NH), 1668, 1641 (C=O) cm⁻¹;
¹H NMR (CDCl₃/TMS) δ 0. 92 (t, *J* = 7. 2 Hz, 3 H, CH₂CH₂C*H*₃), 1. 47-1. 59 (m, 2 H, CH₂C*H*₂CH₃), 1. 52 (t, *J* = 6. 6 Hz, 3 H, OCH₂C*H*₃), 2. 34 (t, *J* = 7. 5 Hz, 2 H, C*H*₂CH₂CH₃), 3. 85 (s, 3 H, NCH₃), 4. 29 (q, *J*= 6. 6 Hz, 2 H, OC*H*₂CH₃), 6. 52 (s, I H, H-2), 7. 04 (d, *J*= 8. 4 Hz, 1 H, H-3'), 7. 09-7. 15 (m, 1 H, H-5'), 7. 51 (ddd, *J*= 8. 4 Hz, 7. 2 Hz, 1. 8 Hz, I H, H-4'), 8. 29 (dd, *J* = 8. 1 Hz, 1. 8 Hz, 1 H, H-6'), 9. 37 (br s, 1 H, NH); MS (FAB) *m*/*z* 330 (MH⁺).

### Preparative Example 29

### Preparation of 1-methyl-4-(2-n-propoxybenzamido)-3-n-propylpyrrole-5-carboxamide (a compound of the formula (12) wherein R¹ = CH₃, R² = H, R³ = CH₂CH₂CH₃, R⁴ = CH₂CH₂CH₃)

The titled compound was prepared as described in Preparative Example 28 by using 2-*n*-propoxybenzoyl chloride in place of 2-ethoxybenzoyl chloride.
yield: 91%
mp 136-137 °C (CHCl₃/Et₂O/hexanes);
IR (neat) 3338, 3159 (NH), 1646 (C=O) cm⁻¹;
¹H NMR (CDCl₃/TMS) δ 0. 91 (t, *J* = 7. 2 Hz, 3 H, CH₂CH₂C*H*₃), 1. 06 (t, *J =* 7. 5 Hz, 3 H, OCH₂CH₂C*H*₃), 1. 47-1. 57 (m, 2 H, CH₂C*H*₂CH₃), 1. 85-1. 95 (m, 2 H, OCH₂C*H*₂CH₃), 2. 33 (t, *J* = 7. 5 Hz, 2 H, C*H*₂CH₂CH₃), 3. 85 (s, 3 H, NCH₃), 4. 18 (t, *J* = 6. 6 Hz, 2 H, OC*H*₂CH₂CH₃), 6. 52 (s, 1 H, H-2), 7. 04 (d, *J*= 8. 4 Hz, 1 H, H-3'), 7. 12 (td, *J=* 8. 1 Hz, 0. 9 Hz, 1 H, H-5'), 7. 48-7. 54 (m, 1 H, H-4'), 8. 29 (dd, *J=* 8. 1 Hz, 2. 1 Hz, 1 H, H-6'), 9. 36 (br s, 1 H, NH); MS (FAB) *m*/*z* 344 (MH⁺).

### Preparative Example 30

### Preparation of 4-(2-ethoxy-5-(4-(3-fluoropropyl)piperazin-1-ylsulfonyl)benzamido)-3-ethyl-1-methyl pyrrole-5-carboxamide (a compound of the formula (25) wherein R' = CH₃, R² = H, R³ = CH₂CH₃, R⁴ = CH₂CH₃, R¹⁶ = 4-(3-fluoropropyl)piperazin-1-ylsulfonyl)

To a mixture of 4-amino-3-ethyl-1-methylpyrrole-5-carboxamide (125 mg, 0.74 mmol), 2-ethoxy-5-(4-(3-fluoropropyl)piperazin-1-ylsulfonyl)benzoic acid (279 mg, 0.74 mmol), HOBT (151 mg, 1.12 mmol), and DMAP (18 mg, 0. 15 mmol) in anhydrous pyridine (5 mL) at room temperature under nitrogen atmosphere was slowly added EDC (214 mg, 1.12 mmol) over a period of 5 minutes, and the reaction mixture was stirred for 2 h. Pyridine was removed under vacuum and the resulting residue was diluted with brine (100 mL). Extraction with 5% MeOH in CHCl₃ (2 x 100 mL) was performed and the combined organic layer was dried (MgSO₄), and filtered. The filtrate was evaporated to dryness in vacuo to afford a brown solid, and the crude product was purified by MPLC on silica gel (gradient elution: 2% MeOH in CHCl₃ followed by 3% MeOH in CHCl₃) to afford the titled compound (310 mg, 80%) as a yellowish solid. Analytically pure compound was obtained by crystallization from CH₂Cl₂/MeOH/hexanes.
mp 182. 5-183 °C;
IR (neat) 3353 (NH), 1648 (C=O), 1170 (SO₂) cm⁻¹;
¹H NMR (CDCl₃/TMS) δ 1. 16 (t, *J=* 7. 5 Hz, 3 H, CH₂C*H*₃), 1. 58 (t, *J* = 7. 2 Hz, 3
H, OCH₂C*H*₃), 1. 72-1. 90 (m, 2 H, C*H*₂CH₂F), 2. 39 (q, *J=* 7. 5 Hz, 2 H, C*H*₂CH₃), 2. 47 (t, *J=* 7. 2 Hz, 2 H, NC*H*₂CH₂), 2. 53 (dd, *J =* 4. 8 Hz, 4. 5 Hz, 4 H, 2 NCH₂), 3. 05 (dd, *J =* 4. 8 Hz, 4. 5 Hz, 4 H, 2 SO₂NCH₂), 3. 85 (s, 3 H, NCH₃), 4. 37 (q, *J =* 7. 2 Hz, 2 H, OC*H*₂CH₃), 4. 42 (dt, *J* = 47. 4 Hz, 6. 0 Hz, 2 H, CH₂C*H*₂F), 6. 54 (s, 1 H, H-2), 7. 16 (d, *J*= 8. 7 Hz, 1 H, H-3'), 7. 89 (dd, *J*= 8. 7 Hz, 2. 7 Hz, 1 H, H-4'), 8. 64 (d, *J=* 2. 7 Hz, 1 H, H-6'), 9. 19 (br s, 1 H, NH); MS (FAB) *m*/*z* 524 (MH⁺).

### Preparative Example 31

### Preparation of 4-(2-ethoxy-5-(4-(3-fluoropropyl)piperazin-1-ylsulfonyl)benzamido)-3-(3-fluoropropy 1)-1-methylpyrrole-5-carboxamide (a compound of the formula (25) wherein R¹ = CH₃, R² = H, R³ = CH₂CH₂CH₂F, R⁴ = CH₂CH₃, R¹⁶ = 4-(3-fluoropropyl)piperazin-1-ylsulfonyl)

The titled compound was prepared as described in Preparative Example 30 by using 4-amino-3-(3-fluoropropyl)-1-methylpyrrole-5-carboxamide in place of 4-amino-3-ethyl-1-methylpyrrole-5-carboxamide.
yield: 86%
mp 169-171 °C (CH₂Cl₂/hexanes);
IR (neat) 3352 (NH), 1647 (C=O), 1170 (SO₂) cm⁻¹;
¹H NMR (CDCl₃/TMS) δ 1. 57 (t, *J* = 7. 2 Hz, 3 H, OCH₂C*H*₃), 1. 72-1. 98 (m, 4 H, 2 C*H*₂CH₂F), 2. 47 (t, *J* = 7. 5 Hz, 2 H, NC*H*₂CH₂), 2. 46-2. 60 (m, 6 H, C*H*₂CH₂CH₂F and 2 NCH₂), 3. 06 (br s, 4 H, 2 SO₂NCH₂), 3. 86 (s, 3 H, NCH₃), 4. 37 (q, *J=* 7. 2 Hz, 2 H, OC*H*₂CH₃), 4. 42 (dt, *J =* 47. 4 Hz, 5. 1 Hz, 4 H, 2 CH₂C*H*₂F), 6. 56 (s, 1 H, H-2), 7. 16 (d, *J=* 9. 0 Hz, 1 H, H-3'), 7. 89 (dd, *J=* 9. 0 Hz, 2. 4 Hz, 1 H, H-4'), 8. 62 (d, *J* = 2. 4 Hz, 1 H, H-6'), 9. 20 (br s, 1 H, NH); MS (FAB) *m*/*z* 556 (MH⁺).

### Preparative Example 32

### Preparation of 3-cyclopropylmethyl-4-(2-ethoxy-5-(4-(3-fluoropropyl)piperazin-1-ylsulfonyl)benzam ido)-1-methylpyrrole-5-carboxamide (a compound of the formula (25) wherein R¹ = CH₃, R² = H, R³ = cyclopropylmethyl, R⁴ = CH₂CH₃, R¹⁶ = 4-(3-fluoropropyl)piperazin-1-ylsulfonyl)

The titled compound was prepared as described in Preparative Example 30 by using 4-amino-3-cyclopropylmethyl-1-methylpyrrole-5-carboxamide in place of 4-amino-3-ethyl-1-methylpyrrole-5-carboxamide.
yield: 91%
mp 198-199 °C (CH₂Cl₂/ether);
IR (neat) 3359, 3288 (NH), 1642 (C=O), 1170 (SO₂) cm⁻¹;
¹H NMR (CDCl₃/TMS) δ 0. 09-0. 14 (m, 2 H, c-C₃H₅), 0. 45-0. 51 (m, 2 H, c-C₃H₅), 0. 83-0. 97 (m, 1 H, c-C₃H₅), 1. 57 (t, *J =* 6. 9 Hz, 3 H, OCH₂C*H*₃), 1. 73-1. 90 (m, 2 H, C*H*₂CH₂F), 2. 29 (d, *J* = 6. 9 Hz, 2 H, CHC*H*₂), 2. 47 (t, *J* = 7. 5 Hz, 2 H, NC*H*₂CH₂), 2. 54 (dd, *J =* 4. 8 Hz, 4. 5 Hz, 4 H, 2 NCH₂), 3. 07 (dd, *J =* 4. 8 Hz, 4. 5 Hz, 4 H, 2 SO₂NCH₂), 3. 87 (s, 3 H, NCH₃), 4. 37 (q, *J* = 6. 9 Hz, 2 H, OC*H*₂CH₃), 4. 44 (dt, *J* = 47. 4 Hz, 6. 0 Hz, 2 H, CH₂C*H*₂F), 6. 66 (s, 1 H, H-2), 7. 16 (d, *J=* 8. 7 Hz, 1 H, H-3'), 7. 90 (dd, *J* = 8. 7 Hz, 2. 4 Hz, 1 H, H-4'), 8. 64 (d, *J =* 2. 4 Hz, 1 H, H-6'), 9. 18 (br s, 1 H, NH); MS (FAB) *m*/*z* 550 (MH⁺).

### Preparative Example 33

### Preparation of 4-(2-ethoxy-5-(4-(3-fluoropropyl)piperazin-1-ylsulfonyl)benzamido)-1-ethyl-3-ethylp yrrole-5-carboxamide (a compound of the formula (25) wherein R¹ = CH₂CH₃, R² = H, R³ = CH₂CH₃, R⁴ = CH₂CH₃, R¹⁶ = 4-(3-fluoropropyl)piperazin-1-ylsulfonyl)

The titled compound was prepared as described in Preparative Example 30 by using 4-amino-1-ethyl-3-ethylpyrrole-5-carboxamide and 2-ethoxy-5-(4-ethylpiperazin-1-ylsulfonyl)benzoic acid in place of 4-amino-3-ethyl-1-methylpyrrole-5-carboxamide and 2-ethoxy-5-(4-(3-fluoropropyl)piperazin-1-ylsulfonyl)benzoic acid.
yield: 68%
mp 203-204 °C (CH₂Cl₂/ether);
IR (neat) 3346 (NH), 1646 (C=O), 1173 (SO₂) cm⁻¹;
¹H NMR (CDCl₃/TMS) δ 1. 17 (t, *J=* 7. 5 Hz, 3 H, CH₂C*H*₃), 1. 41 (t, J = 7. 2 Hz, 3
H, NCH₂C*H*₃), 1. 58 (t, *J* = 6. 9 Hz, 3 H, OCH₂C*H*₃), 1. 72-1. 89 (m, 2 H, C*H*₂CH₂F), 2. 39 (q, *J =* 7. 5 Hz, 2 H, C*H*₂CH₃), 2. 47 (t, *J =* 7. 2 Hz, 2 H, NC*H*₂CH₂CH₂F), 2. 53 (dd, *J =* 4. 8 Hz, 4. 5 Hz, 4 H, 2 NCH₂), 3. 06 (br dd, *J =* 4. 8 Hz, 4. 5 Hz, 4 H, 2 SO₂NCH₂), 4. 27 (q, *J* = 7. 2 Hz, 2 H, NC*H*₂CH₃), 4. 37 (q, *J* = 6. 9 Hz, 2 H, OC*H*₂CH₃), 4. 44 (dt, *J* = 47. 1 Hz, 6. 0 Hz, 2 H, CH₂C*H*₂F), 6. 61 (s, 1 H, H-2), 7. 15 (d, *J =* 8. 7 Hz, 1 H, H-3'), 7. 89 (dd, *J =* 8. 7 Hz, 2. 7 Hz, 1 H, H-4'), 8. 64 (d, *J* = 2. 7 Hz, 1 H, H-6'), 9. 18 (br s, 1 H, NH); MS (FAB) *m*/*z* 538 (MH⁺).

### Preparative Example 34

### Preparation of 4-(2-ethoxy-5-(4-ethylpiperazin-1-ylsulfonyl)benzamido)-1-ethyl-3-n-propylpyrrole-5 -carboxamide (a compound of the formula (25) wherein R¹ = CH₂CH₃, R² = H, R³ = CH₂CH₂CH₃, R⁴ = CH₂CH₃, R¹⁶ = 4-ethylpiperazin-1-ylsulfonyl)

The titled compound was prepared as described in Preparative Example 30 by using 4-amino-1-ethyl-3-*n*-propylpyrrole-5-carboxamide and 2-ethoxy-5-(4-ethylpiperazin-1-ylsulfonyl)benzoic acid in place of 4-amino-3-ethyl-1-methylpyrrole-5-carboxamide and 2-ethoxy-5-(4-(3-fluoropropyl)piperazin-1-ylsulfonyl)benzoic acid.
yield: 87%
mp 157. 5-158. 5 °C (CH₂Cl₂/EtOAc/ether/hexanes);
IR (neat) 3366 (NH), 1644 (C=O), 1172 (SO₂) cm⁻¹;
¹H NMR (CDCl₃/TMS) 8 0. 93 (t, *J=* 7. 5 Hz, 3 H, CH₂CH₂C*H*₃), 1. 03 (t, *J=* 7. 2 Hz, 3 H, CH₂NCH₂C*H*₃), 1. 41 (t, *J=* 7. 2 Hz, 3 H, NCH₂C*H*₃), 1. 57 (t, *J=* 6. 9 Hz, 3 H, OCH₂C*H*₃), 1. 48-1. 61 (m, 2 H, CH₂C*H*₂CH₃), 2. 34 (t, *J* = 7. 5 Hz, 2 H, C*H*₂ CH₂CH₃), 2. 41 (q, *J* = 7. 2 Hz, 2 H, CH₂NC*H*₂CH₃), 2. 53 (dd, *J =* 5. 1 Hz, 4. 8 Hz, 4 H, 2 NCH₂), 3. 07 (br dd, *J=* 5. 1 Hz, 4. 8 Hz, 4 H, 2 SO₂NCH₂), 4. 27 (q, *J=* 7. 2 Hz, 2 H, NC*H*₂CH₃), 4. 37 (q, *J* = 6.9 Hz, 2 H, OC*H*₂CH₃), 6. 60 (s, 1 H, H-2), 7. 15 (d, *J =* 8. 7 Hz, 1 H, H-3'), 7. 90 (dd, *J* = 8. 7 Hz, 2.7 Hz, I H, H-4'), 8. 66 (d, *J =* 2.7 Hz, 1 H, H-6'), 9. 18 (br s, I H, NH); MS (FAB) *m*/*z* 520 (MH⁺).

### Preparative Example 35

### Preparation of 4-(2-ethoxy-5-(4-isopropylpiperazin-1-ylsulfonyl)benzamido)-1-ethyl-3-n-propylpyrro le-5-carboxamide (a compound of the formula (25) wherein R¹ = CH₂CH₃, R² = H, R³ = CH₂CH₂CH₃, R⁴ = CH₂CH₃, R¹⁶ = 4-isopropylpiperazin-1-ylsulfonyl)

The titled compound was prepared as described in Preparative Example 30 by using 4-amino-1-ethyl-3-*n*-propylpyrrole-5-carboxamide and 2-ethoxy-5-(4-isopropylpiperazin-1-ylsulfonyl)benzoic acid in place of 4-amino-3-ethyl-1-methylpyrrole-5-carboxamide and 2-ethoxy-5-(4-(3-fluoropropyl)piperazin-1-ylsulfonyl)benzoic acid
yield: 64%
mp 174-175°C (CH₂Cl₂/EtOAc/ether);
IR (neat) 3313 (NH), 1646 (C=O), 1169 (SO₂) cm⁻¹;
¹H NMR (CDCl₃/TMS) δ 0. 93 (t, *J* = 7. 5 Hz, 3 H, CH₂CH₂C*H*₃), 1. 00 (t, *J =* 6. 6 Hz, 6 H, CH(C*H*₃)₂), 1. 41 (t, *J* = 7. 2 Hz, 3 H, NCH₂C*H*₃), 1. 48-1. 61 (m, 2 H, CH₂C*H*₂CH₃), 1. 57 (t, *J* = 6. 9 Hz, 3 H, OCH₂C*H*₃), 2. 34 (t, *J* = 7. 5 Hz, 2 H, C*H*₂ CH₂CH₃), 2. 60 (dd, *J* = 4. 8 Hz, 4. 5 Hz, 4 H, 2 NCH₂), 2. 63-2. 71 (m, 1 H, C*H*(CH₃)₂), 3. 05 (br dd, *J=* 4. 8 Hz, 4. 5 Hz, 4 H, 2 SO₂NCH₂), 4. 26 (q, *J* = 7. 2 Hz, 2 H, NC*H*₂CH₃), 4. 36 (q, *J*= 6. 9 Hz, 2 H, OC*H*₂CH₃), 6. 60 (s, I H, H-2), 7. 14 (d, *J=* 8. 7 Hz, 1 H, H-3'), 7. 89 (dd, *J=* 8. 7 Hz, 2. 4 Hz, I H, H-4'), 8. 65 (d, *J =* 2. 4 Hz, 1 H, H-6'), 9. 17 (br s, 1 H, NH); MS (FAB) *m*/*z* 534 (MH⁺).

### Preparative Example 36

### Preparation of 4-(2-ethoxy-5-(4-(3-fluoropropyl)piperazin-1-ylsulfonyl)benzamido)-1-ethyl-3-n-prop ylpyrrole-5-carboxamide (a compound of the formula (25) wherein R¹ = CH₂CH₃, R² = H, R³ = CH₂CH₂CH₃, R⁴ = CH₂CH₃, R¹⁶ = 4-(3-fluoropropyl)piperazin-1-ylsulfonyl)

The titled compound was prepared as described in Preparative Example 30 by using 4-amino-1-ethyl-3-*n*-propylpyrrole-5-carboxamide in place of 4-amino-3-ethyl-1-methylpyrrole-5-carboxamide.
yield: 91%
mp 176-176. 5°C (CH₂Cl₂/ether);
IR (neat) 3358 (NH), 1649 (C=O), 1172 (SO₂) cm⁻¹;
¹H NMR (CDCl₃/TMS) δ 0. 93 (t, *J*= 7.5 Hz, 3 H, CH₂CH₂C*H*₃), 1.41 (t, *J*= 7. 2 Hz, 3 H, NCH₂C*H*₃), 1. 48-1. 61 (m, 2 H, CH₂C*H*₂CH₃), 1. 58 (t, *J =* 6. 9 Hz, 3 H, OCH₂C*H*₃), 1. 72-1. 89 (m, 2 H, NCH₂C*H*₂CH₂F), 2. 34 (q, *J* = 7. 5 Hz, 2 H, C*H*₂ CH₂CH₃), 2. 47 (t, *J=* 7.2 Hz, 2 H, NC*H*₂CH₂CH₂F), 2. 53 (dd, *J=* 4. 8 Hz, 4.5 Hz, 4 H, 2 NCH₂), 3. 07 (br dd, *J =* 4. 8 Hz, 4. 5 Hz, 4 H, 2 SO₂NCH₂), 4. 27 (q, *J =* 7. 2 Hz, 2 H, NC*H*₂CH₃), 4. 37 (q, *J =* 6. 9 Hz, 2 H, OC*H*₂CH₃), 4. 44 (dt, *J=* 47. 1 Hz, 6. 0 Hz, 2 H, CH₂C*H*₂F), 6. 60 (s, 1 H, H-2), 7. 16 (d, *J* = 8. 7 Hz, 1 H, H-3'), 7. 89 (dd, *J =* 8. 7 Hz, 2. 7 Hz, 1 H, H-4'), 8. 65 (d, *J =* 2. 7 Hz, I H, H-6'), 9. 17 (br s, 1 H, NH); MS (FAB) *m*/*z* 552 (MH⁺).

### Preparative Example 37

### Preparation of 4-(2-ethoxy-5-(4-(2-hydroxyethyl)piperazin-1-ylsulfonyl)benzamido)-1-ethyl-3-n-propylpyrrole-5-carboxamide (a compound of the formula (25) wherein R¹ = CH₂CH₃, R² = H, R³ = CH₂CH₂CH₃, R⁴ = CH₂CH₃, R¹⁶ = 4-(2-hydroxyethylpiperazin-1-ylsulfonyl)

The titled compound was prepared as described in Preparative Example 30 by using 4-amino-1-ethyl-3-*n*-propylpyrrole-5-carboxamide and 2-ethoxy-5-(4-(2-hydroxyethyl)piperazin-1-ylsulfonyl)benzoic acid in place of 4-amino-3-ethyl-1-methylpyrrole-5-carboxamide and 2-ethoxy-5-(4-(3-fluoropropyl)piperazin-1-ylsulfonyl)benzoic acid
yield: 92%
mp 184-185°C (CH₂Cl₂/ether);
IR (neat) 3349 (NH), 1650 (C=O), 1170 (SO₂) cm⁻¹;
¹H NMR (CDCl₃/TMS) δ 0. 93 (t, *J* = 7. 5 Hz, 3 H, CH₂CH₂C*H*₃), 1. 41 (t, *J* = 7. 2 Hz, 3 H, NCH₂C*H*₃), 1. 49-1. 61 (m, 2 H, CH₂C*H*₂CH₃), 1. 58 (t, *J* = 6. 9 Hz, 3 H, OCH₂C*H*₃), 2. 34 (t, *J* = 7. 5 Hz, 2 H, C*H*₂ CH₂CH₃), 2. 55 (t, *J* = 5. 4 Hz, 2 H, NC*H*₂CH₂OH), 2. 61 (dd, *J* = 5. 1 Hz, 4. 8 Hz, 4 H, 2 NCH₂), 3. 09 (br dd, *J* = 5. 1 Hz, 4. 8 Hz, 4 H, 2 SO₂NCH₂), 3. 59 (t, *J* = 5. 4 Hz, 2 H, NCH₂C*H*₂OH), 4. 27 (q, *J* = 7. 2 Hz, 2 H, NC*H*₂CH₃), 4. 39 (q, *J* = 6. 9 Hz, 2 H, OC*H*₂CH₃), 6. 61 (s, 1 H, H-2), 7. 18 (d, *J* = 8. 7 Hz, 1 H, H-3'), 7. 91 (dd, *J* = 8. 7 Hz, 2.7 Hz, 1 H, H-4'), 8. 66 (d, *J* = 2. 4 Hz, 1 H, H-6'), 9. 18 (br s, 1 H, NH); MS (FAB) *m*/*z* 536 (MH⁺).

### Preparative Example 38

### Preparation of 4-(2-ethoxy-5-(4-ethylpiperazin-1-ylsulfonyl)benzamido)-1-ethyl-3-(3-fluoropropyl)p yrrole-5-carboxamide (a compound of the formula (25) wherein R' = CH₂CH₃, R² = H, R³ = CH₂CH₂CH₃F, R⁴ = CH₂CH₃, R¹⁶ = 4-ethylpiperazin-1-ylsulfonyl)

The titled compound was prepared as described in Preparative Example 30 by using 4-amino-1-ethyl-3-(3-fluoropropyl)pyrrole-5-carboxamide and 2-ethoxy-5-(4-ethylpiperazin-1-ylsulfonyl)benzoic acid in place of 4-amino-3-ethyl-1-methylpyrrole-5-carboxamide and 2-ethoxy-5-(4-(3-fluoropropyl)piperazin-1-ylsulfonyl)benzoic acid
yield: 87%
mp 151-152°C (CH₂Cl₂/EtOAc/ether/hexanes);
IR (neat) 3354 (NH), 1664 (C=O), 1170 (SO₂) cm⁻¹;
¹H NMR (CDCl₃/TMS) δ 1. 03 (t, *J =* 7. 2 Hz, 3 H, CH₂NCH₂C*H*₃), 1. 41 (t, *J =* 7. 2 Hz, 3 H, CHNCH₂C*H*₃), 1. 57 (t, *J* = 6. 9 Hz, 3 H, OCH₂C*H*₃), 1. 81-1. 98 (m, 2 H, CH₂C*H*₂CH₂F), 2. 41 (q, *J* = 7. 2 Hz, 2 H, CH₂NC*H*₂CH₃), 2. 49-2. 54 (m, 6 H, C*H*₂CH₂CH₂F and 2 NCH₂), 3. 07 (m, 4 H, 2 SO₂NCH₂), 4. 27 (q, *J =* 7. 2 Hz, 2 H, CHNC*H*₂CH₃), 4. 36 (q, *J* = 6. 9 Hz, 2 H, OC*H*₂CH₃), 4. 44(dt, *J =* 47. 4 Hz, 5. 7Hz, 2H, CH₂CH₂C*H*₂F), 6. 63 (s, 1 H, H-2), 7. 15 (d, *J=* 8. 7 Hz, 1 H, H-3'), 7. 90 (dd, *J=* 8. 7 Hz, 2. 4 Hz, 1 H, H-4'), 8. 64 (d, *J =* 2. 4 Hz, I H, H-6'), 9. 19 (br s, 1 H, NH); MS (FAB) *m*/*z* 538 (MH⁺).

### Preparative Example 39

### Preparation of 4-(5-(4-ethylpiperazin-1-ylsulfonyl)-2-n-propoxybenzamido)-1-ethyl-3-(3-fluoropropyl)pyrrole-5-carboxamide (a compound of the formula (25) wherein R¹ = CH₂CH₃, R² = H, R³ = CH₂CH₂CH₃F, R⁴ = CH₂CH₂CH₃, R¹⁶ = 4-ethylpiperazin-1-ylsulfonyl)

The titled compound was prepared as described in Preparative Example 30 by using 4-amino-1-ethyl-3-(3-fluoropropyl)pyrrole-5-carboxamide and 5-(4-ethylpiperazin-1-ylsulfonyl)-2-*n*-propoxybenzoic acid in place of 4-amino-3-ethyl-1-methylpyrrole-5-carboxamide and 2-ethoxy-5-(4-(3-fluoropropyl)piperazin-1-ylsulfonyl)benzoic acid
yield: 77%
mp 155-156°C (EtOAc/ether/hexanes);
IR (neat) 3361 (NH), 1641 (C=O), 1163 (SO₂) cm⁻¹;
¹H NMR (CDCl₃/TMS) δ 1. 03 (t, *J* = 7. 5 Hz, 3 H, OCH₂CH₂C*H*₃), 1. 08 (t, *J* = 7. 2 Hz, 3 H, CH₂NCH₂C*H*₃), 1. 41 (t, *J =* 7. 2 Hz, 3 H, CHNCH₂C*H*₃), 1. 80-2. 01 (m, 4 H, CH₂C*H*₂CH₂F and OCH₂C*H*₂CH₃), 2. 41 (q, *J =* 7. 2 Hz, 2 H, CH₂NC*H*₂CH₃), 2. 48-2. 55 (m, 6 H, C*H*₂CH₂CH₂F and 2 NCH₂), 3. 07 (br dd, *J =* 5. 1 Hz, 4. 8 Hz, 4 H, 2 SO₂NCH₂), 4. 25 (t, *J* = 6. 3 Hz, 2 H, OC*H*₂CH₂CH₃), 4. 28 (q, *J* = 7. 2 Hz, 2 H, CHNC*H*₂CH₃), 4. 43 (dt, *J* = 47. 4 Hz, 5. 7 Hz, 2 H, CH₂CH₂C*H*₂F), 6. 63 (s, 1 H, H-2), 7.15 (d, *J =* 8. 7 Hz, 1 H, H-3'), 7. 89 (dd, *J* = 8. 7 Hz, 2. 4 Hz, 1 H, H-4'), 8. 63 (d, *J=* 2.4 Hz, 1 H, H-6'), 9. 18 (br s, 1 H, NH); MS (FAB) *m*/*z* 534 (MH⁺).

### Preparative Example 40

### Preparation of 4-(2-ethoxy-5-(4-isopropylpiperazin-1-ylsulfonyl)benzamido)-1-ethyl-3-(3-fluoroprop yl)pyrrole-5-carboxamide (a compound of the formula (25) wherein R' = CH₂CH₃, R² = H, R³ = CH₂CH₂CH₂F, R⁴ = CH₂CH₃, R¹⁶ = 4-isopropylpiperazin-1-ylsulfonyl)

The titled compound was prepared as described in Preparative Example 30 by using 4-amino-1-ethyl-3-(3-fluoropropyl)pyrrole-5-carboxamide and 2-ethoxy-5-(4-isopropylpiperazin-1-ylsulfonyl)benzoic acid in place of 4-amino-3-ethyl-1-methylpyrrole-5-carboxamide and 2-ethoxy-5-(4-(3-fluoropropyl)piperazin-1-ylsulfonyl)benzoic acid
yield: 80%
mp 168-169°C (EtOAc/ether/hexanes);
IR (neat) 3353 (NH), 1648 (C=O), 1177 (SO₂) cm⁻¹;
¹H NMR (CDCl₃/TMS) δ 1. 00 (d, *J* = 6. 6 Hz, 6 H, CH(C*H*₃)₂), 1. 41 (t, *J =* 7. 2 Hz, 3 H, NCH₂C*H*₃), 1. 57 (t, *J* = 6. 9 Hz, 3 H, OCH₂C*H*₃), 1. 81-1. 99 (m, 2 H, CH₂C*H*₂CH₂F), 2. 52 (t, *J* = 7. 5 Hz, 2 H, C*H*₂CH₂CH₂F), 2. 60 (dd, *J* = 5. 1 Hz, 4. 5 Hz, 4 H, 2 NCH₂), 2. 63-2. 71 (m, 1 H, C*H*(CH₃)₂), 3.05 (br dd, *J* = 5. 1 Hz, 4. 5 Hz, 4 H, 2 SO₂NCH₂), 4. 27 (q, *J* = 7. 2 Hz, 2 H, NC*H*₂CH₃), 4. 36 (q, *J* = 6. 9 Hz, 2 H, OC*H*₂CH₃), 4. 44(dt, *J* = 47. 4 Hz, 5. 7 Hz, 2 H, CH₂CH₂C*H*₂F), 6. 63 (s, 1 H, H-2), 7. 14 (d, *J* = 8. 7 Hz, 1 H, H-3'), 7. 89 (dd, *J* = 8. 7 Hz, 2. 7 Hz, 1 H, H-4'), 8. 63 (d, *J =* 2. 7 Hz, I H, H-6'), 9. 19 (br s, 1 H, NH); MS (FAB) *m*/*z* 552 (MH⁺).

### Preparative Example 41

### Preparation of 4-(2-ethoxy-5-(4-(3-fluoropropyl)piperazin-1-ylsulfonyl)benzamido)-1-ethyl-3-(3-fluo ropropyl)pyrrole-5-carboxamide (a compound of the formula (25) wherein R¹ = CH₂CH₃, R² = H, R³ = CH₂CH₂CH₂F, R⁴ = CH₂CH₃, R¹⁶ = 4-(3-fluoropropyl)piperazin-1-ylsulfonyl)

The titled compound was prepared as described in Preparative Example 30 by using 4-amino-1-ethyl-3-(3-fluoropropyl)pyrrole-5-carboxamide in place of 4-amino-3-ethyl-1-methylpyrrole-5-carboxamide
yield: 85%
mp 165-166°C (EtOAc/ether);
IR (neat) 3354 (NH), 1648 (C=O), 1172 (SO₂) cm⁻¹;
¹H NMR (CDCl₃/TMS) δ 1. 42 (t, *J=* 7. 2 Hz, 3 H, NCH₂C*H*₃), 1. 57 (t, *J=* 6. 9 Hz, 3 H, OCH₂C*H*₃), 1. 72-1. 98 (m, 4 H, CH₂C*H*₂CH₂F and NCH₂C*H*₂CH₂F), 2. 45-2. 55(m, 8H, 2 NCH₂, CH₂C*H*₂CH₂F and NCH₂C*H*₂CH₂F), 3. 07 (m, 4 H, 2 SO₂NCH₂), 4. 27 (q, *J* = 7. 2 Hz, 2 H, NC*H*₂CH₃), 4. 37 (q, *J =* 6. 9 Hz, 2 H, OC*H*₂CH₃), 4. 44(dt, *J*= 47. 4 Hz, 6. 0 Hz, 4 H, CH₂CH₂C*H*₂F and NCH₂CH₂C*H*₂F), 6. 63 (s, 1 H, H-2), 7. 16 (d, *J*= 8. 7 Hz, 1 H, H-3'), 7. 89 (dd, *J* = 8. 7 Hz, 2. 4 Hz, 1 H, H-4'), 8. 63 (d, *J=* 2. 4 Hz, 1 H, H-6'), 9. 19 (br s, 1 H, NH); MS (FAB) *m*/*z* 570 (MH⁺).

### Preparative Example 42

### Preparation of 4-(5-(4-(3-fluoropropyl)piperazin-1-ylsulfonyl)-2-n-propoxybenzamido)-1-ethyl-3-(3-fluoropropyl)pyrrole-5-carboxamide (a compound of the formula (25) wherein R¹ = CH₂CH₃, R² = H, R³ = CH₂CH₂CH₂F, R⁴ = CH₂CH₂CH₃, R¹⁶ = 4-(3-fluoropropyl)piperazin-1-ylsulfonyl)

The titled compound was prepared as described in Preparative Example 30 by using 4-amino-1-ethyl-3-(3-fluoropropyl)pyrrole-5-carboxamide and 5-(4-(3-fluoropropyl)piperazin-1-ylsulfonyl)-2-*n*-propoxybenzoic acid in place of 4-amino-3-ethyl-1-methylpyrrole-5-carboxamide and 2-ethoxy-5-(4-(3-fluoropropyl)piperazin-1-ylsulfonyl)benzoic acid
yield: 83%
mp 162. 5-163. 5°C (CH₂Cl₂/EtOAc/hexanes);
IR (neat) 3359 (NH), 1651 (C=O), 1171 (SO₂) cm⁻¹;
¹H NMR (CDCl₃/TMS) δ 1. 08 (t, *J* = 7. 5 Hz, 3 H, OCH₂CH₂C*H*₃), 1. 41 (t, *J =* 7. 2 Hz, 3 H, NCH₂C*H*₃), 1. 72-2. 02 (m, 6 H, OCH₂C*H*₂CH₃, CH₂C*H*₂CH₂F and NCH₂C*H*₂CH₂F), 2. 45-2. 58 (m, 8H, 2 NCH₂, C*H*₂CH₂CH₂F and NC*H*₂CH₂CH₂F), 3. 07 (m, 4 H, 2 SO₂NCH₂), 4. 25 (t, *J* = 6. 6 Hz, 2 H, OC*H*₂CH₂CH₃), 4. 28 (t, *J =* 7. 2 Hz, 2 H, NC*H*₂CH₃), 4. 43(dt, *J* = 47. 2 Hz, 6. 0 Hz, 4 H, CH₂CH₂C*H*₂F and NCH₂CH₂C*H*₂F), 6.63 (s, 1 H, H-2), 7. 16 (d, *J* = 8. 7 Hz, 1 H, H-3'), 7. 89 (dd, *J* = 8. 7 Hz, 2. 4 Hz, 1 H, H-4'), 8. 63 (d, *J* = 2. 4 Hz, 1 H, H-6'), 9. 18 (br s, 1 H, NH); MS (FAB) *m*/*z* 584 (MH⁺).

### Preparative Example 43

### Preparation of 4-(2-ethoxy-5-(4-(2-hydroxyethyl)piperazin-1-ylsulfonyl)benzamido)-1-ethyl-3-(3-flu oropropyl)pyrrole-5-carboxamide (a compound of the formula (25) wherein R¹ = CH₂CH₃, R² = H, R³ = CH₂CH₂CH₂F, R⁴ = CH₂CH₃, R¹⁶ = 4-(2-hydroxyethyl)piperazin-1-ylsulfonyl)

The titled compound was prepared as described in Preparative Example 30 by using 4-amino-1-ethyl-3-(3-fluoropropyl)pyrrole-5-carboxamide and 2-ethoxy-5-(4-(2-hydroxyethyl)piperazin-1-ylsulfonyl)benzoic acid in place of 4-amino-3-ethyl-1-methylpyrrole-5-carboxamide and 2-ethoxy-5-(4-(3-fluoropropyl)piperazin-1-ylsulfonyl)benzoic acid
yield: 81%
IR (neat) 3355 (NH), 1662 (C=O), 1170 (SO₂) cm⁻¹;
¹H NMR (CDCl₃/TMS) δ 1. 42 (t, *J =* 7. 2 Hz, 3 H, NCH₂C*H*₃), 1. 57 (t, *J* = 6. 9 Hz, 3 H, OCH₂C*H*₃), 1. 81-1. 98 (m, 2 H, CH₂C*H*₂CH₂F), 2. 52 (t, *J* = 7. 5 Hz, 2 H, C*H*₂CH₂CH₂F), 2. 55 (t, *J* = 5.4 Hz, 2 H, C*H*₂CH₂OH), 2. 60 (dd, *J* = 5. 1 Hz, 4. 8 Hz, 4 H, 2 NCH₂), 3. 08 (br dd, *J =* 5. 1 Hz, 4. 8 Hz, 4 H, 2 SO₂NCH₂), 3. 58 (t, *J* = 5. 4 Hz, 2 H, CH₂C*H*₂OH), 4. 27 (q, *J =* 7. 2 Hz, 2 H, NC*H*₂CH₃), 4.37 (q, *J* = 6. 9 Hz, 2 H, OC*H*₂CH₃), 4. 44 (dt, *J* = 47. 4 Hz, 6. 0 Hz, 2 H, CH₂CH₂C*H*₂F), 6. 63 (s, 1 H, H-2), 7.16 (d, *J =* 8. 7 Hz, 1 H, H-3'), 7. 89 (dd, *J=* 8. 7 Hz, 2. 7 Hz, 1 H, H-4'), 8. 63 (d, *J=* 2. 7 Hz, 1 H, H-6'), 9. 19 (br s, 1 H, NH); MS (FAB) *m*/*z* 554 (MH⁺).

### Preparative Example 44

### Preparation of 4-(5-(4-(2-hydroxyethyl)piperazin-1-ylsulfonyl)-2-n-propoxybenzamido)-1-ethyl-3-(3-fluoropropyl)pyrrole-5-carboxamide (a compound of the formula (25) wherein R¹ = CH₂CH₃, R² = H, R³ = CH₂CH₂CH₂F, R⁴ = CH₂CH₂CH₃, R¹⁶ = 4-(2-hydroxyethyl)piperazin-1-ylsulfonyl)

The titled compound was prepared as described in Preparative Example 30 by using 4-amino-1-ethyl-3-(3-fluoropropyl)pyrrole-5-carboxamide and 5-(4-(2-hydroxyethyl)piperazin-1-ylsulfonyl)-2-*n*-propoxybenzoic acid in place of 4-amino-3-ethyl-1-methylpyrrole-5-carboxamide and 2-ethoxy-5-(4-(3-fluoropropyl)piperazin-1-ylsulfonyl)benzoic acid
yield: 65%
IR (neat) 3355 (NH), 1669 (C=O), 1165 (SO₂) cm⁻¹;
¹H NMR (CDCl₃/TMS) δ 1. 08 (t, *J =* 7. 5 Hz, 3 H, CH₂CH₂C*H*₃), 1. 41 (t, *J* = 7. 2 Hz, 3 H, NCH₂C*H*₃), 1. 80-2. 02 (m, 4 H, CH₂C*H*₂CH₃ and CH₂C*H*₂CH₂F), 2. 51 (t, *J =* 7. 5 Hz, 2 H, C*H*₂CH₂CH₂F), 2. 56 (t, *J* = 5. 4 Hz, 2 H, C*H*₂CH₂OH), 2. 61 (dd, *J* = 5. 1 Hz, 4. 8 Hz, 4 H, 2 NCH₂), 3. 09 (br dd, *J=* 5. 1 Hz, 4. 8 Hz, 4 H, 2 SO₂NCH₂), 3. 59 (t, *J =* 5. 4 Hz, 2 H, CH₂C*H*₂OH), 4. 26 (q, *J =* 6. 6 Hz, 2 H, C*H*₂CH₂CH₃), 4. 27 (q, *J* = 7. 2 Hz, 2 H, NC*H*₂CH₃), 4. 44 (dt, *J =* 47. 1 Hz, 5. 7 Hz, 2 H, CH₂CH₂C*H*₂F), 6. 63 (s, 1 H, H-2), 7. 18 (d, *J =* 8. 7 Hz, 1 H, H-3'), 7. 89 (dd, *J =* 8. 7 Hz, 2. 4 Hz, 1 H, H-4'), 8. 62 (d, *J =* 2. 4 Hz, 1 H, H-6'), 9. 18 (br s, 1 H, NH); MS (FAB) *m*/*z* 568 (MH⁺).

### Preparative Example 45

### Preparation of 2-(2-ethoxyphenyl)-5-methyl-7-n-propyl-3,5-dihydro-4H-pyrrolo[3,2-d]pyrimidin-4-one (a compound of the formula (9) wherein R¹ = CH₃, R² = H, R³ = CH₂CH₂CH₃, R⁴ = CH₂CH₃)

A suspension of 4-(2-ethoxybenzamido)-1-methyl-3-*n*-propylpyrrole-5-carboxamide (5. 01 g, 15. 21 mmol) and NaOH (3. 04 g, 76. 05 mmol) in a mixture of water (50 mL) and MeOH (100 mL) was heated at 80 °C under nitrogen atmosphere for 5 h. The reaction mixture was cooled to room temperature, and MeOH was removed under vacuum. The resulting aqueous layer was acidified to about pH 9-10 with 1 N aqueous HCl solution, and was extracted with 2% MeOH in CHCl₃ (2 x 300 mL). Combined organic layer was dried (MgSO₄), filtered, and evaporated to dryness *in vacuo* to afford a yellow solid. The crude product was purified by MPLC on silica gel (gradient elution: 1% MeOH in CHCl₃ followed by 2% MeOH in CHCl₃) to afford the titled compound (3. 62 g, 77%) as a white solid. Analytically pure compound was obtained by crystallization from CHCl₃/Et₂O/hexanes.
mp 128. 5-129 °C;
IR (neat) 3319 (NH); 1675 (C=O) cm⁻¹;
¹H NMR (CDCl₃/TMS) δ 1.01 (t, *J =* 7. 5 Hz, 3 H, CH₂CH₂C*H*₃), 1. 59 (t, *J =* 6. 9 Hz, 3 H, OCH₂C*H*₃), 1. 68-1. 81 (m, 2 H, CH₂C*H*₂CH₃), 2. 72 (t, *J* = 7. 5 Hz, 2 H, C*H*₂CH₂CH₃), 4. 08 (s, 3 H, NCH₃), 4. 27 (q, *J =* 6. 9 Hz, 2 H, OC*H*₂CH₃), 6. 86 (s, 1 H, H-2), 7. 02 (d, *J =* 8. 4 Hz, 1 H, H-3'), 7. 09-7. 15 (m, 1 H, H-5'), 7. 41 (ddd, *J =* 8. 4 Hz, 7. 2 Hz, 1. 8 Hz, 1 H, H-4'), 8. 49 (dd, *J =* 8. 1 Hz, 1. 8 Hz, 1 H, H-6'), 10. 91 (br s, I H, NH); MS (FAB) *m*/*z* 312 (MH⁺).

### Preparative Example 46

### Preparation of 5-methyl-2-(2-n-propoxyphenyl)-7-n-propyl-3,5-dihydro-4H-pyrrolo[3,2-d]pyrimidin-4-one (a compound of the formula (9) wherein R¹ = CH₃, R² = H, R³ = CH₂CH₂CH₃, R⁴ = CH₂CH₂CH₃)

The titled compound was prepared as described in Preparative Example 46 by using 1-methyl-4-(2-*n*-propoxybenzamido)-3-*n*-propylpyrrole-5-carboxamide in place of 4-(2-ethoxybenzamido)-1-methyl-3-*n*-propylpyrrole-5-carboxamide.
yield: 94%
mp 108-108. 5 °C (CHCl₃/Et₂O/hexanes);
IR (neat) 3326 (NH), 1684 (C=O) cm⁻¹;
¹H NMR (CDCl₃/TMS) δ 1. 01 (t, *J=* 7. 2 Hz, 3 H, CH₂CH₂C*H*₃), 1. 16 (t, *J=* 7. 5 Hz, 3 H, OCH₂CH₂C*H*₃), 1. 70-1. 82 (m, 2 H, CH₂C*H*₂CH₃), 1. 94-2. 05 (m, 2 H, OCH₂C*H*₂CH₃), 2. 72 (t, *J* = 7. 5 Hz, 2 H, C*H*₂CH₂CH₃), 4. 08 (s, 3 H, NCH₃), 4. 16 (t, *J=* 6. 6 Hz, 2 H, OC*H*₂CH₂CH₃), 6. 86 (s, 1 H, H-2), 7. 02 (d, *J=* 8. 1 Hz, 1 H, H-3'), 7. 12 (t, *J=* 7. 2 Hz, 1 H, H-5'), 7. 41 (ddd, *J=* 8. 1 Hz, 7. 2 Hz, 1. 8 Hz, I H, H-4'), 8. 50 (dd, *J =* 8. 1 Hz, 1. 8 Hz, 1 H, H-6'), 10. 94 (br s, 1 H, NH); MS (FAB) *m*/*z* 326 (MH⁺).

### Prenarative Example 47

### Preparation of 2-(5-chlorosulfonyl-2-ethoxyphenyl)-5-methyl-7-n-propyl-3,5-dihydro-4H-pyrrolo[3,2 - d]pyrimidin-4-one (a compound of the formula (2) wherein Y = Cl, R¹ = CH₃, R² = H, R³ = CH₂CH₂CH₃, R⁴ = CH₂CH₃)

To a stirred and cooled chlorosulfonic acid (6 mL) in an ice bath under nitrogen atmosphere was added portionwise 2-(2-ethoxyphenyl)-5-methyl-7-*n*-propyl-3,5-dihydro-4*H*-pyrrolo[3,2-*d*]pyrimidin-4-o ne (1. 51 g, 4. 85 mmol), and the reaction mixture was stirred in an ice bath for 1 h. Then, the mixture was warmed to room temperature gradually and stirring was continued for additional over 1 h at room temperature. Resulting mixture was transferred dropwise to the well-stirred mixture of CHCl₃ (50 mL) and ice (50 g), and was extracted with 5% MeOH in CHCl₃ (2 × 100 mL). Combined extracts were dried (Na₂SO₄), filtered, and evaporated to dryness under reduced pressure to give the desired sulfonyl chloride as a yellow solid. The crude product was solidified by dissolving in CHCl₃ (20 mL), followed by diluting with diethyl ether (30 mL) and hexanes (100 mL) to afford the titled compound (1. 90 g, 96%) as a pale yellow solid. Analytically pure compound was obtained by crystallization from CHCl₃/Et₂O/hexanes.
mp 164. 5-166 °C dec;
IR (neat) 3341 (NH), 1693 (C=O), 1174 (SO₂) cm⁻¹;
¹H NMR (CDCl₃/TMS) δ 1. 02 (t, *J* = 7. 2 Hz, 3 H, CH₂CH₂C*H*₃), 1. 66 (t, *J* = 6. 9 Hz, 3 H, OCH₂C*H*₃), 1. 68-1. 80 (m, 2 H, CH₂C*H*₂CH₃), 2. 75 (t, *J* = 7. 5 Hz, 2 H, C*H*₂CH₂CH₃), 4. 09 (s, 3 H, NCH₃), 4. 42 (q, *J=* 6. 9 Hz, 2 H, OC*H*₂CH₃), 6. 92 (s, 1 H, H-2), 7. 20 (d, *J=* 9. 0 Hz, I H, H-3'), 8. 08 (dd, *J=* 9. 0 Hz, 2. 4 Hz, 1 H, H-4'), 9. 13 (d, *J=* 2.4 Hz, 1 H, H-6'), 10. 61 (br s, 1 H, NH); MS (FAB) *m*/*z* 392 (MH⁺-H₂O).

### Preparative Example 48

### Preparation of 2-(5-chlorosulfonyl-2-n-propoxyphenyl)-5-methyl-7-n-propyl-3,5-dihydro-4H-pyrrolo [3,2-d]pyrimidin-4-one (a compound of the formula (2) wherein Y = CI, R¹ = CH₃, R² = H, R³ = CH₂CH₂CH₃, R⁴ = CH₂CH₂CH₃)

The titled compound was prepared as described in Preparative Example 48 by using 5-methyl-2-(2-*n*-propoxyphenyl)-7-*n*-propyl-3,5-dihydro-4*H*-pyrrolo[3,2-*d*]pyrimidin-4-one in place of 2-(2-ethoxyphenyl)-5-methyl-7-*n*-propyl-3,5-dihydro-4*H*-pyrrolo[3,2-*d*]pyrimidin-4-o ne.
yield: 94%
mp 136 °C dec (CHCl₃/Et₂O);
IR (neat) 3330 (NH), 1665 (C=O) , 1174 (SO₂) cm⁻¹;
¹H NMR (CDCl₃/TMS) δ 1. 02 (t, *J*= 7. 5 Hz, 3 H, CH₂CH₂C*H*₃), 1. 18 (t, *J*= 7. 2 Hz, 3 H, OCH₂CH₂C*H*₃), 1. 68-1. 80 (m, 2 H, CH₂C*H*₂CH₃), 1. 98-2. 12 (m, 2 H, OCH₂C*H*₂CH₃), 2. 76 (t, *J* = 7. 5 Hz, 2 H, C*H*₂CH₂CH₃), 4. 09 (s, 3 H, NCH₃), 4. 30 (t, *J =* 6. 6 Hz, 2 H, OC*H*₂CH₂CH₃), 6. 93 (s, 1 H, H-2), 7. 21 (d, *J=* 9. 0 Hz, 1 H, H-3'), 8. 07 (dd, *J* = 9. 0 Hz, 2. 4 Hz, 1 H, H-4'), 9. 11 (d, *J* = 2. 4 Hz, 1 H, H-6'); MS (FAB) *m*/*z* 424 (MH⁺).

### Preparative Example 49

### Preparation of 4-(2-(2-fluoroethoxy)benzamido)-1-methyl-3-n-propylpyrrole-5-carboxamide (a compound of the formula (12) wherein R' = CH₃, R² = H, R³ = CH₂CH₂CH₃, R⁴ = CH₂CH₂F)

The titled compound was prepared as described in Preparative Example 28 by using 2-(2-fluoroethoxy)benzoyl chloride in place of 2-ethoxybenzoyl chloride.
yield: 67%
mp 132-132. 5 °C (ethyl acetate/hexanes);
IR (neat) 3344, 3164 (NH), 1664, 1640 (C=O) cm⁻¹;
¹H NMR (CDCl₃/TMS) δ 0. 91 (t, *J =* 7. 2 Hz, 3 H, CH₂CH₂C*H*₃), 1. 49-1. 62 (m, 2 H, CH₂C*H*₂CH₃), 2. 33 (t, *J* = 7. 5 Hz, 2 H, C*H*₂CH₂CH₃), 3. 85 (s, 3 H, NCH₃), 4. 38-4. 51 (m, 2 H, OC*H*₂CH₂F), 4. 72-4. 91 (m, 2 H, OCH₂C*H*₂F), 6. 52 (s, 1 H, H-2), 7. 03 (d, *J =* 8. 1 Hz, 1 H, H-3'), 7. 17 (td, *J =* 8. 1 Hz, 1. 2 Hz, 1 H, H-5'), 7. 50-7.56 (m, 1 H, H-4'), 8. 28 (dd, *J* = 7. 8 Hz, 1. 8 Hz, 1 H, H-6'), 9. 11 (br s, 1 H, NH); MS (FAB) *m*/*z* 348 (MH⁺).

### Preparative Example 50

### Preparation of 2-(2-(2-fluoroethoxy)phenyl)-5-methyl-7-n-propyl-3,5-dihydro-4H-pyrrolo[3,2-d]pyri midin-4-one (a compound of the formula (9) wherein R¹ = CH₃, R² = H, R³ = CH₂CH₂CH₃, R⁴ = CH₂CH₂F)

A suspension of 4-(2-(2-fluoroethoxy)benzamido)-1-methyl-3-*n*-propylpyrrole-5-carboxamide (1. 60 g, 4. 60 mmol) and potassium *tert*-butoxide (1. 03 g, 9. 21 mmol) in *tert*-BuOH (25 mL) was heated at 60 °C under nitrogen atmosphere for 5 h. The reaction mixture was cooled to room temperature, diluted with water (25 mL) and *tert*-BuOH was removed under vacuum. The resulting aqueous layer was acidified to about pH 5-6 with 1 N aqueous HCl solution, and was extracted with 5% MeOH in CHCl₃ (2 × 100 mL). Combined organic layer was dried (MgSO₄), filtered, and evaporated to dryness in vacuo to afford a yellow solid. The crude product was purified by MPLC on silica gel (1% MeOH in CHCl₃) to afford the titled compound (1. 24 g, 82%) as a pale yellow solid. Analytically pure compound was obtained by crystallization from ethyl acetate/hexanes.
mp 116-117 °C;
IR (neat) 3348 (NH), 1676 (C=O) cm⁻¹;
¹H NMR (CDCl₃/TMS) δ 1. 01 (t, *J* = 7. 5 Hz, 3 H, CH₂CH₂C*H*₃), 1. 70-1. 78 (m, 2 H, CH₂C*H*₂CH₃), 2. 72 (t, *J* = 7. 5 Hz, 2 H, C*H*₂CH₂CH₃), 4. 07 (s, 3 H, NCH₃), 4. 36-4. 48 (m, 2 H, OC*H*₂CH₂F), 4. 77-4. 96 (m, 2 H, OCH₂C*H*₂F), 6. 86 (s, 1 H, H-2), 7. 02 (d, *J*= 8. 1 Hz, 1 H, H-3'), 7. 17 (td, *J*= 8. 1 Hz, 0. 9 Hz, 1 H, H-5'), 7. 39-7.46 (m, 1 H, H-4'), 8. 44 (dd, *J* = 8. 1 Hz, 1. 8 Hz, 1 H, H-6'), 10. 60 (br s, 1 H, NH); MS (FAB) *m*/*z* 330 (MH⁺).

### Preparative Example 51

### Preparation of 2-(5-chlorosulfonyl-2-(2-fluoroethoxy)phenyl)-5-methyl-7-n-propyl-3,5-dihydro-4H-p yrrolo[3,2-d]pyrimidin-4-one (a compound of the formula (2) wherein Y = Cl, R¹ = CH₃, R² = H, R³ = CH₂CH₂CH₃, R⁴ = CH₂CH₂F)

The titled compound was prepared as described in Preparative Example 48 by using 2-(2-(2-fluoroethoxy)phenyl)-5-methyl-7-*n*-propyl-3,5-dihydro-4*H*-pyrrolo[3,2-*d*]pyri midin-4-one in place of 2-(2-ethoxyphenyl)-5-methyl-7-*n*-propyl-3,5-dihydro-4*H*-pyrrolo[3,2-*d*]pyrimidin-4-o ne.
yield: 85%
mp 156. 5-157. 5 °C (ethyl acetate/hexanes);
IR (neat) 3344 (NH), 1680 (C=O), 1174 (SO₂) cm⁻¹;
¹H NMR (CDCl₃/TMS) δ 1. 02 (t, *J* = 7. 5 Hz, 3 H, CH₂CH₂C*H*₃), 1. 70-1. 78(m, 2 H, CH₂C*H*₂CH₃), 2. 75 (t, *J* = 7. 5 Hz, 2 H, C*H*₂CH₂CH₃), 4. 09 (s, 3 H, NCH₃), 4. 51-4. 63 (m, 2 H, OC*H*₂CH₂F), 4. 84-5. 02 (m, 2 H, OCH₂C*H*₂F), 6. 92 (s, 1 H, H-2), 7. 23 (d, *J =* 9. 0 Hz, 1 H, H-3'), 8. 10 (dd, *J =* 9. 0 Hz, 2. 7 Hz, 1 H, H-4'), 9. 09 (d, *J =* 2. 7 Hz, 1 H, H-6'), 10. 46 (br s, 1 H, NH); MS (FAB) *m*/*z* 428 (MH⁺).

### Preparative Example 52

### Preparation of N-(2-cyano-1-methyl-2-n-propylvinyl)-N-methylglycine ethyl ester (a compound of the formula (20) wherein R¹ = R² = CH₃, R³ = CH₂CH₂CH₃)

The titled compound was prepared as described in Preparative Example 7 by using 3-cyano-2-hexanone in place of 2-cyanopentanal.
yield: 55%
IR (neat) 2181 (CN), 1743 (C=O) cm⁻¹;
¹H NMR (CDCl₃/TMS) δ 0. 93 (t, *J =* 7. 5 Hz, 3 H, CH₂CH₂C*H*₃), 1. 30(t, *J =* 7. 2 Hz, 3 H, OCH₂C*H*₃), 1. 46-1. 58 (m, 2 H, CH₂C*H*₂CH₃), 1. 90(s, 3 H, CH₃), 2. 14(t, *J* = 7. 5 Hz, 2 H, C*H*₂CH₂CH₃), 3. 11 (s, 3 H, NCH₃), 4. 05(s, 2 H, NCH₂CO), 4. 22(q, *J=* 7. 2 Hz, 2 H, OC*H*₂CH₃) - Z isomer and 0. 94(t, *J=* 7. 5 Hz, 3 H, CH₂CH₂C*H*₃), 1. 29(t, *J=* 7. 2 Hz, 3 H, OCH₂C*H*₃), 1. 52-1. 65 (m, 2 H, CH₂C*H*₂CH₃), 2. 11 (d, *J=* 7. 5 Hz, 2 H, C*H*₂CH₂CH₃), 2. 19 (s, 3 H, CH₃), 2. 90 (s, 3 H, NCH₃), 3. 80 (s, 2 H, NCH₂CO), 4. 21 (q, *J =* 7. 2 Hz, 2 H, OC*H*₂CH₃) - E isomer; MS (FAB) *m*/*z* 225 (MH⁺).

### Preparative Example 53

### Preparation of N-(2-cyano-1-methyl-2-n-propylvinyl)-N-methylglycine amide (a compound of the formula (19) wherein R¹ = R² = CH₃, R³ = n-propyl)

A suspension of *N*-(2-cyano-1-methyl-2-*n*-propylvinyl)-*N*-methylglycine ethyl ester (2. 03 g, 9. 05 mmol in 29% aqueous ammonia solution (13 mL) and MeOH (7 mL) was stirred overnight at room temperature. The reaction mixture was concentrated *in vacuo*, and the aqueous layer was extracted with CHCl₃ (40 mL x 3). Combined organic layer was dried (Na₂SO₄), filtered and the filtrate was evaporated to dryness under reduced pressure. Resulting residue was purified by MPLC on silica gel (gradient elution: 1:1 ethyl acetate/hexanes containing 1% Et₃N followed by 5% MeOH in CHCl₃) to afford the titled compound (1. 03 g, 56%) as a yellowish oil.
IR (neat) 3323, 3208 (NH), 2180 (CN), 1670 (C=O) cm⁻¹;
¹H NMR (CDCl₃/TMS) δ 0. 99 (t, *J=* 7. 5 Hz, 3 H, CH₂CH₂C*H*₃), 1. 48-1. 61(m, 2 H, CH₂C*H*₂CH₃), 1. 84 (t, *J*= 7. 5 Hz, 2 H, C*H*₂CH₂CH₃), 1. 93 (s, 3 H, CH₃), 3. 04 (s, 3 H, NCH₃), 3. 79 (s, 2 H, NCH₂CO), 5. 67 (br s, 1 H, NH), 6. 34 (br s, 1 H, NH) - Z isomer and 0. 95(t, *J* = 7. 5 Hz, 3 H, CH₂CH₂CH₃), 1. 45-1. 58 (m, 2 H, CH₂C*H*₂CH₃), 2. 15 (t, *J =* 7. 5 Hz, 2 H, C*H*₂CH₂CH₃), 2. 20 (s, 3 H, CH₃), 2. 86 (s, 3 H, NCH₃), 3. 66 (s, 2 H, NCH₂CO), 5. 76 (br s, 1 H, NH), 6. 19 (br s, 1 H, NH) - E isomer; MS (FAB) *m*/*z* 196 (MH⁺).

### Preparative Example 54

### Preparation of 4-amino-1,2-dimethyl-3-n-propylpyrrole-5-carboxamide (a compound of the formula (15) wherein R¹ = R² = CH₃, R³ = CH₂CH₂CH₃)

The titled compound was prepared as described in Preparative Example 21 by using *N*-(2-cyano-1-methyl-2-*n*-propylvinyl)-*N*-methylglycine amide in place of *N*-(2-cyano-2-*n*-propylvinyl)-*N*-methylglycine amide.
yield: 34%
mp 135 °C dec (CHCl₃/hexanes);
IR (neat) 3357, 3171 (NH₂), 1639 (C=O) cm⁻¹;
¹H NMR (CDCl₃/TMS) δ 0. 92 (t, *J=* 7. 5 Hz, 3 H, CH₂CH₂C*H*₃), 1. 39-1. 51(m, 2 H, CH₂C*H*₂CH₃), 2. 11 (s, 3 H, CH₃), 2. 31 (t, *J=* 7. 5 Hz, 2 H, C*H*₂CH₂CH₃), 3. 23 (br s, 2 H, NH₂), 3. 78 (s, 3 H, NCH₃), 6. 16(br s, 2 H, CONH₂); MS (FAB) *m*/*z* 196 (MH⁺).

### Preparative Example 55

### Preparation of 1,2-dimethyl-4-(2-ethoxybenzamido)-3-n-propylpyrrole-5-carboxamide (a compound of the formula (12) wherein R¹ = R² = CH₃, R³ = CH₂CH₂CH₃, R⁴ = CH₂CH₃)

The titled compound was prepared as described in Preparative Example 28 by using 4-amino-1,2-dimethyl-3-*n*-propylpyrrole-5-carboxamide in place of 4-amino-1-methyl-3-*n*-propylpyrrole-5-carboxamide.
yield: 73%
mp 171. 5 °C dec (CHCl₃/Et₂O/hexanes);
IR (neat) 3348, 3148 (NH), 1649 (C=O) cm⁻¹;
¹H NMR (CDCl₃/TMS) δ 0. 87 (t, *J*= 7. 5 Hz, 3 H, CH₂CH₂C*H*₃), 1. 38-1. 49(m, 2 H, CH₂C*H*₂CH₃), 1. 51 (t, *J*= 6. 9 Hz, 3 H, OCH₂C*H*₃), 2. 17(s, 3 H, CH₃), 2. 33(t, *J* = 7. 5 Hz, 2 H, C*H*₂CH₂CH₃), 3. 77 (s, 3 H, NCH₃), 4. 29 (q, *J* = 6. 9 Hz, 2 H, OC*H*₂CH₃), 7. 04 (d, *J* = 8. 4 Hz, 1 H, H-3'), 7. 09-7. 14 (m, 1 H, H-5'), 7. 47-7. 53(m, 1 H, H-4'), 8. 29 (dd, *J* = 7. 8 Hz, 1. 8 Hz, 1 H, H-6'), 9. 37 (br s, 1 H, NH); MS (FAB) *m*/*z 344* (MH⁺).

### Preparative Example 56

### Preparation of 1,2-dimethyl-4-(2-n-propoxybenzamido)-3-n-propylpyrrole-5-carboxamide (a compound of the formula (12) wherein R¹ = R² = CH₃, R³ = CH₂CH₂CH₃, R⁴ = CH₂CH₂CH₃)

The titled compound was prepared as described in Preparative Example 28 by using 4-amino-1,2-dimethyl-3-*n*-propylpyrrole-5-carboxamide and 2-n-propoxybenzoyl chloride in place of 4-amino-1-methyl-3-*n*-propylpyrrole-5-carboxamide and 2-ethoxybenzoyl chloride.
yield: 69%
mp 188-189 °C (CHCl₃/Et₂O);
IR (neat) 3344, 3155 (NH), 1643 (C=O) cm⁻¹;
¹H NMR (CDCl₃/TMS) δ 0. 85 (t, *J =* 7. 2 Hz, 3 H, CH₂CH₂C*H*₃), 1. 05 (t, *J* = 7. 5 Hz, 3 H, OCH₂CH₂C*H*₃), 1. 36-1. 49 (m, 2 H, CH₂C*H*₂CH₃), 1. 84-1. 95 (m, 2 H, OCH₂C*H*₂CH₃), 2. 17 (s, 3 H, CH₃), 2. 32 (t, *J* = 7. 5 Hz, 2 H, C*H*₂CH₂CH₃), 3. 77 (s, 3 H, NCH₃), 4. 17 (t, *J =* 6. 6 Hz, 2 H, OC*H*₂CH₂CH₃), 7. 04 (d, *J =* 8. 4 Hz, 1 H, H-3'), 7. 11 (td, *J* = 7. 8 Hz, 1. 2 Hz, 1 H, H-5'), 7. 50 (ddd, *J =* 8. 4 Hz, 7. 8 Hz, 1. 8 Hz, 1 H, H-4'), 8. 29 (dd, *J =* 7. 8 Hz, 1. 8 Hz, 1 H, H-6'), 9. 35 (br s, 1 H, NH); MS (FAB) *m*/*z* 358 (MH⁺).

### Preparative Example 57

### Preparation of 5,6-dimethyl-2-(2-ethoxyphenyl)-7-n-propyl-3,5-dihydro-4H-pyrrolo[3,2-d]pyrimidin-4-one (a compound of the formula (9) wherein R¹ = R² = CH₃, R³ = CH₂CH₂CH₃, R⁴ =CH₂CH₃)

The titled compound was prepared as described in Preparative Example 51 by using 1,2-dimethyl-4-(2-ethoxybenzamido)-3-*n*-propylpyrrole-5-carboxamide in place of 4-(2-(2-fluoroethoxy)benzamido)-1-methyl-3-*n*-propylpyrrole-5-carboxamide.
yield: 94%
mp 130 °C dec (CHCl₃/Et₂O/hexanes);
IR (neat) 3175 (NH), 1653 (C=O) cm⁻¹;
¹H NMR (CDCl₃/TMS) δ 0. 97 (t, *J =* 7. 5 Hz, 3 H, CH₂CH₂C*H*₃), 1. 59 (t, *J =* 6. 9 Hz, 3 H, OCH₂C*H*₃), 1. 62-1. 73 (m, 2 H, CH₂C*H*₂CH₃), 2. 30 (s, 3 H, CH₃), 2. 69 (t, *J =* 7. 5 Hz, 2 H, C*H*₂CH₂CH₃), 4. 03 (s, 3 H, NCH₃), 4. 27 (q, *J* = 6. 9 Hz, 2 H, OC*H*₂CH₃), 7. 01 (d, *J*= 8. 4 Hz, 1 H, H-3'), 7. 12 (td, *J*= 7. 8 Hz, 0. 9 Hz, 1 H, H-5'), 7. 37-7. 43 (m, 1 H, H-4'), 8. 49 (dd, *J* = 7. 8 Hz, 1. 8 Hz, 1 H, H-6'), 10. 85 (br s, 1 H, NH); MS (FAB) *m*/*z* 326 (MH⁺).

### Preparative Example 58

### Preparation of 5,6-dimethyl-2-(2-n-propoxyphenyl)-7-n-propyl-3,5-dihydro-4H-pyrrolo[3,2-d]pyrimi din-4-one (a compound of the formula (9) wherein R¹ = R² = CH₃, R³ = CH₂CH₂CH₃, R⁴ = CH₂CH₂CH₃)

The titled compound was prepared as described in Preparative Example 51 by using 1,2-dimethyl-4-(2-*n*-propoxybenzamido)-3-*n*-propylpyrrole-5-carboxamide in place of 4-(2-(2-fluoroethoxy)benzamido)-1-methyl-3-*n*-propylpyrrole-5-carboxamide.
yield: 97%
mp 112-112. 5 °C (CHCl₃/Et₂O/hexanes);
IR (neat) 3334 (NH), 1683 (C=O) cm⁻¹;
¹H NMR (CDCl₃/TMS) δ 0. 97 (t, *J* = 7. 2 Hz, 3 H, CH₂CH₂C*H*₃); 1. 16(t, *J=* 7. 5 Hz, 3 H, OCH₂CH₂C*H*₃), 1. 63-1. 75 (m, 2 H, CH₂C*H*₂CH₃), 1. 94-2. 06 (m, 2 H, OCH₂C*H*₂CH₃), 2. 30 (s, 3 H, CH₃), 2. 70 (t, *J=* 7. 5 Hz, 2 H, C*H*₂CH₂CH₃), 4. 03 (s, 3 H, NCH₃), 4. 16(t, *J=* 6. 6 Hz, 2 H, OC*H*₂CH₂CH₃), 7. 02(d, *J=* 8. 4 Hz, I H, H-3'), 7. 12(td, *J =* 7. 8 Hz, 1. 2 Hz, 1 H, H-5'), 7. 40(ddd, *J =* 8. 4 Hz, 7. 8 Hz, 1. 8 Hz, 1 H, H-4'), 8. 50 (dd, *J =* 7. 8 Hz, 1. 8 Hz, 1 H, H-6'), 10. 89 (br s, 1 H, NH); MS (FAB) *m*/*z* 340 (MH⁺).

### Preparative Example 59

### Preparation of 2-(5-chlorosulfonyl-2-ethoxyphenyl)-5,6-dimethyl-7-n-propyl-3,5-dihydro-4H-pyrrolo [3,2-d]pyrimidin-4-one (a compound of the formula (2) wherein Y = Cl, R¹ = R² = CH₃, R³ = CH₂CH₂CH₃, R⁴ = CH₂CH₃)

The titled compound was prepared as described in Preparative Example 48 by using 5,6-dimethyl-2-(2-ethoxyphenyl)-7-*n*-propyl-3,5-dihydro-4*H*-pyrrolo[3,2-*d*]pyrimidin-4-one in place of 2-(2-ethoxyphenyl)-5-methyl-7-*n*-propyl-3,5-dihydro-4*H*-pyrrolo[3,2-*d*]pyrimidin-4-o ne.
yield: 98% (crude)
¹H NMR (CDCl₃/TMS) δ 0. 97(t, *J* = 7. 5 Hz, 3 H, CH₂CH₂C*H*₃), 1. 66 (t, *J=* 6. 9 Hz, 3 H, OCH₂C*H*₃), 1. 64-1. 71(m, 2 H, CH₂C*H*₂CH₃), 2. 32 (s, 3 H, CH₃), 2. 71(t, *J* = 7. 5 Hz, 2 H, C*H*₂CH₂CH₃), 4. 04 (s, 3 H, NCH₃), 4. 41 (q, *J =* 6. 9 Hz, 2 H, OC*H*₂CH₃), 7. 19 (d, *J* = 9. 0 Hz, 1 H, H-3'), 8. 06 (dd, *J* = 9. 0 Hz, 2. 7 Hz, 1 H, H-4'), 9. 15 (d, *J* = 2. 7 Hz, 1 H, H-6'), 10. 50 (br s, 1 H, NH); MS (FAB) *m*/*z* 424 (MH⁺).

### Preparative Example 60

### Preparation of 2-(5-chlorosulfony-2-n-propoxylphenyl)-5,6-dimethyl-7-n-propyl-3,5-dihydro-4H-pyr rolo[3,2-d]pyrimidin-4-one (a compound of the formula (2) wherein Y = Cl, R¹ = R² = CH₃, R³ = CH₂CH₂CH₃, R⁴ = CH₂CH₂CH₃)

The titled compound was prepared in a slightly impure form as described in Preparative Example 48 by using 5,6-dimethyl-2-(2-*n*-propoxyphenyl)-7-*n*-propyl-3,5-dihydro-4*H*-pyrrolo[3,2-*d*]pyrimi din-4-one in place of 2-(2-ethoxyphenyl)-5-methyl-7-*n*-propyl-3,5-dihydro-4*H*-pyrrolo[3,2-*d*]pyrimidin-4-o ne.
yield: 100% (crude)
¹H NMR (CDCl₃/TMS) δ 0. 97 (t, *J=* 7. 5 Hz, 3 H, CH₂CH₂C*H*₃), 1. 18 (t, *J*= 7. 5 Hz, 3 H, OCH₂CH₂C*H*₃), 1. 64-1. 77 (m, 2 H, CH₂C*H*₂CH₃), 2. 00-2. 10 (m, 2 H, OCH₂C*H*₂CH₃), 2. 33 (s, 3 H, CH₃), 2. 73 (t, *J =* 7. 5 Hz, 2 H, C*H*₂CH₂CH₃), 4. 04 (s, 3 H, NCH₃), 4. 30 (t, *J* = 6. 6 Hz, 2 H, OC*H*₂CH₂CH₃), 7. 20 (d, *J =* 8. 7 Hz, 1 H, H-3'), 8. 07 (dd, *J =* 8. 7 Hz, 2. 4 Hz, 1 H, H-4'), 9. 12 (d, *J =* 2. 4 Hz, 1 H, H-6'), 10. 67 (br s, 1 H, NH).

### Preparative Example 61

### Preparation of 2-chloro-4-(2-ethoxybenzamido)-1-methyl-3-n-propylpyrrole-5-carboxamide (a compound of the formula (12) wherein R¹ = CH₃, R² = Cl, R³ = CH₂CH₂CH₃, R⁴ = CH₂CH₃)

To a stirred solution of 4-(2-ethoxybenzamido)-1-methyl-3-*n*-propylpyrrole-5-carboxamide (1. 02 g, 3. 09 mmol) in CH₂Cl₂ (35 mL) at -20 °C was added *N*-chlorosuccinimide (0. 54 g, 4. 04 mmol) and the mixture was stirred at -10 °C for I h. After standing overnight in a refrigerator (ca. -12 °C), the reaction mixture was stirred at 0 °C for additional 7 h. The reaction was quenched by the addition of dilute Na₂S₂O₃ aqueous solution (3 mL) and water (40 mL), and then the resulting mixture was extracted with CHCl₃ (20 mL × 4). Combined organic layer was dried (MgSO₄), filtered and the filtrate was evaporated to dryness under vacuum to give an oily yellow residue. The crude product was purified by MPLC on silica gel (gradient elution: 1:3 ethyl acetate/CHCl₃ followed by 1:2 ethyl acetate/CHCl₃) to afford the titled compound (0. 72 g, 64%) as a white solid. Analytically pure compound was obtained by crystallization from ethyl acetate/hexanes.
mp 136.5 - 137°C;
IR (neat) 3451, 3333 (NH), 1672, 1657 (C=O) cm⁻¹;
¹H NMR (CDCl₃/TMS) δ 0. 88 (t, *J =* 7. 5 Hz, 3 H, CH₂CH₂C*H*₃), 1. 43-1. 55 (m, 2 H, CH₂C*H*₂CH₃), 1. 52 (t, *J =* 6. 9 Hz, 3 H, OCH₂C*H*₃), 2. 38 (t, *J =* 7. 5 Hz, 2 H, C*H*₂CH₂CH₃), 3. 83 (s, 3 H, NCH₃), 4. 30 (q, *J=* 6. 9 Hz, 2 H, OC*H*₂CH₃), 7. 05 (d, *J* = 8. 4 Hz, 1 H, H-3'), 7. 10-7. 15 (m, I H, H-5'), 7. 49-7. 55 (m, 1 H, H-4'), 8. 29 (dd, *J=* 7. 8 Hz, 1. 8 Hz, 1 H, H-6'), 9. 40 (br s, 1 H, NH); MS (FAB) *m*/*z* 364 (MH⁺).

### Preparative Example 62

### Preparation of 2-chloro-1-methyl-4-(2-n-propoxybenzamido)-3-n-propylpyrrole-5-carboxamide (a compound of the formula (12) wherein R¹ = CH₃, R² = Cl, R³ = CH₂CH₂CH₃, R⁴ = CH₂CH₂CH₃)

The titled compound was prepared as described in Preparative Example 61 by using 1-methyl-4-(2-*n*-propoxybenzamido)-3-*n*-propylpyrrole-5-carboxamide in place of 4-(2-ethoxybenzamido)-1-methyl-3-*n*-propylpyrrole-5-carboxamide.
yield: 82%
mp 139-140 °C (CHCl₃/Et₂O/hexanes);
IR (neat) 3333, 3155 (NH), 1650 (C=O) cm⁻¹;
¹H NMR (CDCl₃/TMS) δ 0. 87 (t, *J=* 7. 2 Hz, 3 H, CH₂CH₂C*H*₃), 1. 05(t, *J* = 7. 5 Hz, 3 H, OCH₂CH₂C*H*₃), 1. 42-1. 54 (m, 2 H, CH₂C*H*₂CH₃), 1. 85-1. 96 (m, 2 H, OCH₂C*H*₂CH₃), 2. 37 (t, *J* = 7. 5 Hz, 2 H, C*H*₂CH₂CH₃), 3. 84(s, 3 H, NCH₃), 4. 18 (t, *J* = 6*.* 6 Hz, 2 H, OC*H*₂CH₂CH₃), 7. 05 (d, *J* = 8. 4 Hz, 1 H, H-3'), 7. 13(t, *J* = 7. 8 Hz, 1 H, H-5'), 7. 49-7. 55 (m, 1 H, H-4'), 8. 29(dd, *J*= 7. 8 Hz, 1. 8 Hz, 1 H, H-6'), 9. 39 (br s, I H, NH); MS (FAB) *m*/*z* 378 (MH⁺).

### Preparative Example 63

### Preparation of 6-chloro-2-(2-ethoxyphenyl)-5-methyl-7-n-propyl-3,5-dihydro-4H-pyrrolo[3,2-d]pyri midin-4-one (a compound of the formula (9) wherein R' = CH₃, R² = Cl, R³ = CH₂CH₂CH₃, R⁴ = CH₂CH₃)

The titled compound was prepared as described in Preparative Example 51 by using 2-chloro-4-(2-ethoxybenzamido)-1-methyl-3-*n*-propylpyrrole-5-carboxamide in place of 4-(2-(2-fluoroethoxy)benzamido)-1-methyl-3-*n*-propylpyrrole-5-carboxamide.
yield: 70%
mp 145. 5-146 °C (ethyl acetate/hexanes);
IR (neat) 3309 (NH), 1678 (C=O) cm⁻¹;
¹H NMR (CDCl₃/TMS) δ 0. 98 (t, *J =* 7. 5 Hz, 3 H, CH₂CH₂C*H*₃), 1. 59 (t, *J=* 6. 9 Hz, 3 H, OCH₂C*H*₃), 1. 68-1. 81 (m, 2 H, CH₂C*H*₂CH₃), 2. 72 (t, *J =* 7. 5 Hz, 2 H, C*H*₂CH₂CH₃), 4. 07 (s, 3 H, NCH₃), 4. 27 (q, *J =* 6. 9 Hz, 2 H, OC*H*₂CH₃), 7. 02 (d, *J* = 8. 1 Hz, 1 H, H-3'), 7. 10-7. 15 (m, 1 H, H-5'), 7. 39-7. 45 (m, 1 H, H-4'), 8. 49 (dd, *J* = 8. 1 Hz, 1. 8 Hz, I H, H-6'), 11. 00 (br s, 1 H, NH); MS (FAB) *m*/*z* 346 (MH⁺).

### Preparative Example 64

### Preparation of 6-chloro-5-methyl-2-(2-n-propoxyphenyl)-7-n-propyl-3,5-dihydro-4H-pyrrolo[3,2-d]p yrimidin-4-one (a compound of the formula (9) wherein R¹ = CH₃, R² = Cl, R³ = CH₂CH₂CH₃, R⁴ = CH₂CH₂CH₃)

The titled compound was prepared as described in Preparative Example 51 by using 2-chloro-1-methyl-4-(2-*n*-propoxybenzamido)-3-*n*-propylpyrrole-5-carboxamide in place of 4-(2-(2-fluoroethoxy)benzamido)-1-methyl-3-*n*-propylpyrrole-5-carboxamide.
yield: 91%
mp 117-117. 5 °C (Et₂O/hexanes);
IR (neat) 3315 (NH), 1687 (C=O) cm⁻¹;
¹H NMR (CDCl₃/TMS) δ 0. 98 (t, *J=* 7. 2 Hz, 3 H, CH₂CH₂C*H*₃), 1. 17 (t, *J=* 7. 5 Hz, 3 H, OCH₂CH₂C*H*₃), 1. 68-1. 80 (m, 2 H, CH₂C*H*₂CH₃), 1. 95-2. 06 (m, 2 H, OCH₂C*H*₂CH₃), 2. 73 (t, *J=* 7. 5 Hz, 2 H, C*H*₂CH₂CH₃), 4. 07 (s, 3 H, NCH₃), 4. 17 (t, *J =* 6. 6 Hz, 2 H, OC*H*₂CH₂CH₃), 7. 03 (d, *J* = 8. 4 Hz, 1 H, H-3'), 7. 13 (td, *J* = 8. 1 Hz, 1. 2 Hz, 1 H, H-5'), 7.40-7.46 (m, 1 H, H-4'), 8. 50 (dd, *J =* 8. 1 Hz, 1. 8 Hz, 1 H, H-6'), 11. 03 (br s, 1 H, NH); MS (FAB) *m*/*z* 360 (MH⁺).

### Preparative Example 65

### Preparation of 6-chloro-2-(5-chlorosulfonyl-2-ethoxyphenyl)-5-methyl-7-n-propyl-3,5-dihydro-4H-p yrrolo[3,2-d]pyrimidin-4-one (a compound of the formula (2) wherein R¹ = CH₃, R² = Y = Cl, R³ = CH₂CH₂CH₃, R⁴ = CH₂CH₃)

The titled compound was prepared as described in Preparative Example 48 by using 6-chloro-2-(2-ethoxyphenyl)-5-methyl-7-*n*-propyl-3,5-dihydro-4*H*-pyrrolo[3,2-*d*]pyri midin-4-one in place of 2-(2-ethoxyphenyl)-5-methyl-7-*n*-propyl-3,5-dihydro-4*H*-pyrrolo[3,2-*d*]pyrimidin-4-o ne.
yield: 96%
mp 164 °C dec (Et₂O/hexanes);
IR (neat) 3343 (NH), 1691 (C=O), 1175 (SO₂) cm⁻¹;
¹H NMR (CDCl₃/TMS) δ 0. 98 (t, *J* = 7. 5 Hz, 3 H, CH₂CH₂C*H*₃), 1. 66 (t, *J =* 6. 9 Hz, 3 H, OCH₂C*H*₃), 1. 67-1. 79 (m, 2 H, CH₂C*H*₂CH₃), 2. 75 (t, *J* = 7. 5 Hz, 2 H, C*H*₂CH₂CH₃), 4. 08 (s, 3 H, NCH₃), 4. 42 (q, *J* = 6. 9 Hz, 2 H, OC*H*₂CH₃), 7. 21(d, *J* = 9. 0 Hz, 1 H, H-3'), 8. 08 (dd, *J =* 9. 0 Hz, 2. 4 Hz, 1 H, H-4'), 9. 12 (d, *J =* 2. 4 Hz, 1 H, H-6'), 10. 65 (br s, 1 H, NH); MS (FAB) *m*/*z* 426 (M⁺ - H₂O).

### Preparative Example 66

### Preparation of 6-chloro-2-(5-chlorosulfonyl-2-n-propoxyphenyl)-5-methyl-7-n-propyl-3,5-dihydro-4 H-pyrrolo[3,2-d]pyrimidin-4-one (a compound of the formula (2) wherein R¹ = CH₃, R² = Y = Cl, R³ = CH₂CH₂CH₃, R⁴ = CH₂CH₂CH₃)

The titled compound was prepared as described in Preparative Example 48 by using 6-chloro-5-methyl-2-(2-*n*-propoxyphenyl)-7-*n*-propyl-3,5-dihydro-4*H*-pyrrolo[3,2-*d*]p yrimidin-4-one in place of 2-(2-ethoxyphenyl)-5-methyl-7-*n*-propyl-3,5-dihydro-4*H*-pyrrolo[3,2-*d*]pyrimidin-4-o ne.
yield: 78%
mp 172. 5 °C dec (CHCl₃/Et₂O/hexanes);
IR (neat) 3330 (NH), 1703 (C=O), 1182 (SO₂) cm⁻¹;
¹H NMR (CDCl₃/TMS) δ 0. 99 (t, *J* = 7. 5 Hz, 3 H, CH₂CH₂C*H*₃), 1. 19 (t, *J* = 7. 5 Hz, 3 H, OCH₂CH₂C*H*₃), 1. 67-1. 82 (m, 2 H, CH₂C*H*₂CH₃), 1. 99-2. 13 (m, 2 H, OCH₂C*H*₂CH₃), 2. 75 (t, *J =* 7. 5 Hz, 2 H, C*H*₂CH₂CH₃), 4. 08 (s, 3 H, NCH₃), 4. 31 (t, *J* = 6. 6 Hz, 2 H, OC*H*₂CH₂CH₃), 7. 22 (d, *J* = 8. 7 Hz, 1 H, H-3'), 8. 09 (dd, *J* = 8. 7 Hz, 2. 4 Hz, 1 H, H-4'), 9. 13 (d, *J* = 2. 4 Hz, 1 H H-6'), 11. 40 (br s, 1 H, NH); MS (FAB) *m*/*z* 440 (M⁺ - H₂O).

### Preparative Example 67

### Preparation of 2-bromo-4-(2-ethoxybenzamido)-1-methyl-3-n-propylpyrrole-5-carboxamide (a compound of the formula (12) wherein R¹ = CH₃, R² = Br, R³ = CH₂CH₂CH₃, R⁴ = CH₂CH₃)

To a stirred solution of 4-(2-ethoxybenzamido)-1-methyl-3-*n*-propylpyrrole-5-carboxamide (636 mg, 1, 93 mmol) and sodium acetate (238 mg, 2. 90 mmol) in acetic acid (13 mL) at room temperature was added dropwise bromine (119 µL, 2. 32 mmol) in acetic acid (6. 5 mL) over a period of 10 minutes, and the mixture was stirred for 10 minutes. The reaction mixture was diluted with water (10 mL), and was extracted with Et₂O (10 mL × 4). Combined organic layer was dried (Na₂SO₄), filtered and the filtrate was evaporated to dryness under vacuum. The crude residue was purified by MPLC on silica gel (gradient elution: 1% MeOH in CHCl₃ followed by 3% MeOH in CHCl₃) to afford the tided compound (544 mg, 69%) as a white solid. Analytically pure compound was obtained by crystallization from CHCl₃/ Et₂O/hexanes.
mp 152. 5 - 153. 5 °C;
IR (neat) 3453, 3192 (NH), 1663, 1644 (C=O) cm⁻¹;
¹H NMR (CDCl₃/TMS) δ 0. 88 (t, *J* = 7. 5 Hz, 3 H, CH₂CH₂C*H*₃), 1. 43-1. 58 (m, 2 H, CH₂C*H*₂CH₃), 1. 51 (t, *J =* 6. 9 Hz, 3 H, OCH₂C*H*₃), 2. 38 (t, *J* = 7. 5 Hz, 2 H, C*H*₂CH₂CH₃), 3. 86 (s, 3 H, NCH₃), 4. 30 (q, *J* = 6. 9 Hz, 2 H, OC*H*₂CH₃), 7. 04 (d, *J* = 8. 1 Hz, 1 H, H-3'), 7. 09-7. 14 (m, 1 H, H-5'), 7. 48-7. 54 (m, 1 H, H-4'), 8. 28 (dd, *J =* 7. 8 Hz, 1. 8 Hz, 1 H, H-6'), 9. 37 (br s, 1 H, NH); MS (FAB) *m*/*z* 408 (M⁺).

### Preparative Example 68

### Preparation of 2-bromo-1-methyl-4-(2-n-propoxybenzamido)-3-n-propylpyrrole-5-carboxamide (a compound of the formula (12) wherein R' = CH₃, R² = Br, R³ = CH₂CH₂CH₃, R⁴ = CH₂CH₂CH₃)

The titled compound was prepared as described in Preparative Example 68 by using 1-methyl-4-(2-*n*-propoxybenzamido)-3-*n*-propylpyrrole-5-carboxamide in place of 4-(2-ethoxybenzamido)-1-methyl-3-*n*-propylpyrrole-5-carboxamide.
yield: 85%
mp 142-143 °C (CHCl₃/Et₂O/hexanes);
IR (neat) 3332, 3149 (NH), 1648 (C=O) cm⁻¹;
¹H NMR (CDCl₃/TMS) δ 0. 87 (t, *J* = 7. 5 Hz, 3 H, CH₂CH₂C*H*₃), 1. 05 (t, *J* = 7. 2 Hz, 3 H, OCH₂CH₂C*H*₃), 1. 42-1. 58 (m, 2 H, CH₂C*H*₂CH₃), 1. 84-1. 96 (m, 2 H, OCH₂C*H*₂CH₃), 2. 37 (t, *J =* 7. 5 Hz, 2 H, C*H*₂CH₂CH₃), 3. 86 (s, 3 H, NCH₃), 4. 18 (t, *J =* 6. 6 Hz, 2 H, OC*H*₂CH₂CH₃), 7. 05 (d, *J* = 8. 4 Hz, 1 H, H-3'), 7. 12 (td, *J =* 8. 1 Hz, 0. 9 Hz, I H, H-5'), 7. 49-7. 55 (m, 1 H, H-4'), 8. 29 (dd, *J=* 8. 1 Hz, 1. 8 Hz, I H, H-6'), 9. 41 (br s, I H, NH); MS (FAB) *m*/*z* 422 (M⁺).

### Prenarative Example 69

### Preparation of 6-bromo-2-(2-ethoxyphenyl)-5-methyl-7-n-propyl-3,5-dihydro-4H-pyrrolo[3,2-d]pyri midin-4-one (a compound of the formula (9) wherein R¹ = CH₃, R² = Br, R³ = CH₂CH₂CH₃, R⁴ = CH₂CH₃)

The titled compound was prepared as described in Preparative Example 51 by using 2-bromo-4-(2-ethoxybenzamido)-1-methyl-3-*n*-propylpyrrole-5-carboxamide in place of 4-(2-(2-fluoroethoxy)benzamido)-1-methyl-3-*n*-propylpyrrole-5-carboxamide.
yield: 100%
mp 152. 5-153 °C (ethyl acetate/hexanes);
IR (neat) 3311 (NH), 1679 (C=O) cm⁻¹;
¹H NMR (CDCl₃/TMS) δ 0. 98 (t, *J* = 7. 5 Hz, 3 H, CH₂CH₂C*H*₃), 1. 59 (t, *J* = 6. 9 Hz, 3 H, OCH₂C*H*₃), 1. 69-1. 81 (m, 2 H, CH₂C*H*₂CH₃), 2. 72 (t, *J* = 7. 5 Hz, 2 H, C*H*₂CH₂CH₃), 4. 09 (s, 3 H, NCH₃), 4. 28 (q, *J =* 6. 9 Hz, 2 H, OC*H*₂CH₃), 7. 02 (d, *J* = 8. 4 Hz, 1 H, H-3'), 7. 09-7. 15 (m, 1 H, H-5'), 7. 39-7. 45 (m, 1 H, H-4'), 8. 49 (dd, *J =* 7. 8 Hz, 1. 8 Hz, 1 H, H-6'), 10. 96 (br s, 1 H, NH); MS (FAB) *m*/*z* 311 (MH⁺ - Br).

### Preparative Example 70

### Preparation of 6-bromo-5-methyl-2-(2-n-propoxyphenyl)-7-n-propyl-3,5-dihydro-4H-pyrrolo[3,2-d]p yrimidin-4-one (a compound of the formula (9) wherein R¹ = CH₃, R² = Br, R³ = CH₂CH₂CH₃, R⁴ = CH₂CH₂CH₃)

The titled compound was prepared as described in Preparative Example 51 by using 2-bromo-1-methyl-4-(2-*n*-propoxybenzamido)-3-*n*-propylpyrrole-5-carboxamide in place of 4-(2-(2-fluoroethoxy)benzamido)-1-methyl-3-*n*-propylpyrrole-5-carboxamide.
yield: 90%
mp 110-112 °C (Et₂O/hexanes);
IR (neat) 3195 (NH), 1665 (C=O) cm⁻¹;
¹H NMR (CDCl₃/TMS) δ 0. 99 (t, *J=* 7. 5 Hz, 3 H, CH₂CH₂C*H*₃), 1. 17 (t, *J* = 7. 2 Hz, 3 H, OCH₂CH₂C*H*₃), 1. 68-1. 81 (m, 2 H, CH₂C*H*₂CH₃), 1. 94-2. 06 (m, 2 H, OCH₂C*H*₂CH₃), 2. 72 (t, *J =* 7. 5 Hz, 2 H, C*H*₂CH₂CH₃), 4. 09 (s, 3 H, NCH₃), 4. 17 (t, *J =* 6. 6 Hz, 2 H, OC*H*₂CH₂CH₃), 7. 03 (d, *J* = 8. 1 Hz, 1 H, H-3'), 7. 13 (td, *J =* 8. 1 Hz, 0. 9 Hz, 1 H, H-5'), 7. 40-7. 46 (m, 1 H, H-4'), 8. 50 (dd, *J =* 8. 1 Hz, 1. 8 Hz, 1 H, H-6'), 11. 02 (br s, 1 H, NH); MS (FAB) *m*/*z* 404 (M⁺).

### Preparative Example 71

### Preparation of 6-bromo-2-(5-chlorosulfonyl-2-ethoxyphenyl)-5-methyl-7-n-propyl-3,5-dihydro-4H-p yrrolo[3,2-d]pyrimidin-4-one (a compound of the formula (2) wherein Y = Cl, R¹ = CH₃, R² = Br, R³ = CH₂CH₂CH₃, R⁴ = CH₂CH₃)

The titled compound was prepared as described in Preparative Example 48 by using 6-bromo-2-(2-ethoxyphenyl)-5-methyl-7-*n*-propyl-3,5-dihydro-4*H*-pyrrolo[3,2-*d*]pyri midin-4-one in place of 2-(2-ethoxyphenyl)-5-methyl-7-*n*-propyl-3,5-dihydro-4*H*-pyrrolo[3,2-*d*]pyrimidin-4-o ne.
yield: 91 %
mp 191-192 °C (Et₂O/hexanes);
IR (neat) 3331 (NH), 1696 (C=O), 1178 (SO₂) cm⁻¹;
¹H NMR (CDCl₃/TMS) δ 0. 99 (t, J= 7. 5 Hz, 3 H, CH₂CH₂C*H*₃), 1. 66 (t, *J* = 6. 9 Hz, 3 H, OCH₂C*H*₃), 1. 67-1. 80 (m, 2 H, CH₂C*H*₂CH₃), 2. 75 (t, *J* = 7. 5 Hz, 2 H, C*H*₂CH₂CH₃), 4. 10 (s, 3 H, NCH₃), 4. 42 (q, *J =* 6. 9 Hz, 2 H, OC*H*₂CH₃), 7. 21(d, *J* = 8. 7 Hz, 1 H, H-3'), 8. 08 (dd, *J=* 8. 7 Hz, 2. 7 Hz, I H, H-4'), 9. 12 (d, *J=* 2. 7 Hz, 1 H, H-6'), 10. 66 (br s, 1 H, NH); MS (FAB) *m*/*z* 470, 472 (M⁺ - H₂O).

### Preparative Example 72

### Preparation of 6-bromo-2-(5-chlorosulfonyl-2-n-propoxyphenyl)-5-methyl-7-n-propyl-3,5-dihydro-4 H-pyrrolo[3,2-d]pyrimidin-4-one (a compound of the formula (2) wherein Y = Cl, R¹ = CH₃, R² = Br, R³ = CH₂CH₂CH₃, R⁴ = CH₂CH₂CH₃)

The titled compound was prepared as described in Preparative Example 48 by using 6-bromo-5-methyl-2-(2-*n*-propoxyphenyl)-7-*n*-propyl-3,5-dihydro-4*H*-pyrrolo[3,2-*d*]p yrimidin-4-one in place of 2-(2-ethoxyphenyl)-5-methyl-7-*n*-propyl-3,5-dihydro-4*H*-pyrrolo[3,2-*d*]pyrimidin-4-o ne.
yield: 84%
mp 178 °C dec (CHCl₃/hexanes);
IR (neat) 3325 (NH), 1691 (C=O), 1178 (SO₂) cm⁻¹;
¹H NMR (CDCl₃/TMS) δ 0. 99 (t, *J=* 7. 5 Hz, 3 H, CH₂CH₂C*H*₃), 1. 19 (t, *J=* 7. 5 Hz, 3 H, OCH₂CH₂C*H*₃), 1. 67-1. 80 (m, 2 H, CH₂C*H*₂CH₃), 2. 00-2. 12 (m, 2 H, OCH₂C*H*₂CH₃), 2. 75 (t, *J* = 7. 5 Hz, 2 H, C*H*₂CH₂CH₃), 4. 10 (s, 3 H, NCH₃), 4. 30 (t, *J =* 6. 3 Hz, 2 H, OC*H*₂CH₂CH₃), 7. 22 (d, *J =* 9. 0 Hz, 1 H, H-3'), 8. 08 (dd, *J =* 9. 0 Hz, 2. 7 Hz, 1 H, H-4'), 9. 14 (d, *J =* 2. 7 Hz, 1 H, H-6'), 10. 66 (br s, 1 H, NH); MS (FAB) *m*/*z* 484, 486 (M⁺ - H₂O).

### Preparative Example 73

### Preparation of 4-(2-ethoxybenzamido)-2-iodo-1-methyl-3-n-propylpyrrole-5-carboxamide (a compound of the formula (12) wherein R¹ = CH₃, R² = I, R³ = CH₂CH₂CH₃, R⁴ = CH₂CH₃)

To a stirred solution of 4-(2-ethoxybenzamido)-1-methyl-3-*n*-propylpyrrole-5-carboxamide (1. 10 g, 3. 34 mmol) in benzene (30 mL) at 0 °C was added portionwise iodine (0. 93 g, 3. 67 mmol) and mercury oxide (0. 62 g, 2. 84 mmol) alternately, and the mixture was stirred for 2 h at 0 °C. The reaction mixture was diluted with ethyl acetate (100 mL), washed once with dilute Na₂S₂O₃ aqueous solution (100 mL), and the aqueous layer was further extracted with ethyl acetate (100 mL). The combined organic layer was dried (MgSO₄), filtered and the filtrate was evaporated to dryness under vacuum. The crude residue was purified by MPLC on silica gel (20% ethyl acetate in CHCl₃) to afford the titled compound (1. 51 g, 99%) as a white solid. Analytically pure compound was obtained by crystallization from ethyl acetate/hexanes.
mp 157. 5 - 158 °C;
IR (neat) 3338, 3186 (NH), 1660 (C=O) cm⁻¹;
¹H NMR (CDCl₃/TMS) δ 0. 90 (t, *J* = 7. 2 Hz, 3 H, CH₂CH₂C*H*₃), 1. 42-1. 54 (m, 2 H, CH₂C*H*₂CH₃), 1. 52 (t, *J* = 6. 9 Hz, 3 H, OCH₂C*H*₃), 2. 37 (t, *J =* 7. 5 Hz, 2 H, C*H*₂CH₂CH₃), 3. 87 (s, 3 H, NCH₃), 4. 30 (q, *J=* 6. 9 Hz, 2 H, OC*H*₂CH₃), 7. 05 (d, *J* = 8. 1 Hz, I H, H-3'), 7. 10-7. 15 (m, 1 H, H-5'), 7. 49-7. 55 (m, 1 H, H-4'), 8. 29 (dd, *J=* 7. 8 Hz, 2. 1 Hz, 1 H, H-6'), 9. 43 (br s, I H, NH); MS (FAB) *m*/*z* 456 (MH⁺).

### Preparative Example 74

### Preparation of 2-iodo-1-methyl-4-(2-n-propoxybenzamido)-3-n-propylpyrrole-5-carboxamide (a compound of the formula (12) wherein R¹ = CH₃, R² = I, R³ = CH₂CH₂CH₃, R⁴ = CH₂CH₂CH₃)

The titled compound was prepared as described in Preparative Example 74 by using 1-methyl-4-(2-*n*-propoxybenzamido)-3-*n*-propylpyrrole-5-carboxamide in place of 4-(2-ethoxybenzamido)-1-methyl-3-*n*-propylpyrrole-5-carboxamide.
yield: 82%
mp 169. 5-170 °C (ethyl acetate/hexanes);
IR (neat) 3340, 3146 (NH), 1642 (C=O) cm⁻¹;
¹H NMR (CDCl₃/TMS) 8 0. 88 (t, *J =* 7. 5 Hz, 3 H, CH₂CH₂C*H*₃), 1. 05 (t, *J* = 7. 5 Hz, 3 H, OCH₂CH₂C*H*₃), 1. 40-1. 53 (m, 2 H, CH₂C*H*₂CH₃), 1. 84-1. 96 (m, 2 H, OCH₂C*H*₂CH₃), 2. 36 (t, *J* = 7. 5 Hz, 2 H, C*H*₂CH₂CH₃), 3. 86 (s, 3 H, NCH₃), 4. 18 (t, *J =* 6. 9 Hz, 2 H, OC*H*₂CH₂CH₃), 7. 05 (d, *J* = 8. 4 Hz, 1 H, H-3'), 7. 09-7. 15 (m, 1 H, H-5'), 7. 52 (ddd, *J=* 8. 4 Hz, 7. 5 Hz, 1. 8 Hz, 1 H, H-4'), 8. 28 (dd, *J=* 8. 0 Hz, 1. 8 Hz, 1 H, H-6'), 9. 42 (br s, 1 H, NH); MS (FAB) *m*/*z* 470 (MH⁺).

### Preparative Example 75

### Preparation of 2-(2-ethoxyphenyl)-6-iodo-5-methyl-7-n-propyl-3,5-dihydro-4H-pyrrolo[3,2-d]pyrimi din-4-one (a compound of the formula (9) wherein R¹ = CH₃, R² = I, R³ = CH₂CH₂CH₃, R⁴ = CH₂CH₃)

The titled compound was prepared as described in Preparative Example 51 by using 4-(2-ethoxybenzamido)-2-iodo-1-methyl-3-*n*-propylpyrrole-5-carboxamide in place of 4-(2-(2-fluoroethoxy)benzamido)-1-methyl-3-*n*-propylpyrrole-5-carboxamide.
yield: 81 %
mp 178-178. 5 °C (Et₂O/hexanes);
IR (neat) 3309 (NH), 1667 (C=O) cm⁻¹;
¹H NMR (CDCl₃/TMS) δ 0. 99 (t, *J=* 7. 2 Hz, 3 H, CH₂CH₂C*H*₃), 1. 60 (t, *J=* 6. 9 Hz, 3 H, OCH₂C*H*₃), 1. 67-1. 79 (m, 2 H, CH₂C*H*₂CH₃), 2. 72 (t, *J* = 7. 5 Hz, 2 H, C*H*₂CH₂CH₃), 4. 10 (s, 3 H, NCH₃), 4. 28 (q, *J=* 6. 9 Hz, 2 H, OC*H*₂CH₃), 7. 02 (d, *J* = 8. 4 Hz, 1 H, H-3'), 7. 13 (td, *J=* 8. 4 Hz, 1. 2 Hz, 1 H, H-5'), 7. 39-7. 45 (m, 1 H, H-4'), 8. 49 (dd, *J =* 7. 8 Hz, 1. 8 Hz, 1 H, H-6'), 10. 96 (br s, 1 H, NH); MS (FAB) *m*/*z* 438 (MH⁺).

### Preparative Example 76

### Preparation of 6-iodo-5-methyl-2-(2-n-propoxyphenyl)-7-n-propyl-3,5-dihydro-4H-pyrrolo[3,2-d]pyr imidin-4-one (a compound of the formula (9) wherein R¹ = CH₃, R² = I, R³ = CH₂CH₂CH₃, R⁴ = CH₂CH₂CH₃)

The titled compound was prepared as described in Preparative Example 51 by using 2-iodo-1-methyl-4-(2-*n*-propoxybenzamido)-3-*n*-propylpyrrole-5-carboxamide in place of 4-(2-(2-fluoroethoxy)benzamido)-1-methyl-3-*n*-propylpyrrole-5-carboxamide.
yield: 98%
mp 141-142 °C (CHCl₃/hexanes);
IR (neat) 3320 (NH), 1678 (C=O) cm⁻¹;
¹H NMR (CDCl₃/TMS) 8 1. 00 (t, *J* = 7. 5 Hz, 3 H, CH₂CH₂C*H*₃), 1. 17 (t, *J* = 7. 5 Hz, 3 H, OCH₂CH₂C*H*₃), 1. 67-1. 79 (m, 2 H, CH₂C*H*₂CH₃), 1. 94-2. 06 (m, 2 H, OCH₂C*H*₂CH₃), 2. 72 (t, *J* = 7. 5 Hz, 2 H, C*H*₂CH₂CH₃), 4. 10 (s, 3 H, NCH₃), 4. 17 (t, *J* = 6. 6 Hz, 2 H, OC*H*₂CH₂CH₃), 7. 03 (d, *J* = 8. 4 Hz, I H, H-3'), 7. 10- 7. 15 (m, 1 H, H-5'), 7. 40-7. 45 (m, 1 H, H-4'), 8. 50 (dd, *J* = 7. 8 Hz, 1. 8 Hz, 1 H, H-6'), 10. 98 (br s, 1 H, NH); MS (FAB) *m*/*z* 452 (MH⁺).

### Preparative Example 77

### Preparation of 2-(5-chlorosulfonyl-2-ethoxyphenyl)-6-iodo-5-methyl-7-n-propyl-3,5-dihydro-4H-pyrr olo[3,2-d]pyrimidin-4-one (a compound of the formula (2) wherein Y = Cl, R¹ = CH₃, R² = I, R³ = CH₂CH₂CH₃, R⁴ = CH₂CH₃)

The titled compound was prepared as described in Preparative Example 48 by using 2-(2-ethoxyphenyl)-6-iodo-5-methyl-7-*n*-propyl-3,5-dihydro-4*H*-pyrrolo[3,2-*d*]pyrimi din-4-one in place of 2-(2-ethoxyphenyl)-5-methyl-7-*n*-propyl-3,5-dihydro-4*H*-pyrrolo[3,2-*d*]pyrimidin-4-o ne.
yield: 75%
mp 197 °C dec (CHCl₃/hexanes);
IR (neat) 3327 (NH), 1670(C=O), 1174 (SO₂) cm⁻¹;
¹H NMR (CDCl₃/TMS) δ 1. 00 (t, *J* = 7. 5 Hz, 3 H, CH₂CH₂C*H*₃), 1. 66 (t, *J* = 6. 9 Hz, 3 H, OCH₂C*H*₃), 1. 67-1. 79 (m, 2 H, CH₂C*H*₂CH₃), 2. 74 (t, *J* = 7. 5 Hz, 2 H, C*H*₂CH₂CH₃), 4. 11 (s, 3 H, NCH₃), 4. 42 (q, *J =* 6. 9 Hz, 2 H, OC*H*₂CH₃), 7. 2 1 (d, *J* = 8. 7 Hz, I H, H-3'), 8. 08 (dd, *J* = 8. 7 Hz, 2. 7 Hz, 1 H, H-4'), 9. 13 (d, *J* = 2. 7 Hz, 1 H, H-6'), 10. 58 (br s, 1 H, NH); MS (FAB) *m*/*z* 518 (MH⁺ - H₂O).

### Preparative Example 78

### Preparation of 2-(5-chlorosulfonyl-2-n-propoxyphenyl)-6-iodo-5-methyl-7-n-propyl-3,5-dihydro-4H-pyrrolo[3,2-d]pyrimidin-4-one (a compound of the formula (2) wherein Y = Cl, R¹ = CH₃, R² = I, R³ = CH₂CH₂CH₃, R⁴ = CH₂CH₂CH₃)

The titled compound was prepared as described in Preparative Example 48 by using 6-iodo-5-methyl-2-(2-*n*-propoxyphenyl)-7-*n*-propyl-3,5-dihydro-4*H*-pyrrolo[3,2-*d*]pyr imidin-4-one in place of 2-(2-ethoxyphenyl)-5-methyl-7-*n*-propyl-3,5-dihydro-4*H*-pyrrolo[3,2-*d*]pyrimidin-4-o ne.
yield: 93%
mp 177 °C dec (CHCl₃/Et₂O);
IR (neat) 3323 (NH), 1679 (C=O), 1175 (SO₂) cm⁻¹;
¹H NMR (CDCl₃/TMS) δ 1. 00 (t, *J* = 7.5 Hz, 3 H, CH₂CH₂C*H*₃), 1. 19 (t, *J* = 7. 5 Hz, 3 H, OCH₂CH₂C*H*₃), 1. 67-1. 79 (m, 2 H, CH₂C*H*₂CH₃), 2. 00-2. 12 (m, 2 H, OCH₂C*H*₂CH₃), 2. 74 *(t, J=* 7. 5 Hz, 2 H, C*H*₂CH₂CH₃), 4. 11 (s, 3 H, NCH₃), 4. 30 (t, *J* = 6. 3 Hz, 2 H, OC*H*₂CH₂CH₃), 7. 21 (d, *J* = 9. 0 Hz, 1 H, H-3'), 8. 08 (dd, *J* = 9. 0 Hz, 2. 4 Hz, 1 H, H-4'), 9. 14 (d, *J =* 2. 4 Hz, 1 H, H-6'), 10. 61 (br s, 1 H, NH); MS (FAB) *m*/*z* 532 (MH⁺ - H₂O).

### Preparative Example 79

### Preparation of 2-(5-(4-(cyanomethyl)piperidin-1-ylsulfonyl)-2-ethoxyphenyl)-5-methyl-7-n-propyl-3, 5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(cyanomethyl)piperidin-1-yl)

To a mixture of 2-(5-chlorosulfonyl-2-ethoxyphenyl)-5-methyl-7-*n*-propyl-3,5-dihydro-4*H*-pyrrolo[3,2 - *d*]pyrimidin-4-one (200 mg, 0. 49 mmol) and 4-(cyanomethyl)piperidine trifluoroacetic acid (139 m g, 0. 59 mmol) in anhydrous dichloromethane (15 mL) in an ice bath was added triethylamine (0. 20 mL, 1. 46 mmol), and the mixture was stirred at 0 °C under nitrogen atmosphere for 2 h. The reaction mixture was evaporated to dryness under reduced pressure, and the resulting oily residue was purified by MPLC on silica gel (gradient elution: 4:1 ethyl acetate/hexanes followed by 2% MeOH in CHCl₃) to afford the titled compound (217 mg, 89%) as a yellow solid. Analytically pure compound was obtained by crystallization from EtOH/CHCl₃.
mp 217. 5-218 °C;
IR (neat) 3325 (NH), 2246 (CN), 1691 (C=O), 1164 (SO₂) cm⁻¹;
¹H NMR (CDCl₃/TMS) δ 1. 00 (t, *J=* 7. 2 Hz, 3 H, CH₂CH₂C*H*₃), 1. 42-1. 68 (m, 3 H, CH and 2 CHₐₓ), 1. 61 (t, *J =* 7. 2 Hz, 3 H, OCH₂C*H*₃), 1. 68-1. 80 (m, 2 H, CH₂C*H*₂CH₃), 1.84-1.93 (m, 2 H, 2 CH_{eq}), 2. 29 (d, *J =* 6. 6 Hz, 2 H, CH₂CN), 2. 40 (td, *J=* 11. 7 Hz, 2. 1 Hz, 2 H, 2 NCHₐₓ), 2. 71 (t, *J=* 7.5 Hz, 2 H, C*H*₂CH₂CH₃), 3. 91 (br d, *J=* 11. 7 Hz, 2 H, 2 NCH_{eq}), 4. 08 (s, 3 H, NCH₃), 4. 36 (q, *J=* 7. 2 Hz, 2 H, OC*H*₂CH₃), 6. 89 (s, 1 H, H-2), 7. 13 (d, *J=* 8. 7 Hz, 1 H, H-3'), 7. 82 (dd, *J=* 8. 7 Hz, 2. 7 Hz, 1 H, H-4'), 8. 85 (d, *J =* 2. 7 Hz, 1 H, H-6'), 10. 62 (br s, 1 H, NH); MS (FAB) *m*/*z* 498 (MH⁺).

### Preparative Example 80

### Preparation of 2-(5-(4-(cyanomethyl)piperidin-1-ylsulfonyl)-2-n-propoxyphenyl)-5-methyl-7-n-propy 1-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(cyanomethyl)piperidin-1-yl)

The titled compound was prepared as described in Preparative Example 80 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one in place of 2-(5-chlorosulfonyl-2-ethoxyphenyl)-5-methyl-7-*n*-propyl-3,5-dihydro-4*H*-pyrrolo[3,2 -*d*]pyrimidin-4-one.
yield: 96%
mp 194-194. 5 °C (EtOAc/hexanes);
IR (neat) 3304 (NH), 2245 (CN), 1691 (C=O), 1166 (SO₂) cm⁻¹;
¹H NMR (CDCl₃/TMS) δ 1. 00 (t, *J=* 7. 5 Hz, 3 H, CH₂CH₂C*H*₃), 1. 18 (t, *J*= 7. 5 Hz, 3 H, OCH₂CH₂C*H*₃), 1. 41-1. 59 (m, 2 H, 2 CHₐₓ), 1. 60-1. 80 (m, 3 H, CH and CH₂C*H*₂CH₃), 1. 89 (br d, *J* = 11. 4 Hz, 2 H, 2 CH_{eq}), 1. 98-2. 10 (m, 2 H, OCH₂C*H*₂CH₃), 2. 29 (d, *J =* 6. 6 Hz, 2 H, CH₂CN), 2. 40 (td, *J=* 12. 0 Hz, 2. 4 Hz, 2 H, 2 NCHₐₓ), 2. 71 (t, *J* = 7. 5 Hz, 2 H, C*H*₂CH₂CH₃), 3. 91 (br d, *J =* 12. 0 Hz, 2 H, 2 NCH_{eq}), 4. 08 (s, 3 H, NCH₃), 4. 25 (t, *J=* 6. 6 Hz, 2 H, OC*H*₂CH₂CH₃), 6. 89 (s, 1 H, H-2), 7. 14 (d, *J=* 8. 7 Hz, 1 H, H-3'), 7. 82 (dd, *J=* 8. 7 Hz, 2. 4 Hz, 1 H, H-4'), 8. 86 (d, *J=* 2. 4 Hz, 1 H, H-6'), 10. 65 (br s, I H, NH); MS (FAB) *m*/*z* 512 (MH⁺).

### Preparative Example 81

### Preparation of 2-(5-(4-(2-cyanoethyl)piperidin-1-ylsulfonyl)-2-ethoxyphenyl)-5-methyl-7-n-propyl-3, 5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-cyanoethyl)piperidin-1-yl)

The titled compound was prepared as described in Preparative Example 80 by using 4-(2-cyanoethyl)piperidine trifluoroacetic acid in place of 4-(cyanomethyl)piperidine trifluoroacetic acid.
yield: 76%
mp 225-225. 5 °C (EtOH/ CHCl₃);
IR (neat) 3325 (NH), 2243 (CN), 1692 (C=O), 1162 (SO₂) cm⁻¹;
¹H NMR (CDCl₃/TMS) δ 1. 00 (t, *J =* 7. 5 Hz, 3 H, CH₂CH₂C*H*₃), 1. 28-1. 46 (m, 3 H, CH and 2 CHₐₓ), 1. 57-1. 79 (m, 6 H, CH₂C*H*₂CH₃, C*H*₂CH₂CN and 2 CH_{eq}), 1. 64 (t, *J* = 6. 9 Hz, 3 H, OCH₂C*H*₃), 2. 33-2. 39 (m, 4 H, CH₂C*H*₂CN and 2 NCHₐₓ), 2. 71 (t, *J* = 7. 5 Hz, 2 H, C*H*₂CH₂CH₃), 3. 87 (br d, *J =* 11. 7 Hz, 2 H, 2 NCH_{eq}), 4. 08 (s, 3 H, NCH₃), 4. 36 (q, *J =* 6. 9 Hz, 2 H, OC*H*₂CH₃), 6. 89 (s, 1 H, H-2), 7. 13 (d, *J =* 9. 0 Hz, 1 H, H-3'), 7. 81 (dd, *J =* 9. 0 Hz, 2. 4 Hz, 1 H, H-4'), 8. 86 (d, *J =* 2. 4 Hz, 1 H, H-6'), 10. 64 (br s, 1 H, NH); MS (FAB) *m*/*z* 512 (MH⁺).

### Preparative Example 82

### Preparation of 2-(5-(4-(2-cyanoethyl)piperidin-1-ylsulfonyl)-2-n-propoxyphenyl)-5-methyl-7-n-propy 1-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-cyanoethyl)piperidin-1-yl)

The titled compound was prepared as described in Preparative Example 80 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 4-(2-cyanoethyl)piperidine trifluoroacetic acid in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 4-(cyanomethyl)piperidine trifluoroacetic acid.
yield: 96%
mp 179-179. 5 °C (EtOAc/hexanes);
IR (neat) 3330 (NH), 2243 (CN), 1692 (C=O), 1165 (SO₂) cm⁻¹;
¹H NMR (CDCl₃/TMS) δ 1. 00 (t, *J* = 7. 5 Hz, 3 H, CH₂CH₂C*H*₃), 1. 19 (t, *J =* 7. 5 Hz, 3 H, OCH₂CH₂C*H*₃), 1. 30-1. 42 (m, 2 H, 2 CHₐₓ), 1. 57-1. 79 (m, 7 H, CH, C*H*₂CH₂CN, CH₂C*H*₂CH₃ and 2 CH_{eq}), 1. 98-2. 10 (m, 2 H, OCH₂C*H*₂CH₃), 2. 33-2. 39 (m, 2 H, 2 NCHₐₓ), 2. 35 (t, *J* = 7. 2 Hz, 2 H, CH₂C*H*₂CN), 2. 71 (t, *J =* 7. 5 Hz, 2 H, C*H*₂CH₂CH₃), 3. 87 (br d, *J* = 11. 7 Hz, 2 H, 2 NCH_{eq}), 4. 08 (s, 3 H, NCH₃), 4. 25 (t, *J* = 6. 3 Hz, 2 H, OC*H*₂CH₂CH₃), 6. 89 (s, 1 H, H-2), 7. 14 (d, *J =* 8. 7 Hz, 1 H, H-3'), 7. 81 (dd, *J* = 8. 7 Hz, 2. 7 Hz, 1 H, H-4'), 8. 87 (d, *J* = 2. 7 Hz, 1 H, H-6'), 10. 69 (br s, 1 H, NH); MS (FAB) *m*/*z* 526 (MH⁺).

### Preparative Example 83

### Preparation of 2-(2-ethoxy-5-(4-(ethoxycarbonylmethyl)piperidin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(ethoxycarbonylmethyl)piperidin-1-yl)

The titled compound was prepared as described in Preparative Example 80 by using 4-(ethoxycarbonylmethyl)piperidine in place of 4-(cyanomethyl)piperidine trifluoroacetic acid.
yield: 74%
mp 146-147 °C (EtOAc/hexanes);
IR (neat) 3316 (NH), 1738, 1695 (C=O), 1161 (SO₂) cm⁻¹;
¹H NMR (CDCl₃/TMS) δ 0. 99 (t, *J=* 7. 5 Hz, 3 H, CH₂CH₂C*H*₃), 1. 22 (t, *J=* 7. 5 Hz, 3 H, CO₂CH₂C*H*₃), 1. 31-1. 46 (m, 2 H, 2 CHₐₓ), 1. 63 (t, *J* = 6. 9 Hz, 3 H, OCH₂C*H*₃), 1. 67-1. 80 (m, 5 H, CH, CH₂C*H*₂CH₃ and 2 CH_{eq}), 2. 21 (d, *J* = 6. 6 Hz, 2 H, CH₂CO₂), 2. 39 (td, *J =* 11. 7 Hz, 2. 1 Hz, 2 H, 2 NCHₐₓ), 2. 71 (t, *J* = 7. 5 Hz, 2 H, C*H*₂CH₂CH₃), 3. 83 (br d, *J =* 11. 7 Hz, 2 H, 2 NCH_{eq}), 4. 08 (s, 3 H, NCH₃), 4. 09 (q, *J* = 7. 2 Hz, 2 H, CO₂C*H*₂CH₃), 4. 36 (q, *J* = 6. 9 Hz, 2 H, OC*H*₂CH₃), 6. 89 s, 1 H, H-2), 7. 12 (d, *J =* 9. 0 Hz, 1 H, H-3'), 7. 81 (dd, *J=* 9. 0 Hz, 2. 4 Hz, 1 H, H-4'), 8. 85 (d, *J* = 2. 4 Hz, 1 H, H-6'), 10. 65 (br s, 1 H, NH); MS (FAB) *m*/*z* 545 (MH⁺).

### Preparative Example 84

### Preparation of 2-(5-(4-(ethoxycarbonylmethyl)piperidin-1-ylsulfonyl)-2-n-propoxyphenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(ethoxycarbonylmethyl)piperidin-1-yl)

The titled compound was prepared as described in Preparative Example 80 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 4-(ethoxycarbonylmethyl)piperidine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 4-(cyanomethyl)piperidine trifluoroacetic acid.
yield: 93%
mp 147. 5-148 °C (EtOAc/CHCl₃);
IR (neat) 3335 (NH), 1732, 1669 (C=O), 1163 (SO₂) cm⁻¹;
¹H NMR (CDCl₃/TMS) δ 1. 00 (t, *J*= 7. 5 Hz, 3 H, CH₂CH₂C*H*₃), 1. 19 (t, *J*= 7. 5 Hz, 3 H, OCH₂CH₂C*H*₃), 1. 22 (t, *J* = 6. 9 Hz, 3 H, CO₂CH₂C*H*₃), 1. 25-1. 46 (m, 2 H, 2 CHₐₓ), 1. 66-1. 85 (m, 5 H, CH, CH₂C*H*₂CH₃ and 2 CH_{eq}), 1. 98-2. 10 (m, 2 H, OCH₂C*H*₂CH₃), 2. 21 (d, *J* = 6. 6 Hz, 2 H, CH₂CO₂), 2. 33-2. 45 (m, 2 H, 2 NCHₐₓ), 2. 71 (t, *J* = 7. 5 Hz, 2 H, C*H*₂CH₂CH₃), 3. 83 (br d, *J* = 11. 7 Hz, 2 H, 2 NCH_{eq}), 4. 08 (s, 3 H, NCH₃), 4. 09 (q, *J* = 6. 9 Hz, 2 H, CO₂C*H*₂CH₃), 4. 25 (t, *J*= 6. 6 Hz, 2 H, OC*H*₂CH₂CH₃), 6. 89 (s, 1 H, H-2), 7. 13 (d, *J* = 8. 7 Hz, 1 H, H-3'), 7. 81 (dd, *J* = 8. 7 Hz, 2. 4 Hz, 1 H, H-4'), 8. 87 (d, *J*= 2. 4 Hz, 1 H, H-6'), 10. 67 (br s, 1 H, NH); MS (FAB) *m*/*z* 559 (MH⁺).

### Preparative Example 85

### Preparation of 2-(2-ethoxy-5-(4-(2-ethoxycarbonylethyl)piperidin-1-ylsulfonyl)phenyl)-5-methyl-7-n -propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-ethoxycarbonylethyl)piperidin-1-yl)

The titled compound was prepared as described in Preparative Example 80 by using 4-(2-ethoxycarbonylethyl)piperidine in place of 4-(cyanomethyl)piperidine trifluoroacetic acid. yield: 77%
mp 139. 5-140 °C (EtOAc/hexanes);
IR (neat) 3322 (NH), 1734, 1694 (C=O), 1162 (SO₂) cm⁻¹;
¹H NMR (CDCl₃/TMS) δ 1. 00 (t, *J* = 7. 5 Hz, 3 H, CH₂CH₂C*H*₃), 1. 17-1. 44 (m, 3 H, CH and 2 CHₐₓ), 1. 22 (t, *J =* 7. 2 Hz, 3 H, CO₂CH₂C*H*₃), 1. 52-1. 68 (m, 2 H, C*H*₂CH₂CO₂), 1. 63 (t, *J* = 7. 2 Hz, 3 H, OCH₂C*H*₃), 1. 69-1. 80 (m, 4 H, CH₂C*H*₂CH₃ and 2 CH_{eq}), 2. 27 (t, *J* = 7. 5 Hz, 2 H, CH₂C*H*₂CO₂), 2. 33 (br t, J = 11. 4 Hz, 2 H, 2 NCHₐₓ), 2. 71 (t, *J* = 7. 5 Hz, 2 H, C*H*₂CH₂CH₃), 3. 83 (br d, *J=* 11. 4 Hz, 2 H, 2 NCH_{eq}), 4. 08 (s, 3 H, NCH₃), 4. 09 (q, *J* = 7. 2 Hz, 2 H, CO₂C*H*₂CH₃), 4. 35 (q, *J* = 7. 2 Hz, 2 H, OC*H*₂CH₃), 6. 89 (s, 1 H, H-2), 7. 12 (d, *J=* 8. 7 Hz, 1 H, H-3'), 7. 81 (dd, *J=* 8. 7 Hz, 2. 4 Hz, 1 H, H-4'), 8. 85 (d, *J* = 2. 4 Hz, 1 H, H-6'), 10. 64 (br s, 1 H, NH); MS (FAB) *m*/*z* 559 (MH⁺).

### Preparative Example 86

### Preparation of 2-(5-(4-(2-ethoxycarbonylethyl)piperidin-1-ylsulfonyl)-2-n-propoxyphenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-ethoxycarbonylethyl)piperidin-1-yl)

The titled compound was prepared as described in Preparative Example 80 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 4-(2-ethoxycarbonylethyl)piperidine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 4-(cyanomethyl)piperidine trifluoroacetic acid.
yield: 80%
mp 134-135 °C (EtOAc/CHCl₃);
IR (neat) 3335, 3300 (NH), 1735, 1688 (C=O), 1163 (SO₂) cm⁻¹;
¹H NMR (CDCl₃/TMS) δ 1. 00 (t, *J=* 7. 2 Hz, 3 H, CH₂CH₂C*H*₃), 1. 19(t, *J =* 7. 5 Hz, 3 H, OCH₂CH₂C*H*₃), 1. 22(t, *J=* 7. 2 Hz, 3 H, CO₂CH₂C*H*₃), 1. 21-1. 40(m, 3 H, CH and 2 CHₐₓ), 1. 49-1. 62(m, 2 H, C*H*₂CH₂CO₂), 1. 67-1. 80(m, 4 H, CH₂C*H*₂CH₃ and 2 CH_{eq}), 1. 98-2. 08 (m, 2 H, OCH₂C*H*₂CH₃), 2. 27 (t, *J=* 7. 5 Hz, 2 H, CH₂C*H*₂CO₂), 2. 29-2. 38 (m, 2 H, 2 NCHₐₓ), 2. 71 (t, *J =* 7. 5 Hz, 2 H, C*H*₂CH₂CH₃), 3. 83 (br d, *J* = 12. 6 Hz, 2 H, 2 NCH_{eq}), 4. 082 (q, *J =* 7. 2 Hz, 2 H, CO₂C*H*₂CH₃), 4. 083 (s, 3 H, NCH₃), 4. 24(t, *J =* 6. 6 Hz, 2 H, OC*H*₂CH₂CH₃), 6. 89 (s, 1 H, H-2), 7. 13(d, *J=* 8. 7 Hz, 1 H, H-3'), 7. 81 (dd, *J* = 8. 7 Hz, 2. 4 Hz, 1 H, H-4'), 8. 86 (d, *J =* 2. 4 Hz, 1 H, H-6'), 10. 66 (br s, 1 H, NH); MS (FAB) *m*/*z* 573 (MH⁺).

### Preparative Example 87 .

### Preparation of 2-(5-((S)-1-benzyloxycarbonyl-2-methylpropyl)aminosulfonyl-2-ethoxyphenyl)-5-met hyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is ((S)-1-benzyloxycarbonyl-2-methylpropyl)amino)

The titled compound was prepared as described in Preparative Example 80 by using L-valine benzyl ester hydrochloride in place of 4-(cyanomethyl)piperidine trifluoroacetic acid. yield: 79%
mp 103-104 °C (EtOAc/Et₂O/hexanes);
IR (neat) 3327 (NH), 1741, 1674 (C=O), 1164 (SO₂) cm⁻¹;
¹H NMR (CDCl₃/FMS) δ 0. 86 (d, *J* = 6. 9 Hz, 3 H, CHC*H*₃), 0. 97 (d, *J* = 6. 9 Hz, 3 H, CHC*H*₃), 1. 00 (t, *J* = 7. 2 Hz, 3 H, CH₂CH₂C*H*₃), 1. 61 (t, *J* = 6. 9 Hz, 3 H, OCH₂C*H*₃), 1. 68-1. 76 (m, 2 H, CH₂C*H*₂CH₃), 2. 05-2. 21 (m, 1 H, C*H*(CH₃)₂), 2. 72 (t, *J* = 7. 5 Hz, 2 H, C*H*₂CH₂CH₃), 3. 87 (dd, *J* = 9. 9 Hz, 5. 1 Hz, 1 H, NCHCO₂), 4. 08 (s, 3 H, NCH₃), 4. 29 (q, *J* = 6. 9 Hz, 2 H, OC*H*₂CH₃), 4. 89 (s, 2 H, OCH₂Ph), 5. 27 (d, *J =* 9. 9 Hz, 1 H, SO₂NH), 6. 89 (s, I H, H-2), 7. 00 (d, *J* = 8. 7 Hz, 1 H, H-3'), 7.10-7.26 (m, 5 H, ArH), 7. 84 (dd, *J* = 8. 7 Hz, 2. 4 Hz, 1 H, H-47, 8. 92 (d, *J =* 2. 4 Hz, 1 H, H-6'), 10.64 (br s, 1 H, NH); MS (FAB) *m*/*z* 581 (MH⁺).

### Preparative Example 88

### Preparation of 2-(5-((S)-1-benzyloxycarbonyl-2-methylpropyl)aminosulfonyl-2-n-propoxyphenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is ((S)-1-benzyloxycarbonyl-2-methylpropyl)amino)

The titled compound was prepared as described in Preparative Example 80 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and L-valine benzyl ester hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 4-(cyanomethyl)piperidine trifluoroacetic acid.
yield: 82%
mp 126-127 °C (EtOAc/Et₂O/hexanes);
IR (neat) 3330 (NH), 1741, 1675 (C=O), 1165 (SO₂) cm⁻¹;
¹H NMR (CDCl₃/TMS) δ 0. 86 (d, *J =* 6. 9 Hz, 3 H, CHC*H*₃), 0. 97 (d, *J =* 6. 9 Hz, 3 H, CHC*H*₃), 1. 01 (t, *J* = 7. 5 Hz, 3 H, CH₂CH₂C*H*₃), 1. 18 (t, *J* = 7. 5 Hz, 3 H, OCH₂CH₂C*H*₃), 1. 69-1. 78 (m, 2 H, CH₂C*H*₂CH₃), 1. 98-2. 14 (m, 3 H, OCH₂C*H*₂CH₃ and C*H*(CH₃)₂), 2. 73 (t, *J=* 7. 5 Hz, 2 H, C*H*₂CH₂CH₃), 3. 88 (dd, *J =* 9. 9 Hz, 5. 1 Hz, 1 H, NCHCO₂), 4. 09 (s, 3 H, NCH₃), 4. 19 (t, *J =* 6. 6 Hz, 2 H, OC*H*₂CH₂CH₃), 4. 89 (s, 2 H, OCH₂Ph), 5. 19 (d, *J=* 9. 9 Hz, 1 H, SO₂NH), 6. 89 (s, 1 H, H-2), 7. 02 (d, *J=* 8. 7 Hz, 1 H, H-3'), 7. 11-7. 33 (m, 5 H, ArH), 7. 85 (dd, *J=* 8. 7 Hz, 2. 7 Hz, 1 H, H-4'), 8. 95 (d, *J* = 2. 7 Hz, 1 H, H-6'), 10. 67 (br s, 1 H, NH); MS (FAB) *m*/*z* 595 (MH⁺).

### Preparative Example 89

### Preparation of 4-(2-ethoxy-5-nitrobenzamido)-1-methyl-3-n-propylpyrrole-5-carboxamide (a compound of the formula (13) wherein R¹ = CH₃, R² = H, R³ = CH₂CH₂CH₃, R⁴ = CH₂CH₃)

The titled compound was prepared as described in Preparative Example 28 by using 2-ethoxy-5-nitrobenzoyl chloride in place of 2-ethoxybenzoyl chloride.
yield: 96%
mp 227-227. 5 °C (CHCl₃/Et₂O);
IR (neat) 3447, 3299 (NH), 1657, 1639 (C=O), 1341, 1288 (NO₂) cm⁻¹;
¹H NMR (CDCl₃/TMS) δ 0. 92 (t, *J* = 7. 5 Hz, 3 H, CH₂CH₂C*H*₃), 1. 46-1. 57 (m, 2 H, CH₂C*H*₂CH₃), 1. 60 (t, *J* = 6. 9 Hz, 3 H, OCH₂C*H*₃), 2. 33 (t, *J* = 7. 5 Hz, 2 H, C*H*₂CH₂CH₃), 3. 85 (s, 3 H, NCH₃), 4. 42 (q, *J*= 6. 9 Hz, 2 H, OC*H*₂CH₃), 6. 54 (s, 1 H, H-2), 7. 16 (d, *J* = 9. 0 Hz, 1 H, H-3'), 8. 39 (dd, *J*= 9. 0 Hz, 2. 7 Hz, 1 H, H-4'), 9. 13 (br s, 1 H, NH), 9. 16 (d, *J=* 2. 7 Hz, 1 H, H-6'); MS (FAB) m/z 375 (MH⁺).

### Preparative Example 90

### Preparation of 4-(5-nitro-2-n-propoxybenzamido)-1-methyl-3-n-propylpyrrole-5-carboxamide (a compound of the formula (13) wherein R¹ = CH₃, R² = H, R³ = CH₂CH₂CH₃, R⁴ = CH₂CH₂CH₃)

The titled compound was prepared as described in Preparative Example 28 by using 5-nitro-2-*n*-propoxybenzoyl chloride in place of 2-ethoxybenzoyl chloride.
yield: 94%
mp 224-224. 5 °C (CHCl₃/Et₂O);
IR (neat) 3388, 3186 (NH), 1659, 1639 (C=O), 1341, 1277 (NO₂) cm⁻¹;
¹H NMR (CDCl₃/TMS) δ 0. 91 (t, *J* = 7. 5 Hz, 3 H, CH₂CH₂C*H*₃), 1. 09 (t, *J* = 7. 5 Hz, 3 H, OCH₂CH₂C*H*₃), 1. 47-1. 59 (m, 2 H, CH₂C*H*₂CH₃), 1. 91-2. 03 (m, 2 H, OCH₂C*H*₂CH₃), 2. 32 (t, *J*= 7. 5 Hz, 2 H, C*H*₂CH₂CH₃), 3. 85 (s, 3 H, NCH₃), 4. 30 (t, *J* = 6. 9 Hz, 2 H, OC*H*₂CH₂CH₃), 6. 54 (s, 1 H, H-2), 7. 16 (d, *J* = 8. 7 Hz, 1 H, H-3'), 8. 39 (dd, *J =* 8. 7 Hz, 3. 0 Hz, 1 H, H-4'), 9. 12 (br s, 1 H, NH), 9. 16 (d, *J =* 3. 0 Hz, 1 H, H-6'); MS (FAB) *m*/*z* 389 (MH⁺).

### Preparative Example 91

### Preparation of 2-(2-ethoxy-5-nitrophenyl)-5-methyl-7-n-propyl-3,5-dihydro-4H-pyrrolo[3,2-d]pyrimi din-4-one (a compound of the formula (10) wherein R¹ = CH₃, R² = H, R³ = CH₂CH₂CH₃, R⁴ = CH₂CH₃)

The titled compound was prepared as described in Preparative Example 51 by using 4-(2-ethoxy-5-nitrobenzamido)-1-methyl-3-*n*-propylpyrrole-5-carboxamide in place of 4-(2-(2-fluoroethoxy)benzamido)-1-methyl-3-yl-propylpyrrole-5-carboxamide.
yield: 77%
mp 211-211. 5 °C (CHCl₃/Et₂O);
IR (neat) 3327 (NH), 1684 (C=O), 1341, 1268 (NO₂) cm⁻¹;
¹H NMR (DMSO-*d*₆) δ 0. 93 (t, *J =* 7. 5 Hz, 3 H, CH₂CH₂C*H*₃), 1. 37 (t, *J* = 6. 9 Hz, 3 H, OCH₂C*H*₃), 1. 58-1. 70 (m, 2 H, CH₂C*H*₂CH₃), 2. 59 (t, *J* = 7. 5 Hz, 2 H, C*H*₂CH₂CH₃), 3. 99 (s, 3 H, NCH₃), 4. 27 (q, *J=* 6. 9 Hz, 2 H, OC*H*₂CH₃), 7. 23 (s, 1 H, H-2), 7. 36 (d, *J* = 9. 3 Hz, 1 H, H-3'), 8. 35 (dd, *J* = 9. 3 Hz, 3. 0 Hz, 1 H, H-4'), 8. 43 (d, *J=* 3. 0 Hz, 1 H, H-6'), 11. 75 (br s, 1 H, NH); MS (FAB) *m*/*z* 357 (MH⁺).

### Preparative Example 92

### Preparation of 5-methyl-2-(5-nitro-2-n-propoxyphenyl)-7-n-propyl-3,5-dihydro-4H-pyrrolo[3,2-d]pyr imidin-4-one (a compound of the formula (10) wherein R¹ = CH₃, R² = H, R³ = CH₂CH₂CH₃, R⁴ = CH₂CH₂CH₃)

The titled compound was prepared as described in Preparative Example 51 by using 1-methyl-4-(5-nitro-2-*n*-propoxybenzamido)-3-*n*-propylpyrrole-5-carboxamide in place of 4-(2-(2-fluoroethoxy)benzamido)-1-methyl-3-*n*-propylpyrrole-5-carboxamide.
yield: 95%
mp 181. 5 °C dec (CHCl₃/Et₂O);
IR (neat) 3324 (NH), 1689 (C=O), 1345, 1273 (NO₂) cm⁻¹;
¹H NMR (CDCl₃/TMS) δ 1. 02 (t, *J =* 7. 5 Hz, 3 H, CH₂CH₂C*H*₃), 1. 19 (t, *J =* 7. 5 Hz,
3 H, OCH₂CH₂C*H*₃), 1. 68-1. 82 (m, 2 H, CH₂C*H*₂CH₃), 1. 91-2. 12 (m, 2 H, OCH₂C*H*₂CH₃), 2. 75 (t, *J* = 7. 5 Hz, 2 H, C*H*₂CH₂CH₃), 4. 09 (s, 3 H, NCH₃), 4. 28 (t, *J=* 6. 3 Hz, 2 H, OC*H*₂CH₂CH₃), 6. 91 (s, 1 H, H-2), 7. 12 (d, *J=* 9. 3 Hz, 1 H, H-3'), 8. 30 (dd, *J=* 9. 3 Hz, 3. 0 Hz, 1 H, H-4'), 9. 36 (d, *J=* 3. 0 Hz, 1 H, H-6'), 10. 61 (br s, 1 H, NH); MS (FAB) *m*/*z* 371 (MH⁺).

### Preparative Example 93

### Preparation of 2-(5-amino-2-ethoxyphenyl)-5-methyl-7-n-propyl-3,5-dihydro-4H-pyrrolo[3,2-d]pyri midin-4-one (a compound of the formula (3) wherein R¹ = CH₃, R² = H, R³ = CH₂CH₂CH₃, R⁴ = CH₂CH₃)

A mixture of 2-(2-ethoxy-5-nitrophenyl)-5-methyl-7-*n*-propyl-3,5-dihydro-4*H*-pyrrolo[3,2-*d*]pyrimi din-4-one (443 mg, 1. 21 mmol) and 5% Pd/C (43 mg) in THF (10 mL) and EtOH (10 mL) was purged with hydrogen gas three times and stirred vigorously under hydrogen atmosphere (1 atm; a balloon) at room temperature for 5 h. The mixture was filtered through a Celite pad, and the filtrate was evaporated to dryness under reduced pressure. The resulting yellow residue was purified by MPLC on silica gel (1% MeOH in CHCl₃) to afford the titled compound (388 mg, 98%) as a pale yellow solid. Analytically pure compound was obtained by crystallization from EtOAc/hexanes.
mp 165. 5-166 °C;
IR (neat) 3542, 3329, 3297 (NH), 1647 (C=O) cm⁻¹;
¹H NMR (CDCl₃/TMS) δ 1. 01 (t, *J=* 7. 5 Hz, 3 H, CH₂CH₂CH₃), 1. 53 (t, *J=* 6. 9 Hz, 3 H, OCH₂C*H*₃), 1. 68-1. 80 (m, 2 H, CH₂C*H*₂CH₃), 2. 72 (t, *J* = 7. 5 Hz, 2 H, C*H*₂CH₂CH₃), 4. 07 (s, 3 H, NCH₃), 4. 16 (q, J = 6. 9 Hz, 2 H, OC*H*₂CH₃), 6. 76 (dd, *J* = 9. 0 Hz, 3. 0 Hz, 1 H, H-4'), 6. 85 (s, 1 H, H-2), 6. 87 (d, *J* = 9. 0 Hz, 1 H, H-3'), 7. 85(d, *J=* 3. 0 Hz, 1 H, H-6'); MS (FAB) *m*/*z* 327 (MH⁺).

### Preparative Example 94

### Preparation of 2-(5-amino-2-n-propoxyphenyl)-5-methyl-7-n-propyl-3,5-dihydro-4H-pyrrolo[3,2-d]p yrimidin-4-one (a compound of the formula (3) wherein R¹ = CH₃, R² = H, R³ = CH₂CH₂CH₃, R⁴ = CH₂CH₂CH₃)

The titled compound was prepared as described in Preparative Example 94 by using 5-methyl-2-(5-nitro-2-*n*-propoxyphenyl)-7-*n*-propyl-3,5-dihydro-4*H*-pyrrolo[3,2-*d*]pyr imidin-4-one in place of 2-(2-ethoxy-5-nitrophenyl)-5-methyl-7-*n*-propyl-3,5-dihydro-4*H*-pyrrolo[3,2-*d*]pyrimi din-4-one.
yield: 63%
mp 94-96. 5 °C (CH₂Cl₂/MeOH/Et₂O);
IR (neat) 3505, 3439, 3312 (NH), 1676 (C=O) cm⁻¹;
¹H NMR (DMSO-*d*₆) δ 1. 19 (t, *J=* 7. 5 Hz, 3 H, CH₂CH₂C*H*₃), 1. 23 (t, *J=* 7. 5 Hz, 3 H, OCH₂CH₂C*H*₃), 1. 84-2. 03 (m, 4 H, CH₂C*H*₂CH₃ and OCH₂C*H*₂CH₃), 2. 84 (t, *J* = 7. 5 Hz, 2 H, C*H*₂CH₂CH₃), 4. 20 (t, *J=* 6. 3 Hz, 2 H, OC*H*₂CH₂CH₃), 4. 22 (s, 3 H, NCH₃), 5. 13 (br s, 2 H, NH₂), 6. 94 (dd, *J =* 8. 7 Hz, 3. 0 Hz, 1 H, H-4'), 7. 15 (d, *J =* 8. 7 Hz, 1 H, H-3'), 7. 44 (s, 1 H, H-2), 7. 45 (d, *J* = 3. 0 Hz, 1 H, H-6'), 11. 57 (br s, 1 H, NH); MS (FAB) *m*/*z* 341 (MH⁺).

### Preparative Example 95

### Preparation of 4-(5-bromo-2-ethoxybenzamido)-1-methyl-3-n-propylpyrrole-5-carboxamide (a compound of the formula (14) wherein R¹ = CH₃, R² = H, R³ = CH₂CH₂CH₃, R⁴ = CH₂CH₃)

The titled compound was prepared as described in Preparative Example 28 by using 5-bromo-2-ethoxybenzoyl chloride in place of 2-ethoxybenzoyl chloride.
yield: 98%
mp 158-159 °C (EtOAc/Et₂O/hexanes);
IR (neat) 3445, 3174 (NH), 1656 (C=O) cm⁻¹;
¹H NMR (CDCl₃/TMS) δ 0. 92 (t, *J =* 7. 5 Hz, 3 H, CH₂CH₂C*H*₃), 1. 47-1. 59 (m, 2 H, CH₂C*H*₂CH₃), 1. 52 (t, *J* = 6. 9 Hz, 3 H, OCH₂C*H*₃), 2. 32 (t, *J* = 7. 5 Hz, 2 H, C*H*₂CH₂CH₃), 3. 85 (s, 3 H, NCH₃), 4. 27 (q, *J* = 6. 9 Hz, 2 H, OC*H*₂CH₃), 6. 52 (s, 1 H, H-2), 6. 93 (d, *J=* 9. 0 Hz, 1 H, H-3'), 7. 59 (dd, *J=* 9. 0 Hz, 2. 7 Hz, 1 H, H-4'), 8. 40 (d, *J=* 2. 7 Hz, 1 H, H-6'), 9. 28 (br s, 1 H, NH); MS (FAB) *m*/*z* 391 (MH⁺ - H₂O).

### Preparative Example 96

### Preparation of 4-(5-bromo-2-n-propxybenzamido)-1-methyl-3-n-propylpyrrole-5-carboxamide (a compound of the formula (14) wherein R¹ = CH₃, R² = H, R³ = CH₂CH₂CH₃, R⁴ = CH₂CH₂CH₃)

The titled compound was prepared as described in Preparative Example 28 by using 5-bromo-2-*n*-propoxybenzoyl chloride in place of 2-ethoxybenzoyl chloride.
yield: 89%
mp 148-150 °C (CHCl₃/hexanes);
IR (neat) 3315, 3174 (NH), 1659, 1642 (C=O) cm⁻¹;
¹H NMR (CDCl₃/TMS) δ 0. 91 (t, *J=* 7. 5 Hz, 3 H, CH₂CH₂C*H*₃), 1. 05 (t, *J=* 7. 5 Hz, 3 H, OCH₂CH₂C*H*₃), 1. 46-1. 56 (m, 2 H, CH₂C*H*₂CH₃), 1. 85-1. 97 (m, 2 H, OCH₂C*H*₂CH₃), 2. 31 (t, *J=* 7. 5 Hz, 2 H, C*H*₂CH₂CH₃), 3. 84 (s, 3 H, NCH₃), 4. 16 (t, *J=* 6. 9 Hz, 2 H, OC*H*₂CH₂CH₃), 6. 53 (s, 1 H, H-2), 6. 94 (d, *J =* 9. 0 Hz, 1 H, H-3'), 7. 59 (dd, *J* = 9. 0 Hz, 2. 7 Hz, 1 H, H-4'), 8.40 (d, *J=* 2. 7 Hz, 1 H, H-6'), 9. 28 (br s, 1 H, NH); MS (FAB) *m*/*z* 405 (MH⁺ - H₂O).

### Preparative Example 97

### Preparation of 2-(5-bromo-2-ethoxyphenyl)-5-methyl-7-n-propyl-3,5-dihydro-4H-pyrrolo[3,2-d]pyri midin-4-one (a compound of the formula (11) wherein R¹ = CH₃, R² = H, R³ = CH₂CH₂CH₃, R⁴ = CH₂CH₃)

The titled compound was prepared as described in Preparative Example 51 by using 4-(5-bromo-2-ethoxybenzamido)-1-methyl-3-*n*-propylpyrrole-5-carboxamide in place of 4-(2-(2-fluoroethoxy)benzamido)-1-methyl-3-*n*-propylpyrrole-5-carboxamide.
yield: 80%
mp 151: 5-152 °C (EtOAc/Et₂O/hexanes);
IR (neat) 3318 (NH), 1690 (C=O) cm⁻¹;
¹H NMR (CDCl₃/TMS) δ 1. 02 (t, *J* = 7. 5 Hz, 3 H, CH₂CH₂C*H*₃), 1. 59 (t, *J =* 6. 9 Hz, 3 H, OCH₂C*H*₃), 1. 67-1. 80 (m, 2 H, CH₂C*H*₂CH₃), 2. 73 (t, *J* = 7. 5 Hz, 2 H, C*H*₂CH₂CH₃), 4. 08 (s, 3 H, NCH₃), 4. 25 (q, *J=* 6. 9 Hz, 2 H, OC*H*₂CH₃), 6. 87 (s, 1 H, H-2), 6. 90 (d, *J =* 9. 0 Hz, 1 H, H-3'), 7. 49 (dd, *J=* 9. 0 Hz, 2. 7 Hz, 1 H, H-4'), 8. 59 (d, *J=* 2. 7 Hz, 1 H, H-6'), 10. 79 (br s, 1 H, NH); MS (FAB) *m*/*z* 390 (M⁺).

### Preparative Example 98

### Preparation of 2-(5-bromo-2-n-propoxyphenyl)-5-methyl-7-n-propyl-3,5-dihydro-4H-pyrrolo[3,2-d]p yrimidin-4-one (a compound of the formula (11) wherein R¹ = CH₃, R² = H, R³ = CH₂CH₂CH₃, R⁴ = CH₂CH₂CH₃)

The titled compound was prepared as described in Preparative Example 51 by using 4-(5-bromo-2-*n*-propoxybenzamido)-1-methyl-3-*n*-propylpyrrole-5-carboxamide in place of 4-(2-(2-fluoroethoxy)benzamido)-1-methyl-3-*n*-propylpyrrole-5-carboxamide. yield: 76%
mp 118-119 °C (EtOAc/hexanes);
IR (neat) 3324 (NH), 1696 (C=O) cm⁻¹;
¹H NMR (CDCl₃/TMS) δ 1. 02 (t, *J* = 7. 5 Hz, 3 H, CH₂CH₂C*H*₃), 1. 15 (t, *J* = 7. 2 Hz, 3 H, OCH₂CH₂C*H*₃), 1. 68-1. 80 (m, 2 H, CH₂C*H*₂CH₃), 1. 93-2. 05 (m, 2 H, OCH₂C*H*₂CH₃), 2. 73 (t, *J* = 7. 5 Hz, 2 H, C*H*₂CH₂CH₃), 4. 08 (s, 3 H, NCH₃), 4. 14 (t, *J* = 6. 6 Hz, 2 H, OC*H*₂CH₂CH₃), 6. 88 (s, 1 H, H-2), 6. 91 (d, *J* = 8. 7 Hz, 1 H, H-3'), 7. 50 (dd, *J* = 8. 7 Hz, 2. 7 Hz, 1 H, H-4'), 8. 60 (d, *J=* 2. 7 Hz, 1 H, H-6'), 10. 82 (br s, 1 H, NH); MS (FAB) *m*/*z* 404 (M⁺).

### Preparative Example 99

### Preparation of 2-(5-cyano-2-ethoxyphenyl)-5-methyl-7-n-propyl-3,5-dihydro-4H-pyrrolo[3,2-d]pyrim idin-4-one (a compound of the formula (4) wherein R¹ = CH₃, R² = H, R³ = CH₂CH₂CH₃, R⁴ = CH₂CH₃)

A mixture of 2-(5-bromo-2-ethoxyphenyl)-5-methyl-7-*n*-propyl-3,5-dihydro-4*H*-pyrrolo[3,2-*d*]pyri midin-4-one (560 mg, 1. 43 mmol) and copper(I) cyanide (2. 57 g, 28. 7 mmol) in 1-methyl-2-pyrrolidinone (10 mL) was heated at 190 °C for 5 h, and then cooled to room temperature. The reaction mixture was poured into a cooled mixture of 29% aqueous ammonia solution (70 mL) and water (140 mL) in an ice bath, and the resulting deep blue suspension was stirred for 1 h in an ice bath. The mixture was extracted with CHCl₃ (80 mL × 3), the combined organic layer was dried (MgSO₄), filtered and the filtrate was evaporated to dryness under reduced pressure. The crude residue was solidified from Et₂O/hexanes, and the resulting solid was purified by MPLC on silica gel (1% MeOH in CHCl₃) to afford the titled compound (376 mg, 78%) as a pale yellow solid. Analytically pure compound was obtained by crystallization from CHCl₃/Et₂O.
mp 232. 5-233 °C;
IR (neat) 3329 (NH), 2228 (CN), 1692 (C=O) cm⁻¹;
¹H NMR (CDCl₃/TMS) δ 1. 02 (t, *J* = 7. 5 Hz, 3 H, CH₂CH₂C*H*₃), 1. 63 (t, *J =* 6. 9 Hz, 3 H, OCH₂C*H*₃), 1. 68-1. 79 (m, 2 H, CH₂C*H*₂CH₃), 2. 73 (t, *J* = 7. 5 Hz, 2 H, C*H*₂CH₂CH₃), 4. 08 (s, 3 H, NCH₃), 4. 35 (q, *J=* 6. 9 Hz, 2 H, OC*H*₂CH₃), 6. 90 (s, 1 H, H-2), 7. 09 (d, *J* = 8. 7 Hz, 1 H, H-3'), 7. 69 (dd, *J* = 8. 7 Hz, 2. 4 Hz, 1 H, H-4'), 8. 82 (d, *J* = 2. 4 Hz, 1 H, H-6'), 10. 62 (br s, 1 H, NH); MS (FAB) *m*/*z* 337 (MH⁺).

### Preparative Example 100

### Preparation of 2-(5-cyano-2-n-propoxyphenyl)-5-methyl-7-n-propyl-3,5-dihydro-4H-pyrrolo[3,2-d]p yrimidin-4-one (a compound of the formula (4) wherein R¹ = CH₃, R² = H, R³ = CH₂CH₂CH₃, R⁴ = CH₂CH₂CH₃)

The titled compound was prepared as described in Preparative Example 100 by using2-(5-bromo-2-*n*-propoxyphenyl)-5-methyl-7-*n*-propyl-3,5-dihydro-4*H*-pyrrolo[3, 2-*d*]pyrimidin-4-one in place of 2-(5-bromo-2-ethoxyphenyl)-5-methyl-7-*n*-propyl-3,5-dihydro-4*H*-pyrrolo[3,2-*d*]pyri midin-4-one.
yield: 84%
mp 179-180. 5 °C (CHCl₃/hexanes);
IR (neat) 3333 (NH), 2225 (CN), 1689 (C=O) cm⁻¹;
¹H NMR (CDCl₃/TMS) δ 1. 02 (t, *J=* 7. 5 Hz, 3 H, CH₂CH₂C*H*₃), 1. 18(t, *J* = 7. 2 Hz, 3 H, OCH₂CH₂C*H*₃), 1. 68-1. 80 (m, 2 H, CH₂C*H*₂CH₃), 1. 98-2. 09 (m, 2 H, OCH₂C*H*₂CH₃), 2. 73 (t, *J* = 7. 5 Hz, 2 H, C*H*₂CH₂CH₃), 4. 08(s, 3 H, NCH₃); 4. 24 (t, *J* = 6. 6 Hz, 2 H, OC*H*₂CH₂CH₃), 6. 90 (s, I H, H-2), 7. 10 (d, *J* = 9. 0 Hz, 1 H, H-3'), 7. 69 (dd, *J=* 9. 0 Hz, 2. 4 Hz, 1 H, H-4'), 8. 83(d, *J=* 2. 4 Hz, 1 H, H-6'), 10. 66(br s, 1 H, NH); MS (FAB) *m*/*z* 351 (MH⁺).

### Example 1

### Preparation of 2-(2-ethoxy-5-(4-methylpiperazin-1-ylsulfonyl)phenyl)-5-methyl-7-n-propyl-3,5-dihy dro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-methylpiperazin-1-yl)

To a mixture of 2-(5-chlorosulfonyl-2-ethoxyphenyl)-5-methyl-7-*n*-propyl-3,5-dihydro-4*H*-pyrrolo[3,2 - *d*]pyrimidin-4-one (170 mg, 0. 41 mmol) and 1-methylpiperazine (69 µL, 0. 62 mmol) in anhydrous CH₂Cl₂ (10 mL) or EtOH (10 mL) was added triethylamine (115 µL, 0. 83 mmol), and the mixture was stirred at room temperature under nitrogen atmosphere for 1-12 h. The reaction mixture was evaporated to dryness under reduced pressure, and the resulting yellow residue was purified by MPLC on silica gel (gradient elution: 2% MeOH in CHCl₃ followed by 3% MeOH in CHCl₃) to afford the titled compound (161 mg, 82%) as a yellowish solid. Analytically pure compound was obtained by crystallization from EtOAc/hexanes.
mp 220. 5-221 °C;
IR (neat) 3334 (NH), 1678 (C=O), 1172 (SO₂) cm⁻¹;
¹H NMR (CDCl₃/TMS) δ 0. 99 (t, *J* = 7. 5 Hz, 3 H, CH₂CH₂C*H*₃), 1. 63 (t, *J* = 6. 9 Hz, 3 H, OCH₂C*H*₃), 1. 67-1. 79 (m, 2 H, CH₂C*H*₂CH₃), 2. 27 (s, 3 H, NCH₃), 2. 49 (dd, *J* = 5. 1 Hz, 4. 8 Hz, 4 H, 2 NCH₂), 2. 71 (t, *J* = 7. 5 Hz, 2 H, C*H*₂CH₂CH₃), 3. 11 (dd, *J* = 5. 1 Hz, 4. 8 Hz, 4 H, 2 SO₂NCH₂), 4. 08 (s, 3 H, NCH₃), 4. 35 (q, *J* = 6. 9 Hz, 2 H, OC*H*₂CH₃), 6. 88 (s, 1 H, H-2), 7. 12 (d, *J* = 8. 7 Hz, 1 H, H-3'), 7. 81 (dd, *J* = 8. 7 Hz, 2. 4 Hz, 1 H, H-4'), 8. 87 (d, *J =* 2. 4 Hz, 1 H, H-6'), 10. 60 (br s, 1 H, NH); MS (FAB) *m*/*z* 474 (MH⁺).

### Example 2

### Preparation of 2-(2-ethoxy-5-(4-methylpiperazin-1-ylsulfonyl)phenyl)-5-methyl-7-n-propyl-3,5-dihy dro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-methylpiperazin-1-yl)

To a solution of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one (77 mg, 0. 16 mmol) in anhydrous CH₂Cl₂ (2 mL) was added 1 M HCl ether solution (180 µL, 0. 18 mmol) at room temperature under nitrogen atmosphere, and the solution was stirred for about 10 minutes. The reaction mixture was poured slowly into anhydrous ether (50 mL), and the resulting white precipitates were collected by filtration. The filtered solid was dissolved in H₂O (20 mL), filtered through a membrane filter (0. 45 µm), and the filtrate was freeze-dried to afford the titled compound (79 mg, 95%) as an off-white floppy solid.
mp 131. 5 °C dec;
IR (neat) 3334 (NH), 1686 (C=O), 1163 (SO₂) cm⁻¹;
¹H NMR (DMSO-*d*₆) δ 0. 93 (t, *J* = 7. 5 Hz, 3 H, CH₂CH₂C*H*₃), 1. 36 (t, *J =* 6. 9 Hz, 3 H, OCH₂C*H*₃), 1. 58-1. 70 (m, 2 H, CH₂C*H*₂CH₃), 2. 58 (t, *J* = 7. 5 Hz, 2 H, C*H*₂CH₂CH₃), 2. 66-2. 83 (br s, 5 H, NCH₃ and 2 SO₂NCHₐₓ), 3. 05-3. 22 (m, 2 H, 2 SO₂NCH_{eq}), 3. 35-3. 54 (m, 2 H, 2 ⁺HNC*H*ₐₓ), 3. 72-3. 88 (m, 2 H, 2 ⁺HNC*H*_{eq}), 3. 99 (s, 3 H, NCH₃), 4. 24 (q, *J* = 6. 9 Hz, 2 H, OC*H*₂CH₃), 7. 23 (s, 1 H, H-2), 7. 41 (d, *J =* 9. 0 Hz, 1 H, H-3'), 7. 86 (dd, *J =* 9. 0 Hz, 2. 7 Hz, 1 H, H-4'), 7. 97 (d, *J =* 2. 7 Hz, 1 H, H-6'), 10. 58 (br s, 1 H, NH⁺), 11. 75 (br s, 1 H, NH).

### Example 3

### Preparation of 2-(5-(4-methylpiperazin-1-ylsulfonyl)-2-n-propoxyphenyl)-5-methyl-7-n-propyl-3,5-di hydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-methylpiperazin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one in place of 2-(5-chlorosulfonyl-2-ethoxyphenyl)-5-methyl-7-*n*-propyl-3,5-dihydro-4*H*-pyrrolo[3,2 - *d*]pyrimidin-4-one.
yield: 81%
mp 183-183. 5 °C (EtOAc/hexanes);
IR (neat) 3297 (NH), 1695 (C=O), 1171 (SO₂) cm⁻¹;
¹H NMR (CDCl₃/TMS) δ 0. 99 (t, *J* = 7. 5 Hz, 3 H, CH₂CH₂C*H*₃), 1. 18 (t, *J =* 7. 5 Hz, 3 H, OCH₂CH₂C*H*₃), 1. 67-1. 79 (m, 2 H, CH₂C*H*₂CH₃), 1. 98-2. 07 (m, 2 H, OCH₂C*H*₂CH₃), 2. 27 (s, 3 H, NCH₃), 2. 49 (dd, *J* = 5. 1 Hz, 4. 8 Hz, 4 H, 2 NCH₂), 2. 71 (t, *J* = 7. 5 Hz, 2 H, C*H*₂CH₂CH₃), 3. 11 (dd, *J* = 5. 1 Hz, 4. 8 Hz, 4 H, 2 SO₂NCH₂), 4. 08 (s, 3 H, NCH₃), 4. 24 (t, *J =* 6. 6 Hz, 2 H, OC*H*₂CH₂CH₃), 6. 88 (s, 1 H, H-2), 7. 13 (d, J = 8. 7 Hz, 1 H, H-3'), 7. 81 (dd, *J* = 8. 7 Hz, 2. 4 Hz, 1 H, H-4'), 8. 88 (d, *J* = 2.4 Hz, 1 H, H-6'), 10.67 (br s, 1 H, NH); MS (FAB) *m*/*z* 488 (MH⁺).

### Example 4

### Preparation of 2-(5-(4-methylpiperazin-1-ylsulfonyl)-2-n-propoxyphenyl)-5-methyl-7-n-propyl-3,5-di hydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-methylpiperazin-1-yl)

The titled compound was prepared as described in Example 2 by using 2-(5-(4-methylpiperazin-1-ylsulfonyl)-2-*n*-propoxyphenyl)-5-methyl-7-*n*-propyl-3,5-di hydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 93%
mp 124. 5 °C dec;
IR (neat) 3348 (NH), 1680 (C=O), 1167 (SO₂) cm⁻¹;
¹H NMR (DMSO-*d*₆) δ 0. 93 (t, *J* = 7. 5 Hz, 3 H, CH₂CH₂C*H*₃), 0. 97 (t, *J* = 7. 2 Hz, 3 H, OCH₂CH₂C*H*₃), 1. 57-1. 70 (m, 2 H, CH₂C*H*₂CH₃), 1. 70-1. 81 (m, 2 H, OCH₂C*H*₂CH₃), 2. 58 (t, *J* = 7. 5 Hz, 2 H, C*H*₂CH₂CH₃), 2. 75 (s, 3 H, NCH₃), 2. 76-2. 80 (m, 2 H, 2 SO₂NCHₐₓ), 3. 08-3.22 (m, 2 H, 2 SO₂NCH_{eq}), 3. 35-3. 50 (m, 2 H, 2 ⁺HNC*H*ₐₓ), 3. 81 (br d, *J* = 12. 3 Hz, 2 H, 2 ⁺HNC*H*_{eq}), 3. 99 (s, 3 H, NCH₃), 4. 14 (t, *J* = 6. 6 Hz, 2 H, OC*H*₂CH₂CH₃), 7. 24 (s, 1 H, H-2), 7. 42 (d, *J* = 9. 0 Hz, I H, H-3'), 7. 87 (dd, *J* = 9. 0 Hz, 2. 4 Hz, 1 H, H-4'), 7. 98 (d, *J* = 2. 4 Hz, 1 H, H-6'), 10. 93 (br s, 1 H, NH⁺), 11. 76 (br s, 1 H, NH).

### Example 5

### Preparation of 2-(2-ethoxy-5-(4-ethylpiperazin-1-ylsulfonyl)phenyl)-5-methyl-7-n-propyl-3,5-dihydr o-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-ethylpiperazin-1-yl)

The titled compound was prepared as described in Example 1 by using 1-ethylpiperazine in place of 1-methylpiperazine.
yield: 89%
mp 200-200. 5 °C (CHCl₃/EtOAc/hexanes);
IR (neat) 3332 (NH), 1680 (C=O), 1172 (SO₂) cm⁻¹;
¹H NMR (CDCl₃/TMS) δ 0. 99 (t, *J* = 7. 5 Hz, 3 H, CH₂CH₂C*H*₃), 1. 02(t, *J* = 7. 2 Hz, 3 H, NCH₂C*H*₃), 1. 63 (t, *J* = 6. 9 Hz, 3 H, OCH₂C*H*₃), 1. 67-1. 77 (m, 2 H, CH₂C*H*₂CH₃), 2. 40 (q, *J =* 7. 2 Hz, 2 H, NC*H*₂CH₃), 2. 53(dd, *J*= 5. 1 Hz, 4. 8 Hz, 4 H, 2 NCH₂), 2. 71 (t, *J =* 7. 5 Hz, 2 H, C*H*₂CH₂CH₃), 3. 11 (dd, *J =* 5. 1 Hz, 4. 8 Hz, 4 H, 2 SO₂NCH₂), 4. 08 (s, 3 H, NCH₃), 4. 35 (q, *J =* 6. 9 Hz, 2 H, OC*H*₂CH₃), 6. 88 (s, 1 H, H-2), 7. 12 (d, *J* = 9. 0 Hz, 1 H, H-3'), 7. 80 (dd, *J* = 9. 0 Hz, 2. 4 Hz, 1 H, H-4'), 8. 87 (d, *J* = 2. 4 Hz, 1 H, H-6'), 10. 62 (br s, 1 H, NH); MS (FAB) *m*/*z* 488 (MH⁺).

### Example 6

### Preparation of 2-(2-ethoxy-5-(4-n-propylpiperazin-1-ylsulfonyl)phenyl)-5-methyl-7-n-propyl-3,5-dih ydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-n-propylpiperazin-1-yl)

The titled compound was prepared as described in Example 1 by using 1-*n*-propylpiperazine in place of 1-methylpiperazine.
yield: 76%
mp 202. 5 °C dec (CHCl₃/Et₂O/hexanes);
IR (neat) 3332 (NH), 1677 (C=O), 1170 (SO₂) cm⁻¹;
¹H NMR (CDCl₃/TMS) δ 0. 90 (t, *J =* 7. 5 Hz, 3 H, NCH₂CH₂C*H*₃), 0. 99 (t, *J =* 7. 5 Hz, 3 H, CH₂CH₂C*H*₃), 1. 37-1.50 (m, 2 H, NCH₂CH₂CH₃), 1. 63 (t, *J* = 6. 9 Hz, 3 H, OCH₂C*H*₃), 1. 64-1. 77 (m, 2 H, CH₂C*H*₂CH₃), 2. 28 (dd, *J* = 7. 8 Hz, 7. 5 Hz, 2 H, NC*H*₂CH₂CH₃), 2. 52 (dd, *J* = 4. 8 Hz, 3. 9 Hz, 4 H, 2 NCH₂), 2. 71 (t, *J* = 7. 5 Hz, 2 H, C*H*₂CH₂CH₃), 3. 10 (dd, *J* = 4. 8 Hz, 3. 9 Hz, 4 H, 2 SO₂NCH₂), 4. 08 (s, 3 H, NCH₃), 4. 35 (q, *J* = 6.9 Hz, 2 H, OC*H*₂CH₃), 6. 88 (s, 1 H, H-2), 7. 12 (d, *J* = 8. 7 Hz, 1 H, H-3'), 7. 80 (dd, *J* = 8. 7 Hz, 2. 4 Hz, 1 H, H-4'), 8. 86 (d, *J* = 2. 4 Hz, 1 H, H-6'), 10. 63 (br s, 1 H, NH); MS (FAB) *m*/*z* 502 (MH⁺).

### Example 7

### Preparation of 2-(2-ethoxy-5-(4-n-propylpiperazin-1-ylsulfonyl)phenyl)-5-methyl-7-n-propyl-3,5-dih ydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-n-propylpiperazin-1-yl)

The titled compound was prepared as described in Example 2 by using 2-(2-ethoxy-5-(4-*n*-propylpiperazin-1-ylsulfonyl)phenyl)-5-methyl-7-*n*-propyl-3,5-dih ydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 98%
mp 232. 5 °C dec;
IR (neat) 3334 (NH), 1686 (C=O), 1163 (SO₂) cm⁻¹;
¹H NMR (DMSO-*d*₆) δ 0. 87 (t, *J* = 7. 5 Hz, 3 H, NCH₂CH₂C*H*₃), 0. 93 (t, *J* = 7. 2 Hz, 3 H, CH₂CH₂C*H*₃), 1. 36 (t, *J* = 6. 9 Hz, 3 H, OCH₂C*H*₃), 1. 56-1. 72 (m, 4 H, CH₂C*H*₂CH₃ and NCH₂C*H*₂CH₃), 2. 58 (t, *J* = 7. 5 Hz, 2 H, C*H*₂CH₂CH₃), 2. 82(br dd, *J* = 12. 3 Hz, 11. 7 Hz, 2 H, 2 SO₂NCHₐₓ), 2. 94-3. 07 (m, 2 H, NC*H*₂CH₂CH₃), 3. 05-3. 17 (m, 2 H, 2 SO₂NCH_{eq}), 3.47-3. 58 (m, 2 H, 2 ⁺HNC*H*ₐₓ), 3. 79 (br d, *J* = 11. 7 Hz, 2 H, 2 ⁺HNC*H*_{eq}), 3. 99 (s, 3 H, NCH₃), 4. 24(q, *J=* 6. 9 Hz, 2 H, OC*H*₂CH₃), 7. 25 (s, 1 H, H-2), 7. 42 (d, *J =* 9. 0 Hz, I H, H-3'), 7. 87 (dd, *J=* 9. 0 Hz, 2. 4 Hz, 1 H, H-4'), 7. 98 (d, *J =* 2.4 Hz, 1 H, H-6'), 10. 69 (br s, 1 H, NH⁺), 11. 82(br s, 1 H, NH).

### Example 8

### Preparation of 2-(2-ethoxy-5-(4-isopropylpiperazin-1-ylsulfonyl)phenyl)-5-methyl-7-n-propyl-3,5-di hydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-isopropylpiperazin-1-yl)

The titled compound was prepared as described in Example 1 by using 1-isopropylpiperazine in place of 1-methylpiperazine.
yield: 87%
mp 241. 5 °C dec (CHCl₃/Et₂O);
IR (neat) 3333 (NH), 1680, 1672 (C=O), 1177 (SO₂) cm⁻¹;
¹H NMR (CDCl₃/TMS) δ 0. 990 (t, *J=* 7. 5 Hz, 3 H, CH₂CH₂C*H*₃), 0. 993 (t, *J=* 6. 6 Hz, 6 H, CH(C*H*₃)₂), 1. 63 (t, *J* = 6. 9 Hz, 3 H, OCH₂C*H*₃), 1. 67-1. 77 (m, 2 H, CH₂C*H*₂CH₃), 2. 60(dd, *J =* 4. 8 Hz, 4. 5 Hz, 4 H, 2 NCH₂), 2. 61-2. 68 (m, 1 H, C*H*(CH₃)₂), 2. 71 (t, *J=* 7. 5 Hz, 2 H, C*H*₂CH₂CH₃), 3. 09 (dd, *J=* 4. 8 Hz, 4. 5 Hz, 4 H, 2 SO₂NCH₂), 4. 08 (s, 3 H, NCH₃), 4. 35 (q, *J* = 6. 9 Hz, 2 H, OC*H*₂CH₃), 6. 88 (s, 1 H, H-2), 7. 11 (d, *J=* 8. 7 Hz, 1 H, H-3'), 7. 80 (dd, *J=* 8. 7 Hz, 2. 7 Hz, I H, H-4'), 8. 86 (d, *J=* 2. 7 Hz, 1 H, H-6'), 10. 62 (br s, 1 H, NH); MS (FAB) *m*/*z* 502 (MH⁺).

### Example 9

### Preparation of 2-(2-ethoxy-5-(4-isopropylpiperazin-1-ylsulfonyl)phenyl)-5-methyl-7-n-propyl-3,5-di hydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-isopropylpiperazin-1-yl)

The titled compound was prepared as described in Example 2 by using 2-(2-ethoxy-5-(4-isopropylpiperazin-1-ylsulfonyl)phenyl)-5-methyl-7-*n*-propyl-3,5-di hydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 98%
mp 244. 5 °C dec;
IR (neat) 3336(NH), 1684 (C=O), 1166 (SO₂) cm⁻¹;
¹H NMR (DMSO-*d*₆) δ 0.93 (t, *J* = 7. 5 Hz, 3 H, CH₂CH₂C*H*₃), 1. 23 (t, *J* = 6. 6 Hz, 6 H, CH(C*H*₃)₂), 1. 37 (t, *J* = 6. 9 Hz, 3 H, OCH₂C*H*₃), 1. 57-1. 72 (m, 2 H, CH₂C*H*₂CH₃), 2. 58 (t, *J* = 7. 5 Hz, 2 H, C*H*₂CH₂CH₃), 2. 76-2. 88 (m, 2 H, 2 SO₂NCHₐₓ), 3. 07-3. 19 (m, 2 H, 2 SO₂NCH_{eq}), 3. 30-3. 56 (m, 3 H, C*H*(CH₃)₂ and 2 ⁺HNC*H*ₐₓ), 3. 82 (br d, *J* = 12. 3 Hz, 2 H, 2 ⁺HNC*H*_{eq}), 3. 99 (s, 3 H, NCH₃), 4. 24 (q, *J* = 6. 9 Hz, 2 H, OC*H*₂CH₃), 7. 24 (s, 1 H, H-2), 7. 42 (d, *J* = 9. 0 Hz, 1 H, H-3'), 7. 88 (dd, *J* = 9. 0 Hz, 2. 4 Hz, 1 H, H-4'), 7. 99 *(d, J* = 2. 4 Hz, 1 H, H-6'), 10. 38 (br s, 1 H, NH⁺), 11. 78 (br s, 1 H, NH).

### Example 10

### Preparation of 2-(2-ethoxy-5-(4-(2-fluoroethyl)piperdzin-1-ylsulfonyl)phenyl)-5-methyl-7-n-propyl-3 ,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-fluoroethyl)piperazin-1-yl)

The titled compound was prepared as described in Example 1 by using 1-(2-fluoroethyl)piperazine hydrochloride in place of 1-methylpiperazine.
yield: 92%
mp 204. 5-205 °C (EtOAc/hexanes);
IR (neat) 3335 (NH), 1678 (C=O), 1169 (SO₂) cm⁻¹;
¹H NMR (CDCl₃/TMS) δ 0. 99 (t, *J* = 7. 5 Hz, 3 H, CH₂CH₂C*H*₃), 1. 64 (t, *J* = 6. 9 Hz, 3 H, OCH₂C*H*₃), 1. 66-1. 79 (m, 2 H, CH₂C*H*₂CH₃), 2. 62-2. 75 (m, 8 H, NC*H*₂CH₂F, 2 NCH₂ and C*H*₂CH₂CH₃), 3. 13 (dd, *J* = 4. 8 Hz, 4. 5 Hz, 4 H, 2 SO₂NCH₂), 4. 08 (s, 3 H, NCH₃), 4. 35 (q, *J* = 6. 9 Hz, 2 H, OC*H*₂CH₃), 4. 49 (ddd, *J* = 47. 4 Hz, 5. 1 Hz, 4. 5 Hz, 2 H, NCH₂C*H*₂F), 6. 89 (s, 1 H, H-2), 7. 12 (d, *J* = 8. 7 Hz, 1 H, H-3'), 7. 80 (dd, *J* = 8. 7 Hz, 2. 4 Hz, 1 H, H-4'), 8. 87 (d, *J* = 2. 4 Hz, 1 H, H-6'), 10. 63 (br s, 1 H, NH); MS (FAB) *ms*/*z* 506 (MH⁺).

### Example 11

### Preparation of 2-(2-ethoxy-5-(4-(2-fluoroethyl)piperazin-1-ylsulfonyl)phenyl)-5-methyl-7-n-propyl-3 ,5-dihydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-fluoroethyl)piperazin-1-yl)

The titled compound was prepared as described in Example 2 by using 2-(2-ethoxy-5-(4-(2-fluoroethyl)piperazin-1-ylsulfonyl)phenyl)-5-methyl-7-*n*-propyl-3 ,5-dihydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 99%
mp 76 °C dec;
IR (neat) 3333 (NH), 1684 (C=O), 1164 (SO₂) cm⁻¹;
¹H NMR (DMSO-*d*₆) δ 0. 93 (t, *J=* 7. 5 Hz, 3 H, CH₂CH₂C*H*₃), 1. 36 (t, *J* = 6. 9 Hz, 3 H, OCH₂C*H*₃), 1. 58-1. 70 (m, 2 H, CH₂C*H*₂CH₃), 2. 58 (t, *J* = 7. 5 Hz, 2 H, C*H*₂CH₂CH₃), 2. 74-2. 91 (m, 2 H, 2 SO₂NCHₐₓ), 3. 18-3. 33 (m, 2 H, 2 SO₂NCH_{eq}), 3. 47-3. 68 (m, 4 H, NC*H*₂CH₂F and 2 ⁺HNC*H*ₐₓ), 3. 73-3. 87 (m, 2 H, 2 ⁺HNC*H*_{eq}), 3. 99 (s, 3 H, NCH₃), 4. 24 (q, *J* = 6. 9 Hz, 2 H, OC*H*₂CH₃), 4. 85 (br d, *J* = 47. 1 Hz, 2 H, NCH₂C*H*₂F), 7. 25 (s, 1 H, H-2), 7. 42 (d, *J* = 8. 7 Hz, 1 H, H-3'), 7. 87 (dd, *J* = 8. 7 Hz, 2. 4 Hz, 1 H, H-4'), 7. 98 (d, *J* = 2. 4 Hz, 1 H, H-6'), 11. 14 (br s, 1 H, NH⁺), 11. 87 (br s, 1 H, NH).

### Example 12

### Preparation of 2-(5-(4-(2-fluoroethyl)piperazin-1-ylsulfonyl)-2-n-propoxyphenyl)-5-methyl-7-n-prop yl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-fluoroethyl)piperazin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(2-fluoroethyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 82%
mp 158-159 °C (EtOAc/hexanes);
IR (neat) 3339 (NH), 1673 (C=O), 1168 (SO₂) cm⁻¹;
¹H NMR (CDCl₃/TMS) δ 0.99 (t, *J* = 7. 5 Hz, 3 H, CH₂CH₂C*H*₃), 1. 19 (t, *J* = 7. 5 Hz, 3 H, OCH₂CH₂C*H*₃), 1. 67-1. 79 (m, 2 H, CH₂C*H*₂CH₃), 1. 98-2. 10 (m, 2 H, OCH₂C*H*₂CH₃), 2. 62-2. 75 (m, 8 H, NC*H*₂CH₂F, 2 NCH₂ and C*H*₂CH₂CH₃), 3. 13 (dd, *J* = 5. 1 Hz, 4. 5 Hz, 4 H, 2 SO₂NCH₂), 4. 08 (s, 3 H, NCH₃), 4. 24 (t, *J* = 6. 5 Hz, 2 H, OC*H*₂CH₂CH₃), 4. 49 (ddd, *J* = 47. 7 Hz, 5. 1 Hz, 4. 5 Hz, 2 H, NCH₂C*H*₂F), 6. 89 (s, I H, H-2), 7. 13 (d, *J* = 9. 0 Hz, 1 H, H-3'), 7. 80 (dd, *J* = 9. 0 Hz, 2. 7 Hz, 1 H, H-4'), 8. 88 (d, *J =* 2. 7 Hz, 1 H, H-6'), 10. 66 (br s, I H, NH); MS (FAB) *m*/*z* 520 (MH⁺).

### Example 13

### Preparation of 2-(5-(4-(2-fluoroethyl)piperazin-1-ylsulfonyl)-2-n-propoxyphenyl)-5-methyl-7-n-prop yl-3,5-dihydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-fluoroethyl)piperazin-1-yl)

The titled compound was prepared as described in Example 2 by using 2-(5-(4-(2-fluoroethyl)piperazin-1-ylsulfonyl)-2-*n*-propoxyphenyl)-5-methyl-7-*n*-prop yl-3,5-dihydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 99%
mp 107 °C dec;
IR (neat) 3351 (NH), 1677 (C=O), 1168 (SO₂) cm⁻¹;
¹H NMR (DMSO-*d*₆) δ 0. 93 (t, *J* = 7. 5 Hz, 3 H, CH₂CH₂C*H*₃), 0. 97 (t, *J* = 7. 5 Hz, 3 H, OCH₂CH₂C*H*₃), 1. 58-1. 70 (m, 2 H, CH₂C*H*₂CH₃), 1. 70-1. 80 (m, 2 H, OCH₂C*H*₂CH₃), 2. 58 (t, *J* = 7. 5 Hz, 2 H, C*H*₂CH₂CH₃), 2. 74-2. 88 (m, 2 H, 2 SO₂NCHₐₓ), 3. 17-3. 34 (m, 2 H, 2 SO₂NCH_{eq}), 3. 46-3. 68 (m, 4 H, NC*H*₂CH₂F and 2 ⁺HNC*H*ₐₓ), 3. 73-3. 87 (m, 2 H, 2 ⁺HNC*H*_{eq}), 3.99 (s, 3 H, NCH₃), 4. 15 (t, *J* = 6. 6 Hz, 2 H, OC*H*₂CH₂CH₃), 4. 84 (br d, *J* = 48. 0 Hz, 2 H, NCH₂C*H*₂F), 7. 24 (s, 1 H, H-2), 7. 43 (d, *J* = 8. 7 Hz, 1 H, H-3'), 7. 87 (dd, *J* = 8. 7 Hz, 2. 4 Hz, 1 H, H-4'), 7. 98 (d, *J=* 2.4 Hz, 1 H, H-6'), 11. 03 (br s, 1 H, NH⁺), 11. 80 (br s, 1 H, NH).

### Example 14

### Preparation of 2-(2-ethoxy-5-(4-(3-fluoropropyl)piperazin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R' = CH₃, R² = H, R³ = CH₂CH₂CH₃, R⁴ = CH₂CH₃; NR⁶R⁷ is 4-(3-fluoropropyl)piperazin-1-yl)

The titled compound was prepared as described in Example 1 by using 1-(3-fluoropropyl)piperazine hydrochloride in place of 1-methylpiperazine.
yield: 85%
mp 217. 5-218 °C (EtOAc/CHCl₃/hexanes);
IR (neat) 3333 (NH), 1676 (C=O), 1169 (SO₂) cm⁻¹;
¹H NMR (CDCl₃/TMS) δ 0. 99 (t, *J* = 7. 5 Hz, 3 H, CH₂CH₂C*H*₃), 1. 64 (t, *J* = 6. 9 Hz, 3 H, OCH₂C*H*₃), 1. 69-1. 89 (m, 4 H, CH₂C*H*₂CH₂F and CH₂C*H*₂CH₃), 2. 47 (t, *J* = 7. 5 Hz, 2 H, NC*H*₂CH₂), 2. 54 (dd, *J* = 4. 8 Hz, 4. 5 Hz, 4 H, 2 NCH₂), 2. 71 (t, *J* = 7. 5 Hz, 2 H, C*H*₂CH₂CH₃), 3. 10 (dd, *J* = 4. 8 Hz, 4. 5 Hz, 4 H, 2 SO₂NCH₂), 4. 08 (s, 3 H, NCH₃), 4. 35 (q, *J* = 6.9 Hz, 2 H, OC*H*₂CH₃), 4. 43 (dt, *J* = 46. 8 Hz, 6. 0 Hz, 2 H, CH₂C*H*₂F), 6. 89 (s, 1 H, H-2), 7. 13 (d, *J* = 8. 7 Hz, 1 H, H-3'), 7. 81 (dd, *J* = 8. 7 Hz, 2. 4 Hz, 1 H, H-4'), 8. 86 (d, *J* = 2. 4 Hz, 1 H, H-6'), 10. 63 (br s, 1 H, NH); MS (FAB) *m*/*z* 520 (MH⁺).

### Example 15

### Preparation of 2-(2-ethoxy-5-(4-(3-fluoropropyl)piperazin-1-ylsulfonyl)phenyl)-7-ethyl-5-methyl-3,5 - dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₃, R⁴ = CH₂CH₃; NR⁶R⁷ is 4-(3-fluoropropyl)piperazin-1-yl)

A mixture of 4-(2-ethoxy-5-(4-(3-fluoropropyl)piperazin-1-ylsulfonyl)benzamido)-3-ethyl-1-methyl pyrrole-5-carboxamide (270 mg, 0.52 mmol) and *t*-BuOK (244 mg, 2.16 mmol) in anhydrous *t*-BuOH (10 mL) was heated at 80 °C under nitrogen atmosphere for 5 h. The reaction mixture was cooled to room temperature, and was evaporated to dryness under vacuum. The resulting residue was diluted with water (20 mL), acidified to about pH 8 with 1 N aqueous HCl solution, and was extracted with CH₂Cl₂ (2 x 50 mL). Combined organic layer was dried (MgSO₄), filtered, and evaporated to dryness *in vacuo* to afford a white solid. The crude product was purified by MPLC on silica gel (3% MeOH in CHCl₃) to afford the titled compound (247 mg, 95%) as a white solid. Analytically pure compound was obtained by crystallization from CH₂Cl₂/EtOAc/hexanes.
mp 193-194 °C;
IR (neat) 3328 (NH), 1670 (C=O), 1165 (SO₂) cm⁻¹;
¹H NMR (CDCl₃/TMS) δ 1. 32 (t, *J* = 7. 5 Hz, 3 H, CH₂C*H*₃), 1. 64 (t, *J* = 7. 2 Hz, 3 H, OCH₂C*H*₃), 1. 71-1. 89 (m, 2 H, C*H*₂CH₂F), 2. 47 (t, *J =* 7. 5 Hz, 2 H, NC*H*₂CH₂), 2. 54 (dd, *J* = 5. 1 Hz, 4. 8 Hz, 4 H, 2 NCH₂), 2. 77 (q, *J* = 7. 5 Hz, 2 H, C*H*₂CH₃), 3.10 (dd, *J* = 5. 1 Hz, 4. 8 Hz, 4 H, 2 SO₂NCH₂), 4. 08 (s, 3 H, NCH₃), 4. 36 (q, *J* = 7. 2 Hz, 2 H, OC*H*₂CH₃), 4. 43 (dt, *J* = 47. 1 Hz, 6. 0 Hz, 2 H, CH₂C*H*₂F), 6. 90 (s, 1 H, H-2), 7. 13 (d, *J =* 9. 0 Hz, 1 H, H-3'), 7. 80 (dd, *J* = 9. 0 Hz, 2. 4 Hz, 1 H, H-4'), 8. 87 (d, *J=* 2. 4 Hz, 1 H, H-6'), 10.64 (br s, 1 H, NH); MS (FAB) *m*/*z* 506 (MH⁺).

### Example 16

### Preparation of 2-(2-ethoxy-5-(4-(3-fluoropropyl)piperazin-1-ylsulfonyl)phenyl)-7-(3-fluoropropyl)-5-methyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₂F, R⁴ = CH₂CH₃; NR⁶R⁷ is 4-(3-fluoropropyl)piperazin-1-yl)

The titled compound was prepared as described in Example 15 by using 4-(2-ethoxy-5-(4-(3-fluoropropyl)piperazin-1-ylsulfonyl)benzamido)-3-(3-fluoropropy 1)-1-methylpyrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 87%
mp 225 °C dec (CH₂Cl₂/ether);
IR (neat) 3330 (NH), 1680 (C=O), 1171 (SO₂) cm⁻¹;
¹H NMR (CDCl₃/TMS) δ 1. 64 (t, *J* = 7. 2 Hz, 3 H, OCH₂C*H*₃), 1. 71-1. 88 (m, 2 H, NCH₂C*H*₂CH₂F), 2. 03-2. 23 (m, 2 H, CH₂C*H*₂CH₂F), 2. 47 (t, *J* = 7. 2 Hz, 2 H, NC*H*₂CH₂), 2. 54 (dd, *J* = 5. 1 Hz, 4. 5 Hz, 4 H, 2 NCH₂), 2. 86 (t, *J* = 7. 5 Hz, 2 H, C*H*₂CH₂CH₂F), 3.10 (dd, *J* = 5. 1 Hz, 4. 5 Hz, 4 H, 2 SO₂NCH₂), 4. 09 (s, 3 H, NCH₃), 4. 36 (q, *J* = 7. 2 Hz, 2 H, OC*H*₂CH₃), 4. 41 (dt, *J* = 47. 1 Hz, 6. 0 Hz, 2 H, NCH₂CH₂C*H*₂F), 4. 52 (dt, *J* = 47. 4 Hz, 6. 0 Hz, 2 H, CH₂CH₂C*H*₂F), 6. 92 (s, 1 H, H-2), 7. 13 (d, *J* = 9. 0 Hz, 1 H, H-3'), 7. 82 (dd, *J =* 9. 0 Hz, 2. 4 Hz, 1 H, H-4'), 8. 86 (d, *J* = 2. 4 Hz, 1 H, H-6'), 10. 67 (br s, 1 H, NH); MS (FAB) *m*/*z* 538 (MH⁺).

### Example 17

### Preparation of 7-cyclopropylmethyl-2-(2-ethoxy-5-(4-(3-fluoropropyl)piperazin-1-ylsulfonyl)phenyl) -5-methyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = cyclopropylmethyl, R⁴ = CH₂CH₃; NR⁶R⁷ is 4-(3-fluoropropyl)piperazin-1-yl)

The titled compound was prepared as described in Example 15 by using 3-cyclopropylmethyl-4-(2-ethoxy-5-(4-(3-fluoropropyl)piperazin-1-ylsulfonyl)benzam ido)-1-methylpyrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 92%
mp 223-224. 5 °C (CH₂Cl₂/EtOAc/hexanes);
IR (neat) 3328 (NH), 1685 (C=O), 1171 (SO₂) cm⁻¹;
¹H NMR (CDCl₃/TMS) δ 0. 27-0. 32 (m, 2 H, c-C₃H₅), 0. 49-0. 55(m, 2 H, c-C₃H₅), 0. 98-1. 12(m, 1 H, c-C₃H₅), 1. 64(t, *J =* 6. 9 Hz, 3 H, OCH₂C*H*₃), 1. 70-1. 89(m, 2H, C*H*₂CH₂F), 2. 47 (t, *J* = 7. 5 Hz, 2 H, NC*H*₂CH₂), 2. 54 (dd, *J* = 4. 8 Hz, 4. 5 Hz, 4 H, 2 NCH₂), 2. 65 (d, *J* = 6. 9 Hz, 2 H, CHC*H*₂), 3. 10 (dd, *J* = 4. 8 Hz, 4. 5 Hz, 4 H, 2 SO₂NCH₂), 4. 10 (s, 3 H, NCH₃), 4. 36 (q, *J* = 6. 9 Hz, 2 H, OC*H*₂CH₃), 4. 43 (dt, *J* = 47. 1 Hz, 6. 0 Hz, 2 H, CH₂C*H*₂F), 7.00 (s, 1 H, H-2), 7.13 (d, *J* = 8. 7 Hz, 1 H, H-3'), 7. 80 (dd, *J =* 8. 7 Hz, 2. 4 Hz, 1 H, H-4'), 8. 87 (d, *J =* 2. 4 Hz, 1 H, H-6'), 10. 65 (br s, 1 H, NH); MS (FAB) *m*/*z* 532 (MH⁺).

### Example 18

### Preparation of 2-(2-ethoxy-5-(4-(3-fluoropropyl)piperazin-1-ylsulfonyl)phenyl)-5-ethyl-7-ethyl-3,5-d ihydro-4H-pyrrolo[3,2-d]pyrimidin-4-one (a compound of the formula (1) wherein R⁵ = SO₂NR⁶R⁷, R¹ = CH₂CH₃, R² = H, R³ = CH₂CH₃, R⁴ = CH₂CH₃; NR⁶R⁷ is 4-(3-fluoropropyl)piperazin-1-yl)

The titled compound was prepared as described in Example 15 by using 4-(2-ethoxy-5-(4-(3-fluoropropyl)piperazin-1-ylsulfonyl)benzamido)-1-ethyl-3-ethylp yrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 87%
mp 182-182. 5 °C (CH₂Cl₂/EtOAc/ether);
IR (neat) 3334 (NH), 1681 (C=O), 1166 (SO₂) cm⁻¹;
¹H NMR (CDCl₃/TMS) δ 1. 33 (t, *J* = 7. 5 Hz, 3 H, CH₂C*H*₃), 1. 48 (t, *J* = 7. 2 Hz, 3 H, NCH₂C*H*₃), 1. 65 (t, *J* = 6. 9 Hz, 3 H, OCH₂C*H*₃), 1. 71-1. 89 (m, 2 H, CH₂C*H*₂CH₂F), 2. 47 (t, *J* = 7. 2 Hz, 2 H, C*H*₂CH₂CH₂F), 2. 54 (dd, *J* = 4. 8 Hz, 4. 5 Hz, 4 H, 2 NCH₂), 2. 78 (q, *J* = 7. 5 Hz, 2 H, C*H*₂CH₃), 3. 08 (dd, *J* = 4. 8 Hz, 4. 5 Hz, 4 H, 2 SO₂NCH₂), 4. 36 (q, *J* = 6. 9 Hz, 2 H, OC*H*₂CH₃), 4. 43 (dt, *J* = 47. 1 Hz, 6. 0 Hz, 2 H, CH₂CH₂C*H*₂F), 4. 46 (q, *J* = 7. 2 Hz, 2 H, NC*H*₂CH₃), 6. 98 (s, 1 H, H-2), 7. 13 (d, *J* = 8. 7 Hz, 1 H, H-3'), 7. 80 (dd, *J* = 8. 7 Hz, 2. 4 Hz, 1 H, H-4'), 8. 89 (d, *J* = 2. 4 Hz, 1 H, H-6'), 10. 67 (br s, 1 H, NH); MS (FAB) *m*/*z* 520 (MH⁺).

### Example 19

### Preparation of 2-(2-ethoxy-5-(4-(3-fluoropropyl)piperazin-1-ylsulfonyl)phenyl)-5-ethyl-7-n-propyl-3, 5-dihydro-4H-pyrrolo[3,2-d]pyrimidin-4-one (a compound of the formula (1) wherein R⁵ = SO₂NR⁶R⁷, R¹ = CH₂CH₃, R² = H, R³ = CH₂CH₂CH₃, R⁴ = CH₂CH₃; NR⁶R⁷ is 4-(3-fluoropropyl)piperazin-1-yl)

The titled compound was prepared as described in Example 15 by using 4-(2-ethoxy-5-(4-(3-fluoropropyl)piperazin-1-ylsulfonyl)benzamido)-1-ethyl-3-*n*-prop yl pyrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 95%
mp 163-164 °C (EtOAc/ether/hexanes);
IR (neat) 3332 (NH), 1671 (C=O), 1174 (SO₂) cm⁻¹;
¹H NMR (CDCl₃/TMS) δ 1. 00 (t, *J* = 7. 5 Hz, 3 H, CH₂CH₂C*H*₃), 1. 02 (t, *J* = 7. 2 Hz, 3 H, CH₂NCH₂C*H*₃), 1. 48 (t, *J* = 7. 2 Hz, 3 H, NCH₂C*H*₃), 1. 64 (t, *J* = 6. 9 Hz, 3 H, OCH₂C*H*₃), 1. 68-1. 80 (m, 2 H, CH₂C*H*₂CH₃), 2. 40 (q, *J* = 7. 2 Hz, 2 H, CH₂NC*H*₂CH₃), 2. 53 (dd, *J* = 5. 1 Hz, 4. 8 Hz, 4 H, 2 NCH₂), 2. 72 (q, *J* = 7. 5 Hz, 2 H, C*H*₂CH₂CH₃), 3. 11 (br dd, *J* = 5. 1 Hz, 4. 8 Hz, 4 H, 2 SO₂NCH₂), 4. 35 (q, *J* = 6. 9 Hz, 2 H, OC*H*₂CH₃), 4. 45 (q, *J* = 7. 2 Hz, 2 H, NC*H*₂CH₃), 6. 69 (s, 1 H, H-2), 7. 12 *(d, J* = 8. 7 Hz, 1 H, H-3'), 7. 80 (dd, *J* = 8. 7 Hz, 2. 4 Hz, 1 H, H-4'), 8. 88 (d, *J* = 2. 4 Hz, 1 H, H-6'), 10. 65 (br s, 1 H, NH); MS (FAB) *m*/*z* 502 (MH⁺).

### Example 20

### Preparation of 2-(2-ethoxy-5-(4-isopropylpiperazin-1-ylsulfonyl)phenyl)-5-ethyl-7-n-propyl-3,5-dihy dro-4H-pyrrolo[3,2-d]pyrimidin-4-one (a compound of the formula (1) wherein R⁵ = SO₂NR⁶R⁷, R¹ = CH₂CH₃, R² = H, R³ = CH₂CH₂CH₃, R⁴ = CH₂CH₃; NR⁶R⁷ is 4-isopropylpiperazin-1-yl)

The titled compound was prepared as described in Example 15 by using 4-(2-ethoxy-5-(4-isopropylpiperazin-1-ylsulfonyl)benzamido)-1-ethyl-3-*n*-propylpyrro le-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 90%
mp 179. 5-180 °C (CH₂Cl₂/EtOAc/hexanes);
IR (neat) 3331 (NH), 1678 (C=O), 1174 (SO₂) cm⁻¹;
¹H NMR (CDCl₃/TMS) δ 0. 99 (t, *J* = 7. 5 Hz, 3 H, CH₂CH₂C*H*₃), 1. 00 (d, *J* = 6. 6 Hz, 6 H, CH(C*H*₃)₂), 1. 48 (t, *J* = 7. 2 Hz, 3 H, NCH₂C*H*₃), 1. 64 (t, *J* = 6. 9 Hz, 3 H, OCH₂C*H*₃), 1. 68-1. 80 (m, 2 H, CH₂C*H*₂CH₃), 2. 60 (dd, *J* = 4. 8 Hz, 4. 5 Hz, 4 H, 2 NCH₂), 2. 65-2. 74 (m, 1H, C*H*(CH₃)₂), 2. 72 (t, *J* = 7. 5 Hz, 2 H, C*H*₂CH₂CH₃), 3. 09 (br dd, *J* = 4. 8 Hz, 4. 5 Hz, 4 H, 2 SO₂NCH₂), 4. 35 (q, *J* = 6. 9 Hz, 2 H, OC*H*₂CH₃), 4. 45 (q, *J* = 7. 2 Hz, 2 H, NC*H*₂CH₃), 6. 96 (s, 1 H, H-2), 7. 11 (d, *J =* 8. 7 Hz, 1 H, H-3'), 7. 80 (dd, *J* = 8. 7 Hz, 2. 4 Hz, 1 H, H-4'), 8. 88 (d, *J* = 2. 4 Hz, 1 H, H-6'), 10. 65 (br s, 1 H, NH); MS (FAB) *m*/*z* 516 (MH⁺).

### Example 21

### Preparation of 2-(2-ethoxy-5-(4-(3-fluoropropyl)piperazin-1-ylsulfonyl)phenyl)-5-ethyl-7-n-propyl-3, 5-dihydro-4H-pyrrolo[3,2-d]pyrimidin-4-one (a compound of the formula (1) wherein R⁵ = SO₂NR⁶R⁷, R¹ = CH₂CH₃, R² = H, R³ = CH₂CH₂CH₃, R⁴ = CH₂CH₃; NR⁶R⁷ is 4-(3-fluoropropyl)piperazin-1-yl)

The titled compound was prepared as described in Example 15 by using 4-(2-ethoxy-5-(4-(3-fluoropropyl)piperazin-1-ylsulfonyl)benzamido)-1-ethyl-3-*n*-prop yl pyrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 93%
mp 187-188 °C (CH₂Cl₂/EtOAc/ether);
IR (neat) 3334 (NH), 1676 (C=O), 1173 (SO₂) cm⁻¹;
¹H NMR (CDCl₃/TMS) δ 1. 00 (t, *J* = 7. 5 Hz, 3 H, CH₂CH₂C*H*₃), 1. 48 (t, *J* = 7. 2 Hz, 3 H, NCH₂C*H*₃), 1. 64 (t, *J* = 6. 9 Hz, 3 H, OCH₂C*H*₃), 1. 68-1. 89 (m, 4 H, CH₂C*H*₂CH₃ and CH₂C*H*₂CH₂F), 2. 47 (t, *J* = 7. 2 Hz, 2 H, C*H*₂CH₂CH₂F), 2. 54 (dd, *J* = 5. 1 Hz, 4. 8 Hz, 4 H, 2 NCH₂), 2. 72 (t, *J* = 7. 5 Hz, 2 H, C*H*₂CH₂CH₃), 3. 10 (br dd, *J* = 5. 1 Hz, 4. 8 Hz, 4 H, 2 SO₂NCH₂), 4. 36 (q, *J* = 6. 9 Hz, 2 H, OC*H*₂CH₃), 4. 43(dt, *J* = 47. 1 Hz, 6. 0 Hz, 2 H, CH₂CH₂C*H*₂F), 4. 46 (q, *J* = 7. 2 Hz, 2 H, NC*H*₂CH₃), 6. 97 (s, 1 H, H-2), 7. 13 (d, *J* = 8. 7 Hz, 1 H, H-3'), 7. 81 (dd, *J* = 8. 7 Hz, 2. 4 Hz, 1 H, H-4'), 8. 89 (d, *J* = 2. 4 Hz, 1 H, H-6'), 10. 67 (br s, 1 H, NH); MS (FAB) *m*/*z* 534 (MH⁺).

### Example 22

### Preparation of 2-(2-ethoxy-5-(4-(2-hydroxyethyl)piperazin-1-ylsulfonyl)phenyl)-5-ethyl-7-n-propyl-3 ,5-dihydro-4H-pyrrolo[3,2-d]pyrimidin-4-one (a compound of the formula (1) wherein R⁵ = SO₂NR⁶R⁷, R¹ = CH₂CH₃, R² = H, R³ = CH₂CH₂CH₃, R⁴ = CH₂CH₃; NR⁶R⁷ is 4-(2-hydroxyethyl)piperazin-1-yl)

The titled compound was prepared as described in Example 15 by using 4-(2-ethoxy-5-(4-(2-hydroxyethyl)piperazin-1-ylsulfonyl)benzamido)-1-ethyl-3-*n*-pro pyl pyrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 93%
mp 159-160. 5 °C (EtOAc/ether/hexanes);
IR (neat) 3324 (NH), 1688 (C=O), 1167 (SO₂) cm⁻¹;
¹H NMR (CDCl₃/TMS) δ 1. 00 (t, *J =* 7. 5 Hz, 3 H, CH₂CH₂C*H*₃), 1. 48 (t, *J* = 7. 2 Hz, 3 H, NCH₂C*H*₃), 1. 65 (t, *J* = 6. 9 Hz, 3 H, OCH₂C*H*₃), 1. 68-1. 81 (m, 2 H, CH₂C*H*₂CH₃), 2. 55 (t, *J* = 5. 4 Hz, 2 H, NC*H*₂CH₂OH), 2. 61 (dd, *J* = 5. 1 Hz, 4. 8 Hz, 4 H, 2 NCH₂), 2. 72 (t, *J* = 7. 5 Hz, 2 H, C*H*₂CH₂CH₃), 3. 12 (br dd, *J* = 5. 1 Hz, 4. 8 Hz, 4 H, 2 SO₂NCH₂), 3. 57 (t, *J* = 5. 4 Hz, 2 H, NCH₂C*H*₂OH), 4. 37 (q, *J* = 6. 9 Hz, 2 H, OC*H*₂CH₃), 4. 46 (q, *J* = 7. 2 Hz, 2 H, NC*H*₂CH₃), 6. 97 (s, 1 H, H-2), 7. 14 (d, *J* = 8. 7 Hz, 1 H, H-3'), 7. 82 (dd, *J* = 8. 7 Hz, 2. 7 Hz, 1 H, H-4'), 8. 89 (d, *J* = 2. 7 Hz, 1 H, H-6'), 10. 67 (br s, 1 H, NH); MS (FAB) *m*/*z* 518 (MH⁺).

### Example 23

### Preparation of 2-(2-ethoxy-5-(4-ethylpiperazin-1-ylsulfonyl)phenyl)-5-ethyl-7-(3-fluoropropyl)-3,5-d ihydro-4H-pyrrolo[3,2-d]pyrimidin-4-one (a compound of the formula (1) wherein R⁵ = SO₂NR⁶R⁷, R¹ = CH₂CH₃, R² = H, R³ = CH₂CH₂CH₂F, R⁴ = CH₂CH₃; NR⁶R⁷ is 4-ethylpiperazin-1-yl)

The titled compound was prepared as described in Example 15 by using 4-(2-ethoxy-5-(4-ethylpiperazin-1-ylsulfonyl)benzamido)-1-ethyl-3-(3-fluoropropyl)p yrrole-5-carboxamide in place of 4-(2-ethoxy-5-(4-(3-fluoropropyl)piperazin-1-ylsulfonyl)benzamido)-3-ethyl-1-methyl pyrrole-5-carboxamide. yield: 93%
mp 161-162 °C (EtOAc/ether/hexanes);
IR (neat) 3330 (NH), 1680 (C=O), 1171 (SO₂) cm⁻¹;
¹H NMR (CDCl₃/TMS) δ 1. 02 (t, *J* = 7. 2 Hz, 3 H, CH₂NCH₂C*H*₃), 1. 49 (t, *J* = 7. 2 Hz, 3 H, CHNCH₂C*H*₃), 1. 65 (t, *J* = 6. 9 Hz, 3 H, OCH₂C*H*₃), 2. 06-2. 22 (m, 2 H, CH₂C*H*₂CH₂F), 2. 40 (t, *J* = 7. 2 Hz, 2 H, CH₂NC*H*₂CH₃), 2. 53 (dd, *J* = 5. 1 Hz, 4. 5 Hz, 4 H, 2 NCH₂), 2. 87 (t, *J* = 7. 5 Hz, 2 H, C*H*₂CH₂CH₂F), 3. 11 (br dd, *J* = 5. 1 Hz, 4. 5 Hz, 4 H, 2 SO₂NCH₂), 4. 36 (q, *J* = 6. 9 Hz, 2 H, OC*H*₂CH₃), 4. 46 (q, *J* = 7. 2 Hz, 2 H, CHNC*H*₂CH₃), 4. 52 (dt, *J* = 47. 4 Hz, 6. 0 Hz, 2 H, CH₂CH₂C*H*₂F), 7. 00 (s, 1 H, H-2), 7. 12 (d, *J* = 8. 7 Hz, 1 H, H-3'), 7. 81 (dd, *J* = 8. 7 Hz, 2. 4 Hz, 1 H, H-4'), 8. 87 (d, *J* = 2. 4 Hz, 1 H, H-6'), 10. 71 (br s, 1 H, NH); MS (FAB) *m*/*z* 520 (MH⁺).

### Example 24

### Preparation of 2-(5-(4-ethylpiperazin-1-ylsulfonyl)-2-n-propoxyphenyl)-5-ethyl-7-(3-fluoropropyl)-3, 5-dihydro-4H-pyrrolo[3,2-d]pyrimidin-4-one (a compound of the formula (1) wherein R⁵ = SO₂NR⁶R⁷, R¹ = CH₂CH₃, R² = H, R³ = CH₂CH₂CH₂F, R⁴ = CH₂CH₂CH₃; NR⁶R⁷ is 4-ethylpiperazin-1-yl)

The titled compound was prepared as described in Example 15 by using 4-(5-(4-ethylpiperazin-1-ylsulfonyl)-2-*n*-propoxybenzamido)-1-ethyl-3-(3-fluoropropy l)pyrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 92%
mp 137-138 °C (EtOAc/hexanes);
IR (neat) 3320 (NH), 1688 (C=O), 1168 (SO₂) cm⁻¹;
¹H NMR (CDCl₃/TMS) δ 1. 02 (t, *J* = 7. 5 Hz, 3 H, OCH₂CH₂C*H*₃), 1. 19 (t, *J* = 7. 2 Hz, 3 H, CH₂NCH₂C*H*₃), 1. 49 (t, *J* = 7. 2 Hz, 3 H, CHNCH₂C*H*₃), 1. 99-2. 23 (m, 4 H, CH₂C*H*₂CH₂F and OCH₂C*H*₂CH₃), 2. 40 (q, *J* = 7. 2 Hz, 2 H, CH₂NC*H*₂CH₃), 2. 53 (dd, *J* = 5. 1 Hz, 4. 8 Hz, 4 H, 2 NCH₂), 2. 87 (t, *J* = 7. 5 Hz, 2 H, C*H*₂CH₂CH₂F), 3. 11 (br dd, *J* = 5. 1 Hz, 4. 8 Hz, 4 H, 2 SO₂NCH₂), 4. 24 (t, *J* = 6. 3 Hz, 2 H, OC*H*₂CH₂CH₃), 4. 46 *(q, J* = 7. 2 Hz, 2 H, CHNC*H*₂CH₃), 4. 52 (dt, *J* = 47. 1 Hz, 6. 0 Hz, 2 H, CH₂CH₂C*H*₂F), 6. 99 (s, 1 H, H-2), 7. 13 (d, *J* = 8. 7 Hz, 1 H, H-3'), 7. 81 (dd, *J* = 8. 7 Hz, 2. 4 Hz, 1 H, H-4'), 8. 88 (d, *J* = 2. 4 Hz, 1 H, H-6'), 10. 73 (br s, 1 H, NH); MS (FAB) *m*/*z* 552 (MH⁺).

### Example 25

### Preparation of 2-(2-ethoxy-5-(4-isopropylpiperazin-1-ylsulfonyl)phenyl)-5-ethyl-7-(3-fluoropropyl)-3,5-dihydro-4H-pyrrolo[3,2-d]pyrimidin-4-one (a compound of the formula (1) wherein R⁵ = SO₂NR⁶R⁷, R¹ = CH₂CH₃, R² = H, R³ = CH₂CH₂CH₂F, R⁴ = CH₂CH₃; NR⁶R⁷ is 4-isopropylpiperazin-1-yl)

The titled compound was prepared as described in Example 15 by using 4-(2-ethoxy-5-(4-isopropylpiperazin-1-ylsulfonyl)benzamido)-1-ethyl-3-(3-fluoroprop yl)pyrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 82%
mp 182-182. 5 °C (CH₂Cl₂/EtOAc/ether);
IR (neat) 3330 (NH), 1677 (C=O), 1173 (SO₂) cm⁻¹;
¹H NMR (CDCl₃/TMS) δ 0. 98 (d, *J* = 6. 6 Hz, 6 H, CH(C*H*₃)₂), 1. 49 (t, *J* = 7. 2 Hz, 3 H, NCH₂C*H*₃), 1. 65 (t, *J* = 6. 9 Hz, 3 H, OCH₂C*H*₃), 2. 04-2. 23 (m, 2 H, CH₂C*H*₂CH₂F), 2. 60 (dd, *J* = 5. 1 Hz, 4. 8 Hz, 4 H, 2 NCH₂), 2. 62-2. 71 (m, 1 H, C*H*(CH₃)₂), 2. 87 (t, *J* = 7. 5 Hz, 2 H, C*H*₂CH₂CH₂F), 3. 09 (br dd, *J* = 5. 1 Hz, 4. 8 Hz, 4 H, 2 SO₂NCH₂), 4. 35 (q, *J* = 6. 9 Hz, 2 H, OC*H*₂CH₃), 4. 45 (q, *J* = 7. 2 Hz, 2 H, NC*H*₂CH₃), 4. 52 (dt, *J* = 47. 4 Hz, 6. 0 Hz, 2 H, CH₂CH₂C*H*₂F), 6. 99 (s, 1 H, H-2), 7. 11 (d, *J* = 8. 7 Hz, 1 H, H-3'), 7. 80 (dd, *J* = 8. 7 Hz, 2. 4 Hz, 1 H, H-4'), 8. 86 (d, *J* = 2. 4 Hz, 1 H, H-6'), 10. 70 (br s, 1 H, NH); MS (FAB) *m*/*z* 534 (MH⁺).

### Example 26

### Preparation of 2-(2-ethoxy-5-(4-(3-fluoropropyl)piperazin-1-ylsulfonyl)phenyl)-5-ethyl-7-(3-fluoropr opyl)-3,5-dihydro-4H-pyrrolo[3,2-d]pyrimidin-4-one (a compound of the formula (1) wherein R⁵ = SO₂NR⁶R⁷, R¹ = CH₂CH₃, R² = H, R³ = CH₂CH₂CH₂F, R⁴ = CH₂CH₃; NR⁶R⁷ is 4-(3-fluoropropyl)piperazin-1-yl)

The titled compound was prepared as described in Example 15 by using 4-(2-ethoxy-5-(4-(3-fluoropropyl)piperazin-1-ylsulfonyl)benzamido)-1-ethyl-3-(3-fluo ropropyl)pyrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 93%
mp 194-194. 5 °C (CH₂Cl₂/EtOAc/ether);
IR (neat) 3330 (NH), 1682 (C=O), 1166 (SO₂) cm⁻¹;
¹H NMR (CDCl₃/TMS) δ 1. 49 (t, *J* = 7. 2 Hz, 3 H, NCH₂C*H*₃), 1. 65 (t, *J* = 6. 9 Hz, 3 H, OCH₂C*H*₃), 1. 71-1. 89 (m, 2 H, NCH₂C*H*₂CH₂F), 2. 05-2. 23 (m, 2 H, CH₂C*H*₂CH₂F), 2. 47 (t, *J* = 7. 2 Hz, 2 H, NC*H*₂CH₂CH₂F), 2. 54 (dd, *J* = 5. 1 Hz, 4. 8 Hz, 4 H, 2 NCH₂), 2. 87 (t, *J* = 7. 5 Hz, 2 H, C*H*₂CH₂CH₂F), 3. 10 (br dd, *J* = 5. 1 Hz, 4. 8 Hz, 4 H, 2 SO₂NCH₂), 4. 36 (q, *J* = 6. 9 Hz, 2 H, OC*H*₂CH₃), 4. 43 (dt, *J* = 47. 4 Hz, 6. 0 Hz, 2 H, NCH₂CH₂C*H*₂F), 4. 46 (q, *J* = 7. 2 Hz, 2 H, NC*H*₂CH₃), 4. 52 (dt, *J* = 47. 4 Hz, 6. 0 Hz, 2 H, CH₂CH₂C*H*₂F), 7. 00 (s, 1 H, H-2), 7. 13 (d, *J* = 8. 7 Hz, 1 H, H-3'), 7. 82 (dd, *J =* 8. 7 Hz, 2. 7 Hz, 1 H, H-4'), 8. 87 (d, *J* = 2. 7 Hz, 1 H, H-6'), 10. 70 (br s, 1 H, NH); MS (FAB) *m*/*z* 552 (MH⁺).

### Example 27

### Preparation of 2-(2-ethoxy-5-(4-(3-fluoropropyl)piperazin-1-ylsulfonyl)phenyl)-5-ethyl-7-(3-fluoropr opyl)-3,5-dihydro-4H-pyrrolo[3,2-d]pyrimidin-4-one hydrogen sulfate (a compound of the formula (1) wherein R⁵ = SO₂NR⁶R⁷, R¹ = CH₂CH₃, R² = H, R³ = CH₂CH₂CH₂F, R⁴ = CH₂CH₃; NR⁶R⁷ is 4-(3-fluoropropyl)piperazin-1-yl)

The titled compound was prepared as described in Example 2 by using 2-(2-ethoxy-5-(4-(3-fluoropropyl)piperazin-1-ylsulfonyl)phenyl)-5-ethyl-7-(3-fluoropr opyl)-3,5-dihydro-4*H*-pyrrolo[3,2-*d*]pyrimidin-4-one and 10% ethanolic H₂SO₄ solution in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one and 1 *N* HCl ethereal solution.
yield: 95%
mp 90. 5-91°C;
IR (neat) 3330 (NH), 1717 (C=O), 1162 (SO₂) cm⁻¹;
¹H NMR (DMSO-*d*₆) δ 1.37 (t, *J* = 7. 1 Hz, 6 H, NCH₂C*H*₃ and OCH₂C*H*₃), 1, 91-2. 12 (m, 4 H, CH₂C*H*₂CH₂F and NCH₂C*H*₂CH₂F), 2. 55-2. 67 (m, 2 H, NC*H*₂CH₂CH₂F), 2. 71 (t, *J* = 7. 5 Hz, 2 H, C*H*₂CH₂CH₂F), 3. 14-3. 28 (m, 4 H, 2 SO₂NCH₂), 3. 52-3. 63 (m, 2 H, 2 ⁺HNC*H*ₐₓ), 3. 75-3. 86 (m, 2 H, 2 ⁺HNC*H*_{eq}), 4. 25 (q, *J* = 6. 9 Hz, 2 H, OC*H*₂CH₃), 4. 38 (q, *J* = 7. 2 Hz, 2 H, NC*H*₂CH₃), 4. 51 (dt, *J* = 47. 4 Hz, 6. 0 Hz, 4 H, CH₂CH₂C*H*₂F and NCH₂CH₂C*H*₂F), 7. 39 (s, 1 H, H-2), 7. 43 (d, *J* = 8. 7 Hz, 1 H, H-3'), 7. 88 (dd, *J* = 8. 7 Hz, 2. 4 Hz, 1 H, H-4'), 7.97 (d, *J* = 2. 4 Hz, 1 H, H-6'), 9. 22 (br s, 1 H, NH), 10. 86 (br s, 1 H, NH⁺).

### Example 28

### Preparation of 2-(5-(4-(3-fluoropropyl)piperazin-1-ylsulfonyl)-2-n-propoxyphenyl)-5-ethyl-7-(3-fluor opropyl)-3,5-dihydro-4H-pyrrolo[3,2-d]pyrimidin-4-one (a compound of the formula (1) wherein R⁵ = SO₂NR⁶R⁷, R' = CH₂CH₃, R² = H, R³ = CH₂CH₂CH₂F, R⁴ = CH₂CH₂CH₃; NR⁶R⁷ is 4-(3-fluoropropyl)piperazin-1-yl)

The titled compound was prepared as described in Example 15 by using 4-(5-(4-(3-fluoropropyl)piperazin-1-ylsulfonyl)-2-*n*-propoxybenzamido)-1-ethyl-3-(3-fluoropropyl)pyrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 94%
mp 143-144 °C (EtOAc/hexanes);
IR (neat) 3318 (NH), 1687 (C=O), 1170 (SO₂) cm⁻¹;
¹H NMR (CDCl₃/TMS) δ 1. 20 (t, *J* = 7. 5 Hz, 3 H, OCH₂CH₂C*H*₃), 1. 49 (t, *J* = 7. 2 Hz, 3 H, NCH₂C*H*₃), 1. 71-1. 88 (m, 2 H, NCH₂C*H*₂CH₂F), 1. 99-2. 23 (m, 4 H, CH₂C*H*₂CH₂F and OCH₂C*H*₂CH₃), 2. 47 (t, *J* = 7. 2 Hz, 2 H, NC*H*₂CH₂CH₂F), 2. 54 (dd, *J* = 4. 8 Hz, 4. 5 Hz, 4 H, 2 NCH₂), 2. 87 (t, *J* = 7. 5 Hz, 2 H, C*H*₂CH₂CH₂F), 3.10 (m, 4 H, 2 SO₂NCH₂), 4. 25 *(t, J* = 6. 3 Hz, 2 H, OC*H*₂CH₂CH₃), 4. 43 (dt, *J* = 47. 1 Hz, 6. 0 Hz, 2 H, NCH₂CH₂C*H*₂F), 4. 45 (t, *J* = 7. 2 Hz, 2 H, NC*H*₂CH₃), 4. 52 (dt, *J* = 47. 4 Hz, 6. 0 Hz, 2 H, CH₂CH₂CH₂F), 7. 00 (s, 1 H, H-2), 7. 14 (d, *J* = 8. 7 Hz, 1 H, H-3'), 7. 82 (dd, *J* = 8. 7 Hz, 2. 4 Hz, 1 H, H-4'), 8. 88 (d, *J* = 2. 4 Hz, 1 H, H-6'), 10. 74 (br s, 1 H, NH); MS (FAB) *m*/*z* 566 (MH⁺).

### Example 29

### Preparation of 2-(2-ethoxy-5-(4-(2-hydroxyethyl)piperazin-1-ylsulfonyl)phenyl)-5-ethyl-7-(3-fluorop ropyl)-3,5-dihydro-4H-pyrrolo[3,2-d]pyrimidin-4-one (a compound of the formula (1) wherein R⁵ = SO₂NR⁶R⁷, R¹ = CH₂CH₃, R² = H, R³ = CH₂CH₂CH₂F, R⁴ = CH₂CH₃; NR⁶R⁷ is 4-(2-hydroxyethyl)piperazin-1-yl)

The titled compound was prepared as described in Example 15 by using 4-(2-ethoxy-5-(4-(2-hydroxyethyl)piperazin-1-ylsulfonyl)benzamido)-1-ethyl-3-(3-flu oropropyl)pyrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 86%
mp 164-165 °C (EtOAc/hexanes);
IR (neat) 3526(OH), 3322 (NH), 1694 (C=O), 1170 (SO₂) cm⁻¹;
¹H NMR (CDCl₃/TMS) δ 1. 49 (t, *J* = 7. 2 Hz, 3 H, NCH₂C*H*₃), 1. 65 (t, *J* = 6. 9 Hz, 3 H, OCH₂C*H*₃), 2. 06-2. 23 (m, 2 H, CH₂C*H*₂CH₂F), 2. 54 (t, *J* = 5. 4 Hz, 2 H, C*H*₂CH₂OH), 2. 61 (dd, *J* = 5. 1 Hz, 4. 8 Hz, 4 H, 2 NCH₂), 2. 87 (t, *J* = 7. 5 Hz, 2 H, C*H*₂CH₂CH₂F), 3. 12 (br dd, *J* = 5. 1 Hz, 4. 8 Hz, 4 H, 2 SO₂NCH₂), 3. 57 (t, *J* = 5. 4 Hz, 2 H, CH₂C*H*₂OH), 4. 37 (q; *J* = 6. 9 Hz, 2 H, OC*H*₂CH₃), 4. 45 (q, *J* = 7. 2 Hz, 2 H, NC*H*₂CH₃), 4. 53 (dt, *J* = 47. 4 Hz, 6. 0 Hz, 2 H, CH₂CH₂C*H*₂F), 7. 00 (s, 1 H, H-2), 7. 14 (d, *J* = 8. 7 Hz, 1 H, H-3'), 7. 82 (dd, *J* = 8. 7 Hz, 2. 7 Hz, 1 H, H-4'), 8. 88 (d, *J* = 2. 7 Hz, 1 H, H-6'), 10. 71 (br s, 1 H, NH); MS (FAB) *m*/*z* 536 (MH⁺).

### Example 30

### Preparation of 2-(5-(4-(2-hydroxyethyl)piperazin-1-ylsulfonyl)-2-n-propoxyphenyl)-5-ethyl-7-(3-fluo ropropyl)-3,5-dihydro-4H-pyrrolo[3,2-d]pyrimidin-4-one (a compound of the formula (1) wherein R⁵ = SO₂NR⁶R⁷, R¹ = CH₂CH₃, R² = H, R³ = CH₂CH₂CH₂F, R⁴ = CH₂CH₂CH₃; NR⁶R⁷ is 4-(2-hydroxyethyl)piperazin-1-yl)

The titled compound was prepared as described in Example 15 by using 4-(5-(4-(2-hydroxyethyl)piperazin-1-ylsulfonyl)-2-*n*-propoxybenzamido)-1-ethyl-3-(3-fluoropropyl)pyrrole-5-carboxamide in place of 4-(2-ethoxy-5-(4-(3-fluoropropyl)piperazin-1-ylsulfonyl)benzamido)-3-ethyl-1-methyl pyrrole-5-carboxamide.
yield: 82%
mp 162-163 °C (EtOAc/hexanes);
IR (neat) 3526(OH), 3325 (NH), 1685 (C=O), 1169 (SO₂) cm⁻¹;
¹H NMR (CDCl₃/TMS) δ 1. 20 (t, *J* = 7. 5 Hz, 3 H, CH₂CH₂C*H*₃), 1. 48 (t, *J* = 7. 2 Hz, 3 H, NCH₂C*H*₃), 2. 00-2. 23 (m, 4 H, CH₂C*H*₂CH₃ and CH₂C*H*₂CH₂F), 2. 35(br s, 1H, OH), 2. 55 (t, *J* = 5. 4 Hz, 2 H, C*H*₂CH₂OH), 2. 62 (dd, *J* = 5. 1 Hz, 4. 8 Hz, 4 H, 2 NCH₂), 2. 87 (t, *J* = 7. 5 Hz, 2 H, C*H*₂CH₂CH₂F), 3. 12 (br dd, *J* = 5. 1 Hz, 4. 8 Hz, 4 H, 2 SO₂NCH₂), 3. 57 (t, *J* = 5. 4 Hz, 2 H, CH₂C*H*₂OH), 4. 26 (t, *J* = 6. 6 Hz, 2 H, C*H*₂ CH₂CH₃), 4. 46 (q, *J* = 7. 2 Hz, 2 H, NC*H*₂CH₃), 4. 53 (dt, *J* = 47. 1 Hz, 5. 7 Hz, 2 H, CH₂CH₂C*H*₂F), 7. 00 (s, 1 H, H-2), 7. 16 (d, *J* = 8. 7 Hz, 1 H, H-3'), 7. 82 (dd, *J* = 8. 7 Hz, 2. 4 Hz, 1 H, H-4'), 8. 89 (d, *J* = 2. 4 Hz, 1 H, H-6'), 10. 75 (br s, 1 H, NH); MS (FAB) *m*/*z* 550 (MH⁺).

### Example 31

### Preparation of 2-(2-ethoxy-5-(4-(3-fluoropropyl)piperazin-1-ylsulfonyl)phenyl)-5-methyl-7-n-propyl-3,5-dihydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(3-fluoropropyl)piperazin-1-yl)

The titled compound was prepared as described in Example 2 by using 2-(2-ethoxy-5-(4-(3-fluoropropyl)piperazin-1-ylsulfonyl)phenyl)-5-methyl-7-*n*-propyl-3,5-dihydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 99%
mp 123 °C dec;
IR (neat) 3318 (NH), 1682 (C=O), 1169 (SO₂) cm⁻¹;
¹H NMR (DMSO-*d*₆) δ 0.93 (t, *J* = 7. 5 Hz, 3 H, CH₂CH₂C*H*₃), 1. 36 (t, *J* = 6. 9 Hz, 3 H, OCH₂C*H*₃), 1. 58-1. 70 (m, 2 H, CH₂C*H*₂CH₃), 2. 01-2. 15 (m, 2 H, CH₂C*H*₂CH₂F), 2. 58 (t, *J* = 7. 5 Hz, 2 H, C*H*₂CH₂CH₃), 2. 81 (br t, *J* = 13. 2 Hz, 2 H, 2 SO₂NCHₐₓ), 3. 08-3. 24 (m, 4 H, NC*H*₂CH₂ and 2 SO₂NCH_{eq}), 3. 49-3. 67 (m, 2 H, 2 ⁺HNC*H*ₐₓ), 3. 81 (br d, *J* = 12. 6 Hz, 2 H, 2 ⁺HNC*H*_{eq}), 3. 99 (s, 3 H, NCH₃), 4. 24 (q, *J* = 6. 9 Hz, 2 H, OC*H*₂CH₃), 4. 51 (dt, *J* = 46. 8 Hz, 5. 7 Hz, 2 H, CH₂C*H*₂F), 7. 25 (s, 1 H, H-2), 7. 42 (d, *J* = 9. 0 Hz, 1 H, H-3'), 7. 88 (dd, *J* = 9. 0 Hz, 2. 4 Hz, 1 H, H-4'), 7. 99 (d, *J* = 2. 4 Hz, 1 H, H-6'), 10. 92 (br s, 1 H, NH⁺), 11. 83 (br s, I H, NH).

### Example 32

### Preparation of 2-(2-ethoxy-5-(4-(3-fluoropropyl)piperazin-1-ylsulfonyl)phenyl)-5-methyl-7-n-propyl-3,5-dihydro-4H-pyrrolo[3,2-d]pyrimidin-4-one hydrogen sulfate (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(3-fluoropropyl)piperazin-1-yl)

The titled compound was prepared as described in Example 2 by using 2-(2-ethoxy-5-(4-(3-fluoropropyl)piperazin-1-ylsulfonyl)phenyl)-5-methyl-7-*n*-propyl-3,5-dihydro-4*H*-pyrrolo[3,2-*d*]pyrimidin-4-one and 10% ethanolic H₂SO₄ solution in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one and 1 N HCl ethereal solution.
yield: 90%
mp 82 °C dec;
IR (neat) 3314 (NH), 1717 (C=O), 1166 (SO₂) cm⁻¹;
¹H NMR (DMSO-*d*₆) δ 0. 93 (t, *J =* 7. 2 Hz, 3 H, CH₂CH₂C*H*₃), 1. 36 (t, *J* = 6. 9 Hz, 3 H, OCH₂C*H*₃), 1. 58-1. 70 (m, 2 H, CH₂C*H*₂CH₃), 1. 93-2. 11 (m, 2 H, CH₂C*H*₂CH₂F), 2. 58 (t, *J* = 7. 5 Hz, 2 H, C*H*₂CH₂CH₃), 2. 56-2. 74 (m, 2 H, 2 SO₂NCHₐₓ), 3. 15-3. 30 (m, 4 H, NC*H*₂CH₂ and 2 SO₂NCH_{eq}), 3. 53-3. 65 (m, 2 H, 2 ⁺HNC*H*ₐₓ), 3. 75-3. 88 (m, 2 H, 2 ⁺HNC*H*_{eq}), 4. 00 (s, 3 H, NCH₃), 4. 25 (q, *J* = 6. 9 Hz, 2 H, OC*H*₂CH₃), 4. 51 (dt, *J =* 47. 4 Hz, 5. 6 Hz, 2 H, CH₂C*H*₂F), 7. 27 (s, 1 H, H-2), 7.44 (d, *J* = 8. 7 Hz, 1 H, H-3'), 7. 89 (dd, *J* = 8. 7 Hz, 2. 4 Hz, 1 H, H-4'), 7. 98 (d, *J* = 2. 4 Hz, 1 H, H-6'), 9. 30 (br s, 1 H, NH⁺).

### Example 33

### Preparation of 2-(2-ethoxy-5-(4-(3-fluoropropyl)piperazin-1-ylsulfonyl)phenyl)-5-methyl-7-n-propyl-3,5-dihydro-4H-pyrrolo[3,2-d]pyrimidin-4-one hydrogen phosphate (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(3-fluoropropyl)piperazin-1-yl)

The titled compound was prepared as described in Example 2 by using 2-(2-ethoxy-5-(4-(3-fluoropropyl)piperazin-1-ylsulfonyl)phenyl)-5-methyl-7-*n*-propyl-3,5-dihydro-4*H*-pyrrolo[3,2-*d*]pyrimidin-4-one and 10% ethanolic H₃PO₄ solution in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one and 1 N HCl ethereal solution.
yield: 91%
mp 83 °C dec;
IR (neat) 3311 (NH), 1662 (C=O), 1166 (SO₂) cm⁻¹;
¹H NMR (DMSO-*d*₆) δ 0. 92 (t, *J* = 7. 5 Hz, 3 H, CH₂CH₂C*H*₃), 1. 35 (t, *J* = 6. 9 Hz, 3 H, OCH₂C*H*₃), 1. 57-1. 70 (m, 2 H, CH₂C*H*₂CH₃), 1. 67-1. 84 (m, 2 H, CH₂C*H*₂CH₂F), 2. 41 (t, *J* = 7. 2 Hz, 2 H, NC*H*₂CH₂), 2. 50 (br s, 4 H, 2 NCH₂), 2. 57 (t, *J* = 7. 5 Hz, 2 H, C*H*₂CH₂CH₃), 2. 93 (br s, 4 H, 2 SO₂NCH₂), 3. 99 (s, 3 H, NCH₃), 4. 22 *(q, J* = 6. 9 Hz, 2 H, OC*H*₂CH₃), 4. 41 (dt, *J* = 47. 4 Hz, 5. 7 Hz, 2 H, CH₂C*H*₂F), 7. 22 (s, 1 H, H-2), 7. 37 (d, *J* = 8. 7 Hz, 1 H, H-3'), 7. 81 (dd, *J* = 8. 7 Hz, 2. 4 Hz, 1 H, H-4'), 7. 88 (d, *J* = 2.4 Hz, 1 H, H-6'), 11. 72 (br s, 1 H, NH).

### Example 34

### Preparation of 2-(2-ethoxy-5-(4-(3-fluoropropyl)piperazin-1-ylsulfonyl)phenyl)-5-methyl-7-n-propyl-3,5-dihydro-4H-pyrrolo[3,2-d]pyrimidin-4-one methanesulfonic acid (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(3-fluoropropyl)piperazin-1-yl)

The titled compound was prepared as described in Example 2 by using 2-(2-ethoxy-5-(4-(3-fluoropropyl)piperazin-1-ylsulfonyl)phenyl)-5-methyl-7-*n*-propyl-3,5-dihydro-4*H*-pyrrolo[3,2-*d*]pyrimidin-4-one and 10% CH₃SO₃H/CH₂Cl₂ solution in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one and 1 *N* HCl ethereal solution.
yield: 90%
mp 74 °C dec;
IR (neat) 3321 (NH), 1683 (C=O), 1173 (SO₂) cm⁻¹;
¹H NMR (DMSO-*d*₆) δ 0. 93 (t, *J* = 7. 5 Hz, 3 H, CH₂CH₂C*H*₃), 1. 36 (t, *J* = 6. 9 Hz, 3 H, OCH₂C*H*₃), 1. 58-1. 70 (m, 2 H, CH₂C*H*₂CH₃), 1. 93-2. 11 (m, 2 H, CH₂C*H*₂CH₂F), 2. 33 (s, 3 H, CH₃SO₃), 2. 58 (t, *J* = 7. 5 Hz, 2 H, C*H*₂CH₂CH₃), 2. 65 (br t, *J* = 11. 4 Hz, 2 H, 2 SO₂NCHₐₓ), 3. 15-3. 30 (m, 4 H, NC*H*₂CH₂ and 2 SO₂NCH_{eq}), 3. 59 (br d, *J* = 12. 9 Hz, 2 H, 2 ^{*+*}HNC*H*ₐₓ), ), 3. 81 (br d, *J* = 12. 9 Hz, 2 H, 2 ⁺HNC*H*_{eq}), 4. 00 (s, 3 H, NCH₃), 4. 24 (q, *J* = 6. 9 Hz, 2 H, OC*H*₂CH₃), 4. 51 (dt, *J* = 47. 4 Hz, 5. 7 Hz, 2 H, CH₂C*H*₂F), 7. 26 (s, 1 H, H-2), 7. 43 (d, *J* = 8. 7 Hz, I H, H-3'), 7. 89 (dd, *J* = 8. 7 Hz, 2. 4 Hz, 1 H, H-4'), 7. 98 (d, *J* = 2. 4 Hz, 1 H, H-6'), 9. 34 (br s, 1 H, NH⁺).

### Example 35

### Preparation of 2-(5-(4-(3-fluoropropyl)piperazin-1-ylsulfonyl)-2-n-propoxyphenyl)-5-methyl-7-n-pro pyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(3-fluoropropyl)piperazin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(3-fluoropropyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 90%
mp 154-154. 5 °C (EtOAc/Et₂O/hexanes);
IR (neat) 3337 (NH), 1684 (C=O), 1168 (SO₂) cm⁻¹;
¹H NMR (CDCl₃/TMS) δ 0. 99 (t, *J* = 7. 5 Hz, 3 H, CH₂CH₂C*H*₃), 1. 19 (t, *J* = 7. 5 Hz, 3 H, OCH₂CH₂C*H*₃), 1. 67-1. 87 (m, 4 H, CH₂C*H*₂CH₂F and CH₂C*H*₂CH₃), 2. 03-2. 10 (m, 2 H, OCH₂C*H*₂CH₃), 2. 47 (t, *J* = 7. 2 Hz, 2 H, NC*H*₂CH₂), 2. 54 (dd, J = 5. 1 Hz, 4. 5 Hz, 4 H, 2 NCH₂), 2. 71 (t, *J* = 7.5 Hz, 2 H, C*H*₂CH₂CH₃), 3. 11 (br dd, *J* = 5. 1 Hz, 4. 5 Hz, 4 H, 2 SO₂NCH₂), 4. 08 (s, 3 H, NCH₃), 4. 24 (t, *J* = 6. 6 Hz, 2 H, OC*H*₂CH₂CH₃), 4. 43 (dt, *J* = 47. 1 Hz, 6. 0 Hz, 2 H, CH₂C*H*₂F), 6. 89 (s, 1 H, H-2), 7. 13 (d, *J* = 8. 7 Hz, 1 H, H-3'), 7. 81 (dd, *J* = 8. 7 Hz, 2. 4 Hz, 1 H, H-4'), 8. 88 (d, *J* = 2. 4 Hz, 1 H, H-6'), 10. 66 (br s, 1 H, NH); MS (FAB) *m*/*z* 534 (MH⁺).

### Example 36

### Preparation of 2-(5-(4-(3-fluoropropyl)piperazin-1-ylsulfonyl)-2-n-propoxyphenyl)-5-methyl-7-n-pro pyl-3,5-dihydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(3-fluoropropyl)piperazin-1-yl)

The titled compound was prepared as described in Example 2 by using 2-(5-(4-(3-fluoropropyl)piperazin-1-ylsulfonyl)-2-*n*-propoxyphenyl)-5-methyl-7-*n*-pro pyl-3,5-dihydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 88%
mp 105 °C dec;
IR (neat) 3318 (NH), 1685 (C=O), 1168 (SO₂) cm⁻¹;
¹H NMR (DMSO-*d*₆) δ 0. 93 (t, *J* = 7. 5 Hz, 3 H, CH₂CH₂C*H*₃), 0. 97 (t, *J* = 7. 5 Hz, 3 H, OCH₂CH₂C*H*₃), 1. 58-1. 70 (m, 2 H, CH₂C*H*₂CH₃), 1. 70-1. 80 (m, 2 H, OCH₂C*H*₂CH₃), 2. 00-2. 17 (m, 2 H, CH₂C*H*₂CH₂F), 2. 58 (t, *J* = 7. 5 Hz, 2 H, C*H*₂CH₂CH₃), 2. 81 (br t, *J* = 10. 8 Hz, 2 H, 2 SO₂NCHₐₓ), 3. 10-3. 24 (m, 4 H, NC*H*₂CH₂ and 2 SO₂NCH_{eq}), 3. 56 (br d, *J* = 12. 0 Hz, 2 H, 2 ⁺HNC*H*ₐₓ), 3. 80 (br d, *J* = 12. 0 Hz, 2 H, 2 ⁺HNC*H*_{eq}), 3. 99 (s, 3 H, NCH₃), 4. 14 (t, *J* = 6. 3 Hz, 2 H, OC*H*₂CH₂CH₃), 4. 51 (dt, *J* = 47. 1 Hz, 5. 7 Hz, 2 H, NCH₂C*H*₂F), 7. 24 (s, 1 H, H-2), 7. 43 (d, J= 9. 0 Hz, 1 H, H-3'), 7. 87 (dd, *J* = 9. 0 Hz, 2. 4 Hz, 1 H, H-4'), 7. 99 (d, *J* = 2. 4 Hz, 1 H, H-6'), 10. 85 (br s, 1 H, NH⁺), 11. 75 (br s, 1 H, NH).

### Example 37

### Preparation of 2-(2-ethoxy-5-(4-((R)-3-fluoro-2-methylpropyl)piperazin-1-ylsulfonyl)phenyl)-5-meth yl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-((R)-3-fluoro-2-methylpropyl)piperazin-1-yl)

The titled compound was prepared as described in Example 1 by using 1-((*R*)-3-fluoro-2-methylpropyl)piperazine hydrochloride in place of 1-methylpiperazine.
yield: 90%
mp 212. 5-213 °C (CHCl₃/Et₂O);
[α]_{D}¹⁶ = - 5. 2 ° (c = 2.0, CHCl₃);
IR (neat) 3332 (NH), 1676 (C=O), 1169 (SO₂) cm⁻¹;
¹H NMR (CDCl₃/TMS) δ 0. 92 (d, *J* = 6. 6 Hz, 3 H, CHC*H*₃), 0. 99 (t, *J* = 7. 5 Hz, 3 H, CH₂CH₂C*H*₃), 1. 64 (t, *J* = 7. 2 Hz, 3 H, OCH₂C*H*₃), 1. 67-1. 79 (m, 2 H, CH₂C*H*₂CH₃), 1. 82-2. 03 (m, 1 H, C*H*CH₃), 2. 16 (ddd, *J* = 12. 6 Hz, 6. 6 Hz, 1. 8 Hz, 1 H, NC*H*₂CH), 2. 33 (dd, *J* = 12. 6 Hz, 8. 4 Hz, 1 H, NC*H*₂CH), 2. 45-2. 58 (m, 4 H, 2 NCH₂), 2. 71 (t, *J* = 7. 5 Hz, 2 H, C*H*₂CH₂CH₃), 3. 07-3. 10 (m, 4 H, 2 SO₂NCH₂), 4. 08 (s, 3 H, NCH₃), 4. 27 (ddd, *J* = 47. 4 Hz, 5. 4 Hz, 2. 7 Hz, 2 H, CHC*H*₂F), 4. 36 (q, *J* = 7. 2 Hz, 2 H, OC*H*₂CH₃), 6. 89 (s, 1 H, H-2), 7. 13 (d, *J* = 8. 7 Hz, 1 H, H-3'), 7. 81 (dd, *J* = 8. 7 Hz, 2. 1 Hz, 1 H, H-4'), 8. 86 (d, *J* = 2. 1 Hz, 1 H, H-6'), 10. 63 (br s, 1 H, NH); MS (FAB) *m*/*z* 534 (MH⁺).

### Example 48

### Preparation of 2-(2-ethoxy-5-(4-((S)-3-fluoro-2-methylpropyl)piperazin-1-ylsulfonyl)phenyl)-5-meth yl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-((S)-3-fluoro-2-methylpropyl)piperazin-1-yl)

The titled compound was prepared as described in Example 1 by using 1-((*S*)-3-fluoro-2-methylpropyl)piperazine hydrochloride in place of 1-methylpiperazine.
yield: 90%
mp 212. 5-213 °C (CHCl₃/Et₂O);
[α]_{D}¹⁶ = + 5. 2 ° (c = 2. 0 , CHCl₃);
IR (neat) 3332 (NH), 1676 (C=O), 1169 (SO₂) cm⁻¹;
¹H NMR (CDCl₃/TMS) δ 0. 92 (d, *J* = 6. 6 Hz, 3 H, CHC*H*₃), 0. 99 (t, *J* = 7. 5 Hz, 3 H, CH₂CH₂C*H*₃), 1. 64 (t, *J* = 7. 2 Hz, 3 H, OCH₂C*H*₃), 1. 67-1. 79 (m, 2 H, CH₂C*H*₂CH₃), 1. 82-2. 03 (m, 1 H, C*H*CH₃), 2. 16 (ddd, *J* = 12. 6 Hz, 6. 6 Hz, 1. 8 Hz, 1 H, NC*H*₂CH), 2. 33 (dd, *J* = 12. 6 Hz, 8. 4 Hz, 1 H, NC*H*₂CH), 2. 45-2. 58 (m, 4 H, 2 NCH₂), 2. 71 (t, *J* = 7. 5 Hz, 2 H, C*H*₂CH₂CH₃), 3. 07-3. 10 (m, 4 H, 2 SO₂NCH₂), 4. 08 (s, 3 H, NCH₃), 4. 27 (ddd, *J* = 47. 4 Hz, 5. 4 Hz, 2. 7 Hz, 2 H, CHC*H*₂F), 4. 36 (q, *J* = 7. 2 Hz, 2 H, OC*H*₂CH₃), 6. 89 (s, 1 H, H-2), 7. 13 (d, *J* = 8. 7 Hz, 1 H, H-3'), 7. 81 (dd, *J* = 8. 7 Hz, 2. 1 Hz, 1 H, H-4'), 8. 86 (d, *J* = 2. 1 Hz, 1 H, H-6'), 10. 63 (br s, 1 H, NH); MS (FAB) *m*/*z* 534 (MH⁺).

### Example 39

### Preparation of 2-(5-(4-(1,3-difluoroisopropyl)piperazin-1-ylsulfonyl)-2-ethoxyphenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(1,3-difluoroisopropyl)piperazin-1-yl)

The titled compound was prepared as described in Example 1 by using 1-(1,3-difluoroisopropyl)piperazine hydrochloride in place of 1-methylpiperazine.
yield: 75%
mp 218. 5-219 °C (CHCl₃/EtOAc/hexanes);
IR (neat) 3338 (NH), 1676 (C=O), 1170 (SO₂) cm⁻¹;
¹H NMR (CDCl₃/TMS) δ 0. 99 (t, *J* = 7. 5 Hz, 3 H, CH₂CH₂C*H*₃), 1. 64 (t, *J* = 6. 9 Hz, 3 H, OCH₂C*H*₃), 1. 67-1. 80 (m, 2 H, CH₂C*H*₂CH₃), 2. 71 (t, *J* = 7. 5 Hz, 2 H, C*H*₂CH₂CH₃), 2. 82 (dd, *J* = 5. 1 Hz, 4. 5 Hz, 4 H, 2 NCH₂), 2. 87-3. 06 (m, 1 H, NCH), 3. 10 (br dd, *J* = 5. 1 Hz, 4. 5 Hz, 4 H, 2 SO₂NCH₂), 4. 08 (s, 3 H, NCH₃), 4. 36 (q, *J* = 6. 9 Hz, 2 H, OC*H*₂CH₃), 4. 56 (dd, *J* = 48. 0 Hz, 5. 1 Hz, 4 H, 2 CHC*H*₂F), 6. 89 (s, 1 H, H-2), 7. 13 (d, *J* = 9. 0 Hz, 1 H, H-3'), 7. 80 (dd, *J* = 9. 0 Hz, 2. 4 Hz, 1 H, H-4'), 8. 86 (d, *J* = 2. 4 Hz, 1 H, H-6'), 10. 62 (br s, 1 H, NH); MS (FAB) *m*/*z* 538 (MH⁺).

### Example 40

### Preparation of 2-(2-ethoxy-5-(4-(4-fluorobutyl)piperazin-1-ylsulfonyl)phenyl)-5-methyl-7-n-propyl-3 ,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(4-fluorobutyl)piperazin-1-yl)

The titled compound was prepared as described in Example 1 by using 1-(4-fluorobutyl)piperazine trifluoroacetic acid in place of 1-methylpiperazine.
yield: 53%
mp 188-189 °C (EtOAc/CHCl₃/hexanes);
IR (neat) 3326 (NH), 1678 (C=O), 1167 (SO₂) cm⁻¹;
¹H NMR (CDCl₃/TMS) δ 0. 99 (t, *J* = 7. 5 Hz, 3 H, CH₂CH₂C*H*₃), 1. 50-1. 70(m, 4 H, CH₂C*H*₂C*H*₂CH₂F), 1. 64 (t, *J* = 6. 9 Hz, 3 H, OCH₂C*H*₃), 1. 67-1. 79 (m, 2 H, CH₂C*H*₂CH₃), 2. 37(dd, *J* = 7. 5 Hz,7. 2 Hz, 2 H, NC*H*₂CH₂), 2. 53(dd, *J* = 5. 1 Hz, 4. 8 Hz, 4 H, 2 NCH₂), 2. 71 (t, *J* = 7. 5 Hz, 2 H, C*H*₂CH₂CH₃), 3. 10 (br dd, *J* = 5. 1 Hz, 4. 8 Hz, 4 H, 2 SO₂NCH₂), 4. 08(s, 3 H, NCH₃), 4. 35(q, *J* = 6. 9 Hz, 2 H, OC*H*₂CH₃), 4. 40 (dt, *J* = 47. 1 Hz, 6. 0 Hz, 2 H, CH₂C*H*₂F), 6. 89 (s, 1 H, H-2), 7. 12 (d, *J* = 8. 7 Hz, 1 H, H-3'), 7. 81 (dd, *J* = 8. 7 Hz, 2. 4 Hz, 1 H, H-4'), 8. 86 (d, *J* = 2. 4 Hz, 1 H, H-6'), 10. 63 (br s, I H, NH); MS (EI) *m*/*z* 533 (M⁺).

### Example 41

### Preparation of 2-(5-(4-(4-fluorobutyl)piperazin-1-ylsulfonyl)-2-n-propoxyphenyl)-5-methyl-7-n-prop yl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(4-fluorobutyl)piperazin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(4-fluorobutyl)piperazine trifluoroacetic acid in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 74%
mp 162-163 °C (EtOAc/Et₂O/hexanes);
IR(neat) 3335 (NH), 1683 (C=O), 1170 (SO₂) cm⁻¹;
¹H NMR (CDCl₃/TMS) δ 0. 99 (t, *J* = 7. 5 Hz, 3 H, CH₂CH₂C*H*₃), 1. 18 (t, *J* = 7. 5 Hz, 3 H, OCH₂CH₂C*H*₃), 1. 50-1. 94 (m, 6 H, CH₂C*H*₂C*H*₂CH₂F and CH₂C*H*₂CH₃), 1. 98-2. 09 (m, 2 H, OCH₂C*H*₂CH₃), 2. 40 (t, *J* = 7. 2 Hz, 2 H, NC*H*₂CH₂), 2. 56 (dd, J = 5. 1 Hz, 4. 8 Hz, 4 H, 2 NCH₂), 2. 71 (t, *J* = 7. 5 Hz, 2 H, C*H*₂CH₂CH₃), 3. 12 (br dd, *J =* 5. 1 Hz, 4. 8 Hz, 4 H, 2 SO₂NCH₂), 4. 08 (s, 3 H, NCH₃), 4. 24 (t, *J* = 6. 6 Hz, 2 H, OC*H*₂CH₂CH₃), 4. 40 (dt, *J* = 47. 4 Hz, 6. 0 Hz, 2 H, CH₂C*H*₂F), 6. 89 (s, 1 H, H-2), 7. 13 (d, *J* = 8. 7 Hz, 1 H, H-3'), 7.81 (dd, *J* = 8. 7 Hz, 2. 4 Hz, 1 H, H-4'), 8. 85 (d, *J* = 2. 4 Hz, 1 H, H-6'), 10.69 (br s, 1 H, NH); MS (FAB) *m*/*z* 548 (MH⁺).

### Example 42

### Preparation of 2-(2-ethoxy-5-(4-(2,2,2-trifluoroethyl)piperazin-1-ylsulfonyl)phenyl)-5-methyl-7-n-pr opyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2,2,2-trifluoroethyl)piperazin-1-yl)

The titled compound was prepared as described in Example 1 by using 1-(2,2,2-trifluoroethyl)piperazine hydrochloride in place of 1-methylpiperazine.
yield: 80%
mp 243-243. 5 °C (EtOAc/hexanes);
IR (neat) 3337 (NH), 1676 (C=O), 1170 (SO₂) cm⁻¹;
¹H NMR (CDCl₃/TMS) δ 0. 99 (t, *J* = 7. 5 Hz, 3 H, CH₂CH₂C*H*₃), 1. 64 (t, *J* = 7. 2 Hz, 3 H, OCH₂C*H*₃), 1. 67-1. 79 (m, 2 H, CH₂C*H*₂CH₃), 2. 71 (t, *J* = 7. 5 Hz, 2 H, C*H*₂CH₂CH₃), 2. 77 (dd, *J* = 5. 1 Hz, 4. 5 Hz, 4 H, 2 NCH₂), 2. 96 (q, *J* = 9. 6 Hz, 2 H, C*H*₂CF₃), 3. 13 (dd, *J* = 5. 1 Hz, 4. 5 Hz, 4 H, 2 SO₂NCH₂), 4. 08 (s, 3 H, NCH₃), 4. 36 (q, *J* = 7. 2 Hz, 2 H, OC*H*₂CH₃), 6. 89 (s, 1 H, H-2), 7. 13 (d, *J* = 8. 7 Hz, 1 H, H-3'), 7. 80 (dd, *J* = 8. 7 Hz, 2. 4 Hz, 1 H, H-4'), 8. 86 (d, *J* = 2. 4 Hz, 1 H, H-6'), 10. 64 (br s, 1 H, NH); MS (FAB) *m*/*z* 542 (MH⁺).

### Example 43

### Preparation of 2-(2-n-propoxy-5-(4-(2,2,2-trifluoroethyl)piperazin-1-ylsulfonyl)phenyl)-5-methyl-7-n -propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2,2,2-trifluoroethyl)piperazin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(2,2,2-trifluoroethyl)piperazine hydrochloride in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 72%
mp 189. 5-190 °C (EtOAc/hexanes);
IR (neat) 3315 (NH), 1681 (C=O), 1172 (SO₂) cm⁻¹;
¹H NMR (CDCl₃/TMS) δ 0. 99 (t, *J* = 7. 5 Hz, 3 H, CH₂CH₂C*H*₃), 1. 19 (t, *J* = 7. 5 Hz, 3 H, OCH₂CH₂C*H*₃), 1. 67-1. 79 (m, 2 H, CH₂C*H*₂CH₃), 1. 98-2. 10 (m, 2 H, OCH₂C*H*₂CH₃), 2. 71 (t, *J* = 7. 5 Hz, 2 H, C*H*₂CH₂CH₃), 2. 77 (dd, *J* = 5. 1 Hz, 4. 8 Hz, 4 H, 2 NCH₂), 2. 96 (q, J = 9. 3 Hz, 3 H, C*H*₂CF₃), 3. 13 (br dd, *J* = 5. 1 Hz, 4. 8 Hz, 4 H, 2 SO₂NCH₂), 4. 08 (s, 3 H, NCH₃), 4. 25 (t, *J* = 6. 6 Hz, 2 H, OC*H*₂CH₂CH₃), 6. 89 (s, 1 H, H-2), 7. 14 (d, *J* = 8. 7 Hz, 1 H, H-3'), 7. 80 (dd, *J* = 8. 7 Hz, 2. 4 Hz, 1 H, H-4'), 8. 87 (d, *J* = 2. 4 Hz, 1 H, H-6'), 10. 67 (br s, 1 H, NH); MS (FAB) *m*/*z* 556 (MH⁺).

### Example 44

### Preparation of 2-(2-ethoxy-5-(4-(3,3,3-trifluoropropyl)piperazin-1-ylsulfonyl)phenyl)-5-methyl-7-n-p ropyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(3,3,3-trifluoropropyl)piperazin-1-yl)

The titled compound was prepared as described in Example 1 by using 1-(3,3,3-trifluoropropyl)piperazine hydrochloride in place of 1-methylpiperazine.
yield: 87%
mp 219-220 °C (EtOAc/hexanes);
IR (neat) 3339 (NH), 1684 (C=O), 1168 (SO₂) cm⁻¹;
¹H NMR (CDCl₃/TMS) δ 0. 99 (t, *J* = 7. 2 Hz, 3 H, CH₂CH₂C*H*₃), 1. 64 (t, *J* = 7. 2 Hz, 3 H, OCH₂C*H*₃), 1. 67-1. 79 (m, 2 H, CH₂C*H*₂CH₃), 2. 14-2. 30 (m, 2 H, CH₂C*H*₂CF₃), 2. 56 (dd, *J* = 5. 1 Hz, 4. 8 Hz, 4 H, 2 NCH₂), 2. 60 (t, *J* = 7. 5 Hz, 2 H, NC*H*₂CH₂CF₃), 2. 71 (t, *J* = 7. 5 Hz, 2 H, C*H*₂CH₂CH₃), 3. 11 (br dd, *J* = 5. 1 Hz, 4. 8 Hz, 4 H, 2 SO₂NCH₂), 4. 08 (s, 3 H, NCH₃), 4. 36 (q, *J* = 7. 2 Hz, 2 H, OC*H*₂CH₃), 6. 89 (s, 1 H, H-2), 7. 13 (d, *J* = 9. 0 Hz, 1 H, H-3'), 7. 81 (dd, *J* = 9. 0 Hz, 2. 4 Hz, 1 H, H-4), 8. 86 (d, *J* = 2. 4 Hz, 1 H, H-6'), 10. 63 (br s, 1 H, NH); MS (FAB) *m*/*z* 556 (MH⁺).

### Example 45

### Preparation of 2-(2-n-propoxy-5-(4-(3,3,3-trifluoropropyl)piperazin-1-ylsulfonyl)phenyl)-5-methyl-7 - n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(3,3,3-trifluoropropyl)piperazin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(3,3,3-trifluoropropyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 81%
mp 177-178 °C (EtOAc/Et₂O);
IR (neat) 3339 (NH), 1676 (C=O), 1171 (SO₂) cm⁻¹;
¹H NMR (CDCl₃/TMS) δ 1. 00 (t, *J* = 7. 5 Hz, 3 H, CH₂CH₂C*H*₃), 1. 19 (t, *J* = 7. 2 Hz, 3 H, OCH₂CH₂C*H*₃), 1. 67-1. 79 (m, 2 H, CH₂C*H*₂CH₃), 1. 98-2. 10 (m, 2 H, OCH₂C*H*₂CH₃), 2. 14-2. 30 (m, 2 H, CH₂C*H*₂CF₃), 2. 56 (dd, *J* = 5. 1 Hz, 4. 8 Hz, 4 H, 2 NCH₂), 2. 60 (t, *J* = 7. 5 Hz, 2 H, NC*H*₂CH₂CF₃), 2. 71 (t, *J* = 7. 5 Hz, 2 H, C*H*₂CH₂CH₃), 3. 11 (br dd, *J* = 5. 1 Hz, 4. 8 Hz, 4 H, 2 SO₂NCH₂), 4. 08 (s, 3 H, NCH₃), 4. 24 (t, *J* = 6. 6 Hz, 2 H, OC*H*₂CH₂CH₃), 6. 89 (s, 1 H, H-2), 7. 14 (d, *J* = 8. 7 Hz, 1 H, H-3'), 7. 81 (dd, *J* = 8. 7 Hz, 2. 4 Hz, 1 H, H-4'), 8. 88 (d, *J* = 2. 4 Hz, 1 H, H-6'), 10.66 (br s, 1 H, NH); MS (FAB) *m*/*z* 570 (MH⁺).

### Example 46

### Preparation of 2-(5-(4-(2-chloroethyl)piperazin-1-ylsulfonyl)-2-ethoxyphenyl)-5-methyl-7-n-propyl-3 ,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-chloroethyl)piperazin-1-yl)

The titled compound was prepared as described in Example 1 by using 1-(2-chloroethyl)piperazine hydrochloride in place of 1-methylpiperazine.
yield: 87% .
mp 226 °C dec (CHCl₃/Et₂O);
IR (neat) 3335 (NH), 1678 (C=O), 1172 (SO₂) cm⁻¹;
¹H NMR (CDCl₃/TMS) δ 0. 99 (t, *J* = 7. 5 Hz, 3 H, CH₂CH₂C*H*₃), 1. 64 (t, *J* = 6. 9 Hz, 3 H, OCH₂C*H*₃), 1. 67-1. 79 (m, 2 H, CH₂C*H*₂CH₃), 2. 62 (dd, *J* = 4. 8 Hz, 4. 5 Hz, 4 H, 2 NCH₂), 2. 71 (t, *J* = 7. 5 Hz, 2 H, C*H*₂CH₂CH₃), 2. 72 (t, *J* = 6. 6 Hz, 2 H, NC*H*₂CH₂Cl), 3. 12 (br dd, *J* = 4. 8 Hz, 4. 5 Hz, 4 H, 2 SO₂NCH₂), 3. 51 (t, *J* = 6. 6 Hz, 2 H, CH₂C*H*₂Cl), 4. 08 (s, 3 H, NCH₃), 4. 36 (q, *J* = 6. 9 Hz, 2 H, OC*H*₂CH₃), 6. 89 (s, 1 H, H-2), 7. 13 (d, *J* = 8. 7 Hz, 1 H, H-3'), 7. 80 (dd, *J* = 8. 7 Hz, 2. 7 Hz, 1 H, H-4'), 8. 86 (d, *J* = 2. 7 Hz, 1 H, H-6'), 10. 63 (br s, 1 H, NH); MS (FAB) *m*/*z* 522 (M⁺).

### Example 47

### Preparation of 2-(5-(4-(3-chloropropyl)piperazin-1-ylsulfonyl)-2-ethoxyphenyl)-5-methyl-7-n-propyl -3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(3-chloropropyl)piperazin-1-yl)

The titled compound was prepared as described in Example 1 by using 1-(3-chloropropyl)piperazine hydrochloride in place of 1-methylpiperazine.
yield: 94%
mp 203 °C dec (CHCl₃/Et₂O);
IR (neat) 3333 (NH), 1679 (C=O), 1171 (SO₂) cm⁻¹;
¹H NMR (CDCl₃/TMS) δ 1. 00 (t, *J* = 7. 5 Hz, 3 H, CH₂CH₂C*H*₃), 1. 64(t, *J* = 6. 9 Hz, 3 H, OCH₂C*H*₃), 1. 67-1. 80 (m, 2 H, CH₂C*H*₂CH₃), 1. 83-1. 91 (m, 2 H, CH₂C*H*₂CH₂Cl), 2. 49 (t, *J* = 6. 9 Hz, 2 H, NC*H*₂CH₂), 2. 54 (dd, *J* = 4. 8 Hz, 4. 2 Hz, 4 H, 2 NCH₂), 2. 71 (t, *J* = 7. 5 Hz, 2 H, C*H*₂CH₂CH₃), 3. 10 (br dd, *J* = 4. 8 Hz, 4. 2 Hz, 4 H, 2 SO₂NCH₂), 3. 52 (t, *J* = 6. 6 Hz, 2 H, CH₂C*H*₂Cl), 4. 08 (s, 3 H, NCH₃), 4. 36 (q, *J* = 6. 9 Hz, 2 H, OC*H*₂CH₃), 6. 89 (s, 1 H, H-2), 7. 13 (d, *J* = 8. 7 Hz, 1 H, H-3'), 7. 81 (dd, *J* = 8. 7 Hz, 2. 4 Hz, 1 H, H-4'), 8. 86 (d, *J* = 2. 4 Hz, 1 H, H-6'), 10. 63 (br s, 1 H, NH); MS (FAB) *m*/*z* 536 (M⁺).

### Example 48

### Preparation of 2-(5-(4-(3-chloropropyl)piperazin-1-ylsulfonyl)-2-ethoxyphenyl)-5-methyl-7-n-propyl -3,5-dihydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(3-chloropropyl)piperazin-1-yl)

The titled compound was prepared as described in Example 2 by using 2-(5-(4-(3-chloropropyl)piperazin-1-ylsulfonyl)-2-ethoxyphenyl)-5-methyl-7-*n*-propyl -3,5-dihydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 99%
mp 135 °C dec;
IR (neat) 3338 (NH), 1682 (C=O), 1166 (SO₂) cm⁻¹;
¹H NMR (DMSO-*d*₆) δ 0. 93 (t, *J* = 7. 5 Hz, 3 H, CH₂CH₂C*H*₃), 1. 36(t, *J* = 6. 9 Hz, 3 H, OCH₂C*H*₃), 1. 58-1. 70 (m, 2 H, CH₂C*H*₂CH₃), 2. 10-2. 19 (m, 2 H, CH₂C*H*₂CH₂Cl), 2. 58 (t, *J* = 7. 2 Hz, 2 H, C*H*₂CH₂CH₃), 2. 80(br t, *J* = 11. 4 Hz, 2 H, 2 SO₂NCHₐₓ), 3. 10-3. 24 (m, 4 H, NC*H*₂CH₂ and 2 SO₂NCH_{eq}), 3. 54-3. 59 (m, 2 H, 2 ⁺HNC*H*ₐₓ), 3. 71 (t, *J* = 6. 3 Hz, 2 H, CH₂C*H*₂Cl), 3. 77-3. 82 (m, 2 H, 2 ⁺HNC*H*_{eq}), 3. 99 (s, 3 H, NCH₃), 4. 24 (q, *J* = 6. 9 Hz, 2 H, OC*H*₂CH₃), 7. 25 (s, 1 H, H-2), 7. 42 (d, *J* = 8. 7 Hz, 1 H, H-3'), 7. 88 (dd, *J* = 8. 7 Hz, 2. 7 Hz, 1 H, H-4'), 7. 99(d, *J* = 2. 7 Hz, 1 H, H-6'), 10. 83 (br s, 1 H, NH⁺).

### Example 49

### Preparation of 2-(2-ethoxy-5-(4-(2-hydroxyethyl)piperazin-1-ylsulfonyl)phenyl)-5-methyl-7-n-propyl -3,5-dihydro-4H-pyrrolo[3,2-d]pyrimidin-4-one (a compound of the formula (1) wherein R⁵ = SO₂NR⁶R⁷, R¹ = CH₃; R² = H, R³ = CH₂CH₂CH₃, R⁴ = CH₂CH₃; NR⁶R⁷ is 4-(2-hydroxyethyl)piperazin-1-yl)

The titled compound was prepared as described in Example 1 by using 1-(2-hydroxyethyl)piperazine in place of 1-methylpiperazine.
yield: 99%
mp 175 °C dec (EtOAc/hexanes);
IR (neat) 3429, 3323 (NH and OH), 1675 (C=O), 1167 (SO₂) cm⁻¹;
¹H NMR (CDCl₃/TMS) δ 1. 00 (t, *J* = 7. 5 Hz, 3 H, CH₂CH₂C*H*₃), 1. 64 (t, *J* = 7. 2 Hz, 3 H, OCH₂C*H*₃), 1. 68-1. 80 (m, 2 H, CH₂C*H*₂CH₃), 2. 36 (br s, 1 H, OH), 2. 55 (t, *J* = 5. 4 Hz, 2 H, NC*H*₂CH₂), 2. 61 (dd, *J* = 5. 1 Hz, 4. 8 Hz, 4 H, 2 NCH₂), 2. 71 (t, *J* = 7. 5 Hz, 2 H, C*H*₂CH₂CH₃), 3. 12 (br dd, *J* = 5. 1 Hz, 4. 8 Hz, 4 H, 2 SO₂NCH₂), 3. 58 (br t, *J* = 5. 4 Hz, 2 H, CH₂C*H*₂OH), 4. 08 (s, 3 H, NCH₃), 4. 39 (q, *J* = 7. 2 Hz, 2 H, OC*H*₂CH₃), 6. 89 (s, 1 H, H-2), 7. 14 (d, *J* = 8. 7 Hz, 1 H, H-3'), 7. 81 (dd, *J* = 8. 7 Hz, 2. 7 Hz, 1 H, H-4'), 8. 86 (d, *J* = 2. 7 Hz, 1 H, H-6'), 10. 64 (br s, 1 H, NH); MS (FAB) *m*/*z* 504 (MH⁺).

### Example 50

### Preparation of 2-(2-ethoxy-5-(4-(2-hydroxyethyl)piperazin-1-ylsulfonyl)phenyl)-5-methyl-7-n-propyl -3,5-dihydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-hydroxyethyl)piperazin-1-yl)

The titled compound was prepared as described in Example 2 by using 2-(2-ethoxy-5-(4-(2-hydroxyethyl)piperazin-1-ylsulfonyl)phenyl)-5-methyl-7-*n*-propyl -3,5-dihydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 99%
mp 96 °C dec;
IR (neat) 3327 (NH and OH), 1679 (C=O), 1166 (SO₂) cm⁻¹;
¹H NMR (DMSO-*d*₆) δ 0. 93 (t, *J* = 7. 5 Hz, 3 H, CH₂CH₂C*H*₃), 1. 36 (t, *J* = 7. 2 Hz, 3 H, OCH₂C*H*₃), 1. 58-1. 74 (m, 2 H, CH₂C*H*₂CH₃), 2. 59 (t, *J* = 7. 5 Hz, 2 H, C*H*₂CH₂CH₃), 2. 91 (br t, *J* = 11. 7 Hz, 2 H, 2 SO₂NCHₐₓ), 3. 10-3. 27 (m, 4 H, NC*H*₂CH₂ and 2 SO₂NCH_{eq}), 3. 58 (br d, J = 11. 7 Hz, 2 H, 2 ⁺HNC*H*ₐₓ), 3. 68-3. 82 (m, 4 H, CH₂C*H*₂OH and 2 ⁺HNC*H*_{eq}), 4. 00 (s, 3 H, NCH₃), 4. 10 (br s, 1 H, OH), 4. 23 (q, *J* = 7. 2 Hz, 2 H, OC*H*₂CH₃), 7. 26 (s, 1 H, H-2), 7. 42 (d, *J* = 9. 0 Hz, 1 H, H-3'), 7. 88 (dd, *J* = 9. 0 Hz, 2. 4 Hz, 1 H, H-4'), 8. 00 (d, *J* = 2. 4 Hz, 1 H, H-6'), 10. 60 (br s, 1 H, NH⁺), 11. 95 (br s, 1 H, NH).

### Example 51

### Preparation of 2-(5-(4-(2-hydroxyethyl)piperazin-1-ylsulfonyl)-2-n-propoxyphenyl)-5-methyl-7-n-pr opyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-hydroxyethyl)piperazin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(2-hydroxyethyl)piperazine in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 98%
mp 228 °C dec (EtOAc/hexanes);
IR (neat) 3539,3338 (NH and OR), 1677 (C=O), 1167 (SO₂) cm⁻¹;
¹H NMR (CDCl₃/TMS) δ 1. 00 (t, *J* = 7. 5 Hz, 3 H, CH₂CH₂C*H*₃), 1. 19 (t, *J* = 7. 5 Hz, 3 H, OCH₂CH₂C*H*₃), 1. 67-1. 80 (m, 2 H, CH₂C*H*₂CH₃), 1. 98-2. 10 (m, 2 H, OCH₂C*H*₂CH₃), 2. 34 (br s, 1 H, OH), 2. 55 (t, *J* = 5. 4 Hz, 2 H, NC*H*₂CH₂), 2. 61 (dd, *J* = 5. 1 Hz, 4. 5 Hz, 4 H, 2 NCH₂), 2. 71 (t, *J* = 7. 5 Hz, 2 H, C*H*₂CH₂CH₃), 3. 12 (br dd, *J* = 5. 1 Hz, 4. 5 Hz, 4 H, 2 SO₂NCH₂), 3. 57 (br s, 2 H, CH₂C*H*₂OH), 4. 08 (s, 3 H, NCH₃), 4. 25 (t, *J* = 6. 3 Hz, 2 H, OC*H*₂CH₂CH₃), 6. 89 (s, 1 H, H-2), 7. 15 (d, *J* = 8. 7 Hz, 1 H, H-3'), 7. 81 (dd, *J* = 8. 7 Hz, 2. 7 Hz, 1 H, H-4'), 8. 88 (d, *J* = 2. 7 Hz, 1 H, H-6'), 10. 67 (br s, 1 H, NH); MS (FAB) *m*/*z* 518 (MH⁺).

### Example 52

### Preparation of 2-(5-(4-(2-hydroxyethyl)piperazin-1-ylsulfonyl)-2-n-propoxyphenyl)-5-methyl-7-n-pr opyl-3,5-dihydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-hydroxyethyl)piperazin-1-yl)

The titled compound was prepared as described in Example 2 by using 2-(5-(4-(2-hydroxyethyl)piperazin-1-ylsulfonyl)-2-*n*-propoxyphenyl)-5-methyl-7-*n*-pr opyl-3,5-dihydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 99%
mp 66. 5 °C dec;
IR (neat) 3332 (NH and OH), 1676 (C=O), 1166 (SO₂) cm⁻¹;
¹H NMR (DMSO-*d*₆) δ 0.92 (t, *J* = 7. 2 Hz, 3 H, CH₂CH₂C*H*₃), 0. 96 (t, *J* = 7. 2 Hz, 3 H, OCH₂CH₂C*H*₃), 1. 56-1. 80 (m, 4 H, 2 CH₂C*H*₂CH₃), 2. 59 (t, *J* = 7. 5 Hz, 2 H, C*H*₂CH₂CH₃), 2. 91 (br t, *J* = 11. 7 Hz, 2 H, 2 SO₂NCHₐₓ), 3. 12-3. 27 (m, 4 H, NC*H*₂CH₂ and 2 SO₂NCH_{eq}), 3. 58 (br d, J = 11. 7 Hz, 2 H, 2 ⁺HNC*H*ₐₓ), 3. 68-3. 85 (m, 4 H, CH₂C*H*₂OH and 2 ⁺HNC*H*_{eq}), 4. 00 (s, 3 H, NCH₃), 4. 15 (t, *J* = 6. 3 Hz, 2 H, OC*H*₂CH₂CH₃), 4. 66 (br s, 1 H, OH), 7. 28 (s, 1 H, H-2), 7. 44 (d, *J* = 9. 0 Hz, 1 H, H-3'), 7. 89 (dd, *J* = 9. 0 Hz, 2. 4 Hz, 1 H, H-4'), 8. 01 (d, *J* = 2. 4 Hz, 1 H, H-6'), 10. 85 (br s, 1 H, NH⁺), 12. 01 (br s, 1 H, NH).

### Example 53

### Preparation of 2-(2-ethoxy-5-(4-(3-hydroxypropyl)piperazin-1-ylsulfonyl)phenyl)-5-methyl-7-n-prop yl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(3-hydroxypropyl)piperazin-1-yl)

The titled compound was prepared as described in Example 1 by using 1-(3-hydroxypropyl)piperazine in place of 1-methylpiperazine.
yield: 99%
mp 180. 5 °C dec (EtOAc/ hexanes);
IR (neat) 3460, 3331 (NH and OH), 1677 (C=O), 1168 (SO₂) cm⁻¹;
¹H NMR (CDCl₃/TMS) δ 1. 00 (t, *J* = 7. 5 Hz, 3 H, CH₂CH₂C*H*₃), 1. 65 (t, *J* = 6. 9 Hz, 3 H, OCH₂C*H*₃), 1. 67-1. 80 (m, 4 H, CH₂C*H*₂CH₂OH and CH₂C*H*₂CH₃), 2. 58-2. 65 (m, 6 H, NC*H*₂CH₂ and 2 NCH₂), 2. 71 (t, *J* = 7. 5 Hz, 2 H, C*H*₂CH₂CH₃), 3. 09 (br s, 4 H, 2 SO₂NCH₂), 3. 71 (t, *J* = 5. 4 Hz, 2 H, CH₂C*H*₂OH), 4. 08 (s, 3 H, NCH₃), 4. 26 (br s, 1 H, OH), 4. 37 (q, *J* = 6. 9 Hz, 2 H, OC*H*₂CH₃), 6. 88 (s, 1 H, H-2), 7. 13 (d, *J* = 8. 7 Hz, 1 H, H-3'), 7. 77 (dd, *J* = 8. 7 Hz, 2. 4 Hz, 1 H, H-4'), 8. 86 (d, *J* = 2. 4 Hz, 1 H, H-6'), 10. 65 (br s, 1 H, NH); MS (FAB) *m*/*z* 518 (MH⁺).

### Example 54

### Preparation of 2-(2-ethoxy-5-(4-(3-hydroxypropyl)piperazin-1-ylsulfonyl)phenyl)-5-methyl-7-n-prop yl-3,5-dihydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(3-hydroxypropyl)piperazin-1-yl)

The titled compound was prepared as described in Example 2 by using 2-(2-ethoxy-5-(4-(3-hydroxypropyl)piperazin-1-ylsulfonyl)phenyl)-5-methyl-7-*n*-prop yl-3,5-dihydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 98%
mp 81 °C dec;
IR (neat) 3333 (NH and OH), 1684 (C=O), 1163 (SO₂) cm⁻¹;
¹H NMR (DMSO-*d*₆) δ 0. 93 (t, *J* = 7. 5 Hz, 3 H, CH₂CH₂C*H*₃), 1. 35 (t, *J* = 7. 2 Hz, 3 H, OCH₂C*H*₃), 1. 58-1. 76 (m, 2 H, CH₂C*H*₂CH₃), 1. 78-1. 90 (m, 2 H, CH₂C*H*₂CH₂OH), 2. 59 (t, *J* = 7. 5 Hz, 2 H, C*H*₂CH₂CH₃), 2. 84 (br t, *J* = 11. 7 Hz, 2 H, 2 SO₂NCHₐₓ), 3. 04-3.20 (m, 4 H, NC*H*₂CH₂ and 2 SO₂NCH_{eq}), 3. 42 (t, *J* = 6. 3 Hz, 2 H, CH₂C*H*₂OH), 3. 52 (br d, *J* = 11. 7 Hz, 2 H, 2 ⁺HNC*H*ₐₓ), 3. 80 (br d, *J* = 11. 7 Hz, 2 H, 2 ⁺HNC*H*_{eq}), 4. 00 (s, 3 H, NCH₃), 4. 21 (br s, 1 H, OH), 4. 23 (q, *J* = 7. 2 Hz, 2 H, OC*H*₂CH₃), 7. 27 (s, 1 H, H-2), 7. 43 (d, *J* = 9. 0 Hz, 1 H, H-3'), 7. 87 (dd, *J* = 9. 0 Hz, 2. 4 Hz, I H, H-4'), 8. 00 (d, *J* = 2. 4 Hz, 1 H, H-6'), 11. 05 (br s, 1 H, NH⁺), 11. 95 (br s, 1 H, NH).

### Example 55

### Preparation of 2-(5-(4-(3-hydroxypropyl)piperazin-1-ylsulfonyl)-2-n-propoxyphenyl)-5-methyl-7-n-p ropyl-3,5-dihydro-4H-pyrrolo[3,2-d]pyrimidin-4-one (a compound of the formula (1) wherein R⁵ = SO₂NR⁶R⁷, R' = CH₃, R² = H, R³ = CH₂CH₂CH₃, R⁴ = CH₂CH₂CH₃; NR⁶R⁷ is 4-(3-hydroxypropyl)piperazin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(3-hydroxypropyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 94%
mp 162. 5 °C dec (EtOAc/hexanes);
IR (neat) 3484, 3302 (NH and OH), 1669 (C=O), 1170 (SO₂) cm⁻¹;
¹H NMR (CDCl₃/TMS) δ 1. 00 (t, *J* = 7. 5 Hz, 3 H, CH₂CH₂C*H*₃), 1. 20 (t, *J* = 7. 5 Hz, 3 H, OCH₂CH₂C*H*₃), 1. 64-1. 80 (m, 4 H, CH₂C*H*₂CH₂OH and CH₂C*H*₂CH₃), 1. 99-2. 11 (m, 2 H, OCH₂C*H*₂CH₃), 2. 58-2. 64 (m, 6 H, NC*H*₂CH₂ and 2 NCH₂), 2. 71 (t, *J* = 7. 5 Hz, 2 H, C*H*₂CH₂CH₃), 3. 08 (br s, 4 H, 2 SO₂NCH₂), 3. 71 (t, *J* = 5. 4 Hz, 2 H, CH₂C*H*₂OH), 4. 08 (s, 3 H, NCH₃), 4. 26 (t, *J* = 6. 3 Hz, 2 H, OC*H*₂CH₂CH₃), 4. 28 (br s, 1 H, OH), 6. 88 (s, 1 H, H-2), 7. 14 (d, *J* = 8. 7 Hz, 1 H, H-3'), 7. 77(dd, *J* = 8. 7 Hz, 2. 7 Hz, 1 H, H-4'), 8. 87 (d, *J* = 2. 7 Hz, 1 H, H-6'), 10. 69 (br s, 1 H, NH); MS (FAB) *m*/*z* 532 (MH⁺).

### Example 56

### Preparation of 2-(5-(4-(3-hydroxypropyl)piperazin-1-ylsulfonyl)-2-n-propoxyphenyl)-5-methyl-7-n-p ropyl-3,5-dihydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(3-hydroxypropyl)piperazin-1-yl)

The titled compound was prepared as described in Example 2 by using 2-(5-(4-(3-hydroxypropyl)piperazin-1-ylsulfonyl)-2-*n*-propoxyphenyl)-5-methyl-7-*n*-p ropyl-3,5-dihydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 99%
mp 62. 5 °C dec;
IR (neat) 3347, 3321 (NH and OH), 1689 (C=O), 1168 (SO₂) cm⁻¹;
¹H NMR (DMSO-*d*₆) δ 0. 93 (t, *J* = 7. 5 Hz, 3 H, CH₂CH₂C*H*₃), 0. 96(t, *J* = 7. 5 Hz, 3 H, OCH₂CH₂C*H*₃), 1. 57-1. 87 (m, 6 H, CH₂C*H*₂CH₂OH and 2 CH₂C*H*₂CH₃), 2. 59 (t, *J* = 7. 5 Hz, 2 H, C*H*₂CH₂CH₃), 2. 89 (br t, *J* = 11. 7 Hz, 2 H, 2 SO₂NCHₐₓ), 3. 01-3. 19(m, 4 H, NC*H*₂CH₂ and 2 SO₂NCH_{eq}), 3. 44(t, *J* = 6. 0 Hz, 2 H, CH₂C*H*₂OH), 3. 52 (br d, *J* = 11. 7 Hz, 2 H, 2 ⁺HNC*H*ₐₓ), 3. 79(br d, *J* = 11. 7 Hz, 2 H, 2 ⁺HNC*H*_{eq}), 4. 00 (s, 3 H, NCH₃), 4. 15 (t, *J* = 6. 6 Hz, 2 H, OC*H*₂CH₂CH₃), 4. 71 (br s, 1 H, OH), 7. 29 (s, 1 H, H-2), 7. 44 (d, *J* = 8. 7 Hz, 1 H, H-3'), 7. 89 (dd, *J* = 8. 7 Hz, 2. 4 Hz, 1 H, H-4'), 8. 02 (d, *J* = 2. 4 Hz, 1 H, H-6'), 11. 13 (br s, 1 H, NH⁺), 12. 05(br s, 1 H, NH).

### Example 57

### Preparation of 2-(2-ethoxy-5-(4-(4-hydroxybutyl)piperazin-1-ylsulfonyl)phenyl)-5-methyl-7-n-propyl - 3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(4-hydroxybutyl)piperazin-1-yl)

The titled compound was prepared as described in Example 1 by using 1-(4-hydroxybutyl)piperazine in place of 1-methylpiperazine.
yield: 72%
mp 196. 5 °C dec (EtOAc/hexanes);
IR (neat) 3332 (NH and OH), 1676 (C=O), 1168 (SO₂) cm⁻¹;
¹H NMR (CDCl₃/TMS) δ 1. 00 (t, *J* = 7. 5 Hz, 3 H, CH₂CH₂C*H*₃), 1. 64 (t, *J* = 6. 9 Hz, 3 H, OCH₂C*H*₃), 1. 60-1. 79 (m, 6 H, CH₂C*H*₂C*H*₂CH₂ and CH₂C*H*₂CH₃), 2. 41 (br s, 2 H, NC*H*₂CH₂), 2. 60 (br t, *J* = 4. 8 Hz, 4 H, 2 NCH₂), 2. 71 (t, *J* = 7. 5 Hz, 2 H, C*H*₂CH₂CH₃), 3. 13 (br s, 4 H, 2 SO₂NCH₂), 3. 49 (br s, 2 H, CH₂C*H*₂OH), 4. 08 (s, 3 H, NCH₃), 4. 36 (q, *J* = 6. 9 Hz, 2 H, OC*H*₂CH₃), 4. 78 (br s, 1 H, OH), 6. 88 (s, 1 H, H-2), 7. 12 (d, *J* = 8. 7 Hz, 1 H, H-3'), 7. 77 (dd, *J* = 8. 7 Hz, 2. 7 Hz, 1 H, H-4'), 8. 86 (d, *J* = 2. 7 Hz, 1 H, H-6'), 10. 66 (br s, 1 H, NH); MS (EI) *m*/*z* 532 (MH⁺).

### Example 58

### Preparation of 2-(2-ethoxy-5-(4-(4-hydroxybutyl)piperazin-1-ylsulfonyl)phenyl)-5-methyl-7-n-propyl -3,5-dihydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(4-hydroxybutyl)piperazin-1-yl)

The titled compound was prepared as described in Example 2 by using 2-(2-ethoxy-5-(4-(3-hydroxybutyl)piperazin-1-ylsulfonyl)phenyl)-5-methyl-7-*n*-propyl -3,5-dihydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 98%
mp 62 °C dec;
IR (neat) 3334 (NH and OH), 1680 (C=O), 1167 (SO₂) cm⁻¹;
¹H NMR (DMSO-*d*₆) δ 0.93 (t, *J* = 7. 5 Hz, 3 H, CH₂CH₂C*H*₃), 1. 36 (t, *J* = 7. 2 Hz, 3 H, OCH₂C*H*₃), 1. 37-1. 52 (m, 2 H, NCH₂CH₂C*H*₂CH₂), 1. 58-1. 80 (m; 4 H, NCH₂C*H*₂CH₂CH₂ and CH₂C*H*₂CH₃), 2. 58 (t, *J* = 7. 5 Hz, 2 H, C*H*₂CH₂CH₃), 2. 84 (br t, *J* = 11. 7 Hz, 2 H, 2 SO₂NCHₐₓ), 3. 00-3. 18 (m, 4 H, NC*H*₂CH₂ and 2 SO₂NCH_{eq}), 3. 39 (t, *J* = 6. 3 Hz, 2 H, CH₂C*H*₂OH), 3. 48 (br d, *J* = 11. 7 Hz, 2 H, 2 ⁺HNC*H*ₐₓ), 3. 79 (br d, *J* = 11. 7 Hz, 2 H, 2 ⁺HNC*H*_{eq}), 3. 99 (s, 3 H, NCH₃), 4. 22 (q, *J* = 7. 2 Hz, 2 H, OC*H*₂CH₃), 7. 26 (s, 1 H, H-2), 7. 42 (d, *J* = 9. 0 Hz, 1 H, H-3'), 7. 84 (dd, *J* = 9. 0 Hz, 2. 4 Hz, 1 H, H-4'), 8. 00 (d, *J* = 2. 4 Hz, 1 H, H-6'), 11. 02 (br s, 1 H, NH⁺), 11. 84 (br s, 1 H, NH).

### Example 59

### Preparation of 2-(5-(4-(4-hydroxybutyl)piperazin-1-ylsulfonyl)-2-n-propoxyphenyl)-5-methyl-7-n-pr opyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(4-hydroxybutyl)piperazin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(4-hydroxybutyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 61%
mp 119 °C dec (EtOAc/Et₂O/hexanes);
IR (neat) 3469, 3300 (NH and OH), 1670 (C=O), 1169 (SO₂) cm⁻¹;
¹H NMR (CDCl₃/TMS) δ 1. 00 (t, *J* = 7. 5 Hz, 3 H, CH₂CH₂C*H*₃), 1. 20 (t, *J* = 7. 5 Hz, 3 H, OCH₂CH₂C*H*₃), 1. 60-1. 79 (m, 6 H, CH₂C*H*₂C*H*₂CH₂ and CH₂C*H*₂CH₃), 1. 99-2. 11 (m, 2 H, OCH₂C*H*₂CH₃), 2. 41 (br s, 2 H, NC*H*₂CH₂), 2. 61 (br t, J = 4. 8 Hz, 4 H, 2 NCH₂), 2. 71 (t, *J* = 7. 5 Hz, 2 H, C*H*₂CH₂CH₃), 3. 13 (br s, 4 H, 2 SO₂NCH₂), 3. 49 (br s, 2 H, CH₂C*H*₂OH), 4. 08 (s, 3 H, NCH₃), 4. 25 (t, *J* = 6. 3 Hz, 2 H, OC*H*₂CH₂CH₃), 4. 83 (br s, 1 H, OH), 6. 88 (s, 1 H, H-2), 7. 13 (d, *J*= 8. 7 Hz, 1 H, H-3'), 7. 77 (dd, *J* = 8. 7 Hz, 2. 4 Hz, 1 H, H-4'), 8. 87 (d, *J* = 2. 4 Hz, 1 H, H-6'), 10. 69 (br s, 1 H, NH); MS (FAB) *m*/*z* 546 (MH⁺).

### Example 60

### Preparation of 2-(5-(4-(4-hydroxybutyl)piperazin-1-ylsulfonyl)-2-n-propoxyphenyl)-5-methyl-7-n-pr opyl-3,5-dihydro-4H-pyrrolo[3,2-d]pyrimidin-4-one hydrochloride (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(4-hydroxybutyl)piperazin-1-yl)

The titled compound was prepared as described in Example 2 by using 2-(5-(4-(4-hydroxybutyl)piperazin-1-ylsulfonyl)-2-*n*-propoxyphenyl)-5-methyl-7-*n*-pr opyl-3,5-dihydro-4*H*-pyrrolo[3,2-*d*]pyrimidin-4-one in place of 2-(2-ethoxy-5-(4-methylpiperazin-1-ylsulfonyl)phenyl)-5-methyl-7-*n*-propyl-3,5-dihy dro-4*H*-pyrrolo[3,2-*d*]pyrimidin-4-one.
yield: 98%
mp 58 °C dec;
IR (neat) 3333 (NH and OH), 1679 (C=O), 1167 (SO₂) cm⁻¹;
¹H NMR (DMSO-*d*₆) δ 0. 93 (t, *J* = 7. 5 Hz, 3 H, CH₂CH₂C*H*₃), 0. 96(t, *J* = 7. 5 Hz, 3 H, OCH₂CH₂C*H*₃), 1. 36-1. 45 (m, 2 H, NCH₂CH₂C*H*₂CH₂), 1. 56-1. 80 (m, 6 H, NCH₂C*H*₂CH₂CH₂ and 2 CH₂C*H*₂CH₃), 2. 59 (t, *J* = 7. 5 Hz, 2 H, C*H*₂CH₂CH₃), 2. 89 (br t, *J* = 11. 7 Hz, 2 H, 2 SO₂NCHₐₓ), 3. 00-3. 18 (m, 4 H, NC*H*₂CH₂ and 2 SO₂NCH_{eq}), 3. 39 (t, *J* = 6. 3 Hz, 2 H, CH₂C*H*₂OH), 3. 51 (br d, *J* = 11. 4 Hz, 2 H, 2 ⁺HNC*H*ₐₓ), 3. 80 (br d, *J* = 11. 4 Hz, 2 H, 2 ⁺HNC*H*_{eq}), 4. 00 (s, 3 H, NCH₃), 4. 15(t, *J* = 6. 3 Hz, 2 H, OC*H*₂CH₂CH₃), 4. 80 (br s, 1 H, OH), 7. 29 (s, 1 H, H-2), 7. 44(d, *J* = 9. 0 Hz, 1 H, H-3'), 7. 89 (dd, *J* = 9. 0 Hz, 2. 4 Hz, 1 H, H-4'), 8. 02 (d, *J* = 2. 4 Hz, 1 H, H-6'), 11. 14 (br s, 1 H, NH⁺), 12. 12 (br s, 1 H, NH).

### Example 61

### Preparation of 2-(2-(2-fluoroethoxy)-5-(4-(2-fluoroethyl)piperazin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂F; NR⁶R⁷ is 4-(2-fluoroethyl)piperazin-1-yl)

The titled compound was prepared as described in Example 1 by using 2-(5-chlorosulfonyl-2-(2-fluoroethoxy)phenyl)-5-methyl-7-*n*-propyl-3,5-dihydro-4*H*-p yrrolo[3,2-*d*]pyrimidin-4-one and 1-(2-fluoroethyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 95%
mp 165. 5-166 °C (EtOAc/Et₂O/hexanes);
IR (neat) 3357 (NH), 1678 (C=O), 1169 (SO₂) cm⁻¹;
¹H NMR (CDCl₃/TMS) δ 0. 99 (t, *J* = 7. 5 Hz, 3 H, CH₂CH₂C*H*₃), 1. 69-1. 77 (m, 2 H, CH₂C*H*₂CH₃), 2. 63-2. 76 (m, 6 H, NC*H*₂CH₂F and 2 NCH₂), 2. 71 (t, *J* = 7. 5 Hz, 2 H, C*H*₂CH₂CH₃), 3. 14 (br dd, *J* = 4. 8 Hz, 4. 5 Hz, 4 H, 2 SO₂NCH₂), 4. 08 (s, 3 H, NCH₃), 4. 40-4. 59 (m, 4 H, OC*H*₂CH₂F and NCH₂C*H*₂F), 4. 82-5. 01 (m, 2 H, OCH₂C*H*₂F), 6. 88 (s, 1 H, H-2), 7. 15 (d, *J* = 8. 7 Hz, 1 H, H-3'), 7. 82 (dd, *J* = 8. 7 Hz, 2. 4 Hz, 1 H, H-4'), 8. 82 (d, *J* = 2. 4 Hz, 1 H, H-6'), 10. 40 (br s, 1 H, NH); MS (FAB) *m*/*z* 524 (MH⁺).

### Example 62

### Preparation of 2-(2-(2-fluoroethoxy)-5-(4-(3-fluoropropyl)piperazin-1-ylsulfonyl)phenyl)-5-methyl-7 -n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂F; NR⁶R⁷ is 4-(3-fluoropropyl)piperazin-1-yl)

The titled compound was prepared as described in Example 1 by using 2-(5-chlorosulfonyl-2-(2-fluoroethoxy)phenyl)-5-methyl-7-*n*-propyl-3,5-dihydro-4*H*-p yrrolo[3,2-*d*]pyrimidin-4-one and 1-(3-fluoropropyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 95%
mp 179-180 °C (EtOAc/Et₂O/hexanes);
IR (neat) 3358 (NH), 1678 (C=O), 1171 (SO₂) cm⁻¹;
¹H NMR (CDCl₃/TMS) δ 0. 99 (t, *J* = 7. 5 Hz, 3 H, CH₂CH₂C*H*₃), 1. 65-1. 89 (m, 4 H, CH₂C*H*₂CH₂F and CH₂C*H*₂CH₃), 2. 48 (t, *J* = 7. 2 Hz, 2 H, NC*H*₂CH₂), 2. 52-2. 58 (m, 4 H, 2 NCH₂), 2. 71 (t, *J* = 7. 5 Hz, 2 H, C*H*₂CH₂CH₃), 3. 06-3. 15 (m, 4 H, 2 SO₂NCH₂), 4. 08 (s, 3 H, NCH₃), 4. 44 (dt, *J* = 46. 8 Hz, 6. 0 Hz, 2 H, NCH₂C*H*₂F), 4. 45-4. 56 (m, 2 H, OC*H*₂CH₂F), 4. 81-5. 00 (m, 2 H, OCH₂C*H*₂F), 6. 89 (s, 1 H, H-2), 7. 15 (d, *J* = 8. 7 Hz, 1 H, H-3'), 7. 83 (dd, *J* = 8. 7 Hz, 2. 4 Hz, 1 H, H-4'), 8. 81 (d, *J*. = 2. 4 Hz, 1 H, H-6'), 10. 39 (br s, 1 H, NH); MS (FAB) *m*/*z* 538 (MH⁺).

### Example 63

### Preparation of 2-(2-ethoxy-5-(4-(2-fluoroethyl)homopiperazin-1-ylsulfonyl)phenyl)-5-methyl-7-n-pr opyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-fluoroethyl)homopiperazin-1-yl)

The titled compound was prepared as described in Example 1 by using 1-(2-fluoroethyl)homopiperazine hydrochloride in place of 1-methylpiperazine.
yield: 92%
mp 118-118. 5 °C (EtOAc/Et₂O);
IR (neat) 3322 (NH), 1693 (C=O), 1147 (SO₂) cm⁻¹;
¹H NMR (CDCl₃/TMS) δ 0. 97 (t, *J* = 7. 5 Hz, 3 H, CH₂CH₂C*H*₃), 1. 63 (t, *J* = 6. 9 Hz, 3 H, OCH₂C*H*₃), 1. 67-1. 77 (m, 2 H, CH₂C*H*₂CH₃), 1. 79-1. 97 (m, 2 H, NCH₂C*H*₂CH₂N), 2. 71 (t, *J* = 7. 5 Hz, 2 H, C*H*₂CH₂CH₃), 2. 80-2. 95 (m, 6 H, NC*H*₂CH₂F and 2 NCH₂), 3. 42-3. 49 (m, 4 H, 2 SO₂NCH₂), 4. 08 (s, 3 H, NCH₃), 4. 35 (q, *J* = 6. 9 Hz, 2 H, OC*H*₂CH₃), 4. 49 (dt, *J* = 47. 1 Hz, 5. 4 Hz, 2 H, NCH₂C*H*₂F), 6. 89 (s, 1 H, H-2), 7. 10 (d, *J* = 8. 7 Hz, 1 H, H-3'), 7. 85 (dd, *J* = 8. 7 Hz, 2. 7 Hz, 1 H, H-4'), 8. 89 (d, *J* = 2. 7 Hz, 1 H, H-6'), 10. 64 (br s, 1 H, NH); MS (FAB) *m*/*z* 520 (MH⁺).

### Example 64

### Preparation of 2-(2-ethoxy-5-(4-(3-fluoropropyl)homopiperazin-1-ylsulfonyl)phenyl)-5-methyl-7-n-p ropyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(3-fluoropropyl)homopiperazin-1-yl)

The titled compound was prepared as described in Example 1 by using 1-(3-fluoropropyl)homopiperazine hydrochloride in place of 1-methylpiperazine.
yield: 74%
mp 107-108 °C (EtOAc/Et₂O);
IR (neat) 3322 (NH), 1686 (C=O), 1148 (SO₂) cm⁻¹;
¹H NMR (CDCl₃/TMS) δ 1. 00 (t, *J* = 7. 5 Hz, 3 H, CH₂CH₂C*H*₃), 1. 63(t, *J* = 6. 9 Hz, 3 H, OCH₂C*H*₃), 1. 72-1. 79 (m, 4 H, CH₂C*H*₂CH₂F and CH₂C*H*₂CH₃), 1. 83-1. 87 (m, 2 H, NCH₂C*H*₂CH₂N), 2.61 (t, *J* = 6. 9 Hz, 2 H, NC*H*₂CH₂), 2. 68-2. 75 (m, 4 H, 2 NCH₂), 2. 71 (t, *J* = 7. 5 Hz, 2 H, C*H*₂CH₂CH₃), 3. 43 (br dd, *J* = 5. 7 Hz, 5. 4 Hz, 4 H, 2 SO₂NCH₂), 4. 08 (s, 3 H, NCH₃), 4. 35 (q, *J* = 6. 9 Hz, 2 H, OC*H*₂CH₃), 4. 46 (dt, *J* = 47. 1 Hz, 5. 7 Hz, 2 H, CH₂C*H*₂F), 6. 89 (s, 1 H, H-2), 7. 10 (d, *J* = 8. 7 Hz, 1 H, H-3'), 7. 84 (dd, *J* = 8. 7 Hz, 2. 4 Hz, 1 H, H-4'), 8. 89 (d, *J* = 2. 4 Hz, 1 H, H-6'), 10. 65 (br s, 1 H, NH); MS (FAB) *m*/*z* 534 (MH⁺).

### Example 65

### Preparation of 2-(2-ethoxy-5-(4-(2-(2-fluoroethoxy)ethyl)piperazin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-(2-fluoroethoxy)ethyl)piperazin-1-yl)

The titled compound was prepared as described in Example 1 by using 1-(2-(2-fluoroethoxy)ethyl)piperazine trifluoroacetic acid in place of 1-methylpiperazine.
yield: 92%
mp 167. 5-169 °C (MeOH/H₂O);
IR (neat) 3325 (NH), 1679 (C=O), 1169 (SO₂) cm⁻¹;
¹H NMR (CDCl₃/TMS) δ 1. 00 (t, *J* = 7. 5 Hz, 3 H, CH₂CH₂C*H*₃), 1. 64 (t, *J =* 6. 9 Hz, 3 H, OCH₂C*H*₃), 1. 67-1. 77 (m, 2 H, CH₂C*H*₂CH₃), 2. 59-2. 63 (m, 6 H, NC*H*₂CH₂F and 2 NCH₂), 2. 71 (t, *J* = 7. 5 Hz, 2 H, C*H*₂CH₂CH₃), 3. 09-3. 13 (m, 4 H, 2 SO₂NCH₂), 3. 57-3. 72 (m, 4 H, 2 OC*H*₂CH₂), 4. 09 (s, 3 H, NCH₃), 4. 36 (q, *J* = 6. 9 Hz, 2 H, OC*H*₂CH₃), 4. 42-4. 60 (m, 2 H, OCH₂C*H*₂F), 6. 89 (s, 1 H, H-2), 7. 12 (d, *J* = 8. 4 Hz, 1 H, H-3'), 7. 81 (dd, *J* = 8. 4 Hz, 2. 4 Hz, 1 H, H-4'), 8. 87 (d, *J* = 2. 4 Hz, 1 H, H-6'), 10. 62 (br s, 1 H, NH); MS (FAB) *m*/*z* 550 (MH⁺).

### Example 66

### Preparation of 2-(5-(4-(t-butylaminocarbonylmethyl)piperazin-1-ylsulfonyl)-2-ethoxyphenyl)-5-meth yl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(t-butylaminocarbonylmethyl)piperazin-1-yl)

The titled compound was prepared as described in Example I by using 1-(*t*-butylaminocarbonylmethyl)piperazine trifluoroacetic acid in place of 1-methylpiperazine.
yield: 69%
mp 192. 5-193 °C (EtOAc/Et₂O/hexanes);
IR (neat) 3320 (NH), 1678 (C=O), 1167 (SO₂) cm⁻¹;
¹H NMR (CDCl₃/TMS) δ 1. 00 (t, *J* = 7. 5 Hz, 3 H, CH₂CH₂CH₃), 1. 29 (s, 9 H, 3 CH₃), 1. 64 (t, *J* = 6. 9 Hz, 3 H, OCH₂C*H*₃), 1. 64-1. 77 (m, 2 H, CH₂C*H*₂CH₃), 2. 61 (br dd, *J* = 5. 4 Hz, 4. 5 Hz, 4 H, 2 NCH₂), 2. 71 (t, *J* = 7. 5 Hz, 2 H, C*H*₂CH₂CH₃), 2. 90 (s, 2 H, CH₂CO), 3. 14 (br dd, *J* = 5. 4 Hz, 4. 5 Hz, 4 H, 2 SO₂NCH₂), 4. 09 (s, 3 H, NCH₃), 4. 37 (q, *J* = 6. 9 Hz, 2 H, OC*H*₂CH₃), 6. 61 (br s, 1 H, CONH), 6. 89 (s, 1 H, H-2), 7. 15 (d, *J* = 8. 7 Hz, 1 H, H-3'), 7. 83 (dd, *J* = 8. 7 Hz, 2. 4 Hz, 1 H, H-4'), 8. 87 (d, *J* = 2.4 Hz, 1 H, H-6'), 10. 61 (br s, 1 H, NH); MS (FAB) *m*/*z* 573 (MH⁺).

### Example 67

### Preparation of 2-(5-(4-(t-butylaminocarbonylmethyl)piperazin-1-ylsulfonyl)-2-n-propoxyphenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(t-butylaminocarbonylmethyl)piperazin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(*t*-butylaminocarbonylmethyl)piperazine trifluoroacetic acid in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 68%
mp 164. 5-165. 5 °C (EtOAc/Et₂O/hexanes);
IR (neat) 3330,3303 (NH), 1684 (C=O), 1169 (SO₂) cm⁻¹;
¹H NMR (CDCl₃/TMS) δ 1. 00 (t, *J* = 7. 5 Hz, 3 H, CH₂CH₂C*H*₃), 1. 19(t, *J* = 7. 5 Hz, 3 H, OCH₂CH₂C*H*₃), 1. 29(s, 9 H, 3 CH₃), 1. 68-1. 78(m, 2 H, CH₂C*H*₂CH₃), 1. 98-2. 08 (m, 2 H, OCH₂C*H*₂CH₃), 2. 58-2. 63 (m, 4 H, 2 NCH₂), 2. 71 (t, *J* = 7. 5 Hz, 2 H, C*H*₂CH₂CH₃), 2. 91 (s, 2 H, CH₂CO), 3. 12-3.17 (m, 4 H, 2 SO₂NCH₂), 4. 09 (s, 3 H, NCH₃), 4. 25 (t, *J* = 6. 6 Hz, 2 H, OC*H*₂CH₂CH₃), 6. 63 (br s, 1 H, CONH), 6. 90(s, 1 H, H-2), 7. 16 (d, *J* = 8. 7 Hz, 1 H, H-3'), 7. 83 (dd, *J* = 8. 7 Hz, 2. 4 Hz, 1 H, H-4'), 8. 88 (d, *J* = 2. 4 Hz, 1 H, H-6'), 10. 64 (br s, 1 H, NH); MS (FAB) *m*/*z* 587 (MH⁺).

### Example 68

### Preparation of 2-(2-ethoxy-5-(4-(4-fluorophenyl)piperazin-1-ylsulfonyl)phenyl)-5-methyl-7-n-propyl -3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(4-fluorophenyl)piperazin-1-yl)

The titled compound was prepared as described in Example 1 by using 1-(4-fluorophenyl)piperazine in place of 1-methytpiperazine.
yield: 95%
mp 226-227 °C (CHCl₃/Et₂O);
IR (neat) 3337 (NH), 1678 (C=O), 1169 (SO₂) cm⁻¹;
¹H NMR (CDCl₃/TMS) δ 1. 00 (t, *J* = 7. 5 Hz, 3 H, CH₂CH₂C*H*₃), 1. 64 (t, *J* = 6. 9 Hz, 3 H, OCH₂C*H*₃), 1. 68-1. 80 (m, 2 H, CH₂C*H*₂CH₃), 2. 72 (t, *J* = 7. 5 Hz, 2 H, C*H*₂CH₂CH₃), 3. 15-3. 26 (m, 8 H, 4 NCH₂), 4. 08 (s, 3 H, NCH₃), 4. 36 (q, *J* = 6. 9 Hz, 2 H, OC*H*₂CH₃), 6. 80-6. 84 (m, 2 H, ArH-2 and 6), 6. 89 (s, 1 H, H-2), 6. 91-6. 97 (m, 2 H, ArH-3 and 5), 7. 15 (d, *J* = 8. 7 Hz, I H, H-3'), 7. 84 (dd, *J* = 8. 7 Hz, 2. 4 Hz, 1 H, H-4'), 8. 90 (d, *J* = 2. 4 Hz, 1 H, H-6'), 10. 63 (br s, 1 H, NH); MS (FAB) *m*/*z* 554 (MH⁺).

### Example 69

### Preparation of 2-(2-ethoxy-5-(4-(2-pyridyl)piperazin-1-ylsulfonyl)phenyl)-5-methyl-7-n-propyl-3,5-d ihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-pyridyl)piperazin-1-yl)

The titled compound was prepared as described in Example 1 by using 1-(2-pyridyl)piperazine in place of 1-methylpiperazine.
yield: 99%
mp 203-204 °C (CHCl₃/Et₂O/hexanes);
IR (neat) 3334 (NH), 1673 (C=O), 1169 (SO₂) cm⁻¹;
¹H NMR (CDCl₃/TMS) δ 1. 01 (t, *J* = 7. 5 Hz, 3 H, CH₂CH₂C*H*₃), 1. 62 (t, *J* = 6. 9 Hz, 3 H, OCH₂C*H*₃), 1. 68-1. 80 (m, 2 H, CH₂C*H*₂CH₃), 2. 72 (t, *J* = 7. 5 Hz, 2 H, C*H*₂CH₂CH₃), 3. 19 (dd, *J* = 5. 4 Hz, 5. 1 Hz, 4 H, 2 NCH₂), 3. 66 (dd, *J* = 5. 4 Hz, 5. 1 Hz, 4 H, 2 SO₂NCH₂), 4. 08 (s, 3 H, NCH₃), 4. 34 (q, *J* = 6. 9 Hz, 2 H, OC*H*₂CH₃), 6. 58 (d, *J* = 8. 7 Hz, 1 H, PyrH-3), 6. 60-6. 64 (m, 1 H, PyrH-5), 6. 89 (s, 1 H, H-2), 7. 13 (d, *J* = 8. 7 Hz, 1 H, H-3'), 7. 45 (ddd, *J* = 8. 7 Hz, 7. 2 Hz, 2. 1 Hz, 1 H, PyrH-4), 7. 82 (dd, *J* = 8. 7 Hz, 2. 4 Hz, 1 H, H-4'), 8. 12-8. 15 (m, 1 H, PyrH-6), 8. 88 (d, *J* = 2.4 Hz, 1 H, H-6'), 10. 62 (br s, 1 H, NH); MS (FAB) *m*/*z* 537 (MH⁺).

### Example 70

### Preparation of 2-(2-ethoxy-5-(4-(2-pyrimidyl)piperazin-1-ylsulfonyl)phenyl)-5-methyl-7-n-propyl-3, 5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-pyrimidyl)piperazin-1-yl)

The titled compound was prepared as described in Example 1 by using 1-(2-pyrimidyl)piperazine dihydrochloride in place of 1-methylpiperazine.
yield: 94%
mp 200-201 °C (CHCl₃/Et₂O);
IR (neat) 3329 (NH), 1679 (C=O), 1168 (SO₂) cm⁻¹;
¹H NMR (CDCl₃/TMS) δ 1. 01 (t, *J* = 7. 5 Hz, 3 H, CH₂CH₂C*H*₃), 1. 62(t, *J* = 6. 9 Hz, 3 H, OCH₂C*H*₃), 1. 68-1. 80 (m, 2 H, CH₂C*H*₂CH₃), 2. 71 (t, *J* = 7. 5 Hz, 2 H, C*H*₂CH₂CH₃), 3. 14 (dd, *J* = 5. 1 Hz, 4. 8 Hz, 4 H, 2 NCH₂), 3. 96 (dd, *J* = 5. 1 Hz, 4. 8 Hz, 4 H, 2 SO₂NCH₂), 4. 08(s, 3 H, NCH₃), 4. 34(q, *J* = 6. 9 Hz, 2 H, OC*H*₂CH₃), 6. 48 (d, *J* = 4. 8 Hz, 1 H, PyrH-5), 6. 88(s, 1 H, H-2), 7. 12(d, *J* = 8. 7 Hz, 1 H, H-3'), 7. 82 (dd, *J* = 8. 7 Hz, 2. 4 Hz, 1 H, H-4'), 8. 25 (d, *J* = 4. 8 Hz, 2 H, PyrH-4 and H-6), 8. 87 (d, *J* = 2. 4 Hz, 1 H, H-6'), 10. 61 (br s, 1 H, NH); MS (FAB) *m*/*z* 538 (MH⁺).

### Example 71

### Preparation of 5,6-dimethyl-2-(2-ethoxy-5-(4-methylpiperazin-1-ylsulfonyl)phenyl)-7-n-propyl-3,5-d ihydro-4H-pyrrolo[3,2-d]pyrimidin-4-one (a compound of the formula (1) wherein R⁵ = SO₂NR⁶R⁷, R¹ = R² = CH₃, R³ = CH₂CH₂CH₃, R⁴ = CH₂CH₃; NR⁶R⁷ is 4-methylpiperazin-1-yl)

The titled compound was prepared as described in Example 1 by using 2-(5-chlorosulfonyl-2-ethoxyphenyl)-5,6-dimethyl-7-*n*-propyl-3,5-dihydro-4*H*-pyrrolo [3,2-*d*]pyrimidin-4-one in place of 2-(5-chlorosulfonyl-2-ethoxyphenyl)-5-methyl-7-*n*-propyl-3,5-dihydro-4*H*-pyrrolo[3,2 - *d*]pyrimidin-4-one.
yield: 84%
mp 232 °C dec (CHCl₃/Et₂O/hexanes);
IR (neat) 3340 (NH), 1672 (C=O), 1172 (SO₂) cm⁻¹;
¹H NMR (CDCl₃/TMS) δ 0. 94 (t, *J* = 7. 5 Hz, 3 H, CH₂CH₂C*H*₃), 1. 63(t, *J* = 7. 2 Hz, 3 H, OCH₂C*H*₃), 1. 64-1. 73(m, 2 H, CH₂C*H*₂CH₃), 2. 28(s, 3 H, NCH₃), 2. 31 (s, 3 H, CH₃), 2. 51 (br dd, *J* = 4. 8 Hz, 4. 5 Hz, 4 H, 2 NCH₂), 2. 68 (t, *J* = 7. 5 Hz, 2 H, C*H*₂CH₂CH₃), 3. 12 (br dd, *J* = 4. 8 Hz, 4. 5 Hz, 4 H, 2 SO₂NCH₂), 4. 04 (s, 3 H, NCH₃), 4. 35 (q, *J* = 7. 2 Hz, 2 H, OC*H*₂CH₃), 7. 11 (d, *J* = 9. 0 Hz, 1 H, H-3'), 7. 80 (dd, *J* = 9. 0 Hz, 2. 4 Hz, 1 H, H-4'), 8. 88 (d, *J* = 2. 4 Hz, 1 H, H-6'), 10. 59 (br s, 1 H, NH); MS (FAB) *m*/*z* 488 (MH⁺).

### Example 72

### Preparation of 5,6-dimethyl-2-(5-(4-methylpiperazin-1-ylsulfonyl)-2-n-propoxyphenyl)-7-n-propyl-3, 5-dihydro-4H-pyrrolo[3,2-d]pyrimidin-4-one (a compound of the formula (1) wherein R⁵ = SO₂NR⁶R⁷, R¹ = R² = CH₃, R³ = CH₂CH₂CH₃, R⁴ = CH₂CH₂CH₃; NR⁶R⁷ is 4-methylpiperazin-1-yl)

The titled compound was prepared as described in Example 1 by using 2-(5-chlorosulfonyl-2-*n*-propoxyphenyl)-5,6-dimethyl-7-*n*-propyl-3,5-dihydro-4*H*-pyr rolo[3,2-*d*]pyrimidin-4-one in place of 2-(5-chlorosulfonyl-2-ethoxyphenyl)-5-methyl-7-*n*-propyl-3,5-dihydro-4*H*-pyrrolo[3,2 -*d*]pyrimidin-4-one.
yield: 82%
mp 199. 5-200 °C (CHCl₃/Et₂O/hexanes);
IR (neat) 3311 (NH), 1677 (C=O), 1175 (SO₂) cm⁻¹;
¹H NMR (CDCl₃/TMS) δ 0. 94 (t, *J* = 7. 5 Hz, 3 H, CH₂CH₂C*H*₃), 1. 18 (t, *J* = 7. 5 Hz, 3 H, OCH₂CH₂C*H*₃), 1. 61-1. 73 (m, 2 H, CH₂C*H*₂CH₃), 1. 98-2. 09 (m, 2 H, OCH₂C*H*₂CH₃), 2. 26 (s, 3 H, NCH₃), 2. 31 (s, 3 H, CH₃), 2. 49 (dd, *J* = 5. 1 Hz, 4. 8 Hz, 4 H, 2 NCH₂), 2. 68 (t, *J* = 7. 5 Hz, 2 H, C*H*₂CH₂CH₃), 3. 11 (br dd, *J* = 5. 1 Hz, 4. 8 Hz, 4 H, 2 SO₂NCH₂), 4. 04 (s, 3 H, NCH₃), 4. 23 (t, *J* = 6. 6 Hz, 2 H, OC*H*₂CH₂CH₃), 7. 12 (d, *J* = 8. 7 Hz, 1 H, H-3'), 7. 80 (dd, *J* = 8. 7 Hz, 2. 4 Hz, 1 H, H-4'), 8. 89 (d, *J* = 2. 4 Hz, 1 H, H-6'), 10. 61 (br s, 1 H, NH); MS (FAB) *m*/*z* 502 (MH⁺).

### Example 73

### Preparation of 5,6-dimethyl-2-(2-ethoxy-5-(4-(2-fluoroethyl)piperazin-1-ylsulfonyl)phenyl)-7-n-prop yl-3,5-dihydro-4H-pyrrolo[3,2-d]pyrimidin-4-one (a compound of the formula (1) wherein R⁵ = SO₂NR⁶R⁷, R¹ = R² = CH₃, R³ = CH₂CH₂CH₃, R⁴ = CH₂CH₃; NR⁶R⁷ is 4-(2-fluoroethyl)piperazin-1-yl)

The titled compound was prepared as described in Example 1 by using 2-(5-chlorosulfonyl-2-ethoxyphenyl)-5,6-dimethyl-7-*n*-propyl-3,5-dihydro-4*H*-pyrrolo [3,2-*d*]pyrimidin-4-one and 1-(2-fluoroethyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 80%
mp 194. 5 °C (CHCl₃/Et₂O/hexanes);
IR (neat) 3340 (NH), 1669 (C=O), 1169 (SO₂) cm⁻¹;
¹H NMR (CDCl₃/TMS) δ 0. 94 (t, *J* = 7.5 Hz, 3 H, CH₂CH₂C*H*₃), 1. 64 (t, *J* = 6. 9 Hz, 3 H, OCH₂C*H*₃), 1. 65-1. 74 (m, 2 H, CH₂C*H*₂CH₃), 2. 31 (s, 3 H, CH₃), 2. 62-2. 76 (m, 8 H, NC*H*₂CH₂F, 2 NCH₂ and C*H*₂CH₂CH₃), 3. 14 (br s, 4 H, 2 SO₂NCH₂), 4. 04 (s, 3 H, NCH₃), 4. 35 (q, *J* = 6. 9 Hz, 2 H, OC*H*₂CH₃), 4. 5 0 (dt, *J* = 47. 4 Hz, 4. 5 Hz, 2 H, NCH₂C*H*₂F), 7. 12 (d, *J* = 9. 0 Hz, 1 H, H-3'), 7. 80 (dd, *J* = 9. 0 Hz, 2. 4 Hz, 1 H, H-4'), 8. 88 (d, *J* = 2. 4 Hz, 1 H, H-6'), 10. 59 (br s, 1 H, NH); MS (FAB) *m*/*z* 520 (MH⁺).

### Example 74

### Preparation of 5,6-dimethyl-2-(5-(4-(2-fluoroethyl)piperazin-1-ylsulfonyl)-2-n-propoxyphenyl)-7-n-p ropyl-3,5-dihydro-4H-pyrrolo[3,2-d]pyrimidin-4-one (a compound of the formula (1) wherein R⁵ = SO₂NR⁶R⁷, R¹ = R² = CH₃, R³ = CH₂CH₂CH₃, R⁴ = CH₂CH₂CH₃; NR⁶R⁷ is 4-(2-fluoroethyl)piperazin-1-yl)

The titled compound was prepared as described in Example 1 by using 2-(5-chlorosulfonyl-2-*n*-propoxyphenyl)-5,6-dimethyl-7-*n*-propyl-3,5-dihydro-4*H*-pyr rolo[3,2-*d*]pyrimidin-4-one and 1-(2'-fluoroethyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 69%
mp 191-192 °C (CHCl₃/Et₂O/hexanes);
IR (neat) 3308 (NH), 1681 (C=O), 1174 (SO₂) cm⁻¹;
¹H NMR (CDCl₃/TMS) δ 0. 94 (t, *J* = 7. 5 Hz, 3 H, CH₂CH₂C*H*₃), 1. 19 (t, *J* = 7. 5 Hz, 3 H, OCH₂CH₂C*H*₃), 1. 61-1. 73 (m, 2 H, CH₂C*H*₂CH₃), 1. 98-2. 09 (m, 2 H, OCH₂C*H*₂CH₃), 2. 31 (s, 3 H, CH₃), 2. 62-2. 75 (m, 8 H, NC*H*₂CH₂F, 2 NCH₂ and C*H*₂CH₂CH₃), 3. 11-3. 15 (m, 4 H, 2 SO₂NCH₂), 4. 04 (s, 3 H, NCH₃), 4. 24 (t, *J* = 6. 6 Hz, 2 H, OC*H*₂CH₂CH₃), 4. 49 (ddd, *J* = 47. 4 Hz, 4. 8 Hz, 4. 5 Hz, 2 H, NCH₂C*H*₂F), 7. 12 (d, *J* = 9. 0 Hz, 1 H, H-3'), 7. 80 (dd, *J* = 9. 0 Hz, 2. 4 Hz, 1 H, H-4'), 8. 89 (d, *J* = 2. 4 Hz, 1 H, H-6'), 10. 61 (br s, 1 H, NH); MS (FAB) *m*/*z* 534 (MH⁺).

### Example 75

### Preparation of 5,6-dimethyl-2-(5-(2-ethoxy-4-(3-fluoropropyl)piperazin-1-ylsulfonyl)phenyl)-7-n-pro pyl-3,5-dihydro-4H-pyrrolo[3,2-d]pyrimidin-4-one (a compound of the formula (1) wherein R⁵ = SO₂NR⁶R⁷, R¹ = R² = CH₃, R³ = CH₂CH₂CH₃, R⁴ = CH₂CH₃; NR⁶R⁷ is 4-(3-fluoropropyl)piperazin-1-yl)

The titled compound was prepared as described in Example 1 by using 2-(5-chlorosulfonyl-2-ethoxyphenyl)-5,6-dimethyl-7-*n*-propyl-3,5-dihydro-4*H*-pyrrolo [3,2-*d*]pyrimidin-4-one and 1-(3-fluoropropyl)piperazine hydrochloride in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 72%
mp 214. 5 °C (CHCl₃/Et₂O/hexanes);
IR (neat) 3340 (NH), 1676 (C=O), 1171 (SO₂) cm⁻¹;
¹H NMR (CDCl₃/TMS) δ 0. 94 (t, *J* = 7. 5 Hz, 3 H, CH₂CH₂C*H*₃), 1. 64(t, *J* = 6. 9 Hz, 3 H, OCH₂C*H*₃), 1. 68-1. 87 (m, 4 H, CH₂C*H*₂CH₂F and CH₂C*H*₂CH₃), 2. 31 (s, 3 H, CH₃), 2. 43-2. 62 (m, 6 H, NC*H*₂CH₂ and 2 NCH₂), 2. 68 (t, *J* = 7. 5 Hz, 2 H, C*H*₂CH₂CH₃), 3. 11 (br s, 4 H, 2 SO₂NCH₂), 4. 04(s, 3 H, NCH₃), 4. 35(q, *J* = 6. 9 Hz, 2 H, OC*H*₂CH₃), 4. 44 (dt, *J* = 47. 4 Hz, 6. 0 Hz, 2 H, CH₂C*H*₂F), 7. 12(d, *J* = 8. 7 Hz, 1 H, H-3'), 7. 80 (dd, *J* = 8. 7 Hz, 2. 4 Hz, 1 H, H-4'), 8. 87 (d, *J* = 2. 4 Hz, 1 H, H-6'), 10. 59 (br s, 1 H, NH); MS (FAB) *m*/*z* 534 (MH⁺).

### Example 76

### Preparation of 5,6-dimethyl-2-(5-(4-(3-fluoropropyl)piperazin-1-ylsulfonyl)-2-n-propoxyphenyl)-7-n -propyl-3,5-dihydro-4H-pyrrolo[3,2-d]pyrimidin-4-one (a compound of the formula (1) wherein R⁵ = SO₂NR⁶R⁷, R¹ = R² = CH₃, R³ = CH₂CH₂CH₃, R⁴ = CH₂CH₂CH₃; NR⁶R⁷ is 4-(3-fluoropropyl)piperazin-1-yl)

The titled compound was prepared as described in Example 1 by using 2-(5-chlorosulfonyl-2-*n*-propoxyphenyl)-5,6-dimethyl-7-*n*-propyl-3,5-dihydro-4*H*-pyr rolo[3,2-*d*]pyrimidin-4-one and 1-(3-fluoropropyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 86%
mp 156-157 °C (CHCl₃/Et₂O/hexanes);
IR (neat) 3312 (NH), 1679 (C=O), 1174 (SO₂) cm⁻¹;
¹H NMR (CDCl₃/TMS) δ 0. 94 (t, *J* = 7. 5 Hz, 3 H, CH₂CH₂C*H*₃), 1. 19(t, *J* = 7. 5 Hz, 3 H, OCH₂CH₂C*H*₃), 1. 61-1. 89 (m, 4 H, CH₂C*H*₂CH₂F and CH₂C*H*₂CH₃), 1. 98-2. 10 (m, 2 H, OCH₂C*H*₂CH₃), 2. 31 (s, 3 H, CH₃), 2. 47 (t, *J* = 7. 2 Hz, 2 H, NC*H*₂CH₂), 2,54 (dd, J = 5. 1 Hz, 4. 8 Hz, 4 H, 2 NCH₂), 2. 68 (t, *J* = 7. 5 Hz, 2 H, C*H*₂CH₂CH₃), 3. 11 (br dd, *J* = 5. 1 Hz, 4. 8 Hz, 4 H, 2 SO₂NCH₂), 4. 04 (s, 3 H, NCH₃), 4. 24 t, *J* = 6. 6 Hz, 2 H, OC*H*₂CH₂CH₃), 4. 43 (dt, *J* = 47. 1 Hz, 6. 0 Hz, 2 H, CH₂C*H*₂F), 7. 13 (d, *J* = 8. 7 Hz, 1 H, H-3'), 7. 80 (dd, *J* = 8. 7 Hz, 2. 4 Hz, 1 H, H-4'), 8. 88 (d, *J* = 2. 4 Hz, 1 H, H-6'), 10.61 (br s, 1 H, NH); MS (FAB) *m*/*z* 548 (MH⁺).

### Example 77

### Preparation of 5,6-dimethyl-2-(2-ethoxy-5-(4-(2-hydroxyethyl)piperazin-1-ylsulfonyl)phenyl)-7-n-pr opyl-3,5-dihydro-4H-pyrrolo[3,2-d]pyrimidin-4-one (a compound of the formula (1) wherein R⁵ = SO₂NR⁶R⁷, R¹ = R² = CH₃, R³ = CH₂CH₂CH₃, R⁴ = CH₂CH₃; NR⁶R⁷ is 4-(2-hydroxyethyl)piperazin-1-yl)

The titled compound was prepared as described in Example 1 by using 2-(5-chlorosulfonyl-2-ethoxyphenyl)-5,6-dimethyl-7-*n*-propyl-3,5-dihydro-4*H*-pyrrolo [3,2-*d*]pyrimidin-4-one and 1-(2-hydroxyethyl)piperazine in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 77%
mp 130-130. 5 °C (CHCl₃/Et₂O/hexanes);
IR (neat) 3555, 3323 (NH and OH), 1663 (C=O), 1168 (SO₂) cm⁻¹;
¹H NMR (CDCl₃/TMS) δ 0. 95 (t, *J* = 7. 5 Hz, 3 H, CH₂CH₂C*H*₃), 1. 64(t, *J* = 6. 9 Hz, 3 H, OCH₂C*H*₃), 1. 58-1. 71 (m, 2 H, CH₂C*H*₂CH₃), 2. 31(s, 3 H, CH₃), 2. 55(t,*J* = 5. 4 Hz, 2 H, NC*H*₂CH₂), 2. 61 (dd, *J* = 5. 1 Hz, 4. 5 Hz, 4 H, 2 NCH₂), 2. 68 (t, *J* = 7. 5 Hz, 2 H, C*H*₂CH₂CH₃), 3. 13(br dd, *J* = 5. 1 Hz, 4. 5 Hz, 4 H, 2 SO₂NCH₂), 3. 57(br t, *J* = 5. 4 Hz, 2 H, CH₂C*H*₂OH), 4. 04 (s, 3 H, NCH₃), 4. 36 (q, *J* = 6. 9 Hz, 2 H, OC*H*₂CH₃), 7. 13 (d, *J* = 8. 7 Hz, 1 H, H-3'), 7. 81(dd, *J* = 8. 7 Hz, 2. 7 Hz, 1 H, H-4'), 8. 88 (d, *J* = 2. 7 Hz, 1 H, H-6'), 10. 58 (br s, 1 H, NH); MS (FAB) *m*/*z* 518 (MH⁺).

### Example 78

### Preparation of 5,6-dimethyl-2-(5-(4-(2-hydroxyethyl)piperazin-1-ylsulfonyl)-2-n-propoxyphenyl)-7-n - propyl-3,5-dihydro-4H-pyrrolo[3,2-d]pyrimidin-4-one (a compound of the formula (1) wherein R⁵ = SO₂NR⁶R⁷, R¹ = R² = CH₃, R³ = CH₂CH₂CH₃, R⁴ = CH₂CH₂CH₃; NR⁶R⁷ is 4-(2-hydroxyethyl)piperazin-1-yl)

The titled compound was prepared as described in Example 1 by using 2-(5-chlorosulfonyl-2-*n*-propoxyphenyl)-5,6-dimethyl-7-*n*-propyl-3,5-dihydro-4*H*-pyr rolo[3,2-*d*]pyrimidin-4-one and 1-(2-hydroxyethyl)piperazine in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 81 %
mp 202-202. 5 °C (CHCl₃/Et₂O/hexanes);
IR (neat) 3402, 3313 (NH and OH), 1662 (C=O), 1173 (SO₂) cm⁻¹;
¹H NMR (CDCl₃/TMS) δ 0. 95 (t, *J* = 7. 5 Hz, 3 H, CH₂CH₂C*H*₃), 1. 19 (t, *J* = 7. 5 Hz, 3 H, OCH₂CH₂C*H*₃), 1. 60-1. 73 (m, 2 H, CH₂C*H*₂CH₃), 1. 98-2. 09 (m, 2 H, OCH₂C*H*₂CH₃), 2. 31 (s, 3 H, CH₃), 2. 56 (t, *J* = 5. 4 Hz, 2 H, NC*H*₂CH₂), 2. 63 (dd, *J* = 4. 8 Hz, 4. 5 Hz, 4 H, 2 NCH₂), 2. 68 (t, *J* = 7. 5 Hz, 2 H, C*H*₂CH₂CH₃), 3. 14 (br dd, *J* = 4. 8 Hz, 4. 5 Hz, 4 H, 2 SO₂NCH₂), 3. 58 (t, *J* = 5. 4 Hz, 2 H, CH₂C*H*₂OH), 4. 04 (s, 3 H, NCH₃), 4. 25 (t, *J* = 6. 6 Hz, 2 H, OC*H*₂CH₂CH₃), 7. 14 (d, *J* = 8. 7 Hz, 1 H, H-3'), 7. 81 (dd, *J* = 8. 7 Hz, 2. 4 Hz, 1 H, H-4'), 8. 88 (d, *J* = 2. 4 Hz, 1 H, H-6'), 10. 62 (br s, 1 H, NH); MS (FAB) *m*/*z* 532 (MH⁺).

### Example 79

### Preparation of 5,6-dimethyl-2-(2-ethoxy-5-(4-(3-hydroxypropyl)piperazin-1-ylsulfonyl)phenyl)-7-n-p ropyl-3,5-dihydro-4H-pyrrolo[3,2-d]pyrimidin-4-one (a compound of the formula (1) wherein R⁵ = SO₂NR⁶R⁷, R¹ = R² = CH₃, R³ = CH₂CH₂CH₃, R⁴ = CH₂CH₃; NR⁶R⁷ is 4-(3-hydroxypropyl)piperazin-1-yl)

The titled compound was prepared as described in Example 1 by using 2-(5-chlorosulfonyl-2-ethoxyphenyl)-5,6-dimethyl-7-*n*-propyl-3,5-dihydro-4*H*-pyrrolo [3,2-*d*]pyrimidin-4-one and 1-(3-hydroxypropyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 80%
mp 209-209. 5 °C (CHCl₃/Et₂O/hexanes);
IR (neat) 3346, 3320 (NH and OH), 1680 (C=O), 1171 (SO₂) cm⁻¹;
¹H NMR (CDCl₃/TMS) δ 0. 95 (t, *J* = 7. 5 Hz, 3 H, CH₂CH₂C*H*₃), 1. 61-1. 79 (m, 4 H, CH₂C*H*₂CH₂OH and CH₂C*H*₂CH₃), 1. 64 (t, *J* = 6. 9 Hz, 3 H, OCH₂C*H*₃), 2. 31 (s, 3 H, CH₃), 2. 65-2. 83 (m, 6 H, NC*H*₂CH₂ and 2 NCH₂), 2. 68 (t, *J* = 7. 5 Hz, 2 H, C*H*₂CH₂CH₃), 3. 22 (br s, 4 H, 2 SO₂NCH₂), 3. 73 (t, *J* = 5. 4 Hz, 2 H, CH₂C*H*₂OH), 4. 04 (s, 3 H, NCH₃), 4. 36 (q, *J* = 6. 9 Hz, 2 H, OC*H*₂CH₃), 7. 13 (d, *J* = 8. 7 Hz, 1 H, H-3'), 7. 76 (dd, *J* = 8. 7 Hz, 2. 1 Hz, 1 H, H-4'), 8. 86 (d, *J* = 2. 1 Hz, 1 H, H-6'), 10. 61 (br s, 1 H, NH); MS (FAB) *m*/*z* 532 (MH⁺).

### Example 80

### Preparation of 5,6-dimethyl-2-(5-(4-(3-hydroxypropyl)piperazin-1-ylsulfonyl)-2-n-propoxyphenyl)-7 - n-propyl-3,5-dihydro-4H-pyrrolo[3,2-d]pyrimidin-4-one (a compound of the formula (1) wherein R⁵ = SO₂NR⁶R⁷, R¹ = R² = CH₃, R³ = CH₂CH₂CH₃, R⁴ = CH₂CH₂CH₃; NR⁶R⁷ is 4-(3-hydroxypropyl)piperazin-1-yl)

The titled compound was prepared as described in Example 1 by using 2-(5-chlorosulfonyl-2-*n*-propoxyphenyl)-5,6-dimethyl-7-*n*-propyl-3,5-dihydro-4*H*-pyr rolo[3,2-*d*]pyrimidin-4-one and 1-(3-hydroxypropyl)piperazine in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 54%
mp 182-183 °C (CHCl₃/Et₂O/hexanes);
IR (neat) 3490, 3310 (NH and OH), 1672 (C=O), 1172 (SO₂) cm⁻¹;
¹H NMR (CDCl₃/TMS) δ 0. 95 (t, *J* = 7. 5 Hz, 3 H, CH₂CH₂C*H*₃), 1. 20(t, *J* = 7. 2 Hz, 3 H, OCH₂CH₂C*H*₃), 1. 61-1. 76(m, 4 H, CH₂C*H*₂CH₂OH and CH₂C*H*₂CH₃), 1. 99-2. 10 (m, 2 H, OCH₂C*H*₂CH₃), 2. 31 (s, 3 H, CH₃), 2. 62-2. 67 (m, 6 H, NC*H*₂CH₂ and 2 NCH₂), 2. 69 (t, *J* = 7. 2 Hz, 2 H, C*H*₂CH₂CH₃), 3. 07-3. 13 (m, 4 H, 2 SO₂NCH₂), 3. 71 (t, *J* = 5. 4 Hz, 2 H, CH₂C*H*₂OH), 4. 04 (s, 3 H, NCH₃), 4. 25 (t, *J* = 6. 3 Hz, 2 H, OC*H*₂CH₂CH₃), 7. 13 (d, *J* = 8. 7 Hz, 1 H, H-3'), 7. 77 (dd, *J* = 8. 7 Hz, 2. 4 Hz, 1 H, H-4'), 8. 87 (d, *J* = 2. 4 Hz, 1 H, H-6'), 10. 65 (br s, 1 H, NH); MS (FAB) *m*/*z* 546 (MH⁺).

### Example 81

### Preparation of 6-chloro-2-(2-ethoxy-5-(4-methylpiperazin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Cl, R³ = CH₂CH₂CH₃, R⁴ = CH₂CH₃; NR⁶R⁷ is 4-methylpiperazin-1-yl)

The titled compound was prepared as described in Example 1 by using 6-chloro-2-(5-chlorosulfonyl-2-ethoxyphenyl)-5-methyl-7-*n*-propyl-3,5-dihydro-4*H*-p yrrolo[3,2-*d*]pyrimidin-4-one in place of 2-(5-chlorosulfonyl-2-ethoxyphenyl)-5-methyl-7-*n*-propyl-3,5-dihydro-4*H*-pyrrolo[3,2 - *d*]pyrimidin-4-one.
yield: 97%
mp 115-116 °C (EtOAc/CHCl₃/hexanes);
IR (neat) 3326 (NH), 1680 (C=O), 1168 (SO₂) cm⁻¹;
¹H NMR (CDCl₃/TMS) δ 0. 96 (t, *J* = 7. 5 Hz, 3 H, CH₂CH₂CH₃), 1. 64 (t, *J* = 6. 9 Hz, 3 H, OCH₂C*H*₃), 1. 68-1. 79 (m, 2 H, CH₂C*H*₂CH₃), 2. 28 (s, 3 H, NCH₃), 2. 49-2. 52 (m, 4 H, 2 NCH₂), 2. 72 (t, *J* = 7. 5 Hz, 2 H, C*H*₂CH₂CH₃), 3. 11-3. 13 (m, 4 H, 2 SO₂NCH₂), 4. 07 (s, 3 H, NCH₃), 4. 36 (q, *J* = 6. 9 Hz, 2 H, OC*H*₂CH₃), 7. 13 (d, *J* = 8. 7 Hz, 1 H, H-3'), 7. 82 (dd, *J* = 8. 7 Hz, 2. 4 Hz, 1 H, H-4'), 8. 85 (d, *J* = 2. 4 Hz, 1 H, H-6'), 10. 72 (br s, 1 H, NH); MS (FAB) *m*/*z* 508 (MH⁺).

### Example 82

### Preparation of 6-chloro-2-(5-(4-methylpiperazin-1-ylsulfonyl)-2-n-propoxyphenyl)-5-methyl-7-n-pro pyl-3,5-dihydro-4H-pyrrolo[3,2-d]pyrimidin-4-one (a compound of the formula (1) wherein R⁵ = SO₂NR⁶R⁷, R¹ = CH₃, R² = Cl, R³ = CH₂CH₂CH₃, R⁴ = CH₂CH₂CH₃; NR⁶R⁷ is 4-methylpiperazin-1-yl)

The titled compound was prepared as described in Example 1 by using 6-chloro-2-(5-chlorosulfonyl-2-*n*-propoxyphenyl)-5-methyl-7-*n*-propyl-3,5-dihydro-4 *H*-pyrrolo[3,2-*d*]pyrimidin-4-one in place of 2-(5-chlorosulfonyl-2-ethoxyphenyl)-5-methyl-7-*n*-propyl-3,5-dihydro-4*H*-pyrrolo[3,2 -*d*]pyrimidin-4-one.
yield: 97%
mp 194-194. 5 °C (EtOAc/CHCl₃/hexanes);
IR (neat) 3306 (NH), 1686 (C=O), 1175 (SO₂) cm⁻¹;
¹H NMR (CDCl₃/TMS) δ 0. 96 (t, *J* = 7. 5 Hz, 3 H, CH₂CH₂C*H*₃), 1. 19 (t, *J* = 7. 5 Hz, 3 H, OCH₂CH₂C*H*₃), 1. 67-1. 79 (m, 2 H, CH₂C*H*₂CH₃), 1. 99-2. 10 (m, 2 H, OCH₂C*H*₂CH₃), 2. 27 (s, 3 H, NCH₃), 2. 49 (dd, *J* = 5. 1 Hz, 4. 5 Hz, 4 H, 2 NCH₂), 2. 72 (t, *J* = 7. 5 Hz, 2 H, C*H*₂CH₂CH₃), 3. 11 (br dd, *J* = 5. 1 Hz, 4. 5 Hz, 4 H, 2 SO₂NCH₂), 4. 07 (s, 3 H, NCH₃), 4. 25 (t, *J* = 6. 6 Hz, 2 H, OC*H*₂CH₂CH₃), 7. 13 (d, *J* = 8. 7 Hz, 1 H, H-3'), 7. 82 (dd, *J* = 8. 7 Hz, 2. 4 Hz, 1 H, H-4'), 8. 87 (d, *J* = 2. 4 Hz, 1 H, H-6'), 10. 74 (br s, 1 H, NH); MS (FAB) *m*/*z* 522 (MH⁺).

### Example 83

### Preparation of 6-chloro-2-(2-ethoxy-5-(4-(2-fluoroethyl)piperazin-1-ylsulfonyl)phenyl)-5-methyl-7-n -propyl-3,5-dihydro-4H-pyrrolo[3,2-d]pyrimidin-4-one (a compound of the formula (1) wherein R⁵ = SO₂NR⁶R⁷, R¹ = CH₃, R² = Cl, R³ = CH₂CH₂CH₃, R⁴ = CH₂CH₃; NR⁶R⁷ is 4-(2-fluoroethyl)piperazin-1-yl)

The titled compound was prepared as described in Example 1 by using 6-chloro-2-(5-chlorosulfonyl-2-ethoxyphenyl)-5-methyl-7-*n*-propyl-3,5-dihydro-4*H*-p yrrolo[3,2-*d*]pyrimidin-4-one and 1-(2-fluoroethyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 96%
mp 222-223 °C (EtOAc/CHCl₃/hexanes);
IR (neat) 3330 (NH), 1674 (C=O), 1168 (SO₂) cm⁻¹;
¹H NMR (CDCl₃/TMS) δ 0. 96 (t, *J* = 7. 5 Hz, 3 H, CH₂CH₂C*H*₃), 1. 65 (t, *J* = 6. 9 Hz, 3 H, OCH₂C*H*₃), 1. 67-1. 79 (m, 2 H, CH₂C*H*₂CH₃), 2. 62-2. 75 (m, 6 H, NC*H*₂CH₂F and 2 NCH₂), 2. 72 (t, *J* = 7. 5 Hz, 2 H, C*H*₂CH₂CH₃), 3. 11-3. 14 (m, 4 H, 2 SO₂NCH₂), 4. 08 (s, 3 H, NCH₃), 4. 37 (q, *J* = 6. 9 Hz, 2 H, OC*H*₂CH₃), 4. 50 (td, *J* = 47. 4 Hz, 4. 5 Hz, 2 H, NCH₂C*H*₂F), 7. 13 (d, *J* = 8. 7 Hz, 1 H, H-3'), 7. 82 (dd, *J* = 8. 7 Hz, 2. 4 Hz, 1 H, H-4'), 8. 86 (d, *J* = 2. 4 Hz, 1 H, H-6'), 10. 70 (br s, 1 H, NH); MS (FAB) *m*/*z* 540 (MH⁺).

### Example 84

### Preparation of 6-chloro-2-(5-(4-(2-fluoroethyl)piperazin-1-ylsulfonyl)-2-n-propoxyphenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Cl, R³ = CH₂CH₂CH₃, R⁴ = CH₂CH₂CH₃; NR⁶R⁷ is 4-(2-fluoroethyl)piperazin-1-yl)

The titled compound was prepared as described in Example 1 by using 6-chloro-2-(5-chlorosulfonyl-2-*n*-propoxyphenyl)-5-methyl-7-*n*-propyl-3,5-dihydro-4 *H*-pyrrolo[3,2-*d*]pyrimidin-4-one and 1-(2-fluoroethyl)piperazine hydrochloride in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 97%
mp 184-184. 5 °C (EtOAc/CHCl₃/hexanes);
IR (neat) 3313 (NH), 1687 (C=O), 1173 (SO₂) cm⁻¹;
¹H NMR (CDCl₃/TMS) δ 0. 96 (t, *J* = 7. 5 Hz, 3 H, CH₂CH₂C*H*₃), 1. 19 (t, *J* = 7. 5 Hz, 3 H, OCH₂CH₂C*H*₃), 1. 67-1. 79 (m, 2 H, CH₂C*H*₂CH₃), 1. 99-2. 10 (m, 2 H, OCH₂C*H*₂CH₃), 2. 62-2. 75 (m, 8 H, NC*H*₂CH₂F, 2 NCH₂ and C*H*₂CH₂CH₃), 3. 10-3. 15 (m, 4 H, 2 SO₂NCH₂), 4. 07 (s, 3 H, NCH₃), 4. 25 (t, *J* = 6. 6 Hz, 2 H, OC*H*₂CH₂CH₃), 4. 49 (ddd, *J* = 47. 7 Hz, 5. 1 Hz, 4. 5 Hz, 2 H, NCH₂C*H*₂F), 7. 14 (d, *J* = 8. 7 Hz, 1 H, H-3'), 7. 82 *(dd, J* = 8. 7 Hz, 2. 4 Hz, 1 H, H-4'), 8. 87 (d, *J* = 2. 4 Hz, 1 H, H-6'), 10. 74 (br s, 1 H, NH); MS (FAB) *m*/*z* 554 (MH⁺).

### Example 85

### Preparation of 6-chloro-2-(2-ethoxy-5-(4-(3-fluoropropyl)piperazin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R' = CH₃, R² = Cl, R³ = CH₂CH₂CH₃, R⁴ = CH₂CH₃; NR⁶R⁷ is 4-(3-fluoropropyl)piperazin-1-yl)

The titled compound was prepared as described in Example 1 by using 6-chloro-2-(5-chlorosulfonyl-2-ethoxyphenyl)-5-methyl-7-*n*-propyl-3,5-dihydro-4*H*-p yrrolo[3,2-*d*]pyrimidin-4-one and 1-(3-fluoropropyl)piperazine hydrochloride in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 94%
mp 181-182 °C (CHCl₃/hexanes);
IR (neat) 3336 (NH), 1681 (C=O), 1170 (SO₂) cm⁻¹;
¹H NMR (CDCl₃/TMS) δ 0. 96 (t, *J* = 7. 5 Hz, 3 H, CH₂CH₂CH₃), 1. 64(t, *J* = 6. 9 Hz, 3 H, OCH₂C*H*₃), 1. 73-1. 84 (m, 4 H, CH₂C*H*₂CH₂F and CH₂C*H*₂CH₃), 2. 47 (t, *J* = 7. 2 Hz, 2 H, NC*H*₂CH₂), 2. 54 (dd, *J* = 5. 4 Hz, 4. 2 Hz, 4 H, 2 NCH₂), 2. 72 (t, *J* = 7. 5 Hz, 2 H, C*H*₂CH₂CH₃), 3. 10(br dd, *J* = 5. 4 Hz, 4. 2 Hz, 4 H, 2 SO₂NCH₂), 4. 08(s, 3 H, NCH₃), 4. 37 (q, *J* = 6. 9 Hz, 2 H, OC*H*₂CH₃), 4. 43 (dt, *J* = 47. 4 Hz, 6. 0 Hz, 2 H, CH₂C*H*₂F), 7. 13 (d, *J* = 8. 7 Hz, 1 H, H-3'), 7. 82(dd, *J* = 8. 7 Hz, 2. 4 Hz, 1 H, H-4'), 8. 86 (d, *J* = 2. 4 Hz, 1 H, H-6'), 10. 71 (br s, 1 H, NH); MS (FAB) *m*/*z* 554 (MH⁺).

### Example 86

### Preparation of 6-chloro-2-(5-(4-(3-fluoropropyl)piperazin-1-ylsulfonyl)-2-n-propoxyphenyl)-5-methy 1-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Cl, R³ = CH₂CH₂CH₃, R⁴ = CH₂CH₂CH₃; NR⁶R⁷ is 4-(3-fluoropropyl)piperazin-1-yl)

The titled compound was prepared as described in Example 1 by using 6-chloro-2-(5-chlorosulfonyl-2-*n*-propoxyphenyl)-5-methyl-7-*n*-propyl-3,5-dihydro-4 *H*-pyrrolo[3,2-*d*]pyrimidin-4-one and 1-(3-fluoropropyl)piperazine hydrochloride in place of 2-(5-chlorosulfonyl-2-ethoxyphenyl)-5-methyl-7-*n*-propyl-3,5-dihydro-4*H*-pyrrolo[3,2 - *d*]pyrimidin-4-one and I -methylpiperazine.
yield: 79%
mp 179-180 °C (EtOAc/CHCl₃/hexanes);
IR (neat) 3315 (NH), 1690 (C=O), 1172 (SO₂) cm⁻¹;
¹H NMR (CDCl₃/TMS) δ 0. 96 (t, *J* = 7. 5 Hz, 3 H, CH₂CH₂C*H*₃), 1. 19 (t, *J* = 7. 5 Hz, 3 H, OCH₂CH₂C*H*₃), 1. 67-1. 89 (m, 4 H, CH₂C*H*₂CH₂F and CH₂C*H*₂CH₃), 1. 99-2. 10 (m, 2 H, OCH₂C*H*₂CH₃), 2. 47 (t, *J* = 7. 2 Hz, 2 H, NC*H*₂CH₂), 2. 54 (dd, *J* = 4. 8 Hz, 4. 5 Hz, 4 H, 2 NCH₂), 2. 72 (t, *J* = 7.5 Hz, 2 H, C*H*₂CH₂CH₃), 3. 10 (br dd, *J* = 4. 8 Hz, 4. 5 Hz, 4 H, 2 SO₂NCH₂), 4. 07 (s, 3 H, NCH₃), 4. 25 (t, *J* = 6. 6 Hz, 2 H, OC*H*₂CH₂CH₃), 4. 43 (dt, *J* = 47. 1 Hz, 6. 0 Hz, 2 H, CH₂C*H*₂F), 7. 14 (d, *J* = 8. 7 Hz, 1 H, H-3'), 7. 82 (dd, *J* = 8. 7 Hz, 2. 4 Hz, 1 H, H-4'), 8. 87 (d, *J* = 2. 4 Hz, 1 H, H-6'), 10. 75 (br s, 1 H, NH); MS (FAB) *m*/*z* 568 (MH⁺).

### Example 87

### Preparation of 6-chloro-2-(2-ethoxy-5-(4-(2-hydroxyethyl)piperazin-1-ylsulfonyl)phenyl)-5-methyl-7 -n-propyl-3,5-dihydro-4H-pyrrolo[3,2-d]pyrimidin-4-one (a compound of the formula (1) wherein R⁵ = SO₂NR⁶R⁷, R¹ = CH₃, R² = Cl, R³ = CH₂CH₂CH₃, R⁴ = CH₂CH₃; NR⁶R⁷ is 4-(2-hydroxyethyl)piperazin-1-yl)

The titled compound was prepared as described in Example 1 by using 6-chloro-2-(5-chlorosulfonyl-2-ethoxyphenyl)-5-methyl-7-*n*-propyl-3,5-dihydro-4*H*-p yrrolo[3,2-*d*]pyrimidin-4-one and 1-(2-hydroxyethyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 96%
mp 201-201. 5 °C (CHCl₃/hexanes);
IR (neat) 3553, 3327 (NH and OH), 1677 (C=O), 1168 (SO₂) cm⁻¹;
¹H NMR (CDCl₃/TMS) δ 0. 96 (t, *J* = 7. 5 Hz, 3 H, CH₂CH₂C*H*₃), 1. 65 (t, *J* = 6. 9 Hz, 3 H, OCH₂C*H*₃), 1. 67-1. 77 (m, 2 H, CH₂C*H*₂CH₃), 2. 57 (t, *J* = 5. 4 Hz, 2 H, NC*H*₂CH₂), 2. 64 (dd, *J* = 4. 8 Hz, 4. 5 Hz, 4 H, 2 NCH₂), 2. 72 (t, *J* = 7. 5 Hz, 2 H, C*H*₂CH₂CH₃), 3. 14 (br dd, *J* = 4. 8 Hz, 4. 5 Hz, 4 H, 2 SO₂NCH₂), 3. 59 (t, *J* = 5. 4 Hz, 2 H, CH₂C*H*₂OH), 4. 07 (s, 3 H, NCH₃), 4. 37 (q, *J* = 6. 9 Hz, 2 H, OC*H*₂CH₃), 7. 15 (d, *J* = 8. 7 Hz, 1 H, H-3'), 7. 82 (dd, *J=* 8. 7 Hz, 2. 7 Hz, 1 H, H-4'), 8. 86 (d, *J* = 2. 7 Hz, 1 H, H-6'), 10.71 (br s, 1 H, NH); MS (FAB) *m*/*z* 538 (MH⁺).

### Example 88

### Preparation of 6-chloro-2-(5-(4-(2-hydroxyethyl)piperazin-1-ylsulfonyl)-2-n-propoxyphenyl)-5-meth yl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Cl, R³ = CH₂CH₂CH₃, R⁴ = CH₂CH₂CH₃; NR⁶R⁷ is 4-(2-hydroxyethyl)piperazin-1-yl)

The titled compound was prepared as described in Example 1 by using 6-chloro-2-(5-chlorosulfonyl-2-*n*-propoxyphenyl)-5-methyl-7-*n*-propyl-3,5-dihydro-4 *H*-pyrrolo[3,2-*d*]pyrimidin-4-one and 1-(2-hydroxyethyl)piperazine in place of 2-(5-chiorosulfonyl-2-ethoxyphenyl)-5-methyl-7-*n*-propyl-3,5-dihydro-4*H*-pyrroio[3,2 -*d*]pyrimidin-4-one and 1-methylpiperazine.
yield: 94%
mp 194-195 °C (EtOAc/CHCl₃/hexanes);
IR (neat) 3452, 3318 (NH and OH), 1687 (C=O), 1173 (SO₂) cm⁻¹;
¹H NMR (CDCl₃/TMS) δ 0. 96 (t, *J* = 7. 5 Hz, 3 H, CH₂CH₂C*H*₃), 1. 19 (t, *J* = 7. 5 Hz, 3 H, OCH₂CH₂C*H*₃), 1. 67-1. 79 (m, 2 H, CH₂C*H*₂CH₃), 1. 99-2. 11 (m, 2 H, OCH₂C*H*₂CH₃), 2.32 (br s, 1 H, OH), 2. 55 (dd, *J* = 5. 4 Hz, 5. 1 Hz, 2 H, NC*H*₂CH₂), 2. 61 (dd, *J* = 5. 1 Hz, 4. 8 Hz, 4 H, 2 NCH₂), 2. 72 (t, *J* = 7.5 Hz, 2 H, C*H*₂CH₂CH₃), 3. 12 (br dd, *J* = 5. 1 Hz, 4. 8 Hz, 4 H, 2 SO₂NCH₂), 3. 57-3. 58 (m, 2 H, CH₂C*H*₂OH), 4. 07 (s, 3 H, NCH₃), 4. 26 (t, *J* = 6. 6 Hz, 2 H, OC*H*₂CH₂CH₃), 7. 15 (d, *J* = 8. 7 Hz, 1 H, H-3'), 7. 83 (dd, *J* = 8. 7 Hz, 2. 4 Hz, 1 H, H-4'), 8. 87 (d, *J* = 2. 4 Hz, 1 H, H-6'), 10. 75 (br s, 1 H, NH); MS (FAB) *m*/*z* 552 (MH⁺).

### Example 89

### Preparation of 6-chloro-2-(2-ethoxy-5-(4-(3-hydroxypropyl)piperazin-1-ylsulfonyl)phenyl)-5-methyl-7-n-propyl-3,5-dihydro-4H-pyrrolo[3,2-d]pyrimidin-4-one (a compound of the formula (I) wherein R⁵ = SO₂NR⁶R⁷, R¹ = CH₃, R² = Cl, R³ = CH₂CH₂CH₃, R⁴ = CH₂CH₃; NR⁶R⁷ is 4-(3-hydroxypropyl)piperazin-1-yl)

The titled compound was prepared as described in Example 1 by using 6-chloro-2-(5-chlorosulfonyl-2-ethoxyphenyl)-5-methyl-7-*n*-propyl-3,5-dihydro-4*H*-p yrrolo[3,2-*d*]pyrimidin-4-one and 1-(3-hydroxypropyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 98%
mp 189-189.5 °C (CHCl₃/hexanes);
IR (neat) 3331 (NH and OH), 1686 (C=O), 1168 (SO₂) cm⁻¹;
¹H NMR (CDCl₃/TMS) δ 0. 96 (t, *J* = 7. 5 Hz, 3 H, CH₂CH₂C*H*₃), 1. 65(t, *J* = 6. 9 Hz, 3 H, OCH₂C*H*₃), 1. 67-1. 79 (m, 4 H, CH₂C*H*₂CH₂OH and CH₂C*H*₂CH₃), 2. 64 (br s, 6 H, NC*H*₂CH₂ and 2 NCH₂), 2. 72 (t, *J* = 7. 2 Hz, 2 H, C*H*₂CH₂CH₃), 3. 10 (br s, 4 H, 2 SO₂NCH₂), 3. 72 (t, *J* = 5. 4 Hz, 2 H, CH₂C*H*₂OH), 4. 08 (s, 3 H, NCH₃), 4. 38 (q, *J* = 6. 9 Hz, 2 H, OC*H*₂CH₃), 7. 14 (d, *J* = 8. 7 Hz, 1 H, H-3'), 7. 79 (dd, *J* = 8. 7 Hz, 2. 7 Hz, 1 H, H-4'), 8. 85 (d, *J* = 2. 7 Hz, 1 H, H-6'), 10. 73 (br s, 1 H, NH); MS (FAB) *m*/*z* 552 (MH⁺).

### Example 90

### Preparation of 6-chloro-2-(5-(4-(3-hydroxypropyl)piperazin-1-ylsulfonyl)-2-n-propoxyphenyl)-5-met hyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Cl, R³ = CH₂CH₂CH₃, R⁴ = CH₂CH₂CH₃; NR⁶R⁷ is 4-(3-hydroxypropyl)piperazin-1-yl)

The titled compound was prepared as described in Example 1 by using 6-chloro-2-(5-chlorosulfonyl-2-*n*-propoxyphenyl)-5-methyl-7-*n*-propyl-3,5-dihydro-4 *H*-pyrrolo[3,2-*d*]pyrimidin-4-one and 1-(3-hydroxypropyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 89%
mp 192-193 °C (EtOAc/CHCl₃/hexanes);
IR (neat) 3419, 3313 (NH and OH), 1688 (C=O), 1173 (SO₂) cm⁻¹;
¹H NMR (CDCl₃/TMS) δ 0. 97 (t, *J* = 7. 5 Hz, 3 H, CH₂CH₂C*H*₃), 1. 20 (t, *J* = 7. 5 Hz, 3 H, OCH₂CH₂C*H*₃), 1. 64-1. 79 (m, 4 H, CH₂C*H*₂CH₂OH and CH₂C*H*₂CH₃), 2. 00-2. 11 (m, 2 H, OCH₂C*H*₂CH₃), 2. 59-2. 62 (m, 6 H, NC*H*₂CH₂ and 2 NCH₂), 2. 72 (t, *J* = 7. 5 Hz, 2 H, CH₂CH₂CH₃), 3. 04-3. 12 (m, 4 H, 2 SO₂NCH₂), 3. 71 (t, *J* = 5. 4 Hz, 2 H, CH₂C*H*₂OH), 4. 08 (s, 3 H, NCH₃), 4. 27 (t, *J* = 6. 6 Hz, 2 H, OC*H*₂CH₂CH₃), 7. 14 (d, *J* = 9. 0 Hz, 1 H, H-3'), 7. 79 (dd, *J* = 9. 0 Hz, 2. 4 Hz, 1 H, H-4'), 8. 86 (d, *J* = 2. 4 Hz, 1 H, H-6'), 10. 77 (br s, 1 H, NH); MS (FAB) *m*/*z* 566 (MH⁺).

### Example 91

### Preparation of 6-bromo-2-(2-ethoxy-5-(4-methylpiperazin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Br, R³ = CH₂CH₂CH₃, R⁴ = CH₂CH₃; NR⁶R⁷ is 4-methylpiperazin-1-yl)

The titled compound was prepared as described in Example 1 by using 6-bromo-2-(5-chlorosulfonyl-2-ethoxyphenyl)-5-methyl-7-*n*-propyl-3,5-dihydro-4*H*-p yrrolo[3,2-*d*]pyrimidin-4-one in place of 2-(5-chlorosulfonyl-2-ethoxyphenyl)-5-methyl-7-*n*-propyl-3,5-dihydro-4*H*-pyrrolo[3,2 - *d*]pyrimidin-4-one.
yield: 87%
mp 202. 5-203 °C dec (EtOAc/CHCl₃/hexanes);
IR (neat) 3334 (NH), 1679 (C=O), 1171 (SO₂) cm⁻¹;
¹H NMR (CDCl₃/TMS) δ 0. 96 (t, *J* = 7. 5 Hz, 3 H, CH₂CH₂C*H*₃), 1. 64(t, *J* = 7. 2 Hz, 3 H, OCH₂C*H*₃), 1. 68-1. 79 (m, 2 H, CH₂C*H*₂CH₃), 2. 27 (s, 3 H, NCH₃), 2. 50 (dd, J = 4. 8 Hz, 4. 2 Hz, 4 H, 2 NCH₂), 2. 71 (t, *J* = 7. 5 Hz, 2 H, C*H*₂CH₂CH₃), 3. 09-3. 13 (m, 4 H, 2 SO₂NCH₂), 4. 10 (s, 3 H, NCH₃), 4. 36 (q, *J* = 7. 2 Hz, 2 H, OC*H*₂CH₃), 7. 13 (d, *J* = 9. 0 Hz, 1 H, H-3'), 7. 82 (dd, *J* = 9. 0 Hz, 2. 4 Hz, 1 H, H-4'), 8. 85 (d, *J* = 2. 4 Hz, 1 H, H-6'), 10. 71 (br s, 1 H, NH); MS (FA8) *m*/*z* 552, 554 (MH⁺).

### Example 92

### Preparation of 6-bromo-2-(5-(4-methylpiperazin-1-ylsulfonyl)-2-n-propoxyphenyl)-5-methyl-7-n-pro pyl-3,5-dihydro-4H-pyrrolo[3,2-d]pyrimidin-4-one (a compound of the formula (1) wherein R⁵ = SO₂NR⁶R⁷, R¹ = CH₃, R² = Br, R³ = CH₂CH₂CH₃, R⁴ = CH₂CH₂CH₃; NR⁶R⁷ is 4-methylpiperazin-1-yl)

The titled compound was prepared as described in Example 1 by using 6-bromo-2-(5-chlorosulfonyl-2-*n*-propoxyphenyl)-5-methyl-7-*n*-propyl-3,5-dihydro-4 *H*-pyrrolo[3,2-*d*]pyrimidin-4-one in place of 2-(5-chlorosulfonyl-2-ethoxyphenyl)-5-methyl-7-*n*-propyl-3,5-dihydro-4*H*-pyrrolo[3,2 -*d*]pyrimidin-4-one.
yield: 92%
mp 260 °C dec (CHCl₃/hexanes);
IR (neat) 3300 (NH), 1683 (C=O), 1173 (SO₂) cm⁻¹;
¹H NMR (CDCl₃/TMS) δ 0. 96 (t, *J* = 7.5 Hz, 3 H, CH₂CH₂CH₃), 1. 19 (t, *J* = 7. 2 Hz, 3 H, OCH₂CH₂C*H*₃), 1. 66-1. 78 (m, 2 H, CH₂C*H*₂CH₃), 1. 98-2. 10 (m, 2 H, OCH₂C*H*₂CH₃), 2. 28 (s, 3 H, NCH₃), 2. 51 (br s, 4 H, 2 NCH₂), 2. 72 (t, *J* = 7. 5 Hz, 2 H, C*H*₂CH₂CH₃), 3. 12 (br s, 4 H, 2 SO₂NCH₂), 4. 10 (s, 3 H, NCH₃), 4. 25 (t; *J* = 6. 3 Hz, 2 H, OC*H*₂CH₂CH₃), 7. 14 (d, *J* = 8. 7 Hz, 1 H, H-3'), 7. 82 (dd, *J* = 8. 7 Hz, 2. 4 Hz, 1 H, H-4'). 8. 87 (d, *J* = 2. 4 Hz, 1 H, H-6'), 10. 73 (br s, 1 H, NH); MS (FAB) *m*/*z* 566, 568 (MH⁺).

### Example 93

### Preparation of 6-bromo-2-(2-ethoxy-5-(4-(2-fluoroethyl)piperazin-1-ylsulfonyl)phenyl)-5-methyl-7-n -propyl-3,5-dihydro-4H-pyrrolo[3,2-d]pyrimidin-4-one (a compound of the formula (1) wherein R⁵ = SO₂NR⁶R⁷, R¹ = CH₃, R² = Br, R³ = CH₂CH₂CH₃, R⁴ = CH₂CH₃; NR⁶R⁷ is 4-(2-fluoroethyl)piperazin-1-yl)

The titled compound was prepared as described in Example 1 by using 6-bromo-2-(5-chlorosulfonyl-2-ethoxyphenyl)-5-methyl-7-*n*-propyl-3,5-dihydro-4*H*-p yrrolo[3,2-*d*]pyrimidin-4-one and 1-(2-fluoroethyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 88%
mp 208 °C dec (EtOAc/hexanes);
IR (neat) 3328 (NH), 1678 (C=O), 1168 (SO₂) cm⁻¹;
¹H NMR (CDCl₃/TMS) δ 0. 96 (t, *J* = 7. 5 Hz, 3 H, CH₂CH₂C*H*₃), 1. 64 (t, *J* = 6. 9 Hz, 3 H, OCH₂C*H*₃), 1. 69-1. 79 (m, 2 H, CH₂C*H*₂CH₃), 2. 64 (dd, *J* = 4. 8 Hz, 4. 5 Hz, 4 H, 2 NCH₂), 2. 62-2. 77 (m, 4 H, NC*H*₂CH₂F and C*H*₂CH₂CH₃), 3. 12 (br dd, *J* = 4. 8 Hz, 4. 5 Hz, 4 H, 2 SO₂NCH₂) 4. 09 (s, 3 H, NCH₃), 4. 36 (q, *J* = 6. 9 Hz, 2 H, OC*H*₂CH₃), 4. 49 (dt, *J* = 47. 7 Hz, 4. 8 Hz, 2 H, NCH₂C*H*₂F), 7. 13 (d, *J* = 8. 7 Hz, 1 H, H-3'), 7. 81 (dd, *J* = 8. 7 Hz, 2. 4 Hz, 1 H, H-4'), 8. 85 (d, *J* = 2. 4 Hz, 1 H, H-6'), 10. 72 (br s, 1 H, NH); MS (FAB) *m*/*z* 584, 586 (MH⁺).

### Example 94

### Preparation of 6-bromo-2-(5-(4-(2-fluoroethyl)piperazin-1-ylsulfonyl)-2-n-propoxyphenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Br, R³ = CH₂CH₂CH₃, R⁴ = CH₂CH₂CH₃; NR⁶R⁷ is 4-(2-fluoroethyl)piperazin-1-yl)

The titled compound was prepared as described in Example 1 by using 6-bromo-2-(5-chlorosulfonyl-2-*n*-propoxyphenyl)-5-methyl-7-*n*-propyl-3,5-dihydro-4 *H*-pyrrolo[3,2-*d*]pyrimidin-4-one and 1-(2-fluoroethyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 84%
mp 195. 5 °C dec (CHCl₃/hexanes);
IR (neat) 3310 (NH), 1685 (C=O), 1173 (SO₂) cm⁻¹;
¹H NMR (CDCl₃/TMS) δ 0. 96 (t, *J* = 7. 5 Hz, 3 H, CH₂CH₂C*H*₃), 1. 19 (t, *J* = 7. 5 Hz, 3 H, OCH₂CH₂C*H*₃), 1. 66-1. 77 (m, 2 H, CH₂C*H*₂CH₃), 2. 00-2. 10 (m, 2 H, OCH₂C*H*₂CH₃), 2. 65 (br s, 4 H, 2 NCH₂), 2. 70-2. 76 (m, 2 H, NC*H*₂CH₂F), 2. 72 (t, *J* = 7. 5 Hz, 2 H, C*H*₂CH₂CH₃), 3. 13 (br s, 4 H, 2 SO₂NCH₂), 4. 10 (s, 3 H, NCH₃), 4. 25 (t, *J* = 6. 3 Hz, 2 H, OC*H*₂CH₂CH₃), 4. 50 (dt, *J* = 48. 0 Hz, 6. 0 Hz, 2 H, NCH₂C*H*₂F), 7. 14 (d, *J* = 9. 0 Hz, 1 H, H-3'), 7. 82 (dd, *J* = 9. 0 Hz, 2. 4 Hz, 1 H, H-4'), 8. 87 (d, *J =* 2. 4 Hz, 1 H, H-6'), 10. 73 (br s, 1 H, NH); MS (FAB) *m*/*z* 598, 600 (MH⁺).

### Example 95

### Preparation of 6-bromo-2-(2-ethoxy-5-(4-(3-fluoropropyl)piperazin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Br, R³ = CH₂CH₂CH₃, R⁴ = CH₂CH₃; NR⁶R⁷ is 4-(3-fluoropropyl)piperazin-1-yl)

The titled compound was prepared as described in Example 1 by using 26-bromo-2-(5-chlorosulfonyl-2-ethoxyphenyl)-5-methyl-7-*n*-propyl-3,5-dihydro-4*H*-pyrrolo[3,2-*d*]pyrimidin-4-one and 1-(3-fluoropropyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 85%
mp 198 °C dec (EtOAc/hexanes);
IR (neat) 3324 (NH), 1678 (C=O), 1169 (SO₂) cm⁻¹;
¹H NMR (CDCl₃/TMS) δ 0. 96 (t, *J* = 7. 5 Hz, 3 H, CH₂CH₂C*H*₃), 1. 64 (t, *J* = 6. 9 Hz, 3 H, OCH₂C*H*₃), 1. 66-1. 88 (m, 4 H, CH₂C*H*₂CH₂F and CH₂C*H*₂CH₃), 2. 47 (t, *J* = 7. 2 Hz, 2 H, NC*H*₂CH₂), 2. 54 (dd, *J* = 4. 8 Hz, 4. 5 Hz, 4 H, 2 NCH₂), 2. 72 (t, *J* = 7. 5 Hz, 2 H, C*H*₂CH₂CH₃), 3. 10 (br dd, *J* = 4. 8 Hz, 4. 5 Hz, 4 H, 2 SO₂NCH₂), 4. 10 (s, 3 H, NCH₃), 4. 37 (q, *J* = 6. 9 Hz, 2 H, OC*H*₂CH₃), 4. 43 (dt, *J =* 47. 1 Hz, 6. 0 Hz, 2 H, CH₂C*H*₂F), 7. 13 (d, *J* = 8. 7 Hz, 1 H, H-3'), 7. 82 (dd, *J* = 8. 7 Hz, 2. 4 Hz, 1 H, H-4'), 8. 85 (d, *J* = 2. 4 Hz, 1 H, H-6'), 10. 71 (br s, 1 H, NH); MS (FAB) *m*/*z* 598, 600 (MH⁺).

### Example 96

### Preparation of 6-bromo-2-(5-(4-(3-fluoropropyl)piperazin-1-ylsulfonyl)-2-n-propoxyphenyl)-5-methy 1-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Br, R³ = CH₂CH₂CH₃, R⁴ = CH₂CH₂CH₃; NR⁶R⁷ is 4-(3-fluoropropyl)piperazin-1-yl)

The titled compound was prepared as described in Example 1 by using 6-bromo-2-(5-chlorosulfonyl-2-*n*-propoxyphenyl)-5-methyl-7-*n*-propyl-3,5-dihydro-4 *H*-pyrrolo[3,2-*d*]pyrimidin-4-one and 1-(3-fluoropropyl)piperazine hydrochloride in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 88%
mp 187 °C dec (CHCl₃/hexanes);
IR (neat) 3314 (NH), 1687 (C=O), 1171 (SO₂) cm⁻¹;
¹H NMR (CDCl₃/TMS) δ 0. 96 (t, *J* = 7. 5 Hz, 3 H, CH₂CH₂C*H*₃), 1. 19(t, *J* = 7. 5 Hz, 3 H, OCH₂CH₂C*H*₃), 1. 66-1. 90 (m, 4 H, CH₂C*H*₂CH₂F and CH₂C*H*₂CH₃), 1. 99-2. 11 (m, 2 H, OCH₂C*H*₂CH₃), 2. 45-2. 65 (m, 6 H, NC*H*₂CH₂ and 2 NCH₂), 2. 72(t, *J* = 7. 5 Hz, 2 H, C*H*₂CH₂CH₃), 3. 11 (br s, 4 H, 2 SO₂NCH₂), 4. 10 (s, 3 H, NCH₃), 4. 25 (t, *J* = 6. 3 Hz, 2 H, OC*H*₂CH₂CH₃), 4. 44 (dt, *J* = 47. 1 Hz, 6. 0 Hz, 2 H, CH₂C*H*₂F), 7. 14 (d, *J* = 8. 7 Hz, 1 H, H-3'), 7. 82 (dd, *J* = 8. 7 Hz, 2. 4 Hz, 1 H, H-4'), 8. 87 (d, *J* = 2. 4 Hz, 1 H, H-6'), 10. 73 (br s, 1 H, NH); MS (FAB) *m*/*z* 612, 614 (MH⁺).

### Example 97

### Preparation of 6-bromo-2-(2-ethoxy-5-(4-(2-hydroxyethyl)piperazin-1-ylsulfonyl)phenyl)-5-methyl-7 -n-propyl-3,5-dihydro-4H-pyrrolo[3,2-d]pyrimidin-4-one (a compound of the formula (1) wherein R⁵ = SO₂NR⁶R⁷, R¹ = CH₃, R² = Br, R³ = CH₂CH₂CH₃, R⁴ = CH₂CH₃; NR⁶R⁷ is 4-(2-hydroxyethyl)piperazin-1-yl)

The titled compound was prepared as described in Example 1 by using 6-bromo-2-(5-chlorosulfonyl-2-ethoxyphenyl)-5-methyl-7-*n*-propyl-3,5-dihydro-4*H*-p yrrolo[3,2-*d*]pyrimidin-4-one and 1-(2-hydroxyethyl)piperazine in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 90%
mp 203. 5-205 °C dec (EtOAc/CHCl₃/hexanes);
IR (neat) 3565, 3327 (NH and OH), 1677 (C=O), 1166 (SO₂) cm⁻¹;
¹H NMR (CDCl₃/TMS) δ 0. 96 (t, *J* = 7. 5 Hz, 3 H, CH₂CH₂C*H*₃), 1. 65 (t, *J* = 6. 9 Hz, 3 H, OCH₂C*H*₃), 1. 67-1. 79 (m, 2 H, CH₂C*H*₂CH₃), 2. 55 (t, *J* = 5. 4 Hz, 2 H, NC*H*₂CH₂), 2. 60 (dd, *J* = 4. 8 Hz, 4. 2 Hz, 4 H, 2 NCH₂), 2. 72 (t, *J* = 7. 5 Hz, 2 H, C*H*₂CH₂CH₃), 3. 12 (br dd, *J* = 4. 8 Hz, 4. 2 Hz, 4 H, 2 SO₂NCH₂), 3. 58 (t, *J* = 5. 4 Hz, 2 H, CH₂C*H*₂OH), 4. 10 (s, 3 H, NCH₃), 4. 37 (q, *J* = 6. 9 Hz, 2 H, OC*H*₂CH₃), 7. 15 (d, *J* = 8. 7 Hz, 1 H, H-3'), 7. 82 (dd, *J* = 8. 7 Hz, 2. 4 Hz, 1 H, H-4'), 8. 85 (d, *J* = 2. 4 Hz, 1 H, H-6'), 10. 72 (br s, 1 H, NH); MS (FAB) *m*/*z* 582, 584 (MH⁺).

### Example 98

### Preparation of 6-bromo-2-(5-(4-(2-hydroxyethyl)piperazin-1-ylsulfonyl)-2-n-propoxyphenyl)-5-meth yl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Br, R³ = CH₂CH₂CH₃, R⁴ = CH₂CH₂CH₃; NR⁶R⁷ is 4-(2-hydroxyethyl)piperazin-1-yl)

The titled compound was prepared as described in Example 1 by using 6-bromo-2-(5-chlorosulfonyl-2-*n*-propoxyphenyl)-5-methyl-7-*n*-propyl-3,5-dihydro-4 *H*-pyrrolo[3,2-*d*]pyrimidin-4-one and 1-(2-hydroxyethyl)piperazine in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine. yield: 87%
mp 198. 5 °C dec (CHCl₃/hexanes);
IR (neat) 3453, 3312 (NH and OH), 1687 (C=O), 1171 (SO₂) cm⁻¹;
¹H NMR (CDCl₃/TMS) δ 0. 96 (t, *J* = 7. 5 Hz, 3 H, CH₂CH₂C*H*₃), 1. 19 (t, *J* = 7. 5 Hz, 3 H, OCH₂CH₂C*H*₃), 1. 66-1. 77 (m, 2 H, CH₂C*H*₂CH₃), 2. 01-2. 11 (m, 2 H, OCH₂C*H*₂CH₃), 2. 57 (t, *J* = 5. 4 Hz, 2 H, NC*H*₂CH₂), 2. 63 (dd, *J* = 4. 8 Hz, 4. 5 Hz, 4 H, 2 NCH₂), 2. 72 (t, *J* = 7. 5 Hz, 2 H, C*H*₂CH₂CH₃), 3. 13 (br dd, *J* = 4. 8 Hz, 4. 5 Hz, 4 H, 2 SO₂NCH₂), 3. 58 (t, *J* = 5. 4 Hz, 2 H, CH₂C*H*₂OH), 4. 10 (s, 3 H, NCH₃), 4. 26 (t, *J* = 6. 3 Hz, 2 H, OC*H*₂CH₂CH₃), 7. 15 (d, *J* = 8. 7 Hz, I H, H-3'), 7. 83 (dd, *J* = 8. 7 Hz, 2. 7 Hz, 1 H, H-4'), 8. 87 (d, *J* = 2. 7 Hz, 1 H, H-6'), 10. 74 (br s, 1 H, NH); MS (FAB) *m*/*z* 596, 598 (MH⁺).

### Example 99

### Preparation of 6-bromo-2-(2-ethoxy-5-(4-(3-hydroxypropyl)piperazin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Br, R³ = CH₂CH₂CH₃, R⁴ = CH₂CH₃; NR⁶R⁷ is 4-(3-hydroxypropyl)piperazin-1-yl)

The titled compound was prepared as described in Example 1 by using 6-bromo-2-(5-chlorosulfonyl-2-ethoxyphenyl)-5-methyl-7-*n*-propyl-3,5-dihydro-4*H*-p yrrolo[3,2-*d*]pyrimidin-4-one and 1-(3-hydroxypropyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 90%
mp 197. 5 °C dec (EtOAc/Et₂O/hexanes);
IR (neat) 3325 (NH and OH), 1678 (C=O), 1167 (SO₂) cm⁻¹;
¹H NMR (CDCl₃/TMS) δ 0. 97 (t, *J* = 7. 5 Hz, 3 H, CH₂CH₂C*H*₃), 1. 57-1. 79(m, 4 H, CH₂C*H*₂CH₂OH and CH₂C*H*₂CH₃), 1. 65 (t, *J* = 7. 2 Hz, 3 H, OCH₂C*H*₃), 2. 51-2. 67 (m, 6 H, NC*H*₂CH₂ and 2 NCH₂), 2. 72 (t, *J* = 7. 5 Hz, 2 H, C*H*₂CH₂CH₃), 3. 08 (br s, 4 H, 2 SO₂NCH₂), 3. 71 (t, *J* = 5. 4 Hz, 2 H, CH₂C*H*₂OH), 4. 10 (s, 3 H, NCH₃), 4. 38 (q, *J* = 7. 2 Hz, 2 H, OC*H*₂CH₃), 7. 13 (d, *J* = 9. 0 Hz, 1 H, H-3'), 7. 78(dd, *J* = 9. 0 Hz, 2.4 Hz, 1 H, H-4'), 8. 84 (d, *J* = 2.4 Hz, 1 H, H-6'), 10. 73 (br s, 1 H, NH); MS (FAB) *m*/*z* 596, 598 (MH⁺).

### Example 100

### Preparation of 6-bromo-2-(5-(4-(3-hydmxypropyl)piperazin-1-ylsulfonyl)-2-n-propoxyphenyl)-5-met hyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = Br, R³ = CH₂CH₂CH₃, R⁴ = CH₂CH₂CH₃; NR⁶R⁷ is 4-(3-hydroxypropyl)piperazin-1-yl)

The titled compound was prepared as described in Example 1 by using 6-bromo-2-(5-chlorosulfonyl-2-*n*-propoxyphenyl)-5-methyl-7-*n*-propyl-3,5-dihydro-4 *H*-pyrrolo[3,2-*d*]pyrimidin-4-one and 1-(3-hydroxypropyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 90%
mp 192 °C dec (CHCl₃/hexanes);
IR (neat) 3423, 3313 (NH and OH), 1684 (C=O), 1171 (SO₂) cm⁻¹;
¹H NMR (CDCl₃/TMS) δ 0.97 (t, *J* = 7. 5 Hz, 3 H, CH₂CH₂C*H*₃), 1. 20(t, *J* = 7. 5 Hz, 3 H, OCH₂CH₂C*H*₃), 1. 66-1. 78(m, 4 H, CH₂C*H*₂CH₂OH and CH₂C*H*₂CH₃), 1. 99-2. 09 (m, 2 H, OCH₂C*H*₂CH₃), 2. 55-2. 70 (m, 6 H, NC*H*₂CH₂ and 2 NCH₂), 2. 72(t, *J* = 7. 5 Hz, 2 H, C*H*₂CH₂CH₃), 3. 10 (br s, 4 H, 2 SO₂NCH₂), 3. 71 (t, *J* = 5. 4 Hz, 2 H, CH₂C*H*₂OH), 4. 10 (s, 3 H, NCH₃), 4. 26(t, *J* = 6. 3 Hz, 2 H, OC*H*₂CH₂CH₃), 7. 14 (d, *J* = 9. 0 Hz, 1 H, H-3'), 7. 79 (dd, *J* = 9. 0 Hz, 2. 4 Hz, 1 H, H-4'), 8. 86(d, *J* = 2. 4 Hz, 1 H, H-6'), 10. 76 (br s, 1 H, NH); MS (FAB) *m*/*z* 610, 612 (MH⁺).

### Example 101

### Preparation of 2-(2-ethoxy-5-(4-methylpiperazin-1-ylsulfonyl)phenyl)-6-iodo-5-methyl-7-n-propyl-3, 5-dihydro-4H-pyrrolo[3,2-d]pyrimidin-4-one (a compound of the formula (1) wherein R⁵ = SO₂NR⁶R⁷, R¹ = CH₃, R² = I, R³ = CH₂CH₂CH₃, R⁴ = CH₂CH₃; NR⁶R⁷ is 4-methylpiperazin-1-yl)

The titled compound was prepared as described in Example 1 by using 2-(5-chlorosulfonyl-2-ethoxyphenyl)-6-iodo-5-methyl-7-*n*-propyl-3,5-dihydro-4*H*-pyrr olo[3,2-*d*]pyrimidin-4-one in place of 2-(5-chlorosulfonyl-2-ethoxyphenyl)-5-methyl-7-*n*-propyl-3,5-dihydro-4*H*-pyrrolo[3,2 -*d*]pyrimidin-4-one.
yield: 97%
mp 197. 5-198 °C (EtOAc/Et₂O);
IR (neat) 3325 (NH), 1679 (C=O), 1172 (SO₂) cm⁻¹;
¹H NMR (CDCl₃/TMS) δ 0. 97 (t, *J* = 7. 5 Hz, 3 H, CH₂CH₂C*H*₃), 1. 64 (t, *J* = 6. 9 Hz, 3 H, OCH₂C*H*₃), 1. 65-1. 78 (m, 2 H, CH₂C*H*₂CH₃), 2. 28 (s, 3 H, NCH₃), 2. 48-2. 53 (m, 4 H, 2 NCH₂), 2. 71 (t, *J* = 7. 5 Hz, 2 H, C*H*₂CH₂CH₃), 3. 07-3. 15 (m, 4 H, 2 SO₂NCH₂), 4. 11 (s, 3 H, NCH₃), 4. 36 (q, *J* = 6. 9 Hz, 2 H, OC*H*₂CH₃), 7. 13 (d, *J* = 8. 7 Hz, 1 H, H-3'), 7. 82 (dd, *J =* 8. 7 Hz, 2. 4 Hz, I H, H-4'), 8. 86 (d, *J* = 2. 4 Hz, 1 H, H-6'), 10. 67 (br s, 1 H, NH); MS (FAB) *m*/*z* 600 (MH⁺).

### Example 102

### Preparation of 6-iodo-2-(5-(4-methylpiperazin-1-ylsulfonyl)-2-n-propoxyphenyl)-5-methyl-7-n-propy 1-3,5-dihydro-4H-pyrrolo[3,2-d]pyrimidin-4-one (a compound of the formula (1) wherein R⁵ = SO₂NR⁶R⁷, R' = CH₃, R² = I, R³ = CH₂CH₂CH₃, R⁴ = CH₂CH₂CH₃; NR⁶R⁷ is 4-methylpiperazin-1-yl)

The titled compound was prepared as described in Example 1 by using 2-(5-chlorosulfonyl-2-*n*-propoxyphenyl)-6-iodo-5-methyl-7-*n*-propyl-3,5-dihydro-4*H*-pyrrolo[3,2-*d*]pyrimidin-4-one in place of 2-(5-chlorosulfonyl-2-ethoxyphenyl)-5-methyl-7-*n*-propyl-3,5-dihydro-4*H*-pyrrolo[3,2 -*d*]pyrimidin-4-one.
yield: 99%
mp 188-188. 5 °C (EtOAc/CHCl₃/hexanes);
IR (neat) 3300 (NH), 1680 (C=O), 1173 (SO₂) cm⁻¹;
¹H NMR (CDCl₃/TMS) δ 0. 97 (t, *J* = 7. 5 Hz, 3 H, CH₂CH₂C*H*₃), 1. 19 (t, *J* = 7. 5 Hz, 3 H, OCH₂CH₂C*H*₃), 1. 65-1. 78 (m, 2 H, CH₂C*H*₂CH₃), 1. 98-2. 10 (m, 2 H, OCH₂C*H*₂CH₃), 2. 27 (s, 3 H, NCH₃), 2. 49 (dd, *J* = 5. 1 Hz, 4. 8 Hz, 4 H, 2 NCH₂), 2. 71 (t, *J* = 7. 5 Hz, 2 H, C*H*₂CH₂CH₃), 3. 11 (br dd, *J* = 5. 1 Hz, 4. 8 Hz, 4 H, 2 SO₂NCH₂), 4. 11 (s, 3 H, NCH₃), 4. 25 (t, *J* = 6. 6 Hz, 2 H, OC*H*₂CH₂CH₃), 7. 13 (d, *J* = 8. 7 Hz, 1 H, H-3'), 7. 82 (dd, *J* = 8. 7 Hz, 2. 4 Hz, 1 H, H-4'), 8. 87 (d, *J* = 2. 4 Hz, 1 H, H-6'), 10. 70 (br s, 1 H, NH); MS (FAB) *m*/*z* 614 (MH⁺).

### Example 103

### Preparation of 2-(2-ethoxy-5-(4-(2-fluoroethyl)piperazin-1-ylsulfonyl)phenyl)-6-iodo-5-methyl-7-n-p ropyl-3,5-dihydro-4H-pyrrolo[3,2-d]pyrimidin-4-one (a compound of the formula (1) wherein R⁵ = SO₂NR⁶R⁷, R' = CH₃, R² = I, R³ = CH₂CH₂CH₃, R⁴ = CH₂CH₃; NR⁶R⁷ is 4-(2-fluoroethyl)piperazin-1-yl)

The titled compound was prepared as described in Example 1 by using 2-(5-chlorosulfonyl-2-ethoxyphenyl)-6-iodo-5-methyl-7-*n*-propyl-3,5-dihydro-4*H*-pyrr olo[3,2-*d*]pyrimidin-4-one and 1-(2-fluoroethyl)piperazine hydrochloride in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 96%
mp 182. 5-183 °C (CHCl₃/hexanes);
IR (neat) 3328 (NH), 1676 (C=O), 1166 (SO₂) cm⁻¹;
¹H NMR (CDCl₃/TMS) δ 0. 97 (t, *J* = 7. 5 Hz, 3 H, CH₂CH₂C*H*₃), 1. 65(t, *J* = 6. 9 Hz, 3 H, OCH₂C*H*₃), 1. 65-1. 77 (m, 2 H, CH₂C*H*₂CH₃), 2. 63-2. 76 (m, 8 H, NC*H*₂CH₂F, C*H*₂CH₂CH₃ and 2 NCH₂), 3. 11-3. 16 (m, 4 H, 2 SO₂NCH₂), 4. 11 (s, 3 H, NCH₃), 4. 36 (q, *J* = 6. 9 Hz, 2 H, OC*H*₂CH₃), 4. 50 (dt, *J* = 47. 7 Hz, 4. 5 Hz, 2 H, NCH₂C*H*₂F), 7. 13 (d, *J* = 8. 7 Hz, 1 H, H-3'), 7. 82 (dd, *J* = 8. 7 Hz, 2. 4 Hz, 1 H, H-4'), 8. 86 (d, *J* = 2.4 Hz, 1 H, H-6'), 10. 66 (br s, 1 H, NH); MS (FAB) *m*/*z* 632 (MH⁺).

### Example 104

### Preparation of 2-(5-(4-(2-fluoroethyl)piperazin-1-ylsulfonyl)-2-n-propoxyphenyl)-6-iodo-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = I, R³ = CH₂CH₂CH₃, R⁴ = CH₂CH₂CH₃; NR⁶R⁷ is 4-(2-fluoroethyl)piperazin-1-yl)

The titled compound was prepared as described in Example 1 by using 2-(5-chlorosulfonyl-2-*n*-propoxyphenyl)-6-iodo-5-methyl-7-*n*-propyl-3,5-dihydro-4*H*-pyrrolo[3,2-*d*]pyrimidin-4-one and 1-(2-fluoroethyl)piperazine hydrochloride in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 96%
mp 209. 5-210 °C (EtOAc/CHCl₃/hexanes);
IR (neat) 3304 (NH), 1686 (C=O), 1172 (SO₂) cm⁻¹;
¹H NMR (CDCl₃/TMS) δ 0. 97 (t, *J* = 7. 2 Hz, 3 H, CH₂CH₂C*H*₃), 1.19 (t, *J* = 7. 5 Hz, 3 H, OCH₂CH₂C*H*₃), 1. 65-1. 78 (m, 2 H, CH₂C*H*₂CH₃), 1. 99-2. 10 (m, 2 H, OCH₂C*H*₂CH₃), 2. 62-2. 75 (m, 8 H, NC*H*₂CH₂F, C*H*₂CH₂CH₃ and 2 NCH₂), 3. 10-3. 15 (m, 4 H, 2 SO₂NCH₂), 4. 11 (s, 3 H, NCH₃), 4. 25 (t, *J* = 6. 6 Hz, 2 H, OC*H*₂CH₂CH₃), 4. 49 (ddd, *J* = 47. 7 Hz, 5. 1 Hz, 4. 5 Hz, 2 H, NCH₂C*H*₂F), 7. 14 (d, *J* = 8. 7 Hz, 1 H, H-3'), 7. 82 (dd, *J* = 8. 7 Hz, 2. 4 Hz, 1 H, H-4'), 8. 87 (d, *J* = 2. 4 Hz, 1 H, H-6'), 10. 70 (br s, 1 H, NH); MS (FAB) *m*/*z* 646 (MH⁺).

### Example 105

### Preparation of 2-(2-ethoxy-5-(4-(3-fluoropropyl)piperazin-1-ylsulfonyl)phenyl)-6-iodo-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = I, R³ = CH₂CH₂CH₃, R⁴ =CH₂CH₃; NR⁶R⁷ is 4-(3-fluoropropyl)piperazin-1-yl)

The titled compound was prepared as described in Example 1 by using 2-(5-chlorosulfonyl-2-ethoxyphenyl)-6-iodo-5-methyl-7-*n*-propyl-3,5-dihydro-4*H*-pyrr olo[3,2-*d*]pyrimidin-4-one and 1-(3-fluoropropyl)piperazine hydrochloride in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 86%
mp 202. 5-203 °C (CHCl₃/hexanes);
IR (neat) 3324 (NH), 1679 (C=O), 1167 (SO₂) cm⁻¹;
¹H NMR (CDCl₃/TMS) δ 0. 97 (t, *J* = 7. 5 Hz, 3 H, CH₂CH₂C*H*₃), 1. 64 (t, *J* = 6. 9 Hz, 3 H, OCH₂C*H*₃), 1. 66-1. 88 (m, 4 H, CH₂C*H*₂CH₂F and CH₂C*H*₂CH₃), 2. 50 (t, *J* = 7. 5 Hz, 2 H, NC*H*₂CH₂), 2. 54-2. 58 (m 4 H, 2 NCH₂), 2. 71 (t, *J* = 7. 5 Hz, 2 H, C*H*₂CH₂CH₃), 3. 08-3. 14 (m, 4 H, 2 SO₂NCH₂), 4. 11 (s, 3 H, NCH₃), 4. 36 (q, *J* = 6. 9 Hz, 2 H, OC*H*₂CH₃), 4. 44 (dt, *J* = 47. 4 Hz, 5. 7 Hz, 2 H, CH₂C*H*₂F), 7. 13 (d, *J* = 8. 7 Hz, 1 H, H-3'), 7. 82 (dd, *J =* 8. 7 Hz, 2. 4 Hz, 1 H, H-4'), 8. 84 (d, *J =* 2. 4 Hz, 1 H, H-6'), 10. 68 (br s, I H, NH); MS (FAB) *m*/*z* 646 (MH⁺).

### Example 106

### Preparation of 2-(5-(4-(3-fluoropropyl)piperazin-1-ylsulfonyl)-2-n-propoxyphenyl)-6-iodo-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = I, R³ = CH₂CH₂CH₃, R⁴ = CH₂CH₂CH₃; NR⁶R⁷ is 4-(3-fluoropropyl)piperazin-1-yl)

The titled compound was prepared as described in Example 1 by using 2-(5-chlorosulfonyl-2-*n*-propoxyphenyl)-6-iodo-5-methyl-7-*n*-propyl-3,5-dihydro-4*H*-pyrrolo[3,2-*d*]pyrimidin-4-one and 1-(3-fluoropropyl)piperazine hydrochloride in place of 2-(5-chlorosulfonyl-2-ethoxyphenyl)-5-methyl-7-*n*-propyl-3,5-dihydro-4*H*-pyrrolo[4,3 - *d*]pyrimidin-4-one and 1-methylpiperazine.
yield: 89%
mp 204-204. 5 °C (EtOAc/CHCl₃/hexanes);
IR (neat) 3310 (NH), 1685 (C=O), 1171 (SO₂) cm⁻¹;
¹H NMR (CDCl₃/TMS) δ 0. 97 (t, *J* = 7.5 Hz, 3 H, CH₂CH₂C*H*₃), 1. 19 (t, *J* = 7. 5 Hz, 3 H, OCH₂CH₂C*H*₃), 1. 65-1. 89 (m, 4 H, CH₂C*H*₂CH₂F and CH₂C*H*₂CH₃), 1. 98-2. 10 (m, 2 H, OCH₂C*H*₂CH₃), ), 2. 47 (t, *J* = 7. 2 Hz, 2 H, NC*H*₂CH₂), 2. 54 (dd, *J* = 5. 4 Hz, 4. 5 Hz, 4 H, 2 NCH₂), 2. 71 (t, *J* = 7. 5 Hz, 2 H, C*H*₂CH₂CH₃), 3. 10 (br dd, *J* = 5. 4 Hz, 4. 5 Hz, 4 H, 2 SO₂NCH₂), 4. 11 (s, 3 H, NCH₃), 4. 25 (t, *J* = 6. 3 Hz, 2 H, OC*H*₂CH₂CH₃), 4. 43 (dt, *J=* 47. 1 Hz, 6. 0 Hz, 2 H, CH₂C*H*₂F), 7. 14 (d, *J* = 9. 0 Hz, 1 H, H-3'), 7. 82 (dd, *J* = 9. 0 Hz, 2. 7 Hz, 1 H, H-4'), 8. 87 (d, *J* = 2. 7 Hz, 1 H, H-6'), 10. 70 (br s, 1 H, NH); MS (FAB) *m*/*z* 660 (MH⁺).

### Example 107

### Preparation of 2-(2-ethoxy-5-(4-(2-hydroxyethyl)piperazin-1-ylsulfonyl)phenyl)-6-iodo-5-methyl-7-n -propyl-3,5-dihydro-4H-pyrrolo[3,2-d]pyrimidin-4-one (a compound of the formula (1) wherein R⁵ = SO₂NR⁶R⁷, R¹ = CH₃, R² = I, R³ = CH₂CH₂CH₃, R⁴ = CH₂CH₃; NR⁶R⁷ is 4-(2-hydroxyethyl)piperazin-1-yl)

The titled compound was prepared as described in Example 1 by using 2-(5-chlorosulfonyl-2-ethoxyphenyl)-6-iodo-5-methyl-7-*n*-propyl-3,5-dihydro-4*H*-pyrr olo[3,2-*d*]pyrimidin-4-one and 1-(2-hydroxyethyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 99%
mp 194-195 °C (CHCl₃/hexanes);
IR (neat) 3442, 3323 (NH and OH), 1677 (C=O), 1171 (SO₂) cm⁻¹;
¹H NMR (CDCl₃/TMS) δ 0. 97 (t, *J* = 7. 5 Hz, 3 H, CH₂CH₂C*H*₃), 1. 65 (t, *J* = 6. 9 Hz, 3 H, OCH₂C*H*₃), 1. 66-1. 78 (m, 2 H, CH₂C*H*₂CH₃), 2. 56 (t, *J* = 5. 4 Hz, 2 H, NC*H*₂CH₂), 2. 62 (dd, *J* = 4. 8 Hz, 4. 5 Hz, 4 H, 2 NCH₂), 2. 71 (t, *J* = 7. 5 Hz, 2 H, C*H*₂CH₂CH₃), 3. 12 (br dd, *J* = 4. 8 Hz, 4. 5 Hz, 4 H, 2 SO₂NCH₂), 3. 58 (t, *J* = 5. 4 Hz, 2 H, CH₂C*H*₂OH), 4. 11 (s, 3 H, NCH₃), 4. 37 (q, *J* = 6. 9 Hz, 2 H, OC*H*₂CH₃), 7. 15 (d, *J* = 8. 7 Hz, 1 H, H-3'), 7. 82 (dd, *J* = 8. 7 Hz, 2. 4 Hz, 1 H, H-4'), 8. 86 (d, *J* = 2. 4 Hz, 1 H, H-6'), 10. 68 (br s, 1 H, NH); MS (FAB) *m*/*z* 630 (MH⁺).

### Example 108

### Preparation of 2-(5-(4-(2-hydroxyethyl)piperazin-1-ylsulfonyl)-2-n-propoxyphenyl)-6-iodo-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = I, R³ = CH₂CH₂CH₃, R⁴ = CH₂CH₂CH₃; NR⁶R⁷ is 4-(2-hydroxyethyl)piperazin-1-yl)

The titled compound was prepared as described in Example 1 by using 2-(5-chlorosulfonyl-2-*n*-propoxyphenyl)-6-iodo-5-methyl-7-*n*-propyl-3,5-dihydro-4*H*-pyrrolo[3,2-*d*]pyrimidin-4-one and 1-(2-hydroxyethyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 91%
mp 183-184 °C (EtOAc/CHCl₃/hexanes);
IR (neat) 3305 (NH and OH), 1686 (C=O), 1172 (SO₂) cm⁻¹;
¹H NMR (CDCl₃/TMS) δ 0. 97 (t, *J* = 7. 5 Hz, 3 H, CH₂CH₂C*H*₃), 1. 19 (t, *J* = 7. 5 Hz, 3 H, OCH₂CH₂C*H*₃), 1. 66-1. 78 (m, 2 H, CH₂C*H*₂CH₃), 1. 99-2. 11 (m, 2 H, OCH₂C*H*₂CH₃), 2. 31 (br s, 1 H, OH), 2. 55 (t, *J* = 5.4 Hz, 2 H, NC*H*₂CH₂), 2. 61 (dd, *J* = 5. 4 Hz, 4. 5 Hz, 4 H, 2 NCH₂), 2. 71 (t, *J* = 7. 5 Hz, 2 H, C*H*₂CH₂CH₃), 3. 12 (br dd, *J* = 5. 4 Hz, 4. 5 Hz, 4 H, 2 SO₂NCH₂), 3. 57 (br t, *J* = 5. 4 Hz, 2 H, CH₂C*H*₂OH), 4. 11 (s, 3 H, NCH₃), 4. 26 (t, *J* = 6. 6 Hz, 2 H, OC*H*₂CH₂CH₃), 7. 15 (d, *J* = 8. 7 Hz, 1 H, H-3'), 7. 83 (dd, *J* = 8. 7 Hz, 2. 4 Hz, 1 H, H-4'), 8. 87 (d, *J* = 2. 4 Hz, 1 H, H-6'), 10. 71 (br s, 1 H, NH); MS (FAB) *m*/*z* 644 (MH⁺).

### Example 109

### Preparation of 2-(2-ethoxy-5-(4-(3-hydroxypropyl)piperazin-1-ylsulfonyl)phenyl)-6-iodo-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = I, R³ = CH₂CH₂CH₃, R⁴ = CH₂CH₃; NR⁶R⁷ is 4-(3-hydroxypropyl)piperazin-1-yl)

The titled compound was prepared as described in Example 1 by using 2-(5-chlorosulfonyl-2-ethoxyphenyl)-6-iodo-5-methyl-7-*n*-propyl-3,5-dihydro-4*H*-pyrr olo[3,2-*d*]pyrimidin-4-one and 1-(3-hydroxypropyl)piperazine in place of 2-(5-chlorosulfonyl-2-ethoxyphenyl)-5-methyl-7-*n*-propyl-3,5-dihydro-4*H*-pyrrolo[3,2 - *d*]pyrimidin-4-one and 1-methylpiperazine.
yield: 96%
mp 120-121 °C (CHCl₃/hexanes);
IR (neat) 3341 (NH and OH), 1678 (C=O), 1172 (SO₂) cm⁻¹;
¹H NMR (CDCl₃/TMS) δ 0. 97 (t, *J* = 7. 5 Hz, 3 H, CH₂CH₂C*H*₃), 1. 53-1. 64 (m, 2 H, CH₂C*H*₂CH₂OH), 1. 63-1. 76 (m, 2 H, CH₂C*H*₂CH₃), 1. 65 (t, *J* = 6. 9 Hz, 3 H, OCH₂C*H*₃), 2. 62-2. 74 (m, 6 H, NC*H*₂CH₂ and 2 NCH₂), 2. 71 (t, *J* = 7. 2 Hz, 2 H, C*H*₂CH₂CH₃), 3. 07-3. 15 (m, 4 H, 2 SO₂NCH₂), 3. 71 (t, *J* = 5. 1 Hz, 2 H, CH₂C*H*₂OH), 4. 11 (s, 3 H, NCH₃), 4. 38 (q, *J* = 6. 9 Hz, 2 H, OC*H*₂CH₃), 7. 13 (d, *J* = 8. 7 Hz, 1 H, H-3'), 7. 79 (dd, *J* = 8. 7 Hz, 2. 4 Hz, 1 H, H-4'), 8. 85 (d, *J* = 2. 4 Hz, 1 H, H-6'), 10. 68 (br s, 1 H, NH); MS (FAB) *m*/*z* 644 (MH⁺).

### Example 110

### Preparation of 2-(5-(4-(3-hydroxypropyl)piperazin-1-ylsulfonyl)-2-n-propoxyphenyl)-6-iodo-5-methy 1-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = I, R³ = CH₂CH₂CH₃, R⁴ = CH₂CH₂CH₃; NR⁶R⁷ is 4-(3-hydroxypropyl)piperazin-1-yl)

The titled compound was prepared as described in Example 1 by using 2-(5-chlorosulfonyl-2-*n*-propoxyphenyl)-6-iodo-5-methyl-7-*n*-propyl-3,5-dihydro-4*H*-pyrrolo[3,2-*d*]pyrimidin-4-one and 1-(3-hydroxypropyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 89%
mp 206-206. 5 °C (EtOAc/CHCl₃/hexanes);
IR (neat) 3423, 3310 (NH and OH), 1683 (C=O), 1171 (SO₂) cm⁻¹;
¹H NMR (CDCl₃/TMS) δ 0. 97 (t, *J* = 7. 5 Hz, 3 H, CH₂CH₂C*H*₃), 1. 20(t, *J* = 7. 5 Hz, 3 H, OCH₂CH₂C*H*₃), 1. 62-1. 78(m, 4 H, CH₂C*H*₂CH₂OH and CH₂C*H*₂CH₃), 1. 99-2. 11 (m, 2 H, OCH₂C*H*₂CH₃), 2. 58-2. 63 (m, 6 H, NC*H*₂CH₂ and 2 NCH₂), 2. 71 (t, *J* = 7. 5 Hz, 2 H, C*H*₂CH₂CH₃), 3. 03-3. 12 (m, 4 H, 2 SO₂NCH₂), 3. 71 (t, *J* = 5. 4 Hz, 2 H, CH₂C*H*₂OH), 4. 11 (s, 3 H, NCH₃), 4. 26 (t, *J* = 6. 6 Hz, 2 H, OC*H*₂CH₂CH₃), 7. 14 *(d, J* = 9. 0 Hz, 1 H, H-3'), 7. 79 *(dd, J* = 9. 0 Hz, 2. 4 Hz, 1 H, H-4'), 8. 86 (d, *J* = 2. 4 Hz, 1 H, H-6'), 10. 72 (br s, 1 H, NH); MS (FAB) *m*/*z* 658 (MH⁺).

### Example 111

### Preparation of 2-(5-(4-(2-aminoethyl)piperidin-1-ylsulfonyl)-2-ethoxyphenyl)-5-methyl-7-n-propyl-3 ,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-aminoethyl)piperidin-1-yl)

A mixture of 2-(5-(4-(cyanomethyl)piperidin-1-ylsulfonyl)-2-ethoxyphenyl)-5-methyl-7-*n*-propyl-3, 5-dihydro-4*H*-pyrrolo[3,2-*d*]pyrimidin-4-one (217 mg, 0. 44 mmol) and Raney Ni (1. 5 g wet; washed with H₂O and EtOH) in glacial acetic acid (20 mL) was purged with H₂ three times, and was vigorously stirred under hydrogen atmosphere (1 atm; a balloon) at room temperature for 22 h. The reaction mixture was filtered through a Celite pad, washed well with 10% MeOH in CHCl₃ (20 mL), and the filtrate was evaporated to dryness under vacuum to give a green oil. The resulting residue was purified by MPLC on silica gel (gradient elution: 10% MeOH in CHCl₃ followed by 10% 2 *N* ammonia methanolic solution in CHCl₃) to afford the titled compound (185 mg, 84%) as a yellow oil. Analytically pure compound was obtained by crystallization from EtOH/Et₂O.
mp 60 °C dec;
IR (neat) 3335 (NH), 1685 (C=O), 1165 (SO₂) cm⁻¹;
¹H NMR (DMSO-*d*₆) δ 0. 92 (t, *J* = 7. 2 Hz, 3 H, CH₂CH₂C*H*₃), 1. 07-1. 35 (m, 5 H, C*H*C*H*₂ and 2 CHₐₓ), 1. 35 (t, *J* = 6. 9 Hz, 3 H, OCH₂C*H*₃), 1. 57-1. 70 (m, 4 H, CH₂C*H*₂CH₃ and 2 CH_{eq}), 2. 23 (br t, *J* = 11. 4 Hz, 2 H, 2 NCHₐₓ), 2. 48-2. 59 (m, 4 H, CH₂C*H*₂NH₂ and C*H*₂CH₂CH₃), 3. 61 (br d, *J* = 11. 4 Hz, 2 H, 2 NCH_{eq}), 3. 98 (s, 3 H, NCH₃), 4. 22 (q, *J* = 6. 9 Hz, 2 H, OC*H*₂CH₃), 7. 22 (s, 1 H, H-2), 7. 35 (d, *J* = 8. 7 Hz, 1 H, H-3'), 7. 80 (dd, *J* = 8. 7 Hz, 2. 7 Hz, 1 H, H-4'), 7. 91 (d, *J* = 2. 7 Hz, 1 H, H-6'); MS (FAB) *m*/*z* 502 (MH⁺).

### Example 112

### Preparation of 2-(5-(4-(2-aminoethyl)piperidin-1-ylsulfonyl)-2-n-propoxyphenyl)-5-methyl-7-n-prop yl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-aminoethyl)piperidin-1-yl)

The titled compound was prepared as described in Example 113 by using 2-(5-(4-(cyanomethyl)piperidin-1-ylsulfonyl)-2-*n*-propoxyphenyl)-5-methyl-7-*n*-propy 1-3,5-dihydro-4*H*-pyrrolo[3,2-*d*]pyrimidin-4-one in place of 2-(5-(4-(cyanomethyl)piperidin-1-ylsulfonyl)-2-ethoxyphenyl)-5-methyl-7-*n*-propyl-3, 5-dihydro-4*H*-pyrrolo[3,2-*d*]pyrimidin-4-one.
yield: 90%
mp 145 °C dec (EtOH/Et₂O);
IR (neat) 3304 (NH), 1673 (C=O), 1152 (SO₂) cm⁻¹;
¹H NMR (DMSO-*d*₆) δ 0. 92 (t, *J* = 7. 5 Hz, 3 H, CH₂CH₂C*H*₃), 0. 97(t, *J* = 7. 2 Hz, 3 H, OCH₂CH₂C*H*₃), 1. 12-1. 28 (m, 3 H, C*H*C*H*₂), 1. 60-1. 79 (m, 8 H, 2 CH₂C*H*₂CH₃ and 2 CH₂), 2. 17-2. 28 (m, 2 H, 2 NCHₐₓ), 2. 44-2. 53 (m, 2 H, CH₂C*H*₂NH₂), 2. 57 *(t, J* = 7. 5 Hz, 2 H, C*H*₂CH₂CH₃), 3. 63 (br d, *J* = 10. 5 Hz, 2 H, 2 NCH_{eq}), 3. 99 (s, 3 H, NCH₃), 4. 13 (t, *J* = 6. 0 Hz, 2 H, OC*H*₂CH₂CH₃), 7. 22 (s, 1 H, H-2), 7. 37 (d, *J* = 8. 7 Hz, 1 H, H-3'), 7. 80 (dd, *J* = 8. 7 Hz, 2. 4 Hz, 1 H, H-4'), 7. 92 (d, *J* = 2. 4 Hz, 1 H, H-6'); MS (FAB) *m*/*z* 516 (MH⁺).

### Example 113

### Preparation of 2-(5-(4-(3-aminopropyl)piperidin-1-ylsulfonyl)-2-ethoxyphenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(3-aminopropyl)piperidin-1-yl)

The titled compound was prepared as described in Example 113 by using 2-(5-(4-(2-cyanoethyl)piperidin-1-ylsulfonyl)-2-ethoxyphenyl)-5-methyl-7-*n*-propyl-3, 5-dihydro-4*H*-pyrrolo[3,2-*d*]pyrimidin-4-one in place of 2-(5-(4-(cyanomethyl)piperidin-1-ylsulfonyl)-2-ethoxyphenyl)-5-methyl-7-*n*-propyl-3, 5-dihydro-4*H*-pyrrolo[3,2-*d*]pyrimidin-4-one.
yield: 87%
mp 55 °C dec (THF/Et₂O);
IR (neat) 3338 (NH), 1683 (C=O), 1162 (SO₂) cm⁻¹;
¹H NMR (DMSO-*d*₆) δ 0. 92 (t, *J* = 7. 5 Hz, 3 H, CH₂CH₂C*H*₃), 1. 08-1. 20 (m, 5 H, C*H*C*H*₂ and 2 CHₐₓ), 1. 27-1. 41 (m, 2 H, CH₂C*H*₂CH₂NH₂), 1. 35 (t, *J* = 6. 9 Hz, 3 H, OCH₂C*H*₃), 1. 57-1. 70 (m, 4 H, CH₂C*H*₂CH₃ and 2 CH_{eq}), 2. 16-2. 29 (m, 2 H, 2 NCHₐₓ), 2. 47-2. 59 (m, 4 H, CH₂C*H*₂NH₂ and C*H*₂CH₂CH₃), 3. 62 (br d, *J* = 11. 2 Hz, 2 H, 2 NCH_{eq}), 3. 99 (s, 3 H, NCH₃), 4. 22 (q, *J* = 6. 9 Hz, 2 H, OC*H*₂CH₃), 7. 22 (s, 1 H, H-2), 7. 35 (d, *J* = 8. 7 Hz, 1 H, H-3'), 7. 79 (dd, *J* = 8. 7 Hz, 2. 4 Hz, 1 H, H-4'), 7. 90 (d, *J* = 2. 4 Hz, 1 H, H-6'); MS (FAB) *m*/*z* 516 (MH⁺).

### Example 114

### Preparation of 2-(5-(4-(3-aminopropyl)piperidin-1-ylsulfonyl)-2-n-propoxyphenyl)-5-methyl-7-n-pro pyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(3-aminopropyl)piperidin-1-yl)

The titled compound was prepared as described in Example 113 by using 2-(5-(4-(2-cyanoethyl)piperidin-1-ylsulfonyl)-2-*n*-propoxyphenyl)-5-methyl-7-*n*-propy 1-3,5-dihydro-4*H*-pyrrolo[3,2-*d*]pyrimidin-4-one in place of 2-(5-(4-(cyanomethyl)piperidin-1-ylsulfonyl)-2-ethoxyphenyl)-5-methyl-7-*n*-propyl-3, 5-dihydro-4*H*-pyrrolo[3,2-*d*]pyrimidin-4-one.
yield: 79%
mp 119-120 °C (EtOH/Et₂O);
IR (neat) 3308 (NH), 1690 (C=O), 1165 (SO₂) cm⁻¹;
¹H NMR (DMSO-*d*₆) δ 0.92 (t, *J* = 7. 5 Hz, 3 H, CH₂CH₂C*H*₃), 0. 97 (t, *J* = 7. 5 Hz, 3 H, OCH₂CH₂C*H*₃), 1. 10-1. 34 (m, 7 H, C*H*C*H*₂C*H*₂ and 2 CHₐₓ), 1. 58-1. 80 (m, 6 H, 2 CH₂C*H*₂CH₃ and 2 CH_{eq}), 2. 21-2. 28 (m, 2 H, 2 NCHₐₓ), 2. 43-2. 52 (m, 2 H, CH₂C*H*₂NH₂), 2. 57 (t, *J* = 7. 5 Hz, 2 H, C*H*₂CH₂CH₃), 3. 63 (br d, *J =* 10. 8 Hz, 2 H, 2 NCH_{eq}), 3. 99 (s, 3 H, NCH₃), 4. 12 (t, *J* = 6. 3 Hz, 2 H, OC*H*₂CH₂CH₃), 7. 22 (s, 1 H, H-2), 7. 37 (d, *J* = 8. 7 Hz, 1 H, H-3'), 7. 79 (dd, *J* = 8. 7 Hz, 2. 4 Hz, 1 H, H-4'), 7. 92 (d, *J* = 2. 4 Hz, 1 H, H-6'); MS (FAB) *m*/*z* 530 (MH⁺).

### Example 115

### Preparation of 2-(5-(4-(dimethylaminomethyl)piperidin-1-ylsulfonyl)-2-n-propoxyphenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(dimethylaminomethyl)piperidin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 4-(dimethylaminomethyl)piperidine trifluoroacetic acid in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 92%
mp 123. 5 °C dec (MeOH/EtOAc/hexanes);
IR (neat) 3342 (NH), 1686 (C=O), 1167 (SO₂) cm⁻¹;
¹H NMR (CDCl₃/TMS) δ 1. 00 (t, *J* = 7. 5 Hz, 3 H, CH₂CH₂C*H*₃), 1. 18 (t, *J* = 7. 5 Hz, 3 H, OCH₂CH₂C*H*₃), 1. 23-1. 46 (m, 3 H, CH and 2 CHₐₓ), 1. 67-1. 77 (m, 2 H, CH₂C*H*₂CH₃), 1. 82 (br d, *J* = 12. 3 Hz, 2 H, 2 CH_{eq}), 1. 97-2. 13 (m, 4 H, OCH₂C*H*₂CH₃ and C*H*₂N(CH₃)₂), 2. 15 (s, 6 H, N(CH₃)₂), 2. 35 (br t, *J* = 12. 3 Hz, 2 H, 2 NCHₐₓ), 2. 71 (t, *J* = 7. 5 Hz, C*H*₂CH₂CH₃), 3. 85 (br d, *J* = 12. 3 Hz, 2 H, 2 NCH_{eq}), 4. 08 (s, 3 H, NCH₃), 4. 24 (t, *J* = 6. 6 Hz, 2 H, OC*H*₂CH₂CH₃), 6. 89 (s, 1 H, H-2), 7. 13 (d, *J* = 8. 7 Hz, 1 H, H-3'), 7. 82 (dd, *J* = 8. 7 Hz, 2. 4 Hz, 1 H, H-4'), 8. 86 (d, *J* = 2. 4 Hz, 1 H, H-6'), 10. 67 (br s, 1 H, NH); MS (FAB) *m*/*z* 530 (MH⁺).

### Example 116

### Preparation of 2-(5-(4-(2-(dimethylamino)ethyl)piperidin-1-ylsulfonyl)-2-n-propoxyphenyl)-5-methy 1-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-(dimethylamino)ethyl)piperidin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 4-(2-(dimethylamino)ethyl)piperidine trifluoroacetic acid in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 71%
mp 160. 5 °C dec (MeOH/EtOAc/hexanes);
IR (neat) 3294 (NH), 1693 (C=O), 1163 (SO₂) cm⁻¹;
¹H NMR (CDCl₃/TMS) δ 1. 00 (t, *J* = 7.2 Hz, 3 H, CH₂CH₂C*H*₃), 1. 19 (t, *J* = 7. 2 Hz, 3 H, OCH₂CH₂C*H*₃), 1. 26-1. 41 (m, 5 H, C*H*C*H*₂ and 2 CHₐₓ), 1. 60-1. 78 (m, 4 H, CH₂C*H*₂CH₃ and 2 CH_{eq}), 1. 98-2. 10 (m, 2 H, OCH₂C*H*₂CH₃), 2. 18 (s, 6 H, N(CH₃)₂), 2. 25 (t, *J* = 7. 5 Hz, 2 H, CH₂C*H*₂N), 2. 35 (br t, *J* = 11. 4 Hz, 2 H, 2 NCHₐₓ), 2. 71 (t, *J* = 7. 5 Hz, 2 H, C*H*₂CH₂CH₃), 3. 81 (br d, *J* = 12. 0 Hz, 2 H, 2 NCH_{eq}), 4. 08 (s, 3 H, NCH₃), 4. 24 (t, *J* = 6. 6 Hz, 2 H, OC*H*₂CH₂CH₃), 6. 89 (s, 1 H, H-2), 7. 12 (d, *J* = 8. 7 Hz, 1 H, H-3'), 7. 81 (dd, *J* = 8. 7 Hz, 2. 4 Hz, 1 H, H-4'), 8. 87 (d, *J =* 2. 4 Hz, 1 H, H-6'), 10. 67 (br s, 1 H, NH); MS (FAB) *m*/*z* 544 (MH⁺).

### Example 117

### Preparation of 2-(2-ethoxy-5-(4-(hydroxycarbonyl)piperidin-1-ylsulfonyl)phenyl)-5-methyl-7-n-prop yl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(hydroxycarbonyl)piperidin-1-yl)

The titled compound was prepared as described in Example 1 by using isonicopetic acid in place of 1-methylpiperazine.
yield: 96%
mp 193-193. 5 °C (EtOAc/hexanes);
IR (neat) 3334, 3104 (NH and CO₂H), 1669 (C=O), 1164 (SO₂) cm⁻¹;
¹H NMR (CDCl₃/TMS) δ 0. 98 (t, *J* = 7.2 Hz, 3 H, CH₂CH₂C*H*₃), 1. 56(t, *J* = 6. 9 Hz, 3 H, OCH₂C*H*₃), 1. 65-1. 77 (m, 2 H, CH₂C*H*₂CH₃), 1. 78-1. 86 (m, 2 H, 2 CHₐₓ), 1. 90-2. 05 (m, 2 H, 2 CH_{eq}), 2. 23-2. 32 (m, 1 H, CHCO₂), 2. 50-2. 59 (m, 2 H, 2 NCHₐₓ), 2. 70 (t, *J* = 7. 5 Hz, 2 H, C*H*₂CH₂CH₃), 3. 67-3. 73(m, 2 H, 2 NCH_{eq}), 4. 07 (s, 3 H, NCH₃), 4. 30(q, *J* = 6. 9 Hz, 2 H, OC*H*₂CH₃), 6. 92 (s, 1 H, H-2), 7. 11(d, *J* = 8.7 Hz, 1 H, H-3'), 7. 81 (dd, *J* = 8. 7 Hz, 2. 1 Hz, 1 H, H-4'), 8. 61(d, *J* = 2. 1 Hz, 1 H, H-6'), 11. 08 (br s, 1 H, NH); MS (FAB) *m*/*z* 503 (MH⁺).

### Example 118

### Preparation of 2-(5-(4-(hydroxycarbonyl)piperidin-1-ylsulfonyl)-2-n-propoxyphenyl)-5-methyl-7-n-p ropyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(hydroxycarbonyl)piperidin-1-yl)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and isonicopetic acid in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 92%
mp 155-155. 5 °C (EtOAc/Et₂O/hexanes);
IR (neat) 3349, 3101 (NH and CO₂H), 1691, 1654 (C=O), 1164 (SO₂) cm⁻¹;
¹H NMR (CDCl₃/TMS) δ 0. 99 (t, *J* = 7. 5 Hz, 3 H, CH₂CH₂C*H*₃), 1. 11 (t, *J* = 7. 5 Hz, 3 H, OCH₂CH₂C*H*₃), 1. 65-1. 77 (m, 2 H, CH₂C*H*₂CH₃), 1. 78-2. 04 (m, 6 H, OCH₂C*H*₂CH₃ and 2 CH₂), 2. 23-2. 35 (m, 1 H, CHCO₂), 2. 53-2. 60 (m, 2 H, 2 NCHₐₓ), 2. 70 (t, *J* = 7. 5 Hz, 2 H, C*H*₂CH₂CH₃), 3. 63-3. 69 (m, 2 H, 2 NCH_{eq}), 4. 07 (s, 3 H, NCH₃), 4.19 (t, *J* = 6. 6 Hz, 2 H, OC*H*₂CH₂CH₃), 6. 93 (s, 1 H, H-2), 7. 13 (d, *J* = 9. 0 Hz, 1 H, H-3'), 7. 81 (dd, *J* = 9. 0 Hz, 2. 7 Hz, 1 H, H-4'), 8. 56 (br s, 1 H, H-6'), 11. 08 (br s, 1 H, NH); MS (FAB) *m*/*z* 517 (MH⁺).

### Example 119

### Preparation of 2-(2-ethoxy-5-(4-(hydroxycarbonylmethyl)piperidin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(hydroxycarbonylmethyl)piperidin-1-yl)

A suspension of 2-(2-ethoxy-5-(4-(ethoxycarbonylmethyl)piperidin-1-ylsulfonyl)phenyl)-5-methyl-7-*n*-propyl-3,5-dihydro-4*H*-pyrrolo[3,2-*d*]pyrimidin-4-one (146 mg, 0. 28 mmol) and 1. 0 N KOH methanolic solution (0. 70 mL, 0. 70 mmol) in a 1:4 mixture of H₂O and EtOH (7 mL) was heated at 50 °C for 2-5 h, and was cooled to room temperature. Ethanol was removed under vacuum, and the mixture was diluted with H₂O (50 mL). The reaction mixture was acidified to pH 4 using 1 N HCl aqueous solution, and was extracted with 5% MeOH in CHCl₃ (70 mL × 2). The combined organic layer was dried (MgSO₄), filtered, and the filtrate was evaporated to dryness under reduced pressure to give a white solid. The resulting residue was purified by MPLC on silica gel (5% MeOH in CHCl₃) to afford the titled compound (136 mg, 99%) as a white solid. Analytically pure compound was obtained by crystallization from CHCl₃/Et₂O.
mp 138. 5-139. 5 °C;
IR (neat) 3328, 3098 (NH and CO₂H), 1685, 1661 (C=O), 1163 (SO₂) cm⁻¹;
¹H NMR (CDCl₃/TMS) δ 0. 98 (t, *J* = 7. 5 Hz, 3 H, CH₂CH₂C*H*₃), 1. 32-1. 46 (m, 2 H, 2 CHₐₓ), 1. 56 (t, *J* = 6. 9 Hz, 3 H, OCH₂C*H*₃), 1. 65-1. 80 (m, 5 H, CH, 2 CH_{eq} and CH₂C*H*₂CH₃), 2. 25 (d, *J* = 6. 6 Hz, 2 H, CH₂CO₂), 2. 37 (td, *J* = 12. 0 Hz, 2. 4 Hz, 2 H, 2 NCHₐₓ), 2. 70 (t, *J* = 7. 5 Hz, 2 H, C*H*₂CH₂CH₃), 3. 82 (br d, *J* = 11. 4 Hz, 2 H, 2 NCH_{eq}), 4. 07 (s, 3 H, NCH₃), 4. 29 (q, *J* = 6. 9 Hz, 2 H, OC*H*₂CH₃), 6. 91 (s, 1 H, H-2), 7. 09 (d, *J* = 9. 0 Hz, 1 H, H-3'), 7. 80 (dd, *J* = 9. 0 Hz, 2. 4 Hz, 1 H, H-4'), 8. 65 (d, *J* = 2. 4 Hz, 1 H, H-6'), 11. 02 (br s, 1 H, NH); MS (FAB) *m*/*z* 517 (MH⁺).

### Example 120

### Preparation of 2-(5-(4-(hydroxycarbonylmethyl)piperidin-1-ylsulfonyl)-2-n-propoxyphenyl)-5-methy 1-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(hydroxycarbonylmethyl)piperidin-1-yl)

The titled compound was prepared as described in Example 121 by using 2-(5-(4-(ethoxycarbonylmethyl)piperidin-1-ylsulfonyl)-2-*n*-propoxyphenyl)-5-methyl-7-*n*-propyl-3,5-dihydro-4*H*-pyrrolo[3,2-*d*]pyrimidin-4-one in place of 2-(2-ethoxy-5-(4-(ethoxycarbonylmethyl)piperidin-1-ylsulfonyl)phenyl)-5-methyl-7-*n*-propyl-3,5-dihydro-4*H*-pyrrolo[3,2-*d*]pyrimidin-4-one.
yield: 89%
mp 181. 5-182 °C (EtOAc/Et₂O/hexanes);
IR (neat) 3350 (NH and CO₂H), 1720, 1658 (C=O), 1157 (SO₂) cm⁻¹;
¹H NMR (CDCl₃/TMS) δ 0. 98 (t, *J* = 7. 5 Hz, 3 H, CH₂CH₂C*H*₃), 1. 13 (t, *J* = 7. 5 Hz, 3 H, OCH₂CH₂C*H*₃), 1. 33-1. 46 (m, 2 H, 2 CHₐₓ), 1. 65-1. 83 (m, 5 H, CH, 2 CH_{eq} and CH₂C*H*₂CH₃), 1. 91-2. 03 (m, 2 H, OCH₂C*H*₂CH₃), 2. 25 (d, *J* = 6. 6 Hz, 2 H, CH₂CO₂), 2. 32-2. 41 (m, 2 H, 2 NCHₐₓ), 2. 70 (t, *J* = 7. 5 Hz, 2 H, C*H*₂CH₂CH₃), 3. 82 (br d, *J* = 11. 1 Hz, 2 H, 2 NCH_{eq}), 4. 07(s, 3 H, NCH₃), 4. 19 (t, *J* = 6. 6 Hz, 2 H, OC*H*₂CH₂CH₃), 6. 91 (s, 1 H, H-2), 7. 11 (d, *J* = 8. 7 Hz, 1 H, H-3'), 7. 80 (dd, *J* = 8. 7 Hz, 2. 4 Hz, 1 H, H-4'), 8. 68 (d, *J=* 2. 4 Hz, 1 H, H-6'), 10. 98 (br s, 1 H, NH); MS (FAB) *m*/*z* 531 (MH⁺).

### Example 121

### Preparation of 2-(2-ethoxy-5-(4-(2-hydroxycarbonylethyl)piperidin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-hydroxycarbonylethyl)piperidin-1-yl)

The titled compound was prepared as described in Example 121 by using 2-(2-ethoxy-5-(4-(2-ethoxycarbonylethyl)piperidin-1-ylsulfonyl)phenyl)-5-methyl-7-*n* - propyl-3,5-dihydro-4*H*-pyrrolo[3,2-*d*]pyrimidin-4-one in place of 2-(2-ethoxy-5-(4-(ethoxycarbonylmethyl)piperidin-1-ylsulfonyl)phenyl)-5-methyl-7-*n*-propyl-3,5-dihydro-4*H*-pyrrolo[3,2-*d*]pyrimidin-4-one.
yield: 92%
mp 129-130. 5 °C (CHCl₃/Et₂O);
IR (neat) 3114, 3044 (NH and CO₂H), 1708, 1671 (C=O), 1164 (SO₂) cm⁻¹;
¹H NMR (CDCl₃/TMS) δ 0. 98 (t, *J* = 7. 5 Hz, 3 H, CH₂CH₂C*H*₃), 1. 17-1. 40 (m, 3 H, CH and 2 CHₐₓ), 1. 56 (t, *J* = 6. 9 Hz, 3 H, OCH₂C*H*₃), 1. 48-1. 64 (m, 2 H, CHC*H*₂CH₂), 1. 65-1. 79 (m, 4 H, 2 CH_{eq} and CH₂C*H*₂CH₃), 2. 30 (t, *J* = 7. 5 Hz, 2 H, CH₂CO₂), 2. 32 (br t, *J* = 12. 0 Hz, 2 H, 2 NCHₐₓ), 2. 70 (t, *J* = 7. 5 Hz, 2 H, C*H*₂CH₂CH₃), 3. 81 (br d, *J* = 12. 0 Hz, 2 H, 2 NCH_{eq}), 4. 07 (s, 3 H, NCH₃), 4. 31 (q, *J* = 6. 9 Hz, 2 H, OC*H*₂CH₃), 6. 90 (s, 1 H, H-2), 7. 10 (d, *J* = 9. 0 Hz, 1 H, H-3'), 7. 78 (dd, *J* = 9. 0 Hz, 2. 1 Hz, 1 H, H-4'), 8. 69 (d, *J* = 2. 1 Hz, 1 H, H-6'), 10. 95 (br s, 1 H, NH); MS (FAB) *m*/*z* 531 (MH⁺).

### Example 122

### Preparation of 2-(5-(4-(2-hydroxycarbonylethyl)piperidin-1-ylsulfonyl)-2-n-propoxyphenyl)-5-methy 1-7-n-propyl-3,5-dibydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-hydroxycarbonylethyl)piperidin-1-yl)

The titled compound was prepared as described in Example 121 by using 2-(5-(4-(2-ethoxycarbonylethyl)piperidin-1-ylsulfonyl)-2-*n*-propoxyphenyl)-5-methyl-7-*n*-propyl-3,5-dihydro-4*H*-pyrrolo[3,2-*d*]pyrimidin-4-one in place of 2-(2-ethoxy-5-(4-(ethoxycarbonylmethyl)piperidin-1-ylsulfonyl)phenyl)-5-methyl-7-*n*-propyl-3,5-dihydro-4*H*-pyrrolo[3,2-*d*]pyrimidin-4-one.
yield: 95%
mp 182-183 °C (EtOAc/Et₂O/hexanes);
IR (neat) 3112, 3039 (NH and CO₂H), 1741, 1706, 1672 (C=O), 1163 (SO₂) cm⁻¹;
¹H NMR (CDCl₃/TMS) δ 0. 99 (t, *J* = 7. 5 Hz, 3 H, CH₂CH₂C*H*₃), 1. 16 (t, *J* = 7. 5 Hz, 3 H, OCH₂CH₂C*H*₃), 1. 23-1. 30 (m, 3 H, CH and 2 CHₐₓ), 1. 48-1. 63 (m, 2 H, CHC*H*₂CH₂), 1. 69-1. 78 (m, 4 H, 2 CH_{eq} and CH₂C*H*₂CH₃), 1. 97-2. 04 (m, 2 H, OCH₂C*H*₂CH₃), 2. 32 (t, *J* = 7. 5 Hz, 2 H, CH₂CO₂), 2. 33-2. 41 (m, 2 H, 2 NCHₐₓ), 2. 71 (t, *J* = 7. 5 Hz, 2 H, C*H*₂CH₂CH₃), 3. 78-3. 84 (m, 2 H, 2 NCH_{eq}), 4. 08 (s, 3 H, NCH₃), 4. 22 (t, *J* = 6. 6 Hz, 2 H, OC*H*₂CH₂CH₃), 6. 90 (s, 1 H, H-2), 7. 12 (d, *J* = 8. 7 Hz, 1 H, H-3'), 7. 81 (dd, *J* = 8. 7 Hz, 2. 4 Hz, 1 H, H-4'), 8. 76 (d, *J* = 2. 4 Hz, I H, H-6'), 10. 80 (br s, 1 H, NH); MS (FAB) *m*/*z* 545 (MH⁺).

### Example 123

### Preparation of 2-(5-(4-(amidinomethyl)piperidin-1-ylsulfonyl)-2-ethoxyphenyl)-5-methyl-7-n-propyl -3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(amidinomethyl)piperidin-1-yl)

A suspension of 2-(5-(4-(cyanomethyl)piperidin-1-ylsulfonyl)-2-ethoxyphenyl)-5-methyl-7-*n*-propyl-3, 5-dihydro-4*H*-pyrrolo[3,2-*d*]pyrimidin-4-one (327 mg, 0. 44 mmol) in anhydrous EtOH (30 mL) was saturated with anhydrous HCl gas at -10 °C for 30 min, and was tightly stoppered. The resulting clear mixture was warmed to room temperature, and was stirred at room temperature for 15-24 h, after which all the volatile materials were completely removed under vacuum. The crude yellow solid was dissolved in anhydrous MeOH (20 mL), saturated with anhydrous ammonia at 0 °C for 30 min, and was tightly stoppered. The reaction mixture was warmed to room temperature, and was stirred at room temperature for 15-24 h. The reaction mixture was evaporated to dryness under reduced pressure and the resulting residue was purified by MPLC on silica gel (gradient elution: 10% MeOH in CHCl₃ followed by 20% 2 *N* ammonia methanolic solution in CHCl₃) to afford the titled compound (197 mg, 58%) as a yellowish solid. Analytically pure compound was obtained by crystallization from EtOH/Et₂O.
mp 168 °C dec;
IR (neat) 3323, 3140 (NH), 1683 (C=O), 1163 (SO₂) cm⁻¹;
¹H NMR (DMSO-*d*₆) δ 0. 92 (t, *J* = 7. 2 Hz, 3 H, CH₂CH₂C*H*₃), 1. 23-1. 32 (m, 2 H, 2 CHₐₓ), 1. 36 (t, *J* = 6. 9 Hz, 3 H, OCH₂C*H*₃), 1. 57-1. 74 (m, 5 H, CH, CH₂C*H*₂CH₃ and 2 CH_{eq}), 2. 25 (br t, *J* = 11. 7 Hz, 2 H, 2 NCHₐₓ), 2. 30 (d, *J* = 7. 2 Hz, 2 H, CHC*H*₂), 2. 57 (t, *J* = 7. 5 Hz, 2 H, C*H*₂CH₂CH₃), 3. 66 (br d, *J* = 11. 7 Hz, 2 H, 2 NCH_{eq}), 3. 99 (s, 3 H, NCH₃), 4. 22 (q, *J* = 6. 9 Hz, 2 H, OC*H*₂CH₃), 7. 23 (s, 1 H, H-2), 7. 36 (d, *J* = 9. 0 Hz, 1 H, H-3'), 7. 81 (dd, *J* = 9. 0 Hz, 2. 1 Hz, 1 H, H-4'), 7. 92 (d, *J* = 2. 1 Hz, 1 H, H-6'), 8. 62 (br s, 1. 5 H, amidine NH), 8. 95 (br s, 1. 5 H, amidine NH), 11. 68 (br s, I H, NH); MS (FAB) *m*/*z* 515 (MH⁺).

### Example 124

### Preparation of 2-(5-(4-(amidinomethyl)piperidin-1-ylsulfonyl)-2-n-propoxyphenyl)-5-methyl-7-n-pro pyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(amidinomethyl)piperidin-1-yl)

The titled compound was prepared as described in Example 125 by using 2-(5-(4-(cyanomethyl)piperidin-1-ylsulfonyl)-2-*n*-propoxyphenyl)-5-methyl-7-*n*-propy 1-3,5-dihydro-4*H*-pyrrolo[3,2-*d*]pyrimidin-4-one in place of 2-(5-(4-(cyanomethyl)piperidin-1-ylsulfonyl)-2-ethoxyphenyl)-5-methyl-7-*n*-propyl-3, 5-dihydro-4*H*-pyrrolo[3,2-*d*]pyrimidin-4-one.
yield: 69%
mp 181. 5 °C dec (EtOH/Et₂O);
IR (neat) 3324, 3089 (NH), 1682 (C=O), 1164 (SO₂) cm⁻¹;
¹H NMR (DMSO-*d*₆) δ 0. 92 (t, *J* = 7. 5 Hz, 3 H, CH₂CH₂C*H*₃), 0. 97(t, *J* = 7. 5 Hz, 3 H, OCH₂CH₂C*H*₃), 1. 21-1. 32 (m, 2 H, 2 CHₐₓ), 1. 60-1. 79 (m, 7 H, CH, 2 CH₂C*H*₂CH₃ and 2 CH_{eq}), 2. 21-2. 31 (m, 4 H, 2 CHC*H*₂ and NCHₐₓ), 2. 57(t, *J* = 7. 5 Hz, 2 H, C*H*₂CH₂CH₃), 3. 66 (br d, *J* = 11. 4 Hz, 2 H, 2 NCH_{eq}), 3. 99 (s, 3 H, NCH₃), 4. 13(t, *J* = 6. 3 Hz, 2 H, OCH₂CH₂CH₃), 7. 22(s, 1 H, H-2), 7. 37(d, *J* = 8. 7 Hz, 1 H, H-3'), 7. 80(dd, *J* = 8. 7 Hz, 2. 4 Hz, 1 H, H-4'), 7. 92(d, *J* = 2. 4 Hz, 1 H, H-6'), 8. 57 (br s, 1. 5 H, amidine NH), 8. 93 (br s, 1. 5 H, amidine NH), 11. 63 (br s, 1 H, NH); MS (FAB) *m*/*z* 529 (MH⁺).

### Example 125

### Preparation of 2-(5-(4-(2-amidinoethyl)piperidin-1-ylsulfonyl)-2-ethoxyphenyl)-5-methyl-7-n-propyl - 3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-amidinoethyl)piperidin-1-yl)

The titled compound was prepared as described in Example 125 by using 2-(5-(4-(2-cyanoethyl)piperidin-1-ylsulfonyl)-2-ethoxyphenyl)-5-methyl-7-*n*-propyl-3, 5-dihydro-4*H*-pyrrolo[3,2-*d*]pyrimidin-4-one in place of 2-(5-(4-(cyanomethyl)piperidin-1-ylsulfonyl)-2-ethoxyphenyl)-5-methyl-7-*n*-propyl-3, 5-dihydro-4*H*-pyrrolo[3,2-*d*]pyrimidin-4-one.
yield: 55%
mp 186-186.5 °C (EtOH/Et₂O);
IR (neat) 3399, 3346, 3080 (NH), 1690 (C=O), 1157 (SO₂) cm⁻¹;
¹H NMR (DMSO-*d*_{*6*}) δ 0. 92 (t, *J* = 7. 5 Hz, 3 H, CH₂CH₂C*H*₃), 1. 15-1. 25 (m, 2 H, 2 CHₐₓ), 1. 35 (t, *J* = 6. 9 Hz, 3 H, OCH₂C*H*₃), 1. 50-1. 75 (m, 7 H, C*H*C*H*₂, CH₂C*H*₂CH₃ and 2 CH_{eq}), 2. 21-2. 27 (m, 2 H, 2 NCHₐₓ), 2. 32-2. 37 (m, 2 H, CH₂C*H*₂C(=NH)NH₂), 2. 57 (t, *J* = 7. 5 Hz, C*H*₂CH₂CH₃), 3. 63 (br d, *J* = 10. 8 Hz, 2 H, 2 NCH_{eq}), 3. 99 (s, 3 H, NCH₃), 4. 22 (q, *J* = 6. 9 Hz, 2 H, OC*H*₂CH₃), 7. 23 (s, 1 H, H-2), 7. 36 (d, *J* = 8. 7 Hz, 1 H, H-3'), 7. 80 (dd, *J* = 8. 7 Hz, 2. 4 Hz, 1 H, H-4'), 7. 91 (d, *J* = 2. 4 Hz, 1 H, H-6'), 8. 55 (br s, 1. 5 H, amidine NH), 8. 93 (br s, 1. 5 H, amidine NH), 11. 68 (br s, 1 H, NH); MS (FAB) *m*/*z* 529 (MH⁺).

### Example 126

### Preparation of 2-(5-(4-(2-amidinoethyl)piperidin-1-ylsulfonyl)-2-n-propoxyphenyl)-5-methyl-7-n-pro pyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-amidinoethyl)piperidin-1-yl)

The titled compound was prepared as described in Example 125 by using 2-(5-(4-(2-cyanoethyl)piperidin-1-ylsulfonyl)-2-*n*-propoxyphenyl)-5-methyl-7-*n*-propy 1-3,5-dihydro-4*H*-pyrrolo[3,2-*d*]pyrimidin-4-one in place of 2-(5-(4-(cyanomethyl)piperidin-1-ylsulfonyl)-2-ethoxyphenyl)-5-methyl-7-*n*-propyl-3, 5-dihydro-4*H*-pyrrolo[3,2-*d*]pyrimidin-4-one.
yield: 55%
mp 135 °C dec (EtOH/Et₂O);
IR (neat) 3331, 3070 (NH), 1685 (C=O), 1161 (SO₂) cm⁻¹;
¹H NMR (DMSO-*d*₆) δ 0. 92 (t, *J* = 7. 5 Hz, 3 H, CH₂CH₂C*H*₃), 0. 97 (t, *J* = 7. 2 Hz, 3 H, OCH₂CH₂C*H*₃), 1. 15-1. 25 (m, 2 H, 2 CHₐₓ), 1. 49-1. 71 (m, 5 H, C*H*C*H*₂ and CH₂C*H*₂CH₃), 1. 72-1. 78 (m, 4 H, OCH₂C*H*₂CH₃ and 2 CH_{eq}), 2. 20-2. 27 (m, 2 H, 2 NCHₐₓ), 2. 32-2. 37 (m, 2 H, CH₂C*H*₂C(=NH)NH₂), 2. 57 (t, *J* = 7. 5 Hz, C*H*₂CH₂CH₃), 3. 63 (br d, *J* = 11. 1 Hz, 2 H, 2 NCH_{eq}), 3. 99 (s, 3 H, NCH₃), 4. 12 (t, *J* = 6. 3 Hz, 2 H, OC*H*₂CH₂CH₃), 7. 22 (s, 1 H, H-2), 7. 36 (d, *J* = 8. 7 Hz, 1 H, H-3'), 7. 80 (dd, *J* = 8. 7 Hz, 2. 4 Hz, 1 H, H-4'), 7. 91 (d, *J* = 2. 4 Hz, 1 H, H-6'), 8. 57 (br s, 1. 5 H, amidine NH), 8. 94 (br s, 1. 5 H, amidine NH), 11. 64 (br s, 1 H, NH); MS (FAB) *m*/*z* 543 (MH⁺).

### Example 127

### Preparation of 2-(2-ethoxy-5-(1H-tetrazol-5-yl)phenyl)-5-methyl-7-n-propyl-3,5-dihydro-4H-pyrrolo[ 3,2-d]pyrimidin-4-one (a compound of the formula (1) wherein R⁵ = 1H-tetrazol-5-yl, R¹ = CH₃, R² = H, R³ = CH₂CH₂CH₃, R⁴ = CH₂CH₃)

A suspension of 2-(5-cyano-2-ethoxyphenyl)-5-methyl-7-*n*-propyl-3,5-dihydro-4*H*-pyrrolo[3,2-*d*]pyrim idin-4-one (150 mg, 0. 45 mmol), tributyltin chloride (133 µL, 0. 49 mmol) and sodium azide (29 mg, 0. 45 mmol) in anhydrous toluene (1. 0 mL) was heated at 120 °C for 70 h, after which the mixture was cooled to room temperature. The reaction mixture was diluted with 5% aqueous NaOH solution (25 mL), and was extracted with CHCl₃ (15 mL × 3). The organic layers were discarded, and the aqueous layer was acidified to pH 2 using 10% aqueous HCl. The resulting white precipitates were collected and was dried under vacuum to afford the titled compound (148 mg, 87%) as a white solid. Analytically pure compound was obtained by crystallization from CHCl₃/MeOH/hexanes.
mp 245 °C dec;
IR (neat) 3325, 3023 (NH), 1666 (C=O) cm⁻¹;
¹H NMR (DMSO-*d*₆) δ 0. 93 (t, *J* = 7. 5 Hz, 3 H, CH₂CH₂C*H*₃), 1. 37 (t, *J* = 6. 9 Hz, 3 H, OCH₂C*H*₃), 1. 59-1. 70 (m, 2 H, CH₂C*H*₂CH₃), 2. 60 (t, *J* = 7. 5 Hz, 2 H, C*H*₂CH₂CH₃), 4. 00 (s, 3 H, NCH₃), 4. 22 (q, *J* = 6. 9 Hz, 2 H, OC*H*₂CH₃), 7. 23 (s, 1 H, H-2), 7. 38 (d, *J* = 8. 7 Hz, 1 H, H-3'), 8. 12 (dd, *J* = 8. 7 Hz, 2. 4 Hz, 1 H, H-4'), 8. 31 (d, *J* = 2.4 Hz, 1 H, H-6'), 11. 69 (br s, 1 H, NH); MS (FAB) *m*/*z* 380 (MH⁺).

### Example 128

### Preparation of 5-methyl-2-(2-n-propoxy-5-(1H-tetrazol-5-yl)phenyl)-7-n-propyl-3,5-dihydro-4H-pyrr olo[3,2-d]pyrimidin-4-one (a compound of the formula (1) wherein R⁵ = 1H-tetrazol-5-yl, R¹ = CH₃, R² = H, R³ = CH₂CH₂CH₃, R⁴ = CH₂CH₂CH₃)

The titled compound was prepared as described in Example 129 by using 2-(5-cyano-2-*n*-propoxyphenyl)-5-methyl-7-*n*-propyl-3,5-dihydro-4*H*-pyrrolo[3,2-*d*]p yrimidin-4-one in place of 2-(5-cyano-2-ethoxyphenyl)-5-methyl-7-*n*-propyl-3,5-dihydra-4*H*-pyrrolo[3,2-*d*]pyrim idin-4-one.
yield: 58%
mp 250. 5 °C dec (CHCl₃/Et₂O);
IR (neat) 3324; 3023 (NH), 1671 (C=O) cm⁻¹;
¹H NMR (DMSO-*d*₆) δ 1. 01 (t, *J* = 7.5 Hz, 3 H, CH₂CH₂C*H*₃), 1. 06 (t, *J* = 7. 5 Hz, 3 H, OCH₂CH₂C*H*₃), 1. 66-1. 78 (m, 2 H, CH₂C*H*₂CH₃), 1. 79-1. 91 (m, 2 H, OCH₂C*H*₂CH₃), 2. 67 (t, *J* = 7. 5 Hz, 2 H, C*H*₂CH₂CH₃), 4. 13 (s, 3 H, NCH₃), 4. 19 (t, *J* = 6. 6 Hz, 2 H, OC*H*₂CH₂CH₃), 7. 30 (s, 1 H, H-2), 7. 44 (d, *J* = 8. 7 Hz, 1 H, H-3'), 8. 19 (dd, *J*= 8. 7 Hz, 2. 4 Hz, 1 H, H-4'), 8. 39 (d, *J* = 2. 4 Hz, 1 H, H-6'), 11. 70 (br s, 1 H, NH); MS (FAB) *m*/*z* 394 (MH⁺).

### Example 129

### Preparation of 2-(2-ethoxy-5-(4-(1H-tetrazol-5-ylmethyl)piperazin-1-ylsulfonyl)phenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(1H-tetrazol-5-ylmethyl)piperazin-1-yl)

A mixture of 1-(*tert*-butoxycarbonyl)-4-(1-trityltetrazol-5-ylmethyl)piperazine (325 mg, 0. 63 mmol) and 1*H*-tetrazole (222 mg, 3. 17 mmol) in tetrahydrofuran (2. 5 mL) was treated with 10% aqueous HCl (1. 2 mL) at room temperature for 2 h, after which it was completely evaporated to dryness under vacuum. The resulting crude 1-(1*H*-tetrazol-5-ylmehthyl)piperazine, 2-(5-chlorosulfonyl-2-ethoxyphenyl)-5-methyl-7-*n*-propyl-3,5-dihydro-4*H*-pyrrolo[3,2 -*d*]pyrimidin-4-one (200 mg, 0. 49 mmol) and triethylamine (0. 44 mL, 3. 17 mmol) were suspended in anhydrous EtOH (8 mL), and the mixture was stirred at room temperature for 1-2 h under nitrogen. The reaction mixture was evaporated to dryness under reduced pressure, and the crude residue was purified by MPLC on silica gel (0. 5% AcOH/7% MeOH in CHCl₃ to obtain a pale yellow solid. The resulting solid was dissolved in 5% MeOH in CHCl₃ (20 mL), washed with H₂O (20 mL), dried (MgSO₄), filtered and the filtrate was evaporated to dryness under reduced pressure to afford the titled compound (265 mg, 99%) as a yellowish solid. Analytically pure compound was obtained by crystallization from CHCl₃/MeOH/Et₂O.
mp 188 °C dec (CHCl₃/MeOH/Et₂O);
IR (neat) 3319, 3104 (NH), 1667 (C=O), 1168 (SO₂) cm⁻¹;
¹H NMR (DMSO-*d*₆) δ 0. 90 (t, *J* = 7. 5 Hz, 3 H, CH₂CH₂C*H*₃), 1. 36 (t, *J* = 6. 9 Hz, 3
H, OCH₂C*H*₃), 1. 56-1. 69 (m, 2 H, CH₂C*H*₂CH₃), 2. 49-2. 59 (m, 6 H, 2 NCH₂ and C*H*₂CH₂CH₃), 2. 91-2. 98 (m, 4 H, 2 SO₂NCH₂), 3. 87 (s, 2 H, NCH₂N), 3. 99 (s, 3 H, NCH₃), 4. 23 (q, *J =* 6. 9 Hz, 2 H, OC*H*₂CH₃), 7. 22 (s, 1 H, H-2), 7. 37 (d, *J* = 8. 7 Hz, 1 H, H-3'), 7. 80 (dd, *J* = 8. 7 Hz, 2. 4 Hz, 1 H, H-4'), 7. 89 (d, *J* = 2. 4 Hz, 1 H, H-6'), 11. 71 (br s, 1 H, NH); MS (FAB) *m*/*z* 542 (MH⁺).

### Example 130

### Preparation of 2-(2-n-propoxy-5-(4-(1H-tetrazol-5-ylmethyl)piperazin-1-ylsulfonyl)phenyl)-5-methyl - 7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(1H-tetrazol-5-ylmethyl)piperazin-1-yl)

The titled compound was prepared as described in Example 131 by using 2-(5-chlorosulfonyl-2-*n*-propoxyphenyl)-5-methyl-7-*n*-propyl-3,5-dihydro-4*H*-pyrrolo [3,2-d]pyrimidin-4-one in place of 2-(5-chlorosulfonyl-2-ethoxyphenyl)-5-methyl-7-*n*-propyl-3,5-dihydro-4*H*-pyrrolo[3,2 - *d*]pyrimidin-4-one.
yield: 86%
mp 150 °C dec (CHCl₃/MeOH/Et₂O);
IR (neat) 3393, 3311 (NH), 1667 (C=O), 1163 (SO₂) cm⁻¹;
¹H NMR (DMSO-*d*₆) δ 0.90 (t, *J* = 7. 5 Hz, 3 H, CH₂CH₂C*H*₃), 0. 97 (t, *J* = 7. 5 Hz, 3 H, OCH₂CH₂C*H*₃), 1. 56-1. 68 (m, 2 H, CH₂C*H*₂CH₃), 1. 69-1. 81 (m, 2 H, OCH₂C*H*₂CH₃), 2. 49-2. 58 (m, 6 H, 2 NCH₂ and C*H*₂CH₂CH₃), 2. 90-2. 98 (m, 4 H, 2 SO₂NCH₂), 3. 88 (s, 2 H, NCH₂N), 3. 99 (s, 3 H, NCH₃), 4. 13 (t, *J* = 6. 3 Hz, 2 H, OC*H*₂CH₂CH₃), 7. 22 (s, 1 H, H-2), 7. 38 (d, *J* = 9. 0 Hz, 1 H, H-3'), 7. 80 (dd, *J* = 9. 0 Hz, 2. 4 Hz, 1 H, H-4'), 7. 89 (d, *J* = 2. 4 Hz, 1 H, H-6'), 11. 67 (br s, 1 H, NH); MS (FAB) *m*/*z* 556 (MH⁺).

### Example 131

### Preparation of 2-(2-ethoxy-5-(4-(2-(1H-tetrazol-5-yl)ethyl)piperazin-1-ylsulfonyl)phenyl)-5-methyl-7 -n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-(1H-tetrazol-5-yl)ethyl)piperazin-1-yl)

The titled compound was prepared as described in Example 131 by using 1-(*tert*-butoxycarbonyl)-4-(2-(1-trityltetrazol-5-yl)ethyl)piperazine in place of 1-(*tert*-butoxycarbonyl)-4-(1-trityltetrazol-5-ylmethyl)piperazine.
yield: 83%
mp 231. 5 °C dec (CHCl₃/MeOH/Et₂O);
IR (neat) 3317, 3245 (NH), 1687 (C=O), 1169 (SO₂) cm⁻¹;
¹H NMR (DMSO-*d*₆) δ 0. 92 (t, *J* = 7. 5 Hz, 3 H, CH₂CH₂C*H*₃), 1. 35 (t, *J* = 6. 9 Hz, 3 H, OCH₂C*H*₃), 1. 57-1. 68 (m, 2 H, CH₂C*H*₂CH₃), 2. 45-2. 54 (m, 4 H, 2 NCH₂), 2. 56 (t, *J* = 7. 5 Hz, 2 H, C*H*₂CH₂CH₃), 2. 68 (t, *J* = 6. 9 Hz, 2 H, NC*H*₂CH₂), 2. 83-2. 92 (m, 4 H, 2 SO₂NCH₂), 2. 99 (t, *J* = 6. 9 Hz, 2 H, NCH₂C*H*₂), 3. 98 (s, 3 H, NCH₃), 4. 21 (q, *J* = 6. 9 Hz, 2 H, OC*H*₂CH₃), 7. 22 (s, 1 H, H-2), 7. 35 (d, *J* = 9. 0 Hz, 1 H, H-3'), 7. 79 (dd, *J* = 9. 0 Hz, 2. 4 Hz, 1 H, H-4'), 7. 88 (d, *J* = 2. 4 Hz, 1 H, H-6'), 11. 68 (br s, 1 H, NH); MS (FAB) *m*/*z* 556 (MH⁺).

### Example 132

### Preparation of 5-methyl-2-(2-n-propoxy-5-(4-(2-(1H-tetrazol-5-yl)ethyl)piperazin-1-ylsulfonyl)pheny 1)-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-(1H-tetrazol-5-yl)ethyl)piperazin-1-yl)

The titled compound was prepared as described in Example 131 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(*tert*-butoxycarbonyl)-4-(2-(1-trityltetrazol-5-yl)ethyl)piperazine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-(*tert*-butoxycarbonyl)-4-(1-trityltetrazol-5-ylmethyl)piperazine
yield: 88%
mp 181 °C dec (CHCl₃/MeOH/Et₂O);
IR (neat) 3317, 3173 (NH), 1678 (C=O), 1166 (SO₂) cm⁻¹;
¹H NMR (DMSO-*d*₆) δ 0. 92 (t, *J* = 7. 2 Hz, 3 H, CH₂CH₂C*H*₃), 0. 96 (t, *J* = 7. 2 Hz, 3 H, OCH₂CH₂C*H*₃), 1. 57-1. 69 (m, 2 H, CH₂C*H*₂CH₃), 1. 68-1. 80 (m, 2 H, OCH₂C*H*₂CH₃), 2. 49-2. 52 (m, 4 H, 2 NCH₂), 2. 56 (t, *J* = 7. 5 Hz, 2 H, C*H*₂CH₂CH₃), 2. 69 (t, *J* = 7. 2 Hz, 2 H, NC*H*₂CH₂), 2. 84-2. 91 (m, 4 H, 2 SO₂NCH₂), 3. 00 (t, *J* = 7. 2 Hz, 2 H, NCH₂C*H*₂), 3. 99 (s, 3 H, NCH₃), 4. 12 (t, *J* = 6. 3 Hz, 2 H, OC*H*₂CH₂CH₃), 7. 22 (s, 1 H, H-2), 7. 36 (d, *J* = 8. 7 Hz, 1 H, H-3'), 7. 79 (dd, *J* = 8. 7 Hz, 2. 4 Hz, 1 H, H-4'), 7. 88 (d, *J* = 2. 4 Hz, 1 H, H-6'), 11. 64 (br s, 1 H, NH); MS (FAB) *m*/*z* 570 (MH⁺).

### Example 133

### Preparation of 2-(2-ethoxy-5-(4-(1H-tetrazol-5-ylmethyl)piperidin-1-ylsulfonyl)phenyl)-5-methyl-7-n -propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(1H-tetrazol-5-ylmethyl)piperidin-1-yl)

The titled compound was prepared as described in Example 131 by using 1-(*tert*-butoxycarbonyl)-4-(1-trityltetrazol-5-ylmethyl)piperidine in place of 1-(*tert*-butoxycarbonyl)-4-(1-trityltetrazol-5-ylmethyl)piperazine.
yield: 83%
mp 215-216 °C dec (CHCl₃/MeOH/Et₂O);
IR (neat) 3396, 3103 (NH), 1662 (C=O), 1166 (SO₂) cm⁻¹;
¹H NMR (DMSO-*d*₆) δ 0. 90 (t, *J* = 7. 5 Hz, 3 H, CH₂CH₂C*H*₃), 1. 22-1. 33 (m, 2 H, 2 CHₐₓ), 1. 36 (t, *J* = 6. 9 Hz, 3 H, OCH₂C*H*₃), 1. 57-1. 69 (m, 5 H, CH, 2 CH_{eq} and CH₂C*H*₂CH₃), 2. 27 (br t, *J* = 11. 1 Hz, 2 H, 2 NCHₐₓ), 2. 56 (t, *J* = 7. 5 Hz, 2 H, C*H*₂CH₂CH₃), 2. 81 (d, *J* = 6. 6 Hz, 2 H, CHC*H*₂), 3. 64 (br d, *J* = 12. 0 Hz, 2 H, 2 NCH_{eq}), 3. 98 (s, 3 H, NCH₃), 4. 22 (q, *J* = 6. 9 Hz, 2 H, OC*H*₂CH₃), 7. 22 (s, 1 H, H-2), 7. 35 (d, *J* = 8. 7 Hz, 1 H, H-3'), 7. 79 (dd, *J* = 8. 7 Hz, 2. 4 Hz, 1 H, H-4'), 7. 91 (d, *J* = 2. 4 Hz, 1 H, H-6'), 11. 66 (br s, 1 H, NH); MS (FAB) *m*/*z* 541 (MH⁺).

### Example 134

### Preparation of 5-methyl-2-(2-n-propoxy-5-(4-(1H-tetrazol-5-ylmethyl)piperidin-1-ylsulfonyl)phenyl) -7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(1H-tetrazol-5-ylmethyl)piperidin-1-yl)

The titled compound was prepared as described in Example 131 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(*tert*-butoxycarbonyl)-4-(1-trityltetrazol-5-ylmethyl)piperidine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-(*tert*-butoxycarbonyl)-4-(1-trityltetrazol-5-ylmethyl)piperazine.
yield: 81%
mp 231 °C dec (CHCl₃/MeOH/Et₂O);
IR (neat) 3313, 3109 (NH), 1665 (C=O), 1167 (SO₂) cm⁻¹;
¹H NMR (DMSO-*d*₆) δ 0. 90 (t, *J* = 7. 5 Hz, 3 H, CH₂CH₂C*H*₃), 0. 97 (t, *J* = 7. 5 Hz, 3 H, OCH₂CH₂C*H*₃), 1. 20-1. 35 (m, 2 H, 2 CHₐₓ), 1. 58-1. 80 (m, 7 H, CH, 2 CH_{eq} and 2 CH₂C*H*₂CH₃), 2. 27 (br t, *J* = 11.1 Hz, 2 H, 2 NCHₐₓ), 2. 56 (t, *J* = 7. 5 Hz, 2 H, C*H*₂CH₂CH₃), 2. 81 (d, *J* = 6. 6 Hz, 2 H, CHC*H*₂), 3. 64 (br d, *J* = 11. 7 Hz, 2 H, 2 NCH_{eq}), 3. 98 (s, 3 H, NCH₃), 4. 12 (t, *J* = 6. 3 Hz, 2 H, OC*H*₂CH₂CH₃), 7. 22 (s, 1 H, H-2), 7. 35 (d, *J* = 8. 7 Hz, 1 H, H-3'), 7. 79 (dd, *J* = 8. 7 Hz, 2. 7 Hz, 1 H, H-4'), 7. 91 *(d, J* = 2. 7 Hz, 1 H, H-6'), 11. 62 (br s, 1 H, NH); MS (FAB) *m*/*z* 555 (MH⁺).

### Example 135

### Preparation of 2-(2-ethoxy-5-(4-(2-(1H-tetrazol-5-yl)ethyl)piperidin-1-ylsulfonyl)phenyl)-5-methyl-7 -n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 4-(2-(1H-tetrazol-5-yl)ethyl)piperidin-1-yl)

The titled compound was prepared as described in Example 131 by using 1-(*tert*-butoxycarbonyl)-4-(2-(1-trityltetrazol-5-yl)ethyl)piperidine in place of 1-(*tert*-butoxycarbonyl)-4-(1-trityltetrazol-5-ylmethyl)piperazine.
yield: 75%
mp 192 °C dec (CHCl₃/MeOH/Et₂O);
IR (neat) 3327, 3227 (NH), 1690 (C=O), 1146 (SO₂) cm⁻¹;
¹H NMR (DMSO-*d*₆) δ 0. 91 (t, *J* = 7. 5 Hz, 3 H, CH₂CH₂C*H*₃), 1. 12-1. 26 (m, 3 H, CH and 2 CHₐₓ), 1. 34 (t, *J* = 6. 9 Hz, 3 H, OCH₂C*H*₃), 1. 54-1. 67 (m, 4 H, CHC*H*₂CH₂ and CH₂C*H*₂CH₃), 1. 67-1. 80 (m, 2 H, 2 CH_{eq}), 2. 20 (br t, *J* = 10. 8 Hz, 2 H, 2 NCHₐₓ), 2. 56 (t, *J* = 7. 5 Hz, 2 H, C*H*₂CH₂CH₃), 2. 85 (t, *J* = 7. 5 Hz, 2 H, CHCH₂C*H*₂), 3. 63 (br d, *J* = 10. 5 Hz, 2 H, 2 NCH_{eq}), 3. 98 (s, 3 H, NCH₃), 4. 21 (q, *J* = 6. 9 Hz, 2 H, OC*H*₂CH₃), 7. 21 (s, 1 H, H-2), 7. 34 (d, *J* = 8. 7 Hz, 1 H, H-3'), 7. 79 (dd, *J* = 8. 7 Hz, 2. 4 Hz, 1 H, H-4'), 7. 90 (d, *J* = 2. 4 Hz, 1 H, H-6'), 11. 65 (br s, 1 H, NH); MS (FAB) *m*/*z* 555 (MH⁺).

### Example 136

### Preparation of 5-methyl-2-(2-n-propoxy-5-(4-(2-(1H-tetrazol-5-yl)ethyl)piperidin-1-ylsulfonyl)pheny 1)-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 4-(2-(1H-tetrazol-5-yl)ethyl)piperidin-1-yl)

The titled compound was prepared as described in Example 131 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and 1-(*tert*-butoxycarbonyl)-4-(2-(1-trityltetrazol-5-yl)ethyl)piperidine in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-(*tert*-butoxycarbonyl)-4-(1-trityltetrazol-5-ylmethyl)piperazine.
yield: 81%
mp 145 °C dec (CHCl₃/MeOH/Et₂O);
IR (neat) 3326, 3224 (NH), 1694 (C=O), 1144 (SO₂) cm⁻¹;
¹H NMR (DMSO-*d*₆) δ 0. 86 (t, *J* = 7. 5 Hz, 3 H, CH₂CH₂C*H*₃), 0. 91 (t, *J* = 7. 5 Hz, 3 H, OCH₂CH₂C*H*₃), 1. 02-1. 24 (m, 3 H, CH and 2 CHₐₓ), 1. 47-1. 62 (m, 4 H, CHC*H*₂CH₂ and CH₂C*H*₂CH₃), 1. 60-1. 78 (m, 4 H, OCH₂C*H*₂CH₃ and 2 CH_{eq}), 2. 14 (br t, *J* = 10. 5 Hz, 2 H, 2 NCHₐₓ), 2. 51 (t, *J* = 7. 5 Hz, 2 H, C*H*₂CH₂CH₃), 2. 74 (t, *J* = 7. 5 Hz, 2 H, CHCH₂C*H*₂), 3. 57 (br d, *J =* 11. 4 Hz, 2 H, 2 NCH_{eq}), 3. 93 (s, 3 H, NCH₃), 4. 07 (t, *J* = 6. 3 Hz, 2 H, OC*H*₂CH₂CH₃), 7. 16 (s, 1 H, H-2), 7. 30 (d, *J* = 9. 0 Hz, 1 H, H-3'), 7. 74 (dd, *J* = 9. 0 Hz, 2. 4 Hz, 1 H, H-4'), 7. 85 (d, *J* = 2. 4 Hz, 1 H, H-6'), 11. 58 (br s, 1 H, NH); MS (FAB) *m*/*z* 569 (MH⁺).

### Example 137

### Preparation of 2-(5-(di(2-fluoroethyl)aminosulfonyl)-2-ethoxyphenyl)-5-methyl-7-n-propyl-3,5-dihyd ro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; R⁶ = R⁷ = 2-fluoroethyl)

The titled compound was prepared as described in Example 1 by using bis(2-fluoroethyl)amine hydrochloride in place of 1-methylpiperazine.
yield: 80%
mp 210-210. 5 °C (MeOH/CHCl₃/Et₂O);
IR (neat) 3320 (NH), 1694 (C=O), 1162 (SO₂) cm⁻¹;
¹H NMR (CDCl₃/TMS) δ 0.92 (t, *J* = 7. 5 Hz, 3 H, CH₂CH₂C*H*₃), 1. 35 (t, *J* = 6. 9 Hz, 3 H, OCH₂C*H*₃), 1. 56-1. 71 (m, 2 H, CH₂C*H*₂CH₃), 2. 57 (t, *J* = 7. 5 Hz, C*H*₂CH₂CH₃), 3. 50 (dt, *J* = 24. 6 Hz, 5. 1 Hz, 4 H, 2 NC*H*₂CH₂F), 3. 99 (s, 3 H, NCH₃), 4. 22 (q, *J* = 6. 9 Hz, 2 H, OC*H*₂CH₃), 4. 54 (dt, *J* = 47. 4 Hz, 5. 1 Hz, 4 H, 2 NCH₂C*H*₂F), 7. 22 (s, 1 H, H-2), 7. 33 (d, *J* = 8. 7 Hz, 1 H, H-3'), 7. 92 (dd, *J* = 8. 7 Hz, 2. 4 Hz, 1 H, H-4'), 8. 02 (d, *J* = 2. 4 Hz, 1 H, H-6'), 11. 69 (br s, 1 H, NH); MS (FAB) *m*/*z* 483 (MH⁺).

### Example 138

### Preparation of 2-(2-ethoxy-5-((S)-1-hydroxycarbonyl-2-methylpropyl)aminosulfonyl)phenyl)-5-meth yl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is ((S)-1-hydroxycarbonyl-2-methylpropyl)amino)

A mixture of 2-(5-((*S*)-1-benzyloxycarbonyl-2-methylpropyl)aminosulfonyl)-2-ethoxyphenyl)-5-me thyl-7-*n*-propyl-3,5-dihydro-4*H*-pyrrolo[3,2-*d*]pyrimidin-4-one (190 mg, 0. 33 mmol) and 5% Pd/C (68 mg) in EtOH was vigorously stirred overnight under H₂ atmosphere (1 atm; a balloon) at room temperature. The reaction mixture was filtered through a Celite pad, and the filtrate was evaporated to dryness under reduced pressure. The resulting residue was purified by MPLC on silica gel (gradient elution: 1% MeOH in CHCl₃ followed by 20% MeOH in CHCl₃) to afford the titled compound (158 mg, 99%) as a pale yellow solid. Analytically pure compound was obtained by crystallization from EtOAc/Et₂O/hexanes.
mp 196-196. 5 °C dec;
IR (neat) 3320 (NH and CO₂H), 1682 (C=O), 1155 (SO₂) cm⁻¹;
¹H NMR (DMSO-*d*₆) δ 0. 80 (d, *J* = 6. 6 Hz, 3 H, CHC*H*₃), 0. 85 (d, *J* = 7. 2 Hz, 3 H, CHC*H*₃), 0. 93 (t, *J* = 7. 5 Hz, 3 H, CH₂CH₂C*H*₃), 1. 35 (t, *J* = 6. 9 Hz, 3 H, OCH₂C*H*₃), 1. 59-1. 69 (m, 2 H, CH₂C*H*₂CH₃), 1. 98-2. 06 (m, 1 H, C*H*(CH₃)₂), 2. 58 (t, *J* = 7. 5 Hz, 2 H, C*H*₂CH₂CH₃), 3. 23 (d, *J* = 6. 6 Hz, 1 H, NCHCO₂), 3. 98 (s, 3 H, NCH₃), 4. 19 (q, *J* = 6. 9 Hz, 2 H, OC*H*₂CH₃), 7. 21 (s, 1 H, H-2), 7. 26 (d, *J* = 8. 7 Hz, 1 H, H-3'), 7. 83 (dd, *J* = 8. 7 Hz, 2. 4 Hz, 1 H, H-4'), 8. 02 (d, *J* = 2. 4 Hz, 1 H, H-6'), 11. 65 (br s, 1 H, NH); MS (FAB) *m*/*z* 491 (MH⁺).

### Example 139

### Preparation of 2-(5-((S)-1-hydroxycarbonyl-2-methylpropyl)aminosulfonyl)-2-n-propoxyphenyl)-5-m ethyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is ((S)-1-hydroxycarbonyl-2-methylpropyl)amino)

The titled compound was prepared as described in Preparative Example 140 by using 2-(5-((*S*)-1-benzyloxycarbonyl-2-methylpropyl)sulfonamido-2-*n*-propoxyphenyl)-5-m ethyl-7-*n*-propyl-3,5-dihydro-4*H*-pyrrolo[3,2-*d*]pyrimidin-4-one in place of 2-(5-((*S*)-1-benzyloxycarbonyl-2-methylpropyl)sulfonamido-2-ethoxyphenyl)-5-methy 1-7-*n*-propyl-3,5-dihydro-4*H*-pyrrolo[3,2-*d*]pyrimidin-4-one.
yield: 99%
mp 193-194 °C (EtOAc/Et₂O/hexanes);
IR (neat) 3322 (NH and CO₂H), 1675 (C=O), 1157 (SO₂) cm⁻¹;
¹H NMR (DMSO-*d*₆) δ 0. 79 (d, *J* = 6. 9 Hz, 3 H, CHC*H*₃), 0. 85 (d, *J* = 6. 9 Hz, 3 H, CHC*H*₃), 0. 92 (t, *J* = 7. 5 Hz, 3 H, CH₂CH₂C*H*₃), 0. 97 (t, *J* = 7. 2 Hz, 3 H, OCH₂CH₂C*H*₃), 1. 57-1. 78 (m, 4 H, 2 CH₂C*H*₂CH₃), 1. 95-2. 05 (m, 1 H, C*H*(CH₃)₂), 2. 58 (t, *J* = 7. 5 Hz, 2 H, C*H*₂CH₂CH₃), 3. 20 (d, *J* = 6. 0 Hz, 1 H, NCHCO₂), 3. 98 (s, 3 H, NCH₃), 4. 09 (t, *J* = 6. 3 Hz, 2 H, OC*H*₂CH₂CH₃), 7. 21 (s, 1 H, H-2), 7. 26 (d, *J* = 8. 7 Hz, 1 H, H-3'), 7. 83 (dd, *J* = 8. 7 Hz, 2. 4 Hz, 1 H, H-4'), 8. 03 (d, *J* = 2. 4 Hz, 1 H, H-6'), 11. 58 (br s, 1 H, NH); MS (FAB) *m*/*z* 405 (MH⁺).

### Example 140

### Preparation of 2-(2-ethoxy-5-(1-imidazolosulfonyl)phenyl)-5-methyl-7-n-propyl-3,5-dihydro-4H-pyrr olo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₃; NR⁶R⁷ is 1-imidazolo)

The titled compound was prepared as described in Example 1 by using imidazole in place of 1-methylpiperazine.
yield: 97%
mp 171-172°C (CHCl₃/Et₂O/hexanes);
IR (neat) 3112 (NH), 1671 (C=O), 1186 (SO₂) cm⁻¹;
¹H NMR (CDCl₃/TMS) δ 1. 04 (t, *J* = 7. 2 Hz, 3 H, CH₂CH₂C*H*₃), 1. 63 (t, *J* = 6. 9 Hz, 3 H, OCH₂C*H*₃), 1. 70-1. 82 (m, 2 H, CH₂C*H*₂CH₃), 2. 74 (t, *J* = 7. 2 Hz, 2 H, C*H*₂CH₂CH₃), 4. 08 (s, 3 H, NCH₃), 4. 36 (q, *J* = 6. 9 Hz, 2 H, OC*H*₂CH₃), 6. 91 (s, 1 H, H-2), 7. 11 (s, 1 H, ImH-4), 7. 14 (d, *J* = 9. 0 Hz, 1 H, H-3'), 7. 35 (s, 1 H, ImH-5), 7. 96 (dd, *J* = 9. 0 Hz, 2. 7 Hz, 1 H, H-4'), 8. 05 (s, 1 H, ImH-2), 9. 05 (d, *J* = 2. 7 Hz, 1 H, H-6'), 10. 52 (br s, 1 H, NH); MS (FAB) *m*/*z* 442 (MH⁺).

### Example 141

### Preparation of 2-(5-(1-imidazolosulfonyl)-2-n-propoxyphenyl)-5-methyl-7-n-propyl-3,5-dihydro-4H-pyrrolo[3,2-d]pyrimidin-4-one (a compound of the formula (1) wherein R⁵ = SO₂NR⁶R⁷, R¹ = CH₃, R² = H, R³ = CH₂CH₂CH₃, R⁴ = CH₂CH₂CH₃; NR⁶R⁷ is 1-imidazolo)

The titled compound was prepared as described in Example 1 by using 2-(5-chlorosulfonyl-2-*n*-propoxyphenyl)-5-methyl-7-*n*-propyl-3,5-dihydro-4*H*-pyrrolo [3,2-*d*]pyrimidin-4-one and imidazole in place of 2-(5-chlorosulfonyl-2-ethoxyphenyl)-5-methyl-7-*n*-propyl-3,5-dihydro-4*H*-pyrrolo[3,2 -*d*]pyrimidin-4-one and 1-methylpiperazine.
yield: 99%
mp 163-164 °C (CHCl₃/Et₂O/hexanes);
IR (neat) 3116 (NH), 1668 (C=O), 1184 (SO₂) cm⁻¹;
¹H NMR (CDCl₃/TMS) δ 1. 04 (t, *J* = 7. 5 Hz, 3 H, CH₂CH₂C*H*₃), 1. 17 (t, *J* = 7. 5 Hz, 3 H, OCH₂CH₂C*H*₃), 1. 70-1. 82 (m, 2 H, CH₂C*H*₂CH₃), 1. 97-2. 08 (m, 2 H, OCH₂C*H*₂CH₃), 2. 75 (t, *J* = 7. 5 Hz, 2 H, C*H*₂CH₂CH₃), 4. 08 (s, 3 H, NCH₃), 4. 25 (t, *J* = 6. 6 Hz, 2 H, OC*H*₂CH₂CH₃), 6. 91 (s, 1 H, H-2), 7. 11 (s, 1 H, ImH-4), 7. 15 (d, J = 9. 0 Hz, 1 H, H-3'), 7. 35 (s, 1 H, ImH-5), 7. 96 (dd, *J* = 9. 0 Hz, 2. 7 Hz, 1 H, H-4'), 8. 06 (s, 1 H, ImH-2), 9. 07 (d, *J =* 2. 7 Hz, 1 H, H-6'), 10. 55 (br s, 1 H, NH); MS (FAB) *m*/*z* 456 (MH⁺).

### Example 142

### Preparation of 2-(2-ethoxy-5-methylsulfonaminophenyl)-5-methyl-7-n-propyl-3,5-dihydro-4H-pyrrol o[3,2-d]pyrimidin-4-one ((a compound of the formula (1) wherein R¹ = CH₃, R² = H, R³ = CH₂CH₂CH₃, R⁴ = CH₂CH₃, R⁵ = methylsulfonamino)

To a stirred solution of 2-(5-amino-2-ethoxyphenyl)-5-methyl-7-*n*-propyl-3,5-dihydro-4*H*-pyrrolo[3,2-*d*]pyri midin-4-one (232 mg, 0. 71 mmol) and triethylamine (0. 20 mL, 1. 42 mmol) in anhydrous CH₂Cl₂ (7 mL) was added methanesulfonyl chloride (0. 13 mL, 1. 62 mmol) at 0 °C, and the mixture was stirred at 0 °C for 2 h. The reaction mixture was evaporated to dryness under reduced pressure, and the resulting residue was dissolved in MeOH (20 mL) and THF (5 mL). The pH of the mixture was adjusted to about 11 using 1 N NaOH aqueous solution, after which it was stirred at room temperature for 30 min. The mixture was acidified to pH 5-6 using 1 N HCl aqueous solution, and was extracted with 10% MeOH in CHCl₃ (100 mL × 5). The combined organic layer was evaporated to dryness under reduced pressure, and the resulting yellow residue was purified by MPLC on silica gel (gradient elution: 2% MeOH in CHCl₃ followed by 3% MeOH in CHCl₃) to afford the titled compound (250 mg, 87%) as a pale yellow solid. Analytically pure compound was obtained by crystallization from CHCl₃/Et₂O.
mp 223. 5-224 °C;
IR (neat) 3287, 3122 (NH), 1657 (C=O) 1147 (SO₂) cm⁻¹;
¹H NMR (CDCl₃/TMS) δ 1. 00 (t, *J* = 7. 5 Hz, 3 H, CH₂CH₂C*H*₃), 1. 58 (t, *J* = 6. 9 Hz, 3 H, OCH₂C*H*₃), 1. 69-1. 79 (m, 2 H, CH₂C*H*₂CH₃), 2. 70 (t, *J* = 7. 5 Hz, 2 H, C*H*₂CH₂CH₃), 3. 02 (s, 3 H, CH₃SO₂), 4. 08 (s, 3 H, NCH₃), 4. 26 (q, *J* = 6. 9 Hz, 2 H, OC*H*₂CH₃), 6. 63 (br s, 1 H, SO₂NH), 6. 87 (s, 1 H, H-2), 7. 02 (d, *J* = 9. 0 Hz, 1 H, H-3'), 7. 47 (dd, *J* = 9. 0 Hz, 3. 0 Hz, 1 H, H-4'), 8. 25 (d, *J* = 3. 0 Hz, 1 H, H-6'), 10. 87 (br s, 1 H, 6-NH); MS (FAB) *m*/*z* 405 (MH⁺).

### Example 143

### Preparation of 2-(5-methylsulfonamino-2-n-propoxyphenyl)-5-methyl-7-n-propyl-3,5-dihydro-4H-py rrolo[3,2-d]pyrimidin-4-one ((a compound of the formula (1) wherein R¹ = CH₃, R² = H, R³ = CH₂CH₂CH₃, R⁴ = CH₂CH₂CH₃, R⁵ = methylsulfonamino)

The titled compound was prepared as described in Example 144 by using 2-(5-amino-2-*n*-propoxyphenyl)-5-methyl-7-*n*-propyl-3,5-dihydro-4*H*-pyrrolo[3,2-*d*]p yrimidin-4-one in place of 2-(5-amino-2-ethoxyphenyl)-5-methyl-7-*n*-propyl-3,5-dihydro-4*H*-pyrrolo[3,2-*d*]pyri midin-4-one.
yield: 54%
mp 219-219. 5 °C (CHCl₃/Et₂O);
IR (neat) 3310, 3231 (NH), 1690 (C=O) 1155 (SO₂) cm⁻¹;
¹H NMR (CDCl₃/TMS) δ 1. 01 (t, *J* = 7. 5 Hz, 3 H, CH₂CH₂C*H*₃), 1. 16(t, *J* = 7. 5 Hz, 3 H, OCH₂CH₂C*H*₃), 1. 67-1. 80 (m, 2 H, CH₂C*H*₂CH₃), 1. 94-2. 05 (m, 2 H, OCH₂C*H*₂CH₃), 2. 71 (t, *J* = 7. 5 Hz, 2 H, C*H*₂CH₂CH₃), 3. 02(s, 3 H, CH₃SO₂), 4. 08 (s, 3 H, NCH₃), 4.16(t, *J* = 6. 3 Hz, 2 H, OC*H*₂CH₂CH₃), 6. 55(s, I H, SO₂NH), 6. 88 (s, 1 H, H-2), 7. 04 (d, *J* = 8. 7 Hz, 1 H, H-3'), 7. 48(dd, *J* = 8. 7 Hz, 3. 0 Hz, 1 H, H-4'), 8. 26 (d, *J* = 3. 0 Hz, 1 H, H-6'), 10. 92 (br s, 1 H, 6-NH); MS (FAB) *m*/*z* 419 (MH⁺).

### Example 144

| Production of tablets (Direct compression) | |
|---|---|
| | mg/tablet |
| Active ingredient | 5. 0 |
| Lactose | 14. 1 |
| Crospovidone USNF | 0. 8 |
| Magnesium Stearate | 0. 1 |
| Total weight | 20 mg |

The active ingredient was sieved and blended with the excipients. The resultant mix was compressed into tablets.

Alternatively, the active ingredient and lactose were dissolved in water and freeze-dried. Then, the dried mixture was blended with the excipients and was compressed into tablets.

### Example 145

| Production of tablets (Wet granulation) | |
|---|---|
| | mg/tablet |
| Active ingredient | 5. 0 |
| Polysorbate 80 | 0. 3 |
| Lactose | 16. 0 |
| Starch | 4.0 |
| Colloidal Silicon Dioxide | 2. 7 |
| Magnesium Stearate | 2. 0 |
| Total weight | 30 mg |

The active ingredient was sieved and blended with the lactose and starch. The polysorbate 80 was dissolved in purified water. Suitable volumes of the polysorbate 80 solution were added and the powders were granulated. After drying, the granules were screened and blended with the colloidal silicon dioxide and magnesium stearate. The granules were then compressed into tablets.

### Example 146

| Production of powder and encapsulated medicine, | |
|---|---|
| | mg/capsule |
| Active ingredient | 5. 0 |
| Lactose | 14.8 |
| Polyvinyl pyrrolidone | 10. 0 |
| Magnesium Stearate | 0. 2 |
| Total weight | 30 mg |

The active ingredient was sieved and blended with the excipients. The mix was filled into No. 5 hard gelatin capsules using suitable equipment.

### Example 147: Biological activity

The rabbit platelet PDE V was prepared using the method described by Hidaka et al. (*Biochim*. *Biophys*. *Acta*., 429: 485 - 497, 1976) with minor modification. The platelet-rich plasma (PRP) was prepared by centrifugation of freshly obtained heparinized whole blood at 360 g. Platelets were isolated from the PRP by centrifugation at 1,200 g. Platelet homogenates were prepared in the homogenization buffer (50 mM Tris-HCl buffer containing 1 mM MgCl₂, pH 7.4) by sonication for 30 s per 1 mL. The homogenized solutions were then centrifuged at 40,000 × g for 2 h at 4 °C. The supernatant was loaded on the DEAE-cellulose column (20 mL bed volume, Sigma) pre-equilibrated with equilibration buffer (50 mM Tris-acetate containing 3.75 mM 2-mercaptoethanol, pH 6.0). The column was then washed with 60 mL of equilibration buffer. The PDE isozymes were eluted using a continuous gradient of 0 to 600 mM sodium acetate in the equilibration buffer (60 mL total volume). The 1.0 mL fractions were collected. A flow rate of 60 mL/h was used throughout the ion-exchange chromatography procedure. PDE activities on cAMP and cGMP were characterized as described below. The characterized fractions were pooled and stored at -80 °C until the inhibition studies.

The cyclic nucleotide PDE V activity was determined using PDE SPA assay kit (Amersham Pharmacia biotech, UK). Each reaction mixture (100 µL total volume) consisted of the column elute containing PDE V (10 µL), [³H]-cGMP (5 µCi/mL), bovine serum albumin (0. 5 mg/mL), and MgCl₂ (5 mM) in Tris-HCl buffer (15 mM, pH 7. 5). The reactions were initiated by the addition of PDE V and the samples were incubated at 30 °C for 30 min, after which the reaction was stopped by the addition of 50 µL of SPA beads. The reaction tube was then settled for 20 min and was counted on the scintillation counter (Tri-carb 1500, Packard, USA). For the inhibition study of PDE V activity, the test compounds were dissolved in dimethyl sulfoxide (DMSO) and was diluted with distilled water. The final concentration of DMSO was less than 0. 2% (v/v). All the inhibition experiments were conducted under the conditions where the level of cGMP hydrolysis did not exceed 15%, and the product formation increased linearly with time and the amount of enzyme.

The following Table illustrates the in vitro activities for a range of the compounds of the invention as inhibitors of cGMP PDE V.

**TABLE**

| EXAMPLE NO. | IC₅₀ (nM) |
|---|---|
| 1 | 2.90 |
| 3 | 0. 59 |
| 14 | 1.36 |
| 18 | 2.23 |
| 26 | 0.62 |
| 30 | 0.18 |
| 31 | 0.33 |
| 37 | 0. 64 |
| 51 | 2.06 |
| 53 | 0.49 |
| 123 | 0.71 |
| 124 | 0.27 |

### Example 148: Safety Profile

Several compounds of the invention have been tested at doses of up to 10 mg/kg p. o. in rats with no untoward effects being observed, and up to 100 mg/kg p. o. in rats with no death being observed.

## Claims

1. A compound of the formula (1) and pharmaceutically acceptable salts and solvates (e. g. hydrates) thereof, 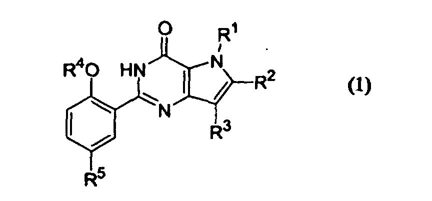 wherein R¹ is H; C₁-C₃ alkyl optionally substituted with one or more fluoro atoms; or C₃-C₆ cycloalkyl;
R² is H; a halogen atom; C₁-C₆ alkyl optionally substituted with OH, C₁-C₃ alkoxy, C₃-C₆ cycloalkyl, or with one or more fluoro atoms; C₃-C₆ cycloalkyl; C₂-C₆ alkenyl; or C₂-C₆ alkynyl;
R³ is H; C₁-C₆ alkyl optionally substituted with OH, C₁-C₃ alkoxy, C₃-C₆ cycloalkyl, or with one or more fluoro atoms; C₃-C₆ cycloalkyl; C₂-C₆ alkenyl; or C₂-C₆ alkynyl;
R⁴ is C₁-C₆ alkyl optionally substituted with C₃-C₆ cycloalkyl or with one or more fluoro atoms; C₂-C₆ alkenyl; C₂-C₆ alkynyl; or C₃-C₆ cycloalkyl;
R⁵ is SO₂NR⁶R⁷; NHSO₂NR⁶R⁷; NHCOCONR⁶R⁷; NHSO₂R⁸; NHCOR⁸; or phenyl or heterocyclyl either of which is optionally substituted with one or more fluoro atoms or C₁-C₃ alkyl;
R⁶ and R⁷ are each independently H or C₁-C₆ alkyl optionally substituted with OH, CO₂H, C₁-C₃ alkoxy, C₃-C₆ cycloalkyl, or with one or more fluoro atoms; or together with the nitrogen atom to which they are attached form either a mono-cyclic ring such as imidazole, aziridene (aziridine), azeridine (azetidine), pyrrolidine, piperidine, morpholine, piperazine and homopiperazine, or a bicyclic ring such as 2,5-diazabicyclo[2. 2. 1]heptane and 3,7-diazabicyclo[3. 3. 0]octane, wherein said group is optionally substituted with R⁹;
R⁸ is C₁-C₆ alkyl optionally substituted with one or more fluoro atoms; or C₃-C₇ cycloalkyl;
R⁹ is C₁-C₆ alkyl optionally substituted with one or more halide atoms, OH, C₁-C₃ alkoxy which is optionally substituted with one or more fluoro atoms, CO₂R¹⁰, NR¹¹R¹², C=NR¹³(NR¹⁴R¹⁵), or with a tetrazole group which is optionally substituted with C₁-C₃ alkyl; or one or more nitrogen containing heteroaryl group which is optionally substituted with one or more fluoro atoms;
R¹⁰ is H; or C₁-C₄ alkyl optionally substituted with OH, NR¹¹R¹², one or more fluoro atoms, or with a nitrogen containing heterocyclic ring such as pyrrolidine, piperidine, piperazine, morpholine, pyrrole, and imidazole wherein nitrogen atom is directly bound to C₁-C₄ alkyl;
R¹¹ and R¹² are each independently H or C₁-C₄ alkyl;
R¹³ is H; C₁-C₄ alkyl optionally substituted with one or more fluoro atoms; or C₃-C₆ cycloalkyl;
R¹⁴ and R¹⁵ are each independently H or C₁-C₄ alkyl optionally substituted with one or more fluoro atoms; C₃-C₆ cycloalkyl; or together with the nitrogen atom to which they are attached form a pyrrolidinyl, piperidino, morpholino, piperazinyl, or homopiperazinyl group wherein said group is optionally substituted with C₁-C₃ alkyl.

2. The compound according to Claim 1, wherein
R¹ is H; methyl; or ethyl;
R² is H; methyl; or a halogen atom;
R³ is C₁-C₄ alkyl optionally substituted with one or more fluoro atoms;
R⁴ is ethyl; *n*-propyl; or allyl;
R⁵ is SO₂NR⁶R⁷; or NHSO₂R⁸;
R⁶ and R⁷ taken together with the nitrogen atom to which they are attached form a piperidino, piperazinyl or homopiperazinyl group wherein said group is substituted with R⁹;
R⁸ is methyl;
R⁹ is C₁-C₄ alkyl optionally substituted with one or more halide atoms, OH, CO₂R¹⁰, or with a tetrazole group which is optionally substituted with C₁-C₃ alkyl; and
R¹⁰ is H.

3. The compound according to Claim 2, wherein
R¹ is methyl; or ethyl;
R² is H;
R³ is ethyl; *n*-propyl; 3-fluoropropyl; or cyclopropylmethyl;
R⁴ is ethyl; or *n*-propyl;
R⁵ is SO₂NR⁶R⁷; or NHSO₂R⁸;
R⁶ and R⁷ taken together with the nitrogen atom to which they are attached form a piperidino or piperazinyl group wherein said group is substituted with R⁹;
R⁸ is methyl;
R⁹ is C₁-C₄ alkyl optionally substituted with one or more fluoro or chloro atoms, OH, CO₂R¹⁰, or with a tetrazole group; and
R¹⁰ is H.

4. The compound according to Claim 3, wherein said compound is selected from the group consisting of:
2-(2-ethoxy-5-(4-methylpiperazin-1-ylsulfonyl)phenyl)-5-methyl-7*-n*-propyl-3,5-d ihydro-4*H*-pyrrolo[3,2-*d*]pyrimidin-4-one;
2-(5-(4-methylpiperazin-1-ylsulfonyl)-2-*n*-propoxyphenyl)-5-methyl-7-*n*-propyl-3, 5-dihydro-4*H*-pyrrolo[3,2-*d*]pyrimidin-4-one;
2-(2-ethoxy-5-(4-*n*-propylpiperazin-1-ylsulfonyl)phenyl)-5-methyl-7-*n*-propyl-3,5-dihydro-4*H*-pyrrolo[3,2-*d*]pyrimidin-4-one;
2-(2-ethoxy-5-(4-isopropylpiperazin-1-ylsulfonyl)phenyl)-5-methyl-7-*n*-propyl-3,5 -dihydro-4*H*-pyrrolo[3,2-*d*]pyrimidin-4-one;
2-(2-ethoxy-5-(4-(2-fluoroethyl)piperazin-1-ylsulfonyl)phenyl)-5-methyl-7-*n*-prop yl-3,5-dihydro-4*H*-pyrrolo[3,2-*d*]pyrimidin-4-one;
2-(5-(4-(2-fluoroethyl)piperazin-1-ylsulfonyl)-2-*n*-propoxyphenyl)-5-methyl-7-*n*-p ropyl-3,5-dihydro-4*H*-pyrrolo[3,2-*d*]pyrimidin-4-one;
2-(2-ethoxy-5-(4-(3-fluoropropyl)piperazin-1-ylsulfonyl)phenyl)-5-methyl-7-*n*-pro pyl-3,5-dihydro-4*H*-pyrrolo[3,2-*d*]pyrimidin-4-one;
2-(2-ethoxy-5-(4-(3-fluoropropyl)piperazin-1-ylsulfonyl)phenyl)-7-ethyl-5-methyl -3,5-dihydro-4*H*-pyrrolo[3,2-*d*]pyrimidin-4-one;
2-(2-ethoxy-5-(4-(3-fluoropropyl)piperazin-1-ylsulfonyl)phenyl)-7-(3-fluoropropyl)-5-methyl-3,5-dihydro-4*H*-pyrrolo[3,2-*d*]pyrimidin-4-one;
7-cyclopropylmethyl-2-(2-ethoxy-5-(4-(3-fluoropropyl)piperazin-1-ylsulfonyl)phenyl)-5-methyl-3,5-dihydro-4*H*-pyrrolo[3,2-*d*]pyrimidin-4-one;
2-(2-ethoxy-5-(4-(3-fluoropropyl)piperazin-1-ylsulfonyl)phenyl)-5-ethyl-7-ethyl-3,5-d ihydro-4*H*-pyrrolo[3,2-*d*]pyrimidin-4-one;
2-(2-ethoxy-5-(4-(3-fluoropropyl)piperazin-1-ylsulfonyl)phenyl)-5-ethyl-7-*n*-propyl-3, 5-dihydro-4*H*-pyrrolo[3,2-*d*]pyrimidin-4-one;
2-(2-ethoxy-5-(4-isopropylpiperazin-1-ylsulfonyl)phenyl)-5-ethyl-7-*n*-propyl-3,5-dihy dro-4*H*-pyrrolo[3,2-*d*]pyrimidin-4-one;
2-(2-ethoxy-5-(4-(3-fluoropropyl)piperazin-1-ylsulfonyl)phenyl)-5-ethyl-7-*n*-propyl-3, 5-dihydro-4*H*-pyrrolo[3,2-*d*]pyrimidin-4-one;
2-(2-ethoxy-5-(4-(2-hydroxyethyl)piperazin-1-ylsulfonyl)phenyl)-5-ethyl-7-*n*-propyl-3 ,5-dihydro-4*H*-pyrrolo[3,2-*d*]pyrimidin-4-one;
2-(2-ethoxy-5-(4-ethylpiperazin-1-ylsulfonyl)phenyl)-5-ethyl-7-(3-fluoropropyl)-3,5-d ihydro-4*H*-pyrrolo[3,2-*d*]pyrimidin-4-one;
2-(5-(4-ethylpiperazin-1-ylsulfonyl)-2-*n*-propoxyphenyl)-5-ethyl-7-(3-fluoropropyl)-3, 5-dihydro-4*H*-pyrrolo[3,2-*d*]pyrimidin-4-one;
2-(2-ethoxy-5-(4-isopropylpiperazin-1-ylsulfonyl)phenyl)-5-ethyl-7-(3-fluoropropyl)-3,5-dihydro-4*H*-pyrrolo[3,2-*d*]pyrimidin-4-one;
2-(2-ethoxy-5-(4-(3-fluoropropyl)piperazin-1-ylsulfonyl)phenyl)-5-ethyl-7-(3-fluoropr opyl)-3,5-dihydro-4*H*-pyrrolo[3,2-*d*]pyrimidin-4-one;
2-(5-(4-(3-fluoropropyl)piperazin-1-ylsulfonyl)-2-*n*-propoxyphenyl)-5-ethyl-7-(3-fluor opropyl)-3,5-dihydro-4*H*-pyrrolo[3,2-*d*]pyrimidin-4-one;
2-(2-ethoxy-5-(4-(2-hydroxyethyl)piperazin-1-ylsulfonyl)phenyl)-5-ethyl-7-(3-fluorop ropyl)-3,5-dihydro-4*H*-pyrrolo[3,2-*d*]pyrimidin-4-one;
2-(5-(4-(2-hydroxyethyl)piperazin-1-ylsulfonyl)-2-*n*-propoxyphenyl)-5-ethyl-7-(3-fluo ropropyl)-3,5-dihydro-4*H*-pyrrolo[3,2-*d*]pyrimidin-4-one;
2-(5-(4-(3-hydroxypropyl)piperazin-1-ylsulfonyl)-2-*n*-propoxyphenyl)-5-ethyl-7-(3-fluoropropyl)-3,5-dihydro-4*H*-pyrrolo[3,2-*d*]pyrimidin-4-one;
2-(5-(4-(2-hydroxyethyl)piperazin-1-ylsulfonyl)-2-*n*-propoxyphenyl)-5-ethyl-7-*n*-p ropyl-3,5-dihydro-4*H*-pyrrolo[3,2-*d*]pyrimidin-4-one;
2-(5-(4-(3-fluoropropyl)piperazin-1-ylsulfonyl)-2-*n*-propoxyphenyl)-5-methyl-7-*n*-propyl-3,5-dihydro-4*H*-pyrrolo[3,2-*d*]pyrimidin-4-one;
2-(2-ethoxy-5-(4-(2-hydroxyethyl)piperazin-1-ylsulfonyl)phenyl)-5-methyl-7-*n*-pr opyl-3,5-dihydro-4*H*-pyrrolo[3,2-*d*]pyrimidin-4-one;
2-(5-(4-(2-hydroxyethyl)piperazin-1-ylsulfonyl)-2-*n*-propoxyphenyl)-5-methyl-7-*n* -propyl-3,5-dihydro-4*H*-pyrrolo[3,2-*d*]pyrimidin-4-one;
2-(5-(2-ethoxy-4-(3-hydroxypropyl)piperazin-1-ylsulfonyl)phenyl)-5-methyl-7-*n*-p ropyl-3,5-dihydro-4*H*-pyrrolo[3,2-*d*]pyrimidin-4-one;
2-(5-(4-(3-hydroxypropyl)piperazin-1-ylsulfonyl)-2-*n*-propoxyphenyl)-5-methyl-7-*n*-propyl-3,5-dihydro-4*H*-pyrrolo[3,2-*d*]pyrimidin-4-one;
2-(2-ethoxy-5-(4-(hydroxycarbonylmethyl)piperidin-1-ylsulfonyl)phenyl)-5-methy 1-7-*n*-propyl-3,5-dihydro-4*H*-pyrrolo[3,2-*d*]pyrimidin-4-one;
2-(5-(4-(hydroxycarbonylmethyl)piperidin-1-ylsulfonyl)-2-*n*-propoxyphenyl)-5-me thyl-7-*n*-propyl-3,5-dihydro-4*H*-pyrrolo[3,2-*d*]pyrimidin-4-one;
2-(2-ethoxy-5-(4-(2-hydroxycarbonylethyl)piperidin-1-ylsulfonyl)phenyl)-5-methy 1-7-*n*-propyl-3,5-dihydro-4*H*-pyrrolo[3,2-*d*]pyrimidin-4-one;
2-(5-(4-(2-hydroxycarbonylethyl)piperidin-1-ylsulfonyl)-2-*n*-propoxyphenyl)-5-m ethyl-7-*n*-propyl-3,5-dihydro-4*H*-pyrrolo[3,2-*d*]pyrimidin-4-one;
2-(2-ethoxy-5-(4-(1*H*-tetrazol-5-ylmethyl)piperazin-1-ylsulfonyl)phenyl)-5-methyl -7-*n*-propyl-3,5-dihydro-4*H*-pyrrolo[3,2-*d*]pyrimidin-4-one;
5-methyl-2-(2-*n*-propoxy-5-(4-(2-(1*H*-tetrazol-5-yl)ethyl)piperazin-1-ylsulfonyl)p henyl)-7-*n*-propyl-3,5-dihydro-4*H*-pyrrolo[3,2-*d*]pyrimidin-4-one;
2-(2-ethoxy-5-(4-(1*H*-tetrazol-5-ylmethyl)piperidin-1-ylsulfonyl)phenyl)-5-methyl -7-*n*-propyl-3,5-dihydro-4*H*-pyrrolo[3,2-*d*]pyrimidin-4-one;
5-methyl-2-(2-*n*-propoxy-5-(4-(1*H*-tetrazol-5-ylmethyl)piperidin-1-ylsulfonyl)phe nyl)-7-*n*-propyl-3,5-dihydro-4*H*-pyrrolo[3,2-*d*]pyrimidin-4-one;
and physiologically acceptable salts and solvates (e. g. hydrates) thereof.

5. A pharmaceutical composition comprising a compound of the formula (**1**) or a pharmaceutically acceptable salt, or a solvate thereof according to any one of Claims 1 to 4, together with a pharmaceutically acceptable diluent or carrier therefor.

6. A compound of the formula **(1)** or a pharmaceutically acceptable salt, or a solvate thereof, or a pharmaceutical composition containing either entity according to any one of Claims 1 to 5, for use in the treatment of impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases **characterized by** disorders of gut motility (e. g. irritable bowel syndrome).

7. The use of a compound of the formula **(1)** or a pharmaceutically acceptable salt, or a solvate thereof, or a pharmaceutical composition containing either entity according to any one of Claims 1 to 5, for the manufacture of a medicament for the treatment of impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases **characterized by** disorders of gut motility (e. g. irritable bowel syndrome).

8. A compound of the formula **(2), (3)** and **(9):** 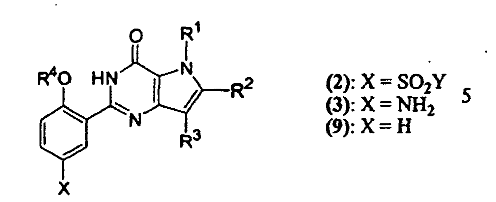 wherein R¹, R², R³ and R⁴ are as previously defined in Claim 1 and Y is a chloro, bromo or fluoro atom.

9. A compound of the formula (**12**) and (**13**): 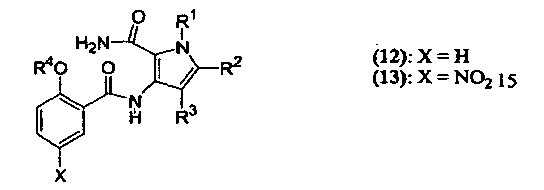 wherein R¹, R², R³ and R⁴ are as previously defined in Claim 1.

10. A compound of the formula (**15**): 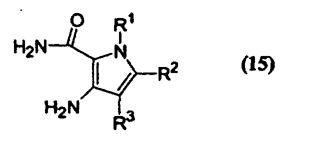 wherein R¹, R² and R³ are as previously defined in Claim 1.

11. Processes for preparing a compound of the formula (1): 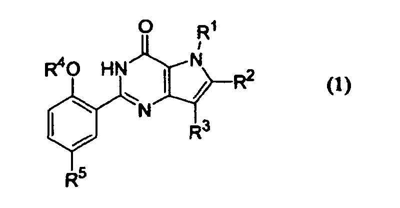 wherein R¹, R², R³, R⁴ and R⁵ are as previously defined in Claim 1, and pharmaceutically acceptable salts thereof, (a) which comprises reacting a compound of the formula (2)-(4): 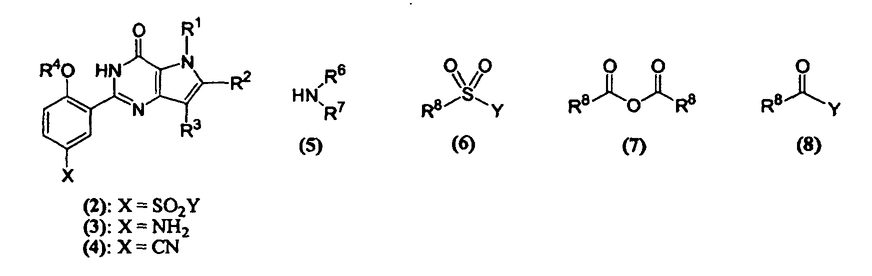 wherein R¹, R² R³ and R⁴ are as previously defined in Claim 1 and Y represents a halogen atom, with an appropriate compound of the formula (**5**) -(**8**) under an appropriate reaction condition, wherein R⁶, R⁷ and R⁸ are as previously defined in Claim 1, or (b) which comprises cyclizing a compound of the formula (**25**): 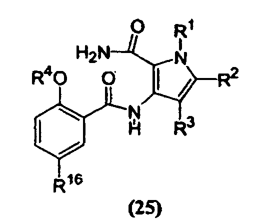 wherein R¹, R², R³ and R⁴ are as previously defined in Claim 1 and R¹⁶ is a group R⁵ as previously defined in Claim 1 or a precursor to a group R⁵ thereof.

12. Processes for preparing a compound of the formula **(2), (3)** and **(9):** 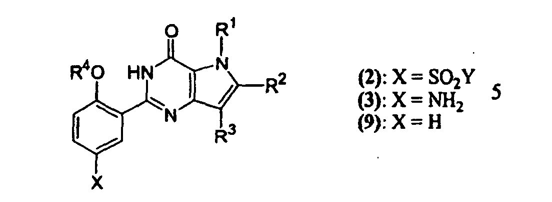 wherein R¹, R², R³ and R⁴ are as previously defined in Claim 1 and Y is a chloro, bromo or fluoro atom, which comprises cyclizing a compound of the formula **(12)** or (13) followed by an appropriate transformation: 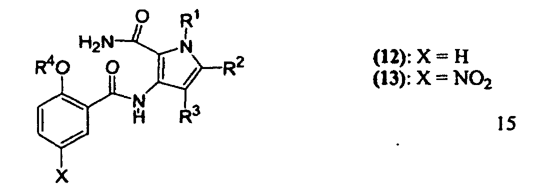 wherein R¹, R², R³ and R⁴ are as previously defined in Claim 1.

13. Processes for preparing a compound of the formula **(12)** and **(13):** 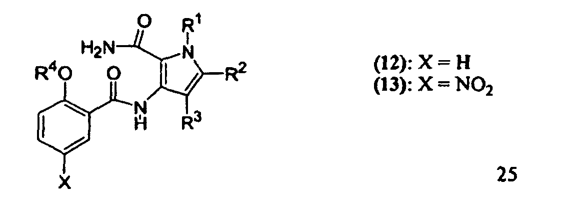 wherein R¹, R², R³ and R⁴ are as previously defined in Claim 1, which comprises reacting a compound of the formula (**15**) with a compound of the formula (**16**) or (**17**): 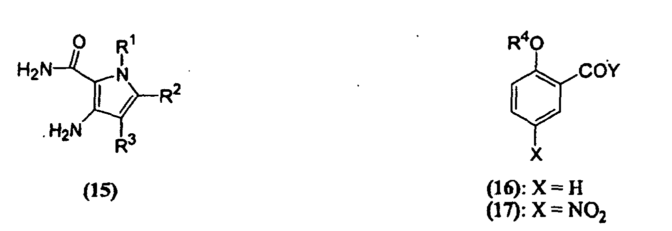 wherein R¹, R², R³ and R⁴ are as previously defined in Claim 1 and Y is a chloro, bromo or fluoro atom.

14. Processes for preparing a compound of the formula **(15):** 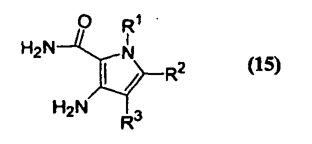 wherein R¹, R² and R³ are as previously defined in Claim 1, which comprises cyclizing a compound of the formula **(19):** 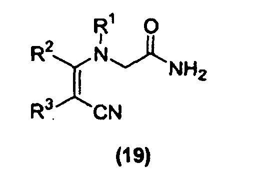 wherein R¹, R² and R³ are as previously defined in Claim 1.

## Patentansprüche

1. Verbindung der Formel (1) und pharmazeutisch verträgliche Salze und Solvate (z.B. Hydrate) davon, 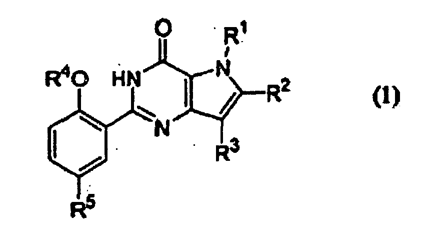 wobei
R¹ H, C₁-C₃-Alkyl, wahlweise substituiert mit einem oder mehreren Fluoratomen, oder C₃-C₆-Cycloalkyl ist,
R² H, ein Halogenatom, C₁-C₆-Alkyl, Wahlweise substituiert mit OH, C₁-C₃-Alkoxy, C₃-C₆-Cycloalkyl oder mit einem oder mehreren Fluoratomen, C₃-C₆-Cycloalkyl, C₂-C₆-Alkenyl oder C₂-C₆-Alkinyl ist,
R³ H, C₁-C₆-Alkyl, wahlweise substituiert mit OH, C₁-C₃-Alkoxy, C₃-C₆-Cycloalkyl oder mit einem oder mehreren Fluoratomen, C₃-C₆-Cycloalkyl, C₂-C₆-Alkenyl oder C₂-C₆-Alkinyl ist,
R⁴ C₁-C₆-Alkyl, wahlweise substituiert mit C₃-C₆-Cycloalkyl oder mit einem oder mehreren Fluoratomen, C₂-C₆-Alkenyl, C₂-C₆-Alkinyl oder C₃-C₆-Cycloalkyl ist,
R⁵ SO₂NR⁶R⁷, NHSO₂NR⁶R⁷, NHCOCONR⁶R⁷, NHSO₂R⁸, NHCOR⁸ oder Phenyl oder Heterocyclyl, von denen jedes wahlweise mit einem oder mehreren Fluoratomen oder C₁-C₃-Alkyl substituiert ist, ist,
R⁶ und R⁷ unabhängig voneinander H oder C₁-C₆-Alkyl, wahlweise substituiert mit OH, CO₂H, C₁-C₃-Alkoxy, C₃-C₆-Cycloalkyl oder mit einem oder mehreren Fluoratomen, sind oder zusammen mit dem Stickstoffatom, an welches sie gebunden sind, entweder einen monozyklischen Ring, wie Imidazol, Aziriden (Aziridin), Azeridin (Azetidin), Pyrrolidin, Piperidin, Morpholin, Piperazin und Homopiperazin, oder einen bizyklischen Ring, wie 2,5-Diazabicyclo-[2.2.1]-heptan und 3,7-Diazabicyclo-[3.3.0]-octan bilden, wobei die Gruppe wahlweise mit R⁹ substituiert ist,
R⁸ C₁-C₆-Alkyl, wahlweise substituiert mit einem oder mehreren Fluoratomen, oder C₃-C₇-Cycloalkyl ist,
R⁹ C₁-C₆-Alkyl, wahlweise substituiert mit einem oder mehreren Halogenatomen, OH, C₁-C₃-Alkoxy, welches wahlweise mit einem oder mehreren Fluoratomen, CO₂R¹⁰, NR¹¹R¹², C=NR¹³(NR¹⁴R¹⁵) oder mit einer Tetrazolgruppe, welche wahlweise mit C₁-C₃-Alkyl substituiert ist, substituiert ist, oder eine Heteroarylgruppe, die einen oder mehrere Stickstoffe enthält und welche wahlweise mit einem oder mehreren Fluoratomen substituiert ist, ist,
R¹⁰ H oder C₁-C₄-Alkyl, wahlweise substituiert mit OH, NR¹¹R¹², einem oder mehreren Fluoratomen oder mit einem stickstoffhaltigen heterozyklischen Ring, wie Pyrrolidin, Piperidin, Piperazin, Morpholin, Pyrrol und Imidazol, worin ein Stickstoffatom direkt an C₁-C₄-Alkyl gebunden ist, ist,
R¹¹ und R¹² unabhängig voneinander H oder C₁-C₄-Alkyl sind,
R¹³ H, C₁-C₄-Alkyl, wahlweise substituiert mit einem oder mehreren Fluoratomen, oder C₃-C₆-Cycloalkyl ist;
R¹⁴ und R¹⁵ unabhängig voneinander H oder C₁-C₄-Alkyl, wahlweise substituiert mit einem oder mehreren Fluoratomen, C₃-C₆-Cycloalkyl sind oder zusammen mit dem Stickstoffatom, an welches sie gebunden sind, eine Pyrrolidinyl-, Piperidino-, Morpholino-, Piperazinyl- oder Homopiperazinylgruppe bilden, wobei die Gruppe wahlweise mit C₁-C₄-Alkyl substituiert ist.

2. Verbindung nach Anspruch 1, wobei
R¹ H, Methyl oder Ethyl ist,
R² H, Methyl oder ein Halogenatom ist,
R³ C₁-C₄-Alkyl, wahlweise substituiert mit einem oder mehreren Fluoratomen, ist,
R⁴ Ethyl, n-Propyl oder Allyl ist,
R⁵ SO₂NR⁶R⁷ oder NHSO₂R⁸ ist,
R⁶ und R⁷ zusammengenommen mit dem Stickstoffatom, an welches sie gebunden sind, eine Piperidino-, Piperazinyl- oder Homopiperazinylgruppe bilden, wobei die Gruppe mit R⁹ substituiert ist,
R⁸ Methyl ist,
R⁹ C₁-C₄-Alkyl, wahlweise substituiert mit einem oder mehreren Halogenatomen, OH, CO₂R¹⁰ oder mit einer Tetrazolgruppe, welche wahlweise mit C₁-C₃-Alkyl substituiert ist, ist, und
R¹⁰ H ist.

3. Verbindung nach Anspruch 2, wobei
R¹ Methyl oder Ethyl ist,
R² H ist,
R³ Ethyl, n-Propyl, 3-Fluoropropyl oder Cyclopropylmethyl ist,
R⁴ Ethyl oder n-Propyl ist,
R⁵ SO₂NR⁶R⁷ oder NHSO₂R⁸ ist,
R⁶ und R⁷ zusammengenommen mit dem Stickstoffatom, an welches sie gebunden sind, eine Piperidino- oder Piperazinylgruppe bilden, wobei die Gruppe mit R⁹ substituiert ist,
R⁸ Methyl ist,
R⁹ C₁-C₄-Alkyl, wahlweise substituiert mit einem oder mehreren Fluor- oder Chloratomen, OH, CO₂R¹⁰ oder mit einer Tetrazolgruppe, ist, und
R¹⁰ H ist.

4. Verbindung nach Anspruch 3, wobei die Verbindung aus der Gruppe ausgewählt ist, bestehend aus:
2-(2-Ethoxy-5-(4-methylpiperazin-1-ylsulfonyl)-phenyl)-5-methyl-7-n-propyl-3,5-dihydro-4Hpyrrolo-[3,2-d]-pyrimidin-4-on,
2-(5-(4-Methylpiperazin-1-ylsulfonyl)-2-n-propoxyphenyl)-5-methyl-7-n-propyl-3,5-dihydro-4H-pyrrolo-[3,2-d]-pyrimidin-4-on,
2-(2-Ethoxy-5-(4-n-propylpiperazin-1-ylsulfonyl)-phenyl)-5-methyl-7-n-propyl-3,5-dihydro-4Hpyrrolo-[3,2-d]-pyrimidin-4-on,
2-(2-Ethoxy-5-(4-isopropylpiperazin-1-ylsulfonyl)-phenyl)-5-methyl-7-n-propyl-3,5-dihydro-4H-pyrrolo-[3,2-d]-pyrimidin-4-on,
2-(2-Ethoxy-5-(4-(2-fluoroethyl)-piperazin-1-ylsulfonyl)-phenyl)-5-methyl-7-n-propyl-3,5-dihydro-4H-pyrrolo-[3,2-d]-pyrimidin-4-on,
2-(5-(4-(2-Fluoroethyl)-piperazin-1-ylsulfonyl)-2-n-propoxyphenyl)-5-methyl-7-n-propyl-3,5-dihydro-4H-pyrrolo-[3,2-d]-pyrimidin-4-on,
2-(2-Ethoxy-5-(4-(3-fluoropropyl)-piperazin-1-ylsulfonyl)-phenyl)-5-methyl-7-n-propyl-3,5-dihydro-4H-pyrrolo-[3,2-d]-pyrimidin-4-on,
2-(2-Ethoxy-5-(4-(3-fluoropropyl)-piperazin-1-ylsulfonyl)-phenyl)-7-ethyl-5-methyl-3,5-dihydro-4H-pyrrolo-[3,2-d]-pyrimidin-4-on,
2-(2-Ethoxy-5-(4-(3-fluoropropyl)-piperazin-1-ylsulfonyl)-phenyl)-7-(3-fluoropropyl)-5-methyl-3,5-dihydro-4H-pyrrolo-[3,2-d]-pyrimidin-4-on,
7-Cyclopropylmethyl-2-(2-ethoxy-5-(4-(3-fluoropropyl)-piperazin-1-ylsulfonyl)-phenyl)-5-methyl-3,5-dihydro-4H-pyrrolo-[3,2-d]-pyrimidin-4-on,
2-(2-Ethoxy-5-(4-(3-fluoropropyl)-piperazin-1-ylsulfonyl)-phenyl)-5-ethyl-7-ethyl-3,5-dihydro-4H-pyrrolo-[3,2-d]-pyrimidin-4-on,
2-(2-Ethoxy-5-(4-(3-fluoropropyl)-piperazin-1-ylsulfonyl)-phenyl)-5-ethyl-7-n-propyl-3,5-dihydro-4H-pyrrolo-[3,2-d]-pyrimidin-4-on,
2-(2-Ethoxy-5-(4-isopropylpiperazin-1-ylsulfonyl)-phenyl)-5-ethyl-7-n-propyl-3,5-dihydro-4Hpyrrolo-[3,2-d]-pyrimidin-4-on,
2-(2-Ethoxy-5-(4-(3-fluoropropyl)-piperazin-1-ylsulfonyl)-phenyl)-5-ethyl-7-n-propyl-3,5-dihydro-4H-pyrrolo-[3,2-d]-pyrimidin-4-on,
2-(2-Ethoxy-5-(4-(2-hydroxyethyl)-piperazin-1-ylsulfonyl)-phenyl)-5-ethyl-7-n-propyl-3,5-dihydro-4H-pyrrolo-[3,2-d]-pyrimidin-4-on,
2-(2-Ethoxy-5-(4-ethylpiperazin-1-ylsulfonyl)-phenyl)-5-ethyl-7-(3-fluoropropyl)-3,5-dihydro-4H-pyrrolo-[3,2-d]-pyrimidin-4-on,
2-(5-(4-Ethylpiperazin-1-ylsulfonyl)-2-n-propoxyphenyl)-5-ethyl-7-(3-fluoropropyl)-3,5-dihydro-4H-pyrrolo-[3,2-d]-pyrimidin-4-on,
2-(2-Ethoxy-5-(4-isopropylpiperazin-1-ylsulfonyl)-phenyl)-5-ethyl-7-(3-fluoropropyl)-3,5-dihydro-4H-pyrrolo-[3,2-d]-pyrimidin-4-on,
2-(2-Ethoxy-5-(4-(3-fluoropropyl)-piperazin-1-ylsulfonyl)-phenyl)-5-ethyl-7-(3-fluororpropyl)-3,5-dihydro-4H-pyrrolo-[3,2-d]-pyrimidin-4-on,
2-(5-(4-(3-Fluoropropyl)-piperazin-1-ylsulfonyl)-2-n-propoxyphenyl)-5-ethyl-7-(3-fluoropropyl)-3,5-dihydro-4H-pyrrolo-[3,2-d]-pyrimidin-4-on,
2-(2-Ethoxy-5-(4-(2-hydroxyethyl)-piperazin-1-ylsulfonyl)-phenyl)-5-ethyl-7-(3-fluoropropyl)-3,5-dihydro-4H-pyrrolo-[3,2-d]-pyrimidin-4-on,
2-(5-(4-(2-Hydroxyethyl)-piperazin-1-ylsulfonyl)-2-n-propoxyphenyl)-5-ethyl-7-(3-fluoropropyl)-3,5-dihydro-4H-pyrrolo-[3,2-d]-pyrimidin-4-on,
2-(5-(4-(3-Hydroxypropyl)-piperazin-1-ylsulfonyl)-2-n-propoxyphenyl)-5-ethyl-7-(3-fluoropropyl)-3,5-dihydro-4H-pyrrolo-[3,2-d]-pyrimidin-4-on,
2-(5-(4-(2-Hydroxyethyl)-piperazin-1-ylsulfonyl)-2-n-propoxyphenyl)-5-ethyl-7-n-propyl-3,5-dihydro-4H-pyrrolo-[3,2-d]-pyrimidin-4-on,
2-(5-(4-(3-Fluoropropyl)-piperazin-1-ylsulfonyl)-2-n-propoxyphenyl)-5-methyl-7-n-propyl-3,5-dihydro-4H-pyrrolo-[3,2-d]-pyrimidin-4-on,
2-(2-Ethoxy-5-(4-(2-hydroxyethyl)-piperazin-1-ylsulfonyl)-phenyl)-5-methyl-7-n-propyl-3,5-dihydro-4H-pyrrolo-[3,2-d]-pyrimidin-4-on,
2-(5-(4-(2-Hydroxyethyl)-piperazin-1-ylsulfonyl)-2-n-propoxyphenyl)-5-methyl-7-n-propyl-3,5-dihydro-4H-pyrrolo-[3,2-d]-pyrimidin-4-on,
2-(5-(2-Ethoxy-4-(3-hydroxypropyl)-piperazin-1-ylsulfonyl)-phenyl)-5-methyl-7-n-propyl-3,5-dihydro-4H-pyrrolo-[3,2-d]-pyrimidin-4-on,
2-(5-(4-(3-Hydroxypropyl)-piperazin-1-ylsulfonyl)-2-n-propoxyphenyl)-5-methyl-7-n-propyl-3,5-dihydro-4H-pyrrolo-[3,2-d]-pyrimidin-4-on,
2-(2-Ethoxy-5-(4-hydroxycarbonylmethyl)-piperidin-1-ylsulfonyl)-phenyl)-5-methyl-7-n-propyl-3,5-dihydro-4H-pyrrolo-[3,2-d]-pyrimidin-4-on,
2-(5-(4-Hydroxycarbonylmethyl)-piperidin-1-ylsulfonyl)-2-n-propoxyphenyl)-5-methyl-7-npropyl-3,5-dihydro-4H-pyrrolo-[3,2-d]-pyrimidin-4-on,
2-(2-Ethoxy-5-(4-(2-hydroxycarbonylethyl)-piperidin-1-ylsulfonyl)-phenyl)-5-methyl-7-npropyl-3,5-dihydro-4H-pyrrolo-[3,2-d]-pyrimidin-4-on,
2-(5-(4-(2-Hydroxycarbonylethyl)-piperidin-1-ylsulfonyl)-2-n-propoxyphenyl)-5-methyl-7-npropyl-3,5-dihydro-4H-pyrrolo-[3,2-d]-pyrimidin-4-on,
2-(2-Ethoxy-5-(4-(1H-tetrazol-5-ylmethyl)-piperazin-1-ylsulfonyl)-phenyl)-5-methyl-7-n-propyl-3,5-dihydro-4H-pyrrolo-[3,2-d]-pyrimidin-4-on,
5-Methyl-(2-(2-n-propoxy-5-(4-(2-(1H-tetrazol-5-yl)-ethyl)-piperazin-1-ylsulfonyl)-phenyl)-7-npropyl-3,5-dihydro-4H-pyrrolo-[3,2-d]-pyrimidin-4-on,
2-(2-Ethoxy-5-(4-(1H-tetrazol-5-ylmethyl)-piperidin-1-ylsulfonyl)-phenyl)-5-methyl-7-n-propyl-3,5-dihydro-4H-pyrrolo-[3,2-d]-pyrimidin-4-on,
5-Methyl-2-(2-n-propoxy-5-(4-(1H-tetrazol-5-ylmethyl)-piperidin-1-ylsulfonyl)-phenyl)-7-npropyl-3,5-dihydro-4H-pyrrolo-[3,2-d]-pyrimidin-4-on,
und pharmazeutisch verträglichen Salzen und Solvaten (z.B. Hydraten) davon.

5. Pharmazeutische Zusammensetzung mit einer Verbindung der Formel (1) oder einem pharmazeutisch verträglichen Salz oder einem Solvat davon nach einem der Ansprüche 1 bis 4 zusammen mit einem pharmazeutisch verträglichen Verdünnungsmittel oder Träger dafür.

6. Verbindung der Formel (1) oder pharmazeutisch verträgliches Salz oder Solvat davon oder pharmazeutische Zusammensetzung, die einen Gegenstand nach einem der Ansprüche 1 bis 5 enthält, zur Verwendung bei der Behandlung von Impotenz, sexueller Störung bei der Frau, stabiler, instabiler und varianter (Prinzmetal) Angina, Bluthochdruck, Lungenhochdruck, Stauungsinsuffizienz des Herzens, Nierenversagen, Arteriosklerose, Zuständen verminderter Blutgefäßdurchgängigkeit (z.B. postperkutane transluminale Koronarangioplastie), Kranzgefäßerkrankung, Gefäßstörungen, wie Raynaud-Syndrom, inflammatorische Erkrankungen, Schlaganfall, Bronchitis, chronischem Asthma, allergischem Asthma, allergischer Rhinitis, Glaukom und Erkrankungen, die durch Störungen der Eingeweidebeweglichkeit gekennzeichnet sind (z.B. reizbares Darmsyndrom).

7. Verwendung einer Verbindung der Formel (1) oder eines pharmazeutisch verträglichen Salzes oder eines Solvates davon oder einer pharmazeutischen Zusammensetzung, die einen Gegenstand nach einem der Ansprüche 1 bis 5 enthält, zur Herstellung eines Medikaments für die Behandlung von Impotenz, sexueller Störung bei der Frau, stabiler, instabiler und varianter (Prinzmetal) Angina, Bluthochdruck, Lungenhochdruck, Stauungsinsuffizienz des Herzens, Nierenversagen, Arteriosklerose, Zuständen verminderter Blutgefäßdurchgängigkeit (z.B. postperkutane transluminale Koronarangioplastie), Kranzgefäßerkrankung, Gefäßstörungen, wie Raynaud-Syndrom, inflammatorische Erkrankungen, Schlaganfall, Bronchitis, chronischem Asthma, allergischem Asthma, allergischer Rhinitis, Glaukom und Erkrankungen, die durch Störungen der Eingeweidebeweglichkeit gekennzeichnet sind (z.B. reizbares Darmsyndrom).

8. Verbindung nach Formel (2), (3) und (9): 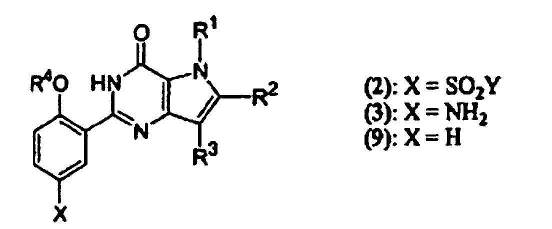 wobei R¹, R², R³ und R⁴ wie zuvor in Anspruch 1 definiert sind und Y ein Chlor-, Brom- oder Fluoratom ist.

9. Verbindung der Formel (12) und (13): 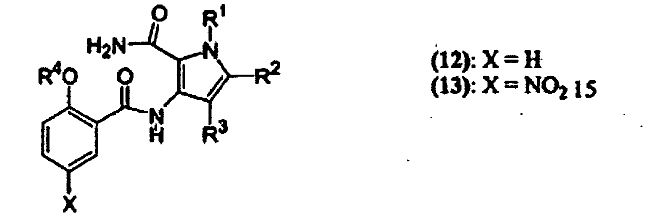 wobei R¹, R², R³ und R⁴ wie zuvor in Anspruch 1 definiert sind.

10. Verbindung der Formel (15): 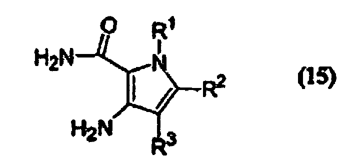 wobei R¹, R² und R³ wie zuvor in Anspruch 1 definiert sind.

11. Verfahren zur Herstellung einer Verbindung der Formel (1 ): 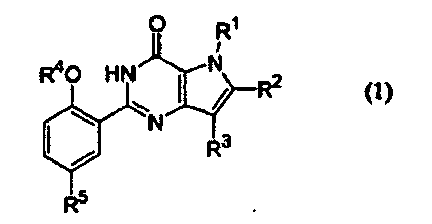 wobei R¹, R², R³, R⁴ und R⁵ wie zuvor in Anspruch 1 definiert sind, und pharmazeutisch verträgliche Salze davon, (a) bei dem man eine Verbindung der Formel (2)-(4) 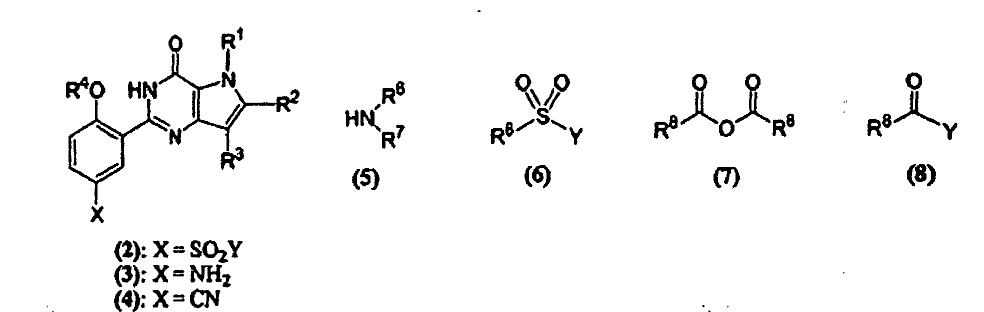 wobei R¹, R², R³ und R⁴ wie zuvor in Anspruch 1 definiert sind und Y ein Halogenatom darstellt, mit einer geeigneten Verbindung der Formel (5)-(8) unter einer geeigneten Reaktionsbedingung umsetzt, wobei R⁶, R⁷ und R⁸ wie zuvor in Anspruch 1 definiert sind, oder (b) bei dem man eine Verbindung der Formel (25) 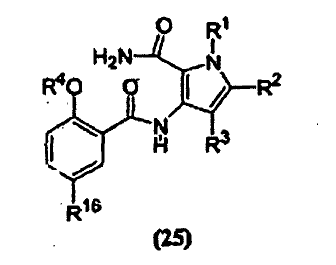 zyklisiert,
wobei R¹, R², R³ und R⁴ wie zuvor in Anspruch 1 definiert sind und R¹⁶ eine Gruppe R⁵ ist, wie sie zuvor in Anspruch 1 definiert wurde, oder ein Vorläufer von einer Gruppe R⁵.

12. Verfahren zur Herstellung einer Verbindung der Formel (2), (3) und (9) 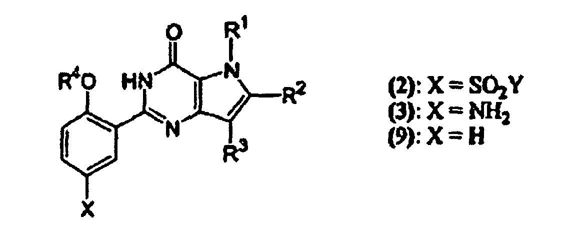 wobei R¹, R², R³ und R⁴ wie zuvor in Anspruch 1 definiert sind und Y ein Chlor-, Brom- oder Fluoratom ist, bei dem man eine Verbindung der Formel (12) oder (13) zyklisiert, gefolgt von einer geeigneten Umwandlung: 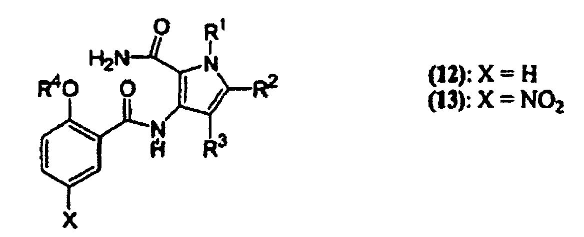 wobei R¹, R², R³ und R⁴ wie zuvor in Anspruch 1 definiert sind.

13. Verfahren zur Herstellung einer Verbindung der Formal (12) und (13) 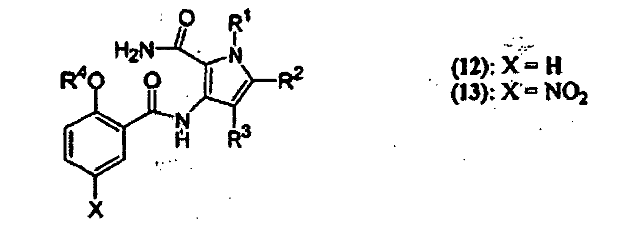 wobei R¹, R², R³ und R⁴ wie zuvor in Anspruch 1 definiert sind, bei dem man eine Verbindung der Formel (15) mit einer Verbindung der Formel (16) oder (17) umsetzt, 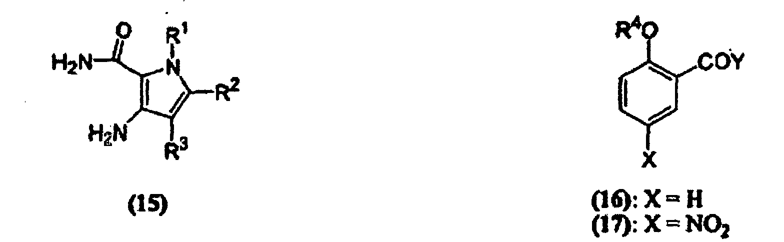 wobei R¹, R², R³ und R⁴ wie zuvor in Anspruch 1 definiert sind und Y ein Chlor-, Brom- oder Fluoratom ist.

14. Verfahren zur Herstellung einer Verbindung der Formel (15), 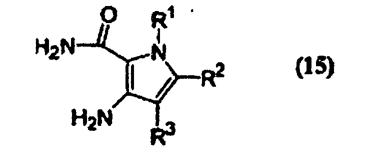 wobei R¹, R² und R³ wie zuvor in Anspruch 1 definiert sind, bei dem man eine Verbindung der Formel (19) zyklisiert, 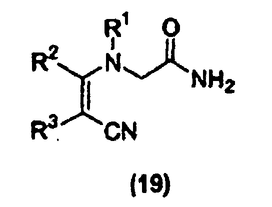 wobei R¹, R² und R³ wie zuvor in Anspruch 1 definiert sind.

## Revendications

1. Composé de formule (1) et sels et solvates (par exemple hydrates) acceptables pharmaceutiquement de ce composé, 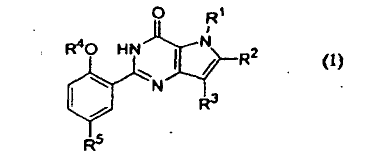 dans laquelle
R¹ désigne H, un groupe alkyle en C₁ à C₃ éventuellement substitué par un ou plusieurs atomes de fluor ou un groupe cycloalkyle en C₃ à C₆,
R² désigne H, un atome d'halogène, un groupe alkyle en C₁ à C₆ éventuellement substitué par OH, un groupe alcoxy en C₁ à C₃, un groupe cycloalkyle en C₃ à C₆ ou par un ou plusieurs atomes de fluor, un groupe cycloalkyle en C₃ à C₆, un groupe alcényle en C₂ à C₆ ou un groupe alcinyle en C₂ à C₆,
R³ désigne H, un groupe alkyle en C₁ à C₆ éventuellement substitué par OH, un groupe alcoxy en C₁ à C₃, un groupe cycloalkyle en C₃ à C₆ ou par un ou plusieurs atomes de fluor, un groupe cycloalkyle en C₃ à C₆, un groupe alcényle en C₂ à C₆ ou un groupe alcinyle en C₂ à C₆,
R⁴ désigne un groupe alkyle en C₁ à C₆ éventuellement substitué par un groupe cycloalkyle en C₃ à C₆ ou par un ou plusieurs atomes de fluor, un groupe alcényle en C₂ à C₆, un groupe alcinyle en C₂ à C₆ ou un groupe cycloalkyle en C₃ à C₆,
R⁵ désigne SO₂NR⁶R⁷, NHSO₂NR⁶R⁷, NHCOCONR⁶R⁷, NHSO₂R⁸, NHCOR⁸ ou un groupe phényle ou hétérocyclyle, l'un quelconque étant éventuellement substitué par un ou plusieurs atomes de fluor ou un groupe alkyle en C₁ à C₃,
R⁶ et R⁷ désignent chacun indépendamment H ou un groupe alkyle en C₁ à C₆ éventuellement substitué par un groupe OH, un groupe CO₂H, un groupe alcoxy en C₁ à C₃, un groupe cycloalkyle en C₃ à C₆ ou par un ou plusieurs atomes de fluor, ou forment, avec l'atome d'azote auquel ils sont fixés, soit un noyau monocyclique, tel qu'un noyau imidazole, aziridène (aziridine), azéridine (azétidine), pyrrolidine, pipéridine, morpholine, pipérazine et homopypérazine, soit un noyau bicyclique tel que le 2,5-diazadicyclo[2.2.1]heptane et le 3,7-diazabicyclo[3.3.0]octane, le groupe étant éventuellement substitué par R⁹,
R⁸ désigne un groupe alkyle en C₁ à C₆ éventuellement substitué par un ou plusieurs atomes de fluor, ou un groupe cycloalkyle en C₃ à C₇,
R⁹ désigne un groupe alkyle en C₁ à C₆ éventuellement substitué par un ou plusieurs atomes d'halogène, OH, un groupe alcoxy en C₁ à C₃ éventuellement substitué par un ou plusieurs atomes de fluor, CO₂R¹⁰, NR¹¹R¹², C=NR¹³(NR¹⁴R¹⁵), ou par un groupe tétrazole qui est éventuellement substitué par un groupe alkyle en C₁ à C₃, ou un ou plusieurs groupes hétéroaryles contenant de l'azote éventuellement substitués par un ou plusieurs atomes de fluor,
R¹⁰ désigne H ou un groupe alkyle en C₁ à C₄ éventuellement substitué par OH, NR¹¹R¹², un ou plusieurs atomes de fluor ou un noyau hétérocyclique contenant de l'azote tel que la pyrrolidine, la pipéridine, la morpholine, le pyrrole, et l'imidazole, l'atome d'azote étant directement lié au groupe alkyle en C₁ à C₄,
R¹¹ et R¹² désignent chacun indépendamment H ou un groupe alkyle en C₁ à C₄,
R¹³ désigne H, un groupe alkyle en C₁ à C₄ éventuellement substitué par un ou plusieurs atomes de fluor, ou un groupe cycloalkyle en C₃ à C₆, et
R¹⁴ et R¹⁵ sont chacun indépendamment H ou un groupe alkyle en C₁ à C₄ éventuellement substitué par un ou plusieurs atomes de carbone, un groupe cycloalkyle en C₃ à C₆ ou forment, avec l'atome d'azote auquel ils sont fixés, un groupe pyrrolidinyle, pypéridino, morpholino, pipérazinyle ou homopipérazinyle dans lequel le groupe est éventuellement substitué par un groupe alkyle en C₁ à C₃.

2. Composé selon la revendication 1, dans lequel
R¹ est H, un groupe méthyle ou éthyle,
R² est H, un groupe méthyle ou un atome d'halogène,
R³ est un groupe alkyle en C₁ à C₄ éventuellement substitué par un ou plusieurs atomes de fluor,
R⁴ est un groupe éthyl, n-propyl ou allyl,
R⁵ est SO₂NR⁶R⁷ ou NHSO₂R⁸,
R⁶ et R⁷ considérés avec l'atome d'azote auquel ils sont fixés forment un groupe pipéridino, pipérazinyle ou homopipérazinyle dans lequel le groupe est substitué par R⁹,
R⁸ est un groupe méthyle,
R⁹ est un groupe alkyle en C₁ à C₄ éventuellement substitué par un ou plusieurs atomes d'halogène, OH, CO₂R¹⁰ ou par un groupe tétrazole éventuellement substitué par un groupe alkyle en C₁ à C₃, et
R¹⁰ est H.

3. Composé selon la revendication 2, dans lequel
R¹ est H, un groupe méthyle ou éthyle,
R² est H,
R³ est un groupe éthyl, n-propyl, 3-fluoropropyl ou cyclopropylméthyl,
R⁴ est un groupe éthyl ou n-propyl,
R⁵ est SO₂NR⁶R⁷ ou NHSO₂R⁸,
R⁶ et R⁷ considérés avec l'atome d'azote auquel ils sont fixés forment un groupe pipéridino ou pipérazinyle dans lequel le groupe est substitué par R⁹,
R⁸ est un groupe méthyle,
R⁹ est un groupe alkyle en C₁ à C₄ éventuellement substitué par un ou plusieurs atomes d'halogène, OH, CO₂R¹⁰ ou par un groupe tétrazole, et
R¹⁰ est H.

4. Composé selon la revendication 3, dans lequel le composé est choisi dans le groupe formé par :
2-(éthoxy-5-(4-méthylpipérazin-1-ylsulfonyl)phényl)-5-méthyl-7-n-propyl-3,5-dihydro-4H-pyrrolo[3,2-d]pyrimidin-4-one,
2-(5-(4-méthylpipérazin-1-ylsulfonyl)-2-n-propoxyphényl)-5-méthyl]-7-n-propyl-3,5-dihydro-4H-pyrrolo[3,2-d]pyrimidin-4-one,
2-(2-éthoxy-5-(4-n-propylpipérazin-1-ylsulfonyl)phényl)-5-méthyl-7-n-propyl-3,5-dihydro-4H-pyrrolo[3,2-d]pyrimidin-4-one,
2-(2-éthoxy-5-(4-isopropylpipérazin-1-ylsulfonyl)phényl)-5-méthyl-7-n-propyl-3,5-dihydro-4H-pyrrolo[3,2-d]pyrimidin-4-one,
2-(2-éthoxy-5-(4-(2(fluoroéthyl)pipérazin-1-ylsulfonyl)phényl)-5-méthyl-7-n-propyl-3,5-dihydro-4H-pyrrolo[3,2-d]pyrimidin-4-one,
2-(5-(4-(2-fluoroéthyl)pipérazin-1-ylsulfonyl)-2-n-propoxyphényl)-5-méthyl-7-n-propyl-3,5-dihydro-4H-pyrrolo[3,2-d]pyrimidin-4-one,
2-(2-éthoxy-5-(4-(3-fluoropropyl)pipérazin-1-ylsulfonyl)phényl)-5-méthyl-7-n-propyl-3,5-dihydro-4H-pyrrolo[3,2-d]pyrimidin-4-one,
2-(2-éthoxy-5-(4-(3-fluoropropyl)pipérazin-1-ylsulfonyl)phényl)-7-éthyl-5-méthyl-3,5-dihydro-4H-pyrrolo[3,2-d]pyrimidin-4-one,
2-(2-éthoxy-5-(4-(3-fluoropropyl)pipérazin-1-ylsulfonyl)phényl)-7-(3-fluoropropyl)-5-méthyl-3,5-dihydro-4H-pyrrolo[3,2-d]pyrimidin-4-one,
7-cyclopropylméthyl-2-(2-éthoxy-5-(4-(3-fluoropropyl)pipérazin-1-ylsulfonyl)phényl)-5-méthyl-3,5-dihydro-4H-pyrrolo[3,2-d]pyrimidin-4-one,
2-(2-éthoxy-5-(4-(3-fluoropropyl)pipérazin-1-ylsulfonyl)phényl)-5-éthyl-7-éthyl-3,5-dihydro-4H-pyrrolo[3,2-d]pyrimidin-4-one,
2-(2-éthoxy-5-(4-(3-fluoropropyl)pipérazin-1-ylsulfonyl)phényl)-5-éthyl-7-n-propyl-3,5-dihydro-4H-pyrrolo[3,2-d]pyrimidin-4-one,
2-(2-éthoxy-5-(4-isopropylpipérazin-1-ylsulfonyl)phényl-5-éthyl-7-n-propyl-3,5-dihydro-4H-pyrrolo[3,2-d]pyrimidin-4-one,
2-(2-éthoxy-5-(4-(3-fluoropropyl)pipérazin-1-ylsulfonyl)phényl)-5-éthyl-7-n-propyl-3,5-dihydro-4H-pyrrolo[3,2-d]pyrimidin-4-one,
2-(2-éthoxy-5-(4-(2-hydroxyéthyl)pipérazin-1-ylsulfonyl)phényl)-5-éthyl-7-n-propyl-3,5-dihydro-4H-pyrrolo[3,2-d]pyrimidin-4-one,
2-(2-éthoxy-5-(4-éthylpipérazin-1-ylsulfonyl)phényl)-5-éthyl-7-(3-fluoropropyl)-3,5-dihydro-4H-pyrrolo[3,2-d]pyrimidin-4-one,
2-(5-(éthylpipérazin-1-ylsulfonyl)-2-n-propoxyphényl)-5-éthyl-7-(3-fluoropropyl)-3,5-dihydro-4H-pyrrolo[3,2-d]pyrimidin-4-one,
2-(2-éthoxy-5-(4-isopropylpipérazin-1-ylsulfonyl)phényl)-5-éthyl-7-(3-fluoropropyl)-3,5-dihydro-4H-pyrrolo[3,2-d]pyridin-4-one,
2-(2-éthoxy-5-(4-(3-fluoropropyl)pipérazin-1-ylsulfonyl)phényl-5-éthyl-7-(3-fluoropropyl)-3,5-dihydro-4H-pyrrolo[3,2-d]pyrimidin-4-one,
2-(5-(4-(3-fluoropropyl)pipérazin-1-ylsulfonyl)-2-n-propoxyphényl-5-éthyl-7-(3-fluoropropyl)-3,5-dihydro-4H-pyrrolo[3,2-d]pyrimidin-4-one,
2-(2-éthoxy-5-(4-(2-hydroxyéthyl)pipérazin-1-ylsulfonyl)phényl-5-éthyl-7(3-fluoropropyl)-3,5-dihydro-4H-pyrrolo[3,2-d]pyrimidin-4-one,
2-(5-(4-hydroxyéthyl)pipérazin-1-ylsulfonyl)-2-n-propoxyphényl)-5-éthyl-7-(3-fluoropropyl)-3,5-dihydro-4H-pyrrolo[3,2-d]pyrimidin-4-one,
2-(5-(4-(3-hydroxypropyl)pipérazin-1-ylsulfonyl)-2-n-propoxyphényl)-5-éthyl-7-(3-fluoropropyl)-3,5-dihydro-4H-pyrrolo[3,2-d]pyrimidin-4-one,
2-(5-(4-(2-hydroxyéthyl)pipérazin-1-ylsulfonyl)-2-n-propoxyphényl)-5-éthyl-7-n-propyl-3,5-dihydro-4H-pyrrolo[3,2-d]pyrimidin-4-one,
2-(5-(4-(3-fluoropropyl)pipérazin-1-ylsulfonyl)-2-n-propoxyphényl)-5-méthyl-7-n-propyl-3,5-dihydro-4H-pyrrolo[3,2-d]pyrimidin-4-one,
2-(2-éthoxy-5-(4-(2-hydroxyéthyl)pipérazin-1-ylsulfonyl)phényl)-5-méthyl-7-n-propyl-3,5-dihydro-4H-pyrrolo[3,2-d]pyrimidin-4-one,
2-(5-(4-(2-hydroxyéthyl)pipérazin-1-ylsulfonyl)-2-n-propoxyphényl)-5-méthyl-7-n-propyl-3,5-dihydro-4H-pyrrolo[3,2-d]pyrimidin-4-one,
2-(5-(2-éthoxy-4-(3-hydroxypropyl)pipérazin-1-ylsulfonyl)phényl-5-méthyl-7-n-propyl-3,5-dihydro-4H-pyrrolo[3,2-d]pyrimidin-4-one,
2-(5-(4-(3-hydroxypropyl)pipérazin-1-ylsulfonyl)-2-n-propoxyphényl)-5-méthyl-7-n-propyl-3,5-dihydro-4H-pyrrolo[3,2-d]pyrimidin-4-one,
2-(2-éthoxy-5-(4-(hydroxycarbonylméthyl)pipéridin-1-ylsulfonyl)phényl)-5-méthyl-7-n-propyl-3,5-dihydro-4H-pyrrolo[3,2-d]pyrimidin-4-one,
2-(5-(4-hydroxycarbonylméthyl)pipéridin-1-ylsulfonyl)-2-n-propoxyphényl)-5-méthyl-7-n-propyl-3,5-dihydro-4H-pyrrolo[3,2-d]pyrimidin-4-one,
2-(2-éthoxy-5-(4-(2-hydroxycarbonyléthyl)pipéridin-1-ylsulfonyl)phényl-5-méthyl-7n-propyl-3,5-dihydro-4H-pyrrolo[3,2-d]pyrimidin-4-one,
2-(5-(4-(2-hydroxycarbonyléthyl)pipéridin-1-ylsulfonyl)-2-n-propoxyphényl)-5-méthyl-7-n-propyl-3,5-dihydro-4H-pyrrolo[3,2-d]pyrimidin-4-one,
2-(2-éthoxy-5-(4-(1H-tétrazol-5-ylméthyl)pipérazin-1-ylsulfonyl)phényl)-5-méthyl-7-n-propyl-3,5-dihydro-4H-pyrrolo[3,2-d]pyrimidin-4-one,
5-méthyl-2-(2-n-propoxy-5-(4-(2-(1H-tétrazol-5-yl)éthylpipérazin-1-ylsulfonyl)phényl)-7-n-propyl-3,5-dihydro-4H-pyrrolo[3,2-d]pyrimidin-4-one,
2-(2-éthoxy-5-(4-(1H-tétrazol-5-ylméthyl)pipéridin-1-ylsulfonyl)phényl)-5-méthyl-7-n-propyl-3,5-dihydro-4H-pyrrolo[3,2-d]pyrimidin-4-one,
5-méthyl-2-(2-n-propoxy-5-(4-(1H-tétrazol-5-ylméthyl)pipéridin-1-ylsulfonyl)phényl)-7-n-propyl-3,5-dihydro-4H-pyrrolo[3,2-d]pyrimidin-4-one, et
leurs sels et solvates (par exemple hydrates) acceptables physiologiquement.

5. Composition pharmaceutique contenant un composé de formule (1) ou un sel ou solvate d'un tel composé acceptable pharmaceutiquement selon l'une quelconque des revendications 1 à 4, avec un véhicule ou diluant acceptable pharmaceutiquement pour ce composé.

6. Composé de formule (I) ou sel ou solvate acceptable pharmaceutiquement d'un tel composé ou composition pharmaceutique contenant une entité quelconque parmi l'une quelconque des revendications 1 à 5, destiné à être utilisé dans le traitement de l'impuissance, du dérèglement sexuel de la femme, de l'angine stable, instable et variante (Prinzmetal), de l'hypertension, de l'hypertension pulmonaire, des troubles cardiaques par congestion, des troubles rénaux, de l'artériosclérose, des états de possibilité réduite des vaisseaux sanguins (par exemple après angioplastie coronaire transluminale percutanée), des maladies vasculaires périphériques, des désordres vasculaires tels que la maladie de Raynaud, des maladies inflammatoires, de l'apoplexie, de la bronchite, de l'asthme chronique, de l'asthme allergique, de la rhinite allergique, du glaucome et des maladies **caractérisées par** des dysfonctionnements de la motilité du système intestinal (par exemple le syndrome des intestins irritables).

7. Application d'un composé de formule (1) ou d'un sel ou solvate d'un tel composé acceptable pharmaceutiquement ou d'une composition pharmaceutique contenant une entité selon l'une quelconque des revendications 1 à 5, à la fabrication d'un médicament destiné à être utilisé dans le traitement de l'impuissance, du dérèglement sexuel de la femme, de l'angine stable, instable et variante (Prinzmetal), de l'hypertension, de l'hypertension pulmonaire, des troubles cardiaques par congestion, des troubles rénaux, de l'artériosclérose, des états de possibilité réduite des vaisseaux sanguins (par exemple après angioplastie coronaire transluminale percutanée), des maladies vasculaires périphériques, des désordres vasculaires tels que la maladie de Raynaud, des maladies inflammatoires, de l'apoplexie, de la bronchite, de l'asthme chronique, de l'asthme allergique, de la rhinite allergique, du glaucome et des maladies **caractérisées par** des dysfonctionnements de la motilité du système intestinal (par exemple le syndrome des intestins irritables).

8. Composé de formules (2), (3), et (9) : 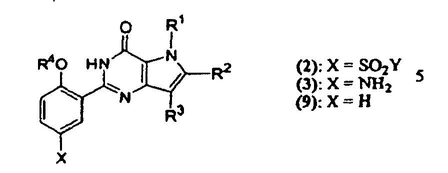 R¹, R², R³ et R⁴ étant tels que définis précédemment dans la revendication 1 et Y étant un atome de chlore, de brome ou de fluor.

9. Composé de formules (12) ou (13) : 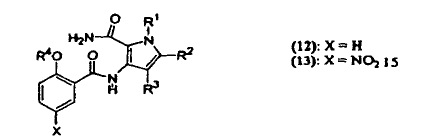 R¹, R², R³ et R⁴ étant tels que définis précédemment dans la revendication 1.

10. Composé de formule (15) : 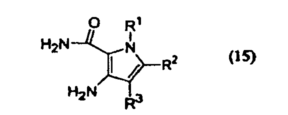 R¹, R² et R³ étant tels que définis précédemment dans la revendication 1.

11. Procédé de préparation d'un composé de formule (1) 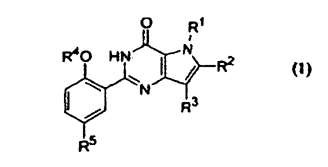 dans laquelle R¹, R², R³, R⁴ et R⁵ sont tels que définis précédemment dans la revendication 1, et leurs sels acceptables pharmaceutiquement, (a) qui comprend la réaction d'un composé de formule (2) à (4) : 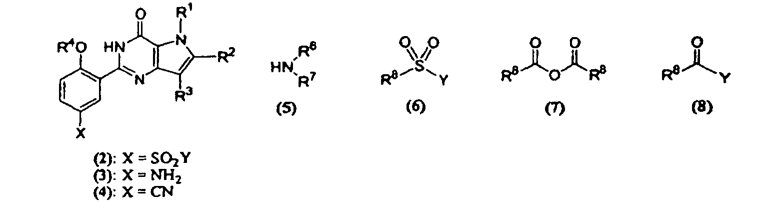 R¹, R², R³ et R⁴ étant tels que définis précédemment dans la revendication 1 et Y représentant un atome d'halogène, avec un composé convenable de formules (5) à (8) dans des conditions réactionnelles appropriées, R⁶, R⁷ et R⁸ étant tels que définis précédemment dans la revendication 1, ou (b) qui comprend la cyclisation d'un composé de formule (25) : 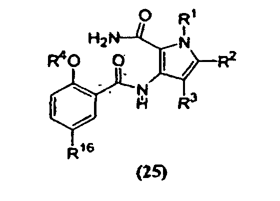 R¹, R², R³ et R⁴ étant tels que définis précédemment dans la revendication 1, et R¹⁶ étant un groupe R⁵ tel que défini précédemment dans la revendication 1 ou un précurseur d'un tel groupe R⁵.

12. Procédé de préparation d'un composé de formule (2), (3) et (9) : 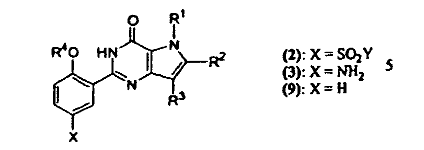 R¹, R², R³ et R⁴ étant tels que définis précédemment dans la revendication 1 et Y représentant un atome de chlore, de brome ou de fluor, qui comprend la cyclisation d'un composé de formule (12) ou (13) suivie d'une transformation appropriée :

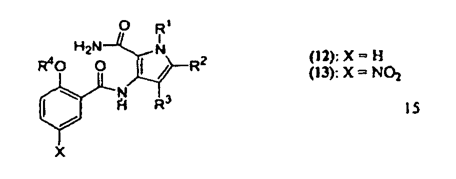

13. Procédé de préparation d'un composé de formule (12) et (13) : 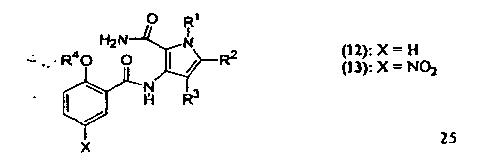 R¹, R², R³ et R⁴ étant tels que définis précédemment dans la revendication 1, qui comprend la réaction d'un composé de formule (15) avec un composé de formule (16) ou (17) : 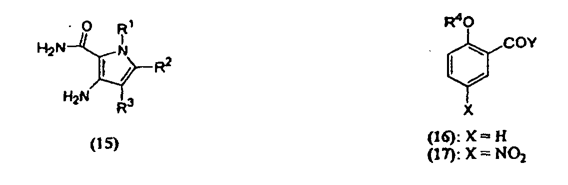 R¹, R², R³ et R⁴ étant tels que définis précédemment dans 1a revendication 1 et Y représentant un atome de chlore, de brome ou de fluor.

14. Procédé de préparation d'un composé de formule (15) : 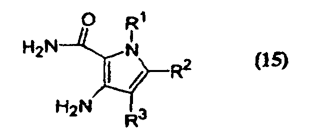 R¹, R² et R³ étant tels que définis précédemment dans la revendication 1, qui comprend la cyclisation d'un composé de formule (19) : 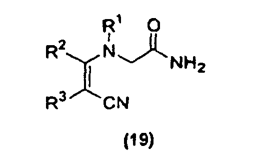 R¹, R² et R³ étant tels que définis précédemment dans la revendication 1
